# EUROPEAN PATENT APPLICATION

(11) **EP 4 303 218 A2**
(43) Date of publication of application: **10.01.2024**
(21) Application number: 23209736.0
(22) Date of filing: 18.12.2019
(51) Int. Cl.: C07D 405/04, C07D 405/10, C07D 405/14, C07D 409/04, C07D 409/10, C07D 409/14, C09K 11/06, H10K 85/60

(54) **BENZO[B]NAPHTHO[2,3-D]FURANYL- OR BENZO[B]NAPHTHO[2,3-D]THIOPHENYL-TRIAZINE COMPOUNDS FOR ORGANIC ELECTRONIC ELEMENTS**

(30) Priority: 20.12.2018 KR 20180166463; 29.04.2019 KR 20190049991; 29.04.2019 KR 20190050099
(62) Divisional of application: 19217541.2
(71) Applicant: Duk San Neolux Co., Ltd., Chungcheongnam-do 31027 (KR)
(72) Inventor: LEE, Yun Suk, 31027 Cheonan-si, Chungcheongnam-do (KR); KIM, Yu Ri, 31027 Cheonan-si, Chungcheongnam-do (KR); JO, Min Ji, 31027 Cheonan-si, Chungcheongnam-do (KR); PARK, Jung Hwan, 31027 Cheonan-si, Chungcheongnam-do (KR); LEE, Bum Sung, 31027 Cheonan-si, Chungcheongnam-do (KR); LEE, Sun Hee, 31027 Cheonan-si, Chungcheongnam-do (KR); MUN, Soung Yun, 31027 Cheonan-si, Chungcheongnam-do (KR)
(74) Representative: Vossius & Partner Patentanwälte Rechtsanwälte mbB

(57) **Abstract**

The present invention provides an organic electric element comprising a first electrode, a second electrode, and an organic material layer formed between the first electrode and the second electrode, and electronic device thereof, and by comprising the mixture of the compound represented by Formula 1 and the compound represented by Formula 2 in the organic material layer, the driving voltage of the organic electronic device can be lowered, and the luminous efficiency and life time of the organic electronic device can be improved.

## Description

### CROSS-REFERENCE TO RELATED APPLICATION

This patent application claims benefit of Korean Patent Application No. 10-2018-0166463, filed on December 20, 2018, Korean Patent Application No. 10-2019-0049991 7 and 10-2019-0050099, filed on April 29, 2019 in the Korean Intellectual Property Office.

### BACKGROUND

### Technical Field

The present invention relates to compounds for organic electric elements, organic electric elements comprising the same, and electronic devices thereof.

### Background Art

In general, an organic light emitting phenomenon refers to a phenomenon in which electric energy is converted into light energy of an organic material. An organic electric element utilizing the organic light emitting phenomenon usually has a structure including an anode, a cathode, and an organic material layer interposed therebetween. In many cases, the organic material layer has a multi-layered structure each composed of different materials in order to improve efficiency and stability of an organic electric element, and for example, may include a hole injection layer, a hole transport layer, a light emitting layer, an electron transport layer, an electron injection layer, or the like.

A material used in an organic material layer of an organic electric element may be classified into a light emitting material and a charge transport material, such as a hole injection material, a hole transport material, an electron transport material, an electron injection material and the like depending on its function. Further, the light emitting material may be divided into a high molecular weight type and a low molecular weight type according to its molecular weight, and may also be divided into a fluorescent material derived from electronic excited singlet states and a phosphorescent material derived from electronic excited triplet states according to its light emitting mechanism. Further, the light emitting material may be divided into blue, green, and red light emitting materials, and yellow and orange light emitting materials required for better natural color reproduction according to its light emitting color.

Meanwhile, when only one material is used as a light emitting material, there occur problems of shift of a maximum luminescence wavelength to a longer wavelength due to intermolecular interactions and lowering of the efficiency of a corresponding element due to the deterioration in color purity or a reduction in luminous efficiency. On account of this, a host/dopant system may be used as the light emitting material in order to enhance the color purity and increase the luminous efficiency through energy transfer. This is based on the principle that if a small amount of dopant having a smaller energy band gap than a host forming a light emitting layer is mixed in the light emitting layer, then excitons generated in the light emitting layer are transported to the dopant, thus emitting light with high efficiency. With regard to this, since the wavelength of the host is shifted to the wavelength band of the dopant, light having a desired wavelength can be obtained according the type of the dopant.

Currently, the power consumption is required more and more as size of display becomes larger and larger in the portable display market. Therefore, the power consumption is a very important factor in the portable display with a limited power source of the battery, and the situation is such that efficiency and life span issue also must be solved.

Efficiency, life span, driving voltage, and the like are correlated with each other. For example, if efficiency is increased, then driving voltage is relatively lowered, and the crystallization of an organic material due to Joule heating generated during operation is reduced as driving voltage is lowered, as a result of which life span shows a tendency to increase. However, efficiency cannot be maximized only by simply improving the organic material layer. This is because long life span and high efficiency can be simultaneously achieved when an optimal combination of energy levels and T1 values, inherent material properties (mobility, interfacial properties, etc.), and the like among the respective organic material layer is given.

Therefore it is required to develop a light emitting material that has high thermal stability and can achieve efficiently a charge balance in the light-emitting layer. That is, in order to allow an organic electric element to fully exhibit the above-mentioned excellent features, the material consisting an organic material layer of the element such as a hole injection material, a hole transport material, a light emitting material, an electron transport material, an electron injection material, or the like should be prerequisite to support by a stable and efficient material, and among them, it is necessary to develop host material for a light emitting layer.

### Object, technical solution and effects of the invention

The present invention is to provide a compound lowering driving voltage of the element, improving luminous efficiency and lifetime of the element, an organic electric element comprising the same, and an electronic device thereof.

In an aspect of the present invention, the present invention provides an organic electric element comprising the compounds represented by the following formulas 1 and 2 and an electric device thereof.

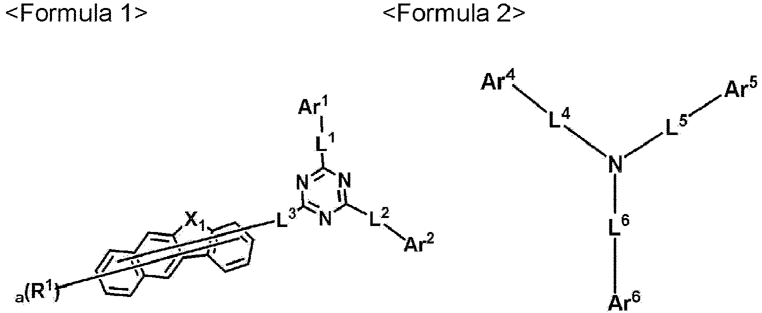

In another aspect of the present invention, the present invention provides the compound represented by the following formula 3, an organic electric element comprising the compound and an electric device thereof.

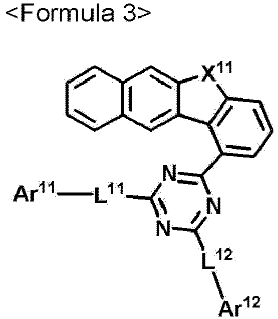

Further, in another aspect of the present invention, the present invention provides an organic electric element comprising the compound represented by formula 3 above and the compound represented by the following formula 4 and an electric device thereof.

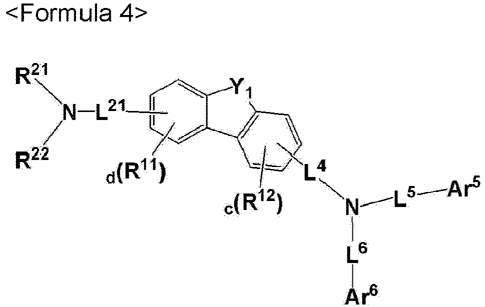

By using the mixture of the compounds represented by formulas 1 and 2 above or by using the compound represented by formula 3, wherein formula 3 is comprised in formula 1, as host of a light emitting layer, the driving voltage of an element can be lowered and the luminous efficiency and lifetime of an element can be improved.

### BRIEF DESCRIPTION OF THE DRAWINGS

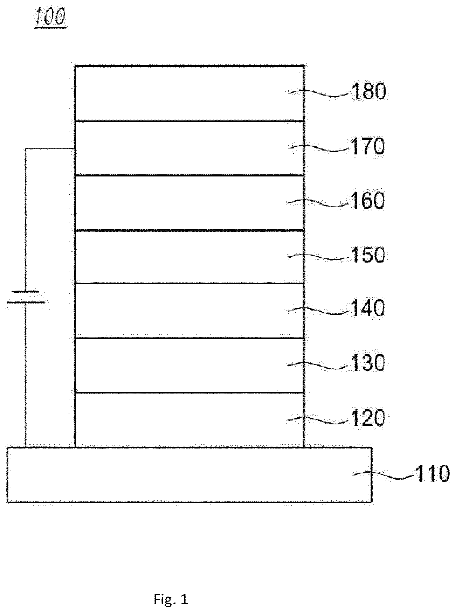

The FIGUREs 1 to 3 illustrate an example of an organic electroluminescent element according to an embodiment of the present invention: 100, 200, 300 are an organic electric element, 110 is a first electrode, 120 is a hole injection layer, 130 is a hole transport layer, 140 is a light emitting layer, 150 is an electron transport layer, 160 is an electron injection layer, 170 is a second electrode, 180 is a light efficiency improving layer, 210 is a buffer layer, 220 is an emission-auxiliary layer, 320 is a first hole injection layer, 330 is a first hole transport layer, 340 is a first light emitting layer, 350 is a first electron transport layer, 360 is a first charge generation layer, 361 is a second charge generation layer, 420 is a second hole injection layer, 430 is a second hole transport layer, 440 is a second light emitting layer, 450 is a second electron transport layer, CGL is a charge generation layer, ST1 is a first stack and ST2 is a second stack.

### DETAILED DESCRIPTION

Unless otherwise stated, the term "aryl group" or "arylene group" as used herein has, but not limited to, 6 to 60 carbon atoms. The aryl group or arylene group in the present ivention may comprise a monocyclic ring, ring assemblies, a fused polycyclic system, spiro compounds and the like. In addition, unless otherwise stated, a fluorenyl group may be comprised in an aryl group and a fluorenylene group may be comprised in an arylene group
Unless otherwise stated, the term "fluorenyl group" or "fluorenylene group" as used herein means "substituted or unsubstituted fluorenyl group" "substituted or unsubstituted fluorenylene group", respectively. "Fluorenyl group" or "fluorenylene group" as used herein may be represented by the following formula, wherein R, R' and R" may be hydrogen or a substituent other than hydrogen and R and R' are linked to each other to form the spiro compound together with the carbon to which they are bonded.

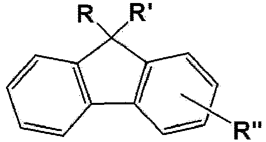

The term "spiro compound" as used herein has a spiro union which means union having one atom as the only common member of two rings. The common atom is designated as 'spiro atom'. The compounds are defined as 'monospiro-', 'dispiro-' or 'trispiro-' depending on the number of spiro atoms in one compound.

The term "heterocyclic group" as used herein means a non-aromatic ring as well as an aromatic ring like "heteroaryl group" or "heteroarylene group", and unless otherwise stated, it means a ring comprising one or more heteroatoms and having 2 to 60 carbon atoms, but not limited thereto. Unless otherwise stated, the term "hetero atom" as used herein represents N, O, S, P or Si, and the heterocyclic group means a monocyclic form, ring assemblies, a fused polycyclic system or a spiro compound comprising heteroatom(s). Also, "heterocyclic group" may comprise the compound comprising a heteroatom group such as SO₂, P=O etc. instead of carbon forming a ring, such as the following compound.

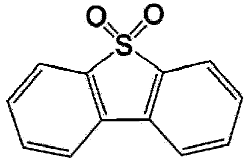

The term "aliphatic ring group" as used herein refers to a cyclic hydrocarbon except for aromatic hydrocarbons, and comprises a monocyclic ring, ring assemblies, a fused polycyclic system, spiro compounds, and the like, and unless otherwise specified, it means a ring of 3 to 60 carbon atoms, but not limited thereto.

In this specification, a 'group name' corresponding to an aryl group, an arylene group, a heterocyclic group, and the like exemplified for each symbol and its substituent may be written in the name of functional group reflecting the valence, and may also be described as the name of a parent compound. For example, in the case of phenanthrene which is a kind of aryl group, it may be described by distinguishing valence such as 'phenanthryl (group)' when it is `monovalent group', and as 'phenanthrylene (group)' when it is 'divalent group', and it may also be described as a parent compound name, 'phenanthrene', regardless of its valence. Similarly, in the case of pyrimidine, it may be described as 'pyrimidine' regardless of its valence, and it may also be described as the name of corresponding functional group such as pyrimidinyl (group) when it is 'monovalent group', and as 'pyrimidylene (group)' when it is 'divalent group'.

In addition, in the present specification, the numbers and alphabets indicating a position may be omitted when describing a compound name or a substituent name, For example, pyrido[4,3-d]pyrimidine, benzopuro[2,3-d] pyrimidine and 9,9-dimethyl-9H-fluorene can be described as pyridopyrimidine, benzofurropyrimidine and dimethylfluorene, respectively. Therefore, both benzo[g]quinoxaline and benzo[f] quinoxaline can be described as benzoquinoxaline.

In addition, unless otherwise expressed, where any formula of the present invention is represented by the following formula, the substituent according to the index may be defined as follows.

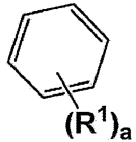

In the above formula, where a is an integer of zero, the substituent R¹ is absent, that is, hydrogen atoms are bonded to all the carbon constituting the benzene ring. Here, chemical formulas or compounds may be written without explicitly describing the hydrogen. In addition, one substituent R¹ is bonded to any carbon of the carbons forming the benzene ring when "a" is an integer of 1. Similarly, where "a" is an integer of 2 or 3, substituents R¹s may be bonded to the carbon of the benzene ring, for example, as followings. Also, where "a" is an integer of 4 to 6, substituents R¹s are bonded to the carbon of the benzene ring in a similar manner. Further, where "a" is an integer of 2 or more, R¹s may be the same or different from each other.

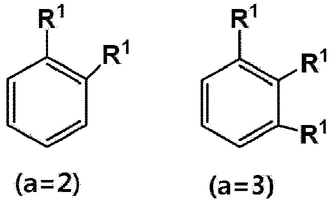

In addition, unless otherwise stated, the ring formed by combining adjacent groups to each other may be selected from the group consisting of a C₆-C₆₀ aromatic ring group, a fluorenyl group, a C₂-C₆₀ heterocyclic group containing at least one heteroatom selected from the group consisting of O, N, S, Si, and P, a C₃-C₆₀ aliphatic ring, a fused ring formed by a C₃-C₆₀ aliphatic ring and a C₆-C₆₀ aromatic ring group and a combination thereof.

Hereinafter, referring to Figures 1~3, a lamination structure of an organic electric element including the compound of the present invention will be described.

In designation of reference numerals to components in respective drawings, it should be noted that the same elements will be designated by the same reference numerals although they are shown in different drawings. Further, in the following description of the present invention, a detailed description of known functions and configurations incorporated herein will be omitted when it may make the subject matter of the present invention rather unclear.

In addition, terms, such as first, second, A, B, (a), (b) or the like may be used herein when describing components of the present invention. Each of these terminologies is not used for defining an essence, order or sequence of a corresponding component but used merely to distinguish the corresponding component from other component(s). It will be understood that the expression "one component is "connected," "coupled" or "joined" to another component" comprises the case where a third component may be "connected," "coupled," and "joined" between the first and second components as well as the case where the first component may be directly connected, coupled or joined to the second component.

In addition, it will be understood that when an element such as a layer, film, region or substrate is referred to as being "on" or "over" another element, it can be directly on the other element or intervening elements may also be present. In contrast, when an element is referred to as being "directly on" another element, there are no intervening elements present.

The Figures 1~3 are laminated stuctures for showing an example of an organic electric element according to an embodiment of the present invention, respectively.

Referring to the FIG. 1, an organic electric element 100 according to an embodiment of the present invention includes a first electrode 110 formed on a substrate(not shown), a second electrode 170, and an organic material layer formed between the first electrode 110 and the second electrode 170.

The first electrode 110 may be an anode (positive electrode), and the second electrode 170 may be a cathode (negative electrode). In the case of an inverted organic electric element, the first electrode may be a cathode, and the second electrode may be an anode.

The organic material layer may comprised a hole injection layer 120, a hole transport layer 130, a light emitting layer 140, an electron transport layer 150, and an electron injection layer 160. Specifically, a hole injection layer 120, a hole transport layer 130, a light emitting layer 140, an electron transport layer 150, and an electron injection layer 160 are formed on the first electrode 110 in sequence.

Preferably, a layer for improving the luminous efficiency 180 may be formed one side of sides of the first electrode 110 and the second electrode 170, wherein one side is not facing the organic material layer, as a result the luminous efficiency of an organic electric element can be improved.

For example, the light efficiency improving layer 180 may be formed on the second electrode 170, as a result, in the case of a top emission organic light emitting diode, the optical energy loss due to surface plasmon polaritons (SPPs) at the second electrode 170 may be reduced and in the case of a bottom emission organic light emitting diode, the light efficiency improving layer 180 may serve as a buffer for the second electrode 170.

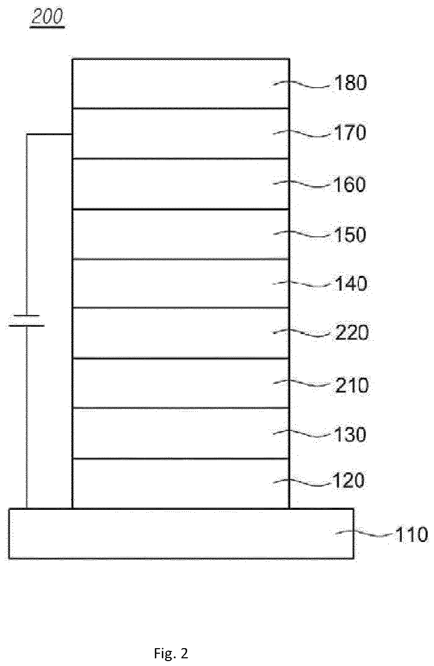

Meanwhile, a buffer layer 210 or an emission-auxiliary layer 220 may be further formed between the hole transport layer 130 and the light emitting layer 140, which will be described with reference to FIG. 2.

Referring to FIG. 2, the organic electric element 200 according to another embodiment of the present invention may comprise a hole injection layer 120, a hole transport layer 130, a buffer layer 210, an emission-auxiliary layer 220, a light emitting layer 140, the electron transport layer 150, the electron injection layer 160, and a second electrode 170 formed on a first electrode 110 in sequence, and a light efficiency improving layer 180 is formed on the second electrode 170.

Although not shown in FIG. 2, an electron transport auxiliary layer may be further formed between the light emitting layer 140 and the electron transport layer 150.

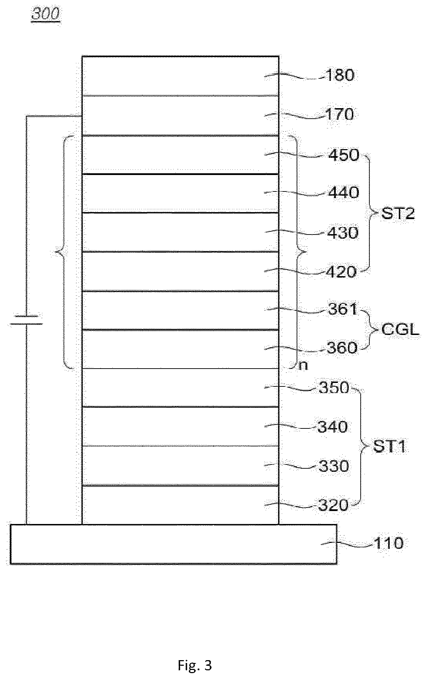

In addition, according to another embodiment of the present invention, the organic material layer may be a form formed by a plurality of stacks, wherein the stacks comprise a hole transport layer, a light emitting layer, and an electron transport layer, respectively. This will be described with reference to FIG. 3.

Referring to FIG. 3, two or more sets of stacks of the organic material layers ST1 and ST2 may be formed between the first electrode 110 and the second electrode 170 in the organic electric element 300 according to another embodiment of the present invention, wherein the organic material layers are consisted of multiple layers, respectively, and the charge generation layer CGL may be formed between the stacks of the organic material layer.

Specifically, the organic electric element according to the embodiment of the present invention may comprise the first electrode 110, the first stack ST1, the charge generation layer CGL, the second stack ST2, and the second electrode 170 and the light efficiency improving layer 180.

The first stack ST1 is an organic layer formed on the first electrode 110, and the first stack ST1 may comprise the first hole injection layer 320, the first hole transport layer 330, the first light emitting layer 340 and the first electron transport layer 350 and the second stack ST2 may comprise a second hole injection layer 420, a second hole transport layer 430, a second light emitting layer 440 and a second electron transport layer 450. As such, the first stack and the second stack may be the organic layers having the same or different stacked structures.

The charge generation layer CGL may be formed between the first stack ST1 and the second stack ST2. The charge generation layer CGL may comprise a first charge generation layer 360 and a second charge generation layer 361. The charge generating layer CGL is formed between the first light emitting layer 340 and the second light emitting layer 440 to increase the current efficiency generated in each light emitting layer and to smoothly distribute charges.

The first light emitting layer 340 may comprise a light emitting material comprising a blue host doped with a blue fluorescent dopant and the second light emitting layer 440 may comprise a light emitting material comprising a green host doped with a greenish yellow dopant and a red dopant together, but the material of the first light emitting layer 340 and the second light emitting layer 440 according to an embodiment of the present invention is not limited thereto.

In FIG. 3, n may be an integer of 1 to 5 and the charge generation layer CGL and the third stack may be further stacked on the second stack ST2 when n is 2.

When a plurality of light emitting layers are formed in a multi-layer stack structure as shown in FIG. 3, it is possible to manufacture an organic electroluminescent element that emits not only white light but also various colors, wherein the white light is emitted by the mixing effect of light emitted from each light emitting layer.

The mixture of the compound represented by Formula 1 and the compound represented by Formula 2, or the compound represented by Formula 3 comprised in Formula 1 can be used as material of a hole injection layer 120, 320, 420, a hole transport layer 130, 330, 430, a buffer layer 210, an emission-auxiliary layer 220, an electron transport layer 150, 350, 450, an electron injection layer 160, a light emitting layer 140, 340, 440, or a layer for improving luminous efficiency 180, preferably as material of an emission-auxiliary layer 220, a light emitting layer 140, 340, 440 or a layer for improving luminous efficiency 180, more preferably, as host material of a light emitting layer 140, 340, 440.

Even if the core is the same core, the band gap, the electrical characteristics, the interface characteristics and the like may be different depending on which substituent is bonded at which position. Therefore, it is necessary to study the selection of the core and the combination of the core and the sub-substituent bonded to the core. In particular, long life span and high efficiency can be simultaneously achieved when the optimal combination of energy levels and T₁ values, inherent material properties (mobility, interfacial properties, etc.), and the like among the respective layers of an organic material layer is achieved.

Therefore, energy level and T₁ value between the respective layers of the organic material layer, inherent material properties (mobility, interfacial properties, etc.) and the like can be optimized by using the mixture of the compound represented by Formula 1 and the compound represented by Formula 2, or by using the compound represented by Formula 3 comprised in Formula 1 as material of a light emitting layer 140, 340, 440 and/or an emission-auxiliary layer 220, and thus it is possible to simultaneously improve the lifetime and efficiency of the organic electric element.

The organic electric element according to an embodiment of the present invention may be manufactured using various deposition methods. The organic electric element according to an embodiment of the present invention may be manufactured using a PVD (physical vapor deposition) method or CVD (chemical vapor deposition) method. For example, the organic electric element may be manufactured by depositing a metal, a conductive metal oxide, or a mixture thereof on the substrate to form the anode 110, forming the organic material layer comprising the hole injection layer 120, the hole transport layer 130, the light emitting layer 140, the electron transport layer 150, and the electron injection layer 160 thereon, and then depositing a material, which can be used as the cathode 170, thereon. Also, an emission-auxiliary layer 220 may be formed between a hole transport layer 130 and a light emitting layer 140, and an electron transport auxiliary layer(not shown) may be further formed between a light emitting layer 140 and an electron transport layer 150 and, as described above, a stack structure may be formed.

Also, the organic material layer may be manufactured in such a manner that the fewer layers are formed using various polymer materials by a soluble process or solvent process, for example, spin coating, nozzle printing, inkjet printing, slot coating, dip coating, roll-to-roll, doctor blading, screen printing, or thermal transfer, instead of deposition. Since the organic material layer according to the present invention may be formed in various ways, the scope of protection of the present invention is not limited by a method of forming the organic material layer.

The organic electric element according to an embodiment of the present invention may be of a top emission type, a bottom emission type, or a dual emission type depending on the material used.

In addition, the organic electric element according to an embodiment of the present invention may be selected from the group consisting of an organic light emitting diode, an organic solar cell, an organic photo conductor, an organic transistor, an element for monochromatic illumination and an element for quantum dot display.

Another embodiment of the present invention provides an electronic device including a display device which includes the above described organic electric element, and a control unit for controlling the display device. Here, the electronic device may be a wired/wireless communication terminal which is currently used or will be used in the future, and covers all kinds of electronic devices including a mobile communication terminal such as a cellular phone, a personal digital assistant (PDA), an electronic dictionary, a point-to-multipoint (PMP), a remote controller, a navigation unit, a game player, various kinds of TVs, and various kinds of computers.

Hereinafter, the organic electric element and the compound according to an aspect of the present invention will be described.

The organic electric element according to an aspect of the present invention comprises a first electrode, a second electrode, and an organic material layer formed between the first electrode and the second electrode, wherein the organic material layer comprises a phosphorescent light emitting layer, and the host of the phosphorescent light emitting layer comprises a first compound represented by the following Formula 1 and a second compound represented by the following Formula 2.

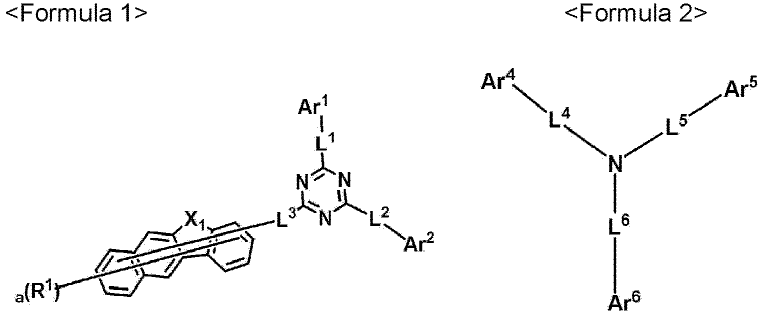

First, Formula 1 will be described.

In the formula 1, each of symbols may be defined as follows.

X₁ is O or S.

Ar¹ and Ar² may be each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring group, a C₁-C₃₀ alkyl group, a C₂-C₃₀ alkenyl group, a C₂-C₃₀ alkynyl group, a C₁-C₃₀ alkoxyl group and a C₆-C₃₀ aryloxy group.

Where Ar¹ and Ar² are an aryl group, Ar¹ and Ar² may be preferably a C₆-C₃₀ aryl group, more preferably a C₆-C₁₈ aryl group, for example, phenyl, biphenyl, naphthyl, terphenyl, phenanthrene, pyrene, triphenylene, anthracene or the like.

When Ar¹ and Ar² are a heterocyclic group, Ar¹ and Ar² may be preferably a C₂-C₃₀ heterocyclic group, more preferably a C₂-C₁₈ heterocyclic group, for example, dibenzothiophene, dibenzofuran, carbazole, phenylcarbazole, benzonaphthofuran, benzonaphthothiophene or the like.

When Ar¹ and Ar² is a fluorenyl group, Ar¹ and Ar² may be 9,9-diphenylfluorene, 9,9-dimethylfluorene or the like.

Where Ar¹ and Ar² are an aliphatic ring group, Ar¹ and Ar² may be preferably a C₃-C₃₆ aliphatic ring group, more preferably a C₃-C₁₂ aliphatic ring group, for example, cyclohexane, cyclohexylcyclohexane or the like.

Where Ar¹ and Ar² are an alkyl group, Ar¹ and Ar² may be preferably a C₁-C₁₀ alkyl group, for example, methyl, t-butyl or the like. Where Ar¹ and Ar² are an alkenyl group, Ar¹ and Ar² may be preferably a C₂-C₁₀ alkenyl group, for example, ethene, propene or the like.

L¹ to L³ may be each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring and a combination thereof.

Where L¹ to L³ are each arylene group, L¹ to L³ may be preferably a C₆-C₃₀ arylene group, more preferably a C₆-C₁₈ arylene group, for example, phenyl, biphenyl, naphthyl, terphenyl or the like. Where L¹ to L³ are a heterocyclic group, L¹ to L³ may be preferably a C₂-C₃₀ heterocyclic group, more preferably a C₂-C₁₈ heterocyclic group, for example, carbazole, phenylcarbazole, dibenzofuran, dibenzothiophene or the like.

R¹ may be selected from the group consisting of hydrogen, deuterium, halogen, a cyano group, a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₅₀ alkyl group, a C₂-C₂₀ alkenyl group, a C₂-C₂₀ alkynyl group, a C₁-C₃₀ alkoxyl group, a C₆-C₃₀ aryloxy group and -L'-N(Rₐ)(R_{b}), and adjacent groups may optionally be linked to each other to form a ring.
a is an integer of 0~9, and where a is an integer of 2 or more, each of a plurality of R¹ is the same as or different from each other.

The ring formed by bonding adjacent R¹s to each other may be a C₆-C₆₀ aromatic ring group, a fluorenyl group, a C₂-C₆₀ heterocyclic group containing at least one heteroatom selected from the group consisting of O, N, S, Si, and P, or a C₃-C₆₀ aliphatic ring or the like. Where adjacent R¹ groups are linked together to each other to form an aromatic ring group, the ring may be preferably a C₆-C₃₀ aromatic ring group, more preferably a C₆-C₁₄ aromatic ring group, for example, benzene ring, naphthalene, phenanthrene or the like.

Where R¹ is an aryl group, R¹ may be preferably a C₆-C₃₀ aryl group, more preferably a C₆-C₁₈ aryl group, for example, phenyl, naphthyl, biphenyl, terphenyl, phenanthrene or the like.

L' may be each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof.

Rₐ and R_{b} may be each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, and a combination thereof.

Ar¹, Ar², L¹~L³, R¹, L', Rₐ, R_{b}, and the ring formed by bonding adjacent R¹s to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group.

Formula 1 may be represented by one of the following Formula 1-A to Formula 1-G:

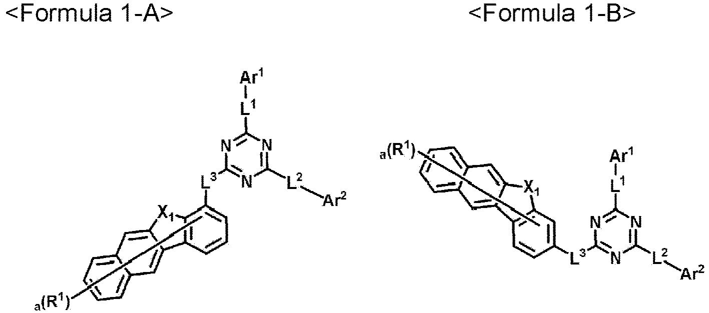

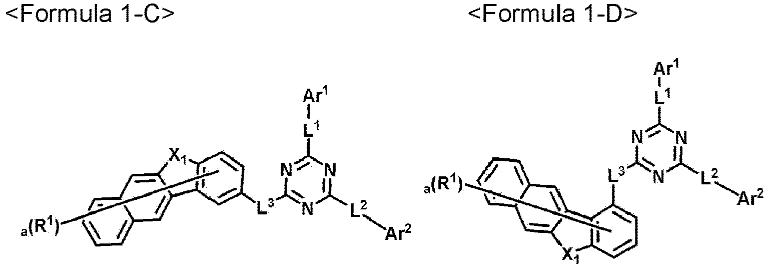

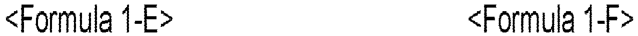

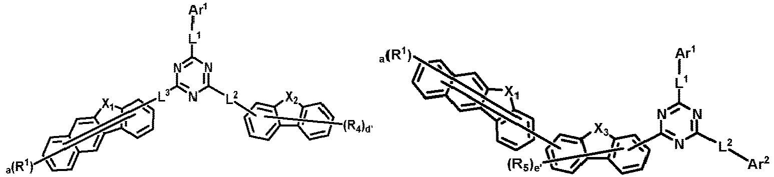

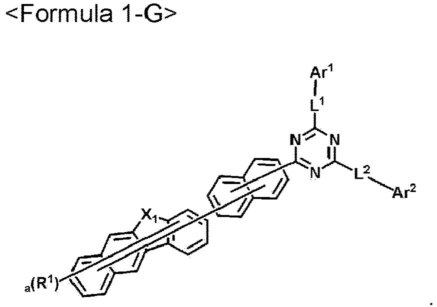

In the formulas 1-A to 1-G, each of symbols may be defined as follows.

Ar¹, Ar², L¹~L³, X₁, R¹ and a are the same as defined in Formula 1. Preferably, in the formulas 1-F and 1-G, Ar¹ and Ar² are different from each other, and preferably, Ar¹ and Ar² may be each independently an aryl group, more preferably naphthyl.

X₂ and X₃ are each independently O or S.

R₄ and R₅ are each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₃₀ aryloxy group, and adjacent groups may optionally be linked to each other to form a ring.

'The ring formed by bonding adjacent groups to each other' may be a C₆-C₆₀ aromatic ring group, a fluorenyl group, a C₂-C₆₀ heterocyclic group containing at least one heteroatom selected from the group consisting of O, N, S, Si, and P, or a C₃-C₆₀ aliphatic ring or the like. Where adjacent R₄ groups or adjacent R₅ groups are linked to each other to form an aromatic ring group, the ring may be preferably a C₆-C₃₀ aromatic ring group, more preferably a C₆-C₁₄ aromatic ring group, for example, benzene, naphthalene, phenanthrene or the like.

d' is an integer of 0~7, e' is an integer of 0~6, and where they are an integer of 2 or more, respectively, each of a plurality of R₄ and each of a plurality of R₅ are the same as or different from each other.

Preferably, in the formulas 1-A to 1-G or the like, Ar¹, Ar², L¹~L³, R¹, R₄, R₅, and the ring formed by bonding adjacent groups to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group.

In addition, preferably, Formula 1 may be represented by the following Formula 3.

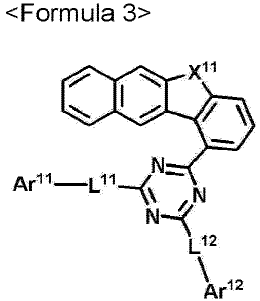

In the formula 3, X¹¹ is O or S, L¹¹ and L¹² are each independently a single bond or a C₆-C₃₀ arylene group, and Ar¹¹ and Ar¹² are each independently C₆-C₃₀ aryl group.

Where L¹¹ and L¹² are each independently an arylene group, preferably, a C₆-C₂₀, a C₆-C₁₉, a C₆-C₁₈, a C₆-C₁₇, a C₆~C₁₆, a C₆-C₁₅, a C₆~C₁₄, a C₆-C₁₃, a C₆~C₁₂, a C₆-C₁₁, a C₆~C₁₀, a C₆, a C₁₀, a C₁₁, a C₁₂, a C₁₃, a C₁₄, a C₁₅, a C₁₆ or a C₁₈ arylene group.

Where Ar¹¹ and Ar¹² are each independently an aryl group, preferably, a C₆-C₂₀, a C₆-C₁₉, a C₆-C₁₈, a C₆-C₁₇, a C₆~C₁₆, a C₆-C₁₅, a C₆~C₁₄, a C₆-C₁₃, a C₆~C₁₂, a C₆-C₁₁, a C₆~C₁₀, a C₆, a C₁₀, a C₁₁, a C₁₂, a C₁₃, a C₁₄, a C₁₅, a C₁₆ or a C₁₈ aryl group.

Preferably, dimethylcyclopentaphenanthrene being a C₁₇ aryl group is excluded from the Formula 3.

In the formula 3, L¹¹, L¹², Ar¹¹ and Ar¹² may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group.

In the formula 3, where X¹¹ is O, Formula 3 may be represented by the following Formula 3-1.

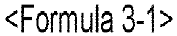

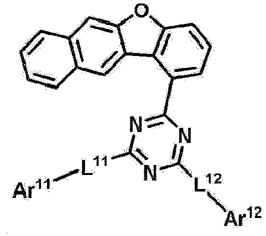

In the formula 3-1, each of symbols may be the same as defined in Formula 3.

Formula 3-1 may be represented by the following Formulas 3-A or 3-B.

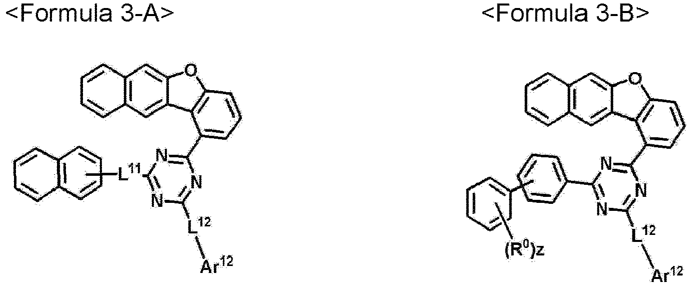

In Formulas 3-A and 3-B, each of symbols may be the same as defined in Formula 3, and R° is the same as Ar¹¹ in Formula 3. z is an integer of 0~5, and where z is an integer of 2 or more, each of a plurality of R° is the same as or different from each other, and adjacent groups may optionally be linked to each other to form a ring. Preferably, R° is hydrogen or a C₆-C₂₀ aryl group, or adjacent R°s may optionally be linked to each other to form a benzene or naphthalene ring.

Specifically, the compound represented by formula 1 may be one of the following compounds.

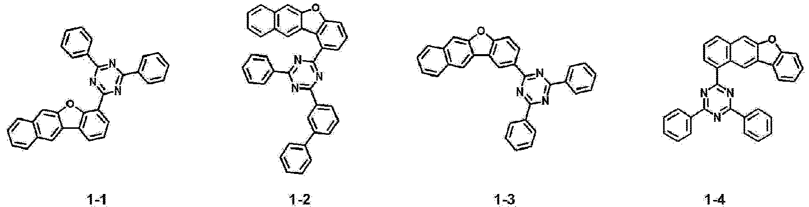

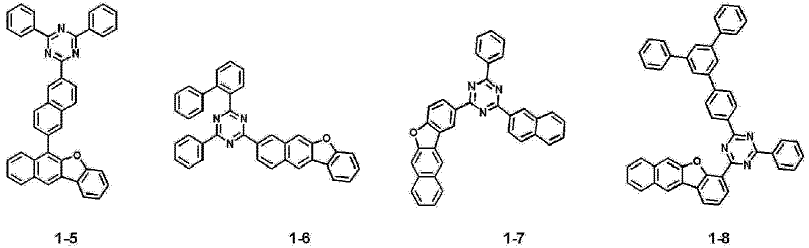

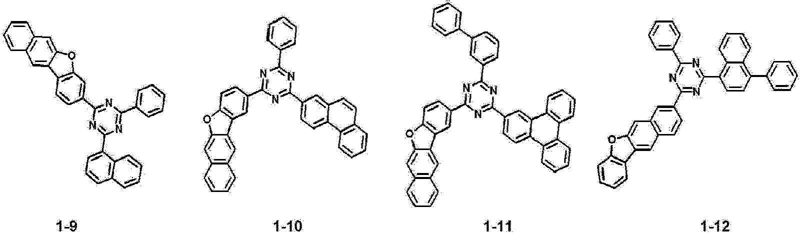

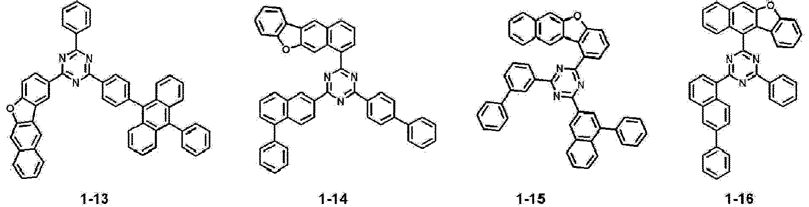

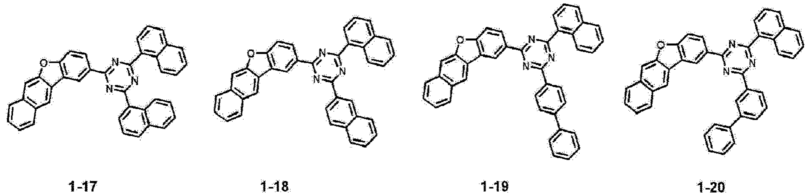

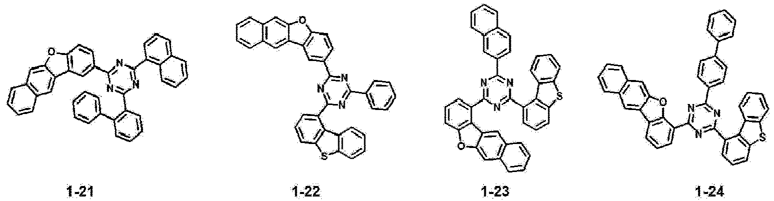

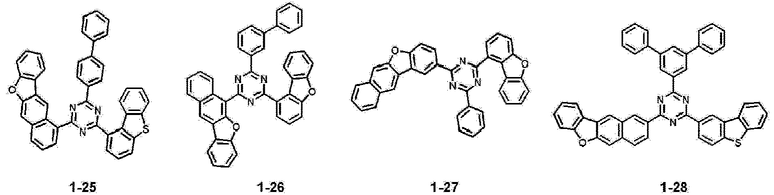

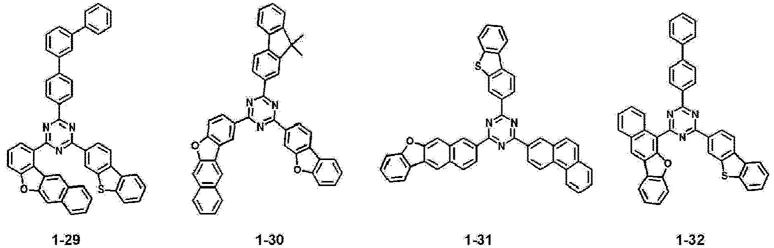

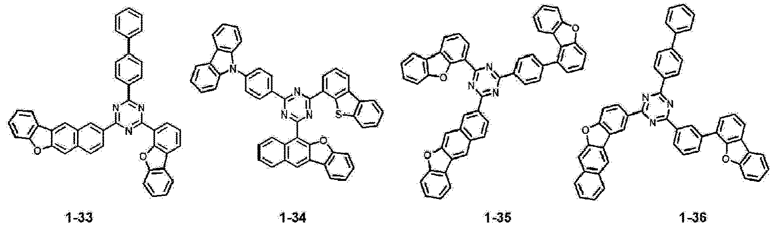

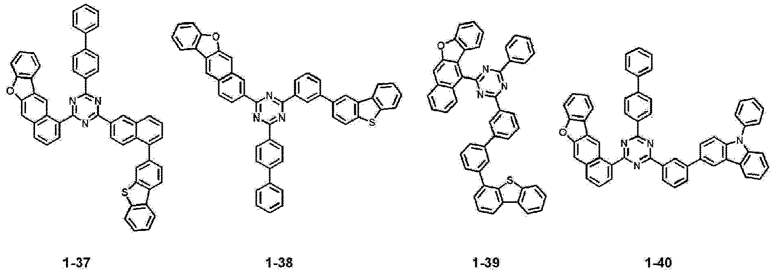

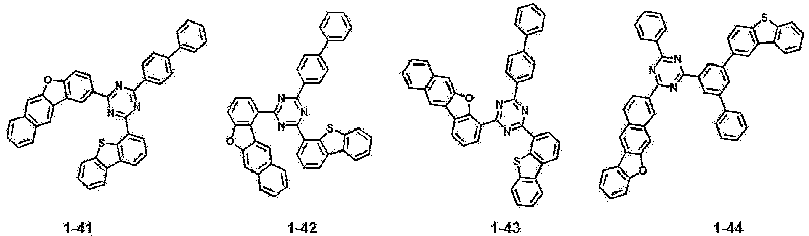

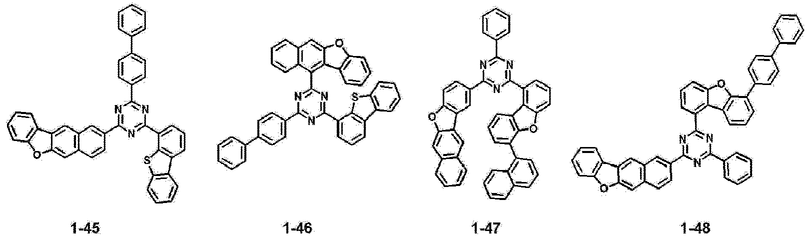

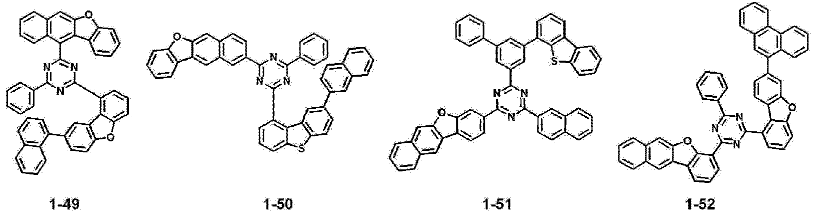

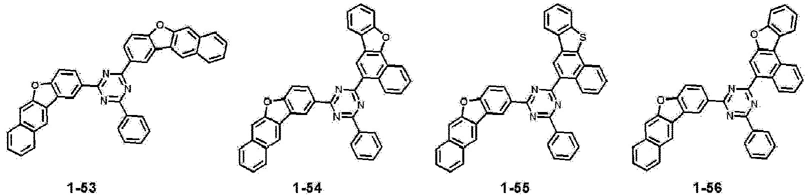

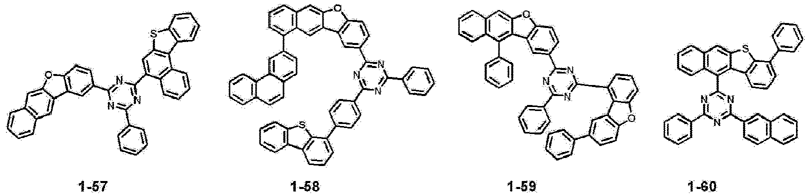

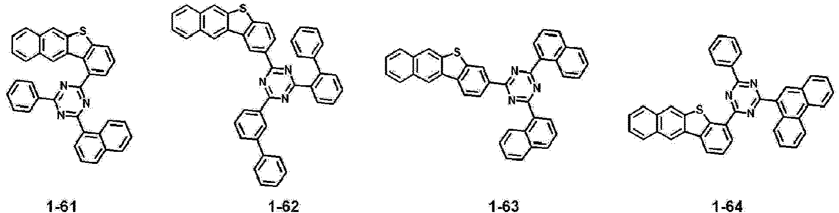

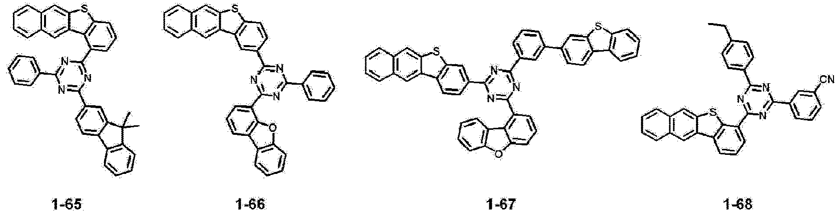

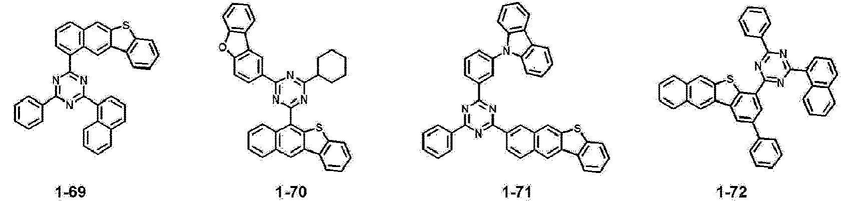

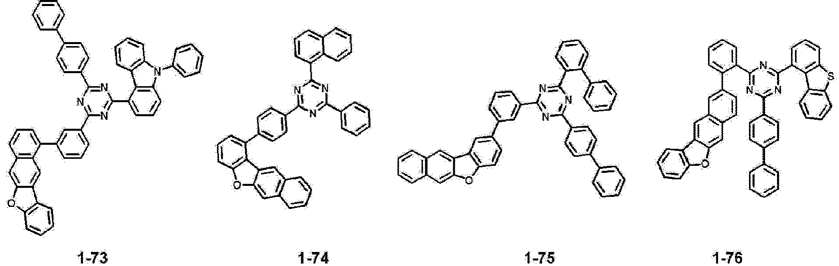

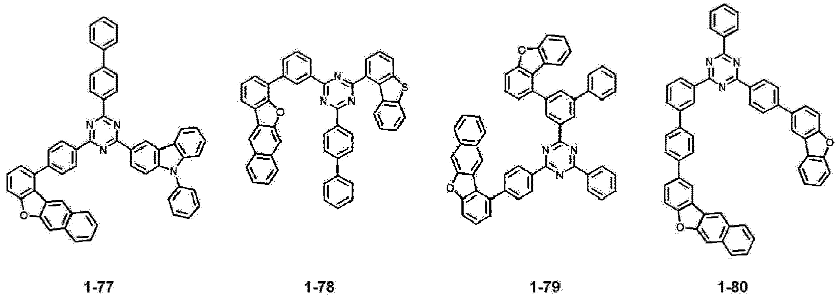

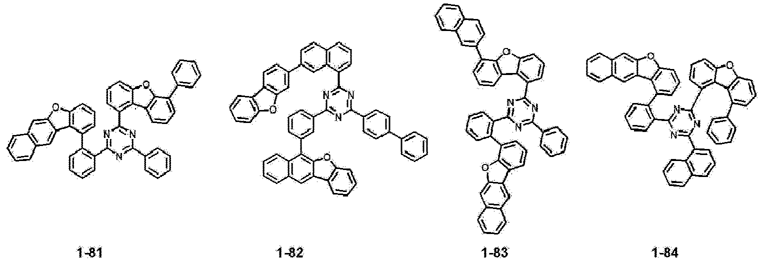

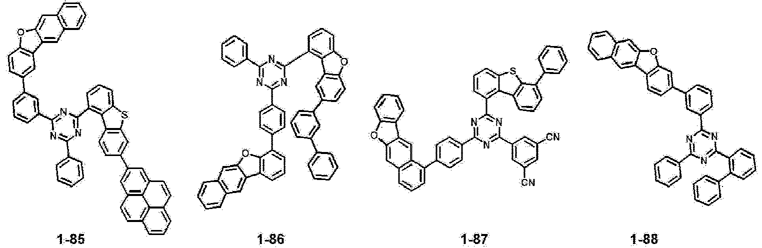

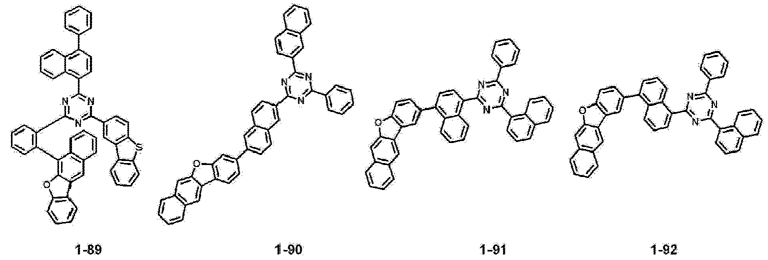

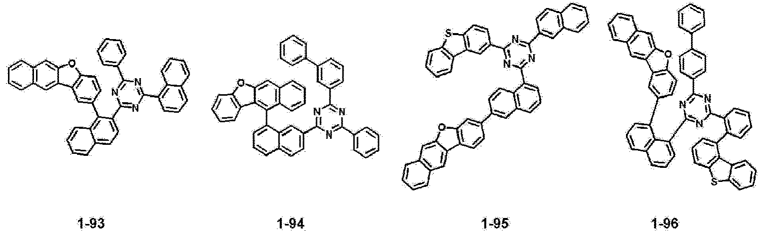

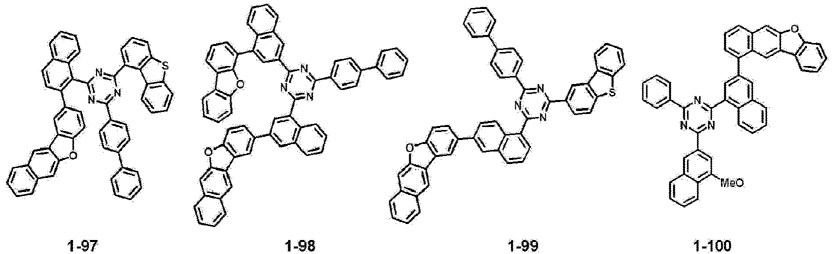

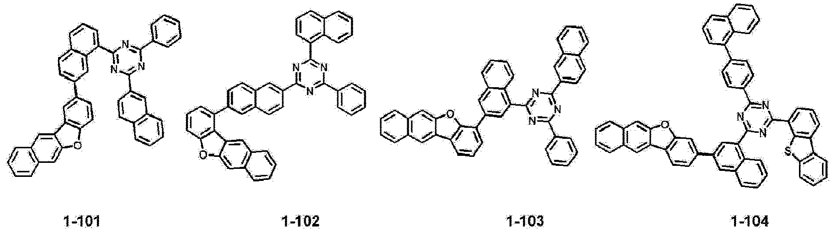

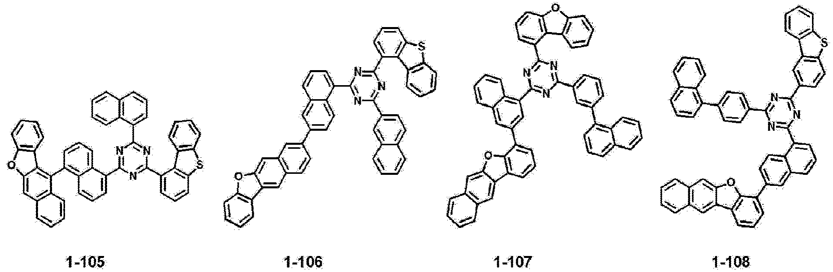

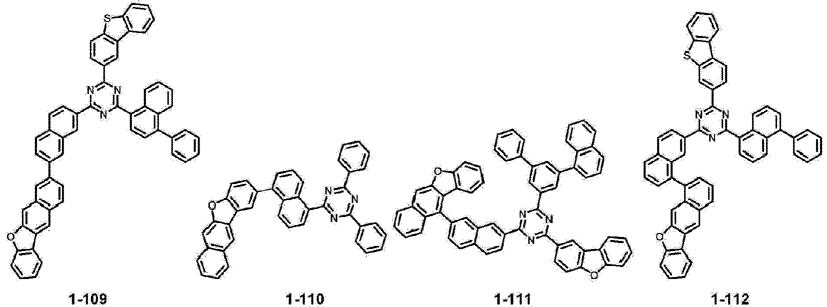

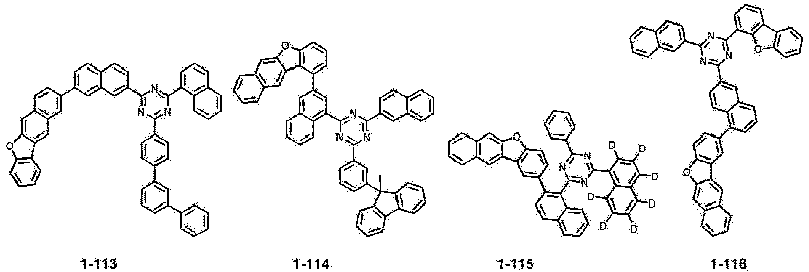

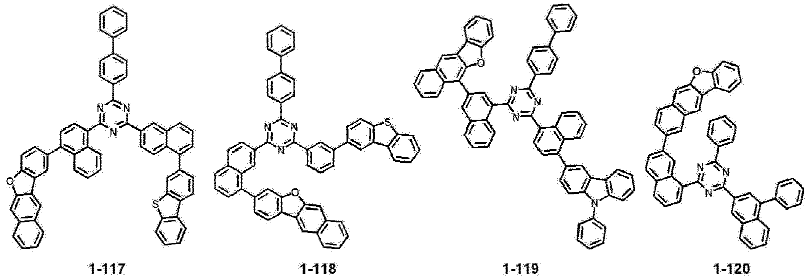

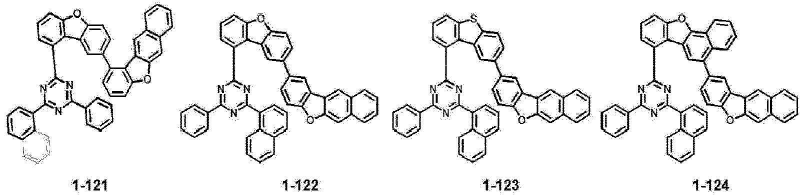

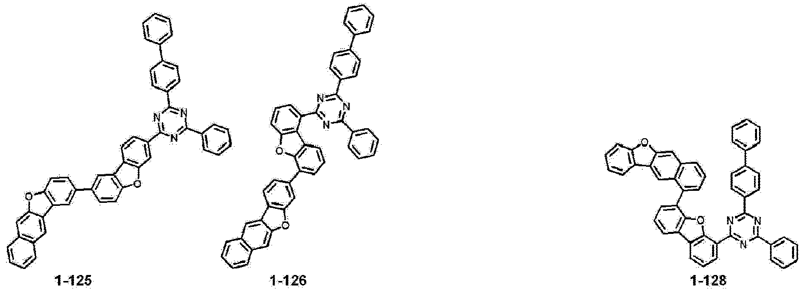

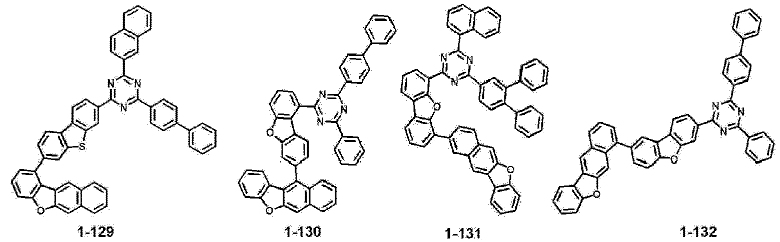

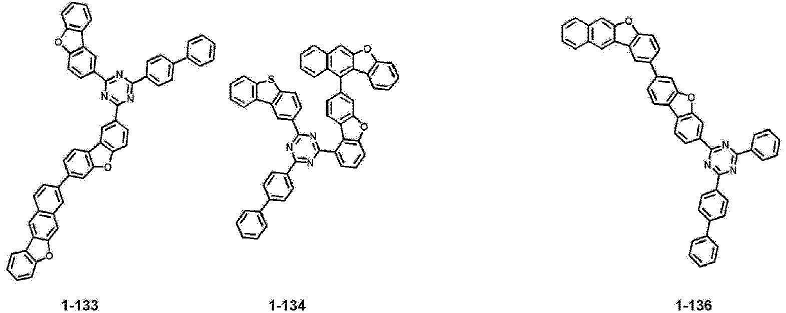

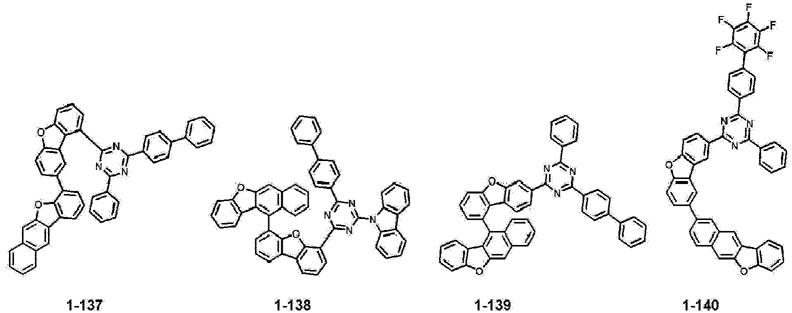

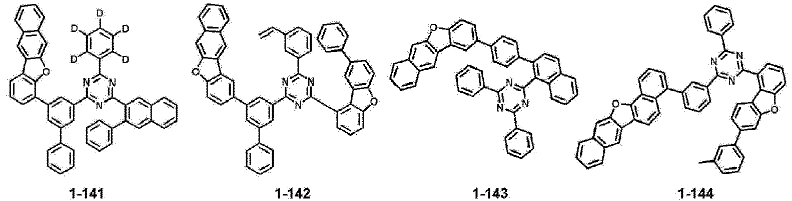

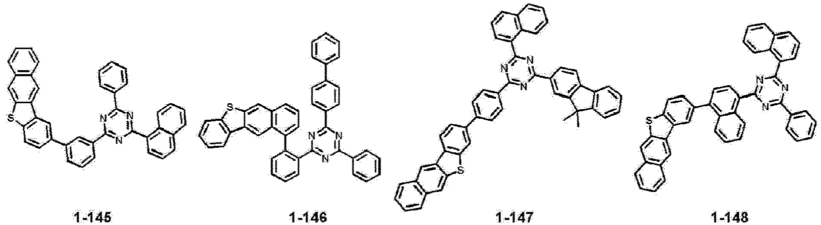

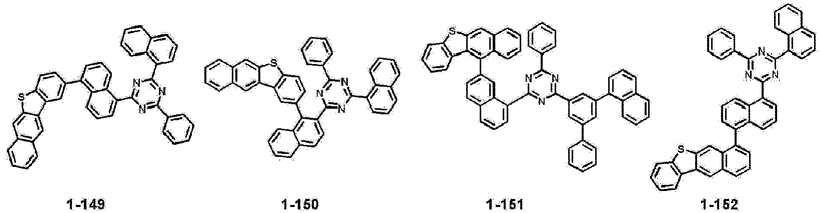

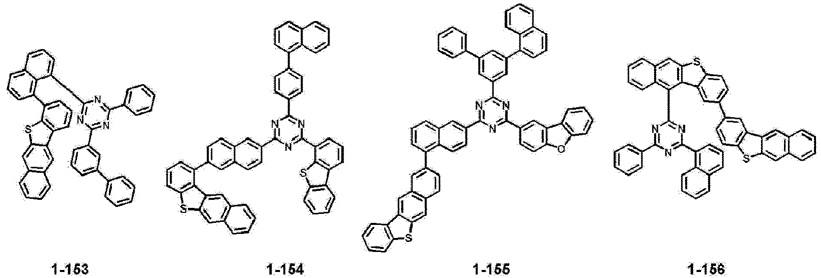

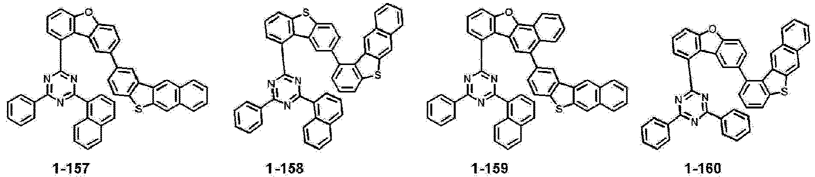

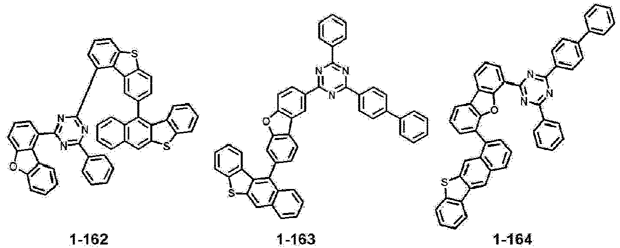

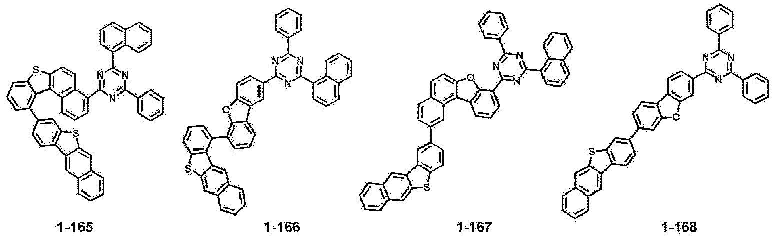

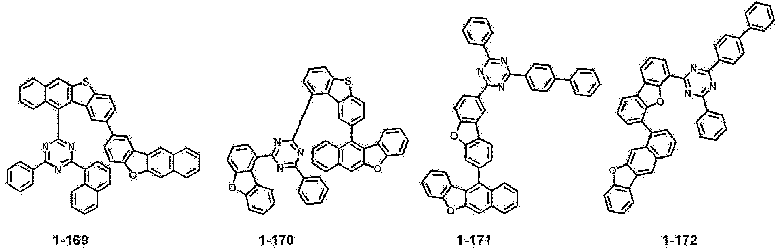

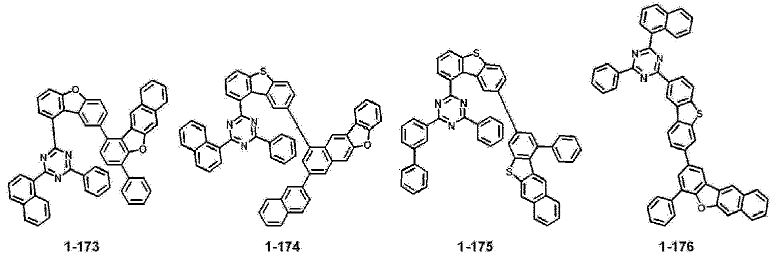

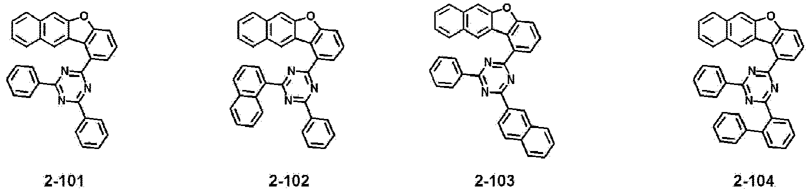

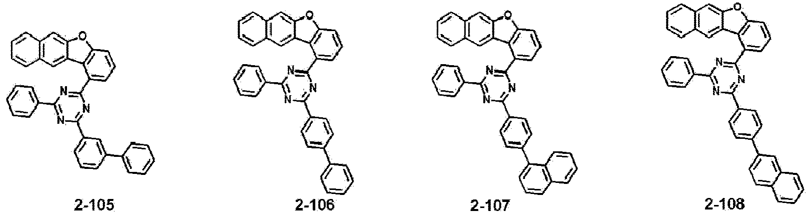

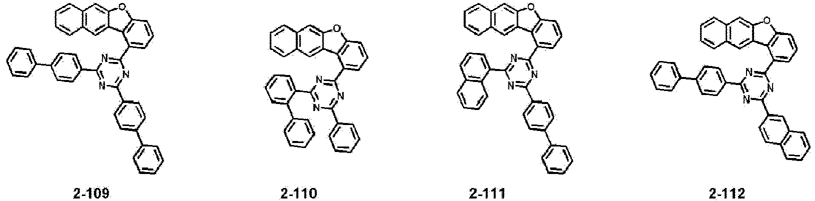

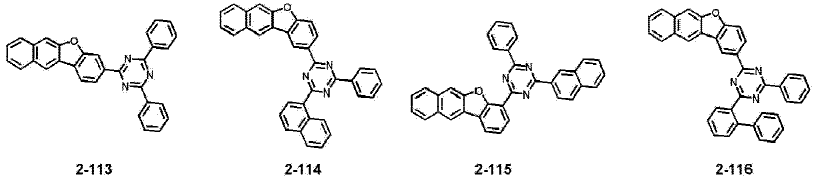

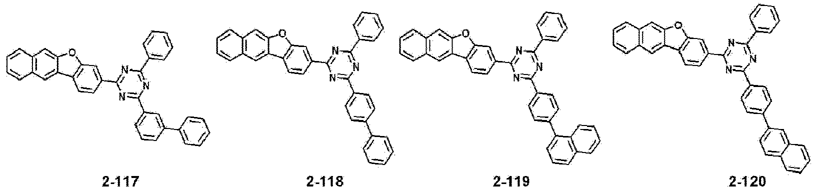

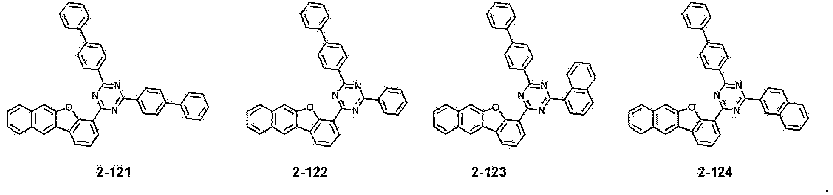

Next, the following Formula 2 will be described.

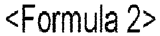

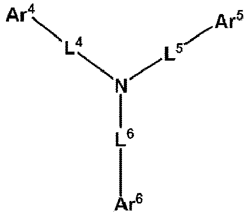

In the formula 2, each of symbols may be defined as follows.

Ar⁴ to Ar⁶ may be each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₃₀ alkyl group, a C₂-C₃₀ alkenyl group, a C₂-C₃₀ alkynyl group, a C₁-C₃₀ alkoxyl group and a C₆-C₃₀ aryloxy group.

Where Ar⁴ to Ar⁶ are an aryl group, Ar⁴ to Ar⁶ may be preferably a C₆-C₃₀ aryl group, more preferably a C₆-C₁₈ aryl group, for example, phenyl, biphenyl, naphthyl, terphenyl, phenanthrene or the like.

When Ar⁴ to Ar⁶ are a heterocyclic group, Ar⁴ to Ar⁶ may be preferably a C₂-C₃₀ heterocyclic group, more preferably a C₂-C₂₀ heterocyclic group, for example, dibenzothiophene, dibenzofuran, benzothienopyrimidine, carbazole, phenylcarbazole, benzonaphthothiophene, benzofurothiophene, dinaphthothiophene, dinaphthofuran or the like.

When Ar⁴ to Ar⁶ are a fluorenyl group, Ar⁴ to Ar⁶ may be 9,9-dimethyl-9H-fluorene, 9,9-diphenyl-9H-fluorene, 9,9'-spirobifluorene or the like.

L⁴ to L⁶ may be each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,

Where L⁴ to L⁶ are each an arylene group, L⁴ to L⁶ may be preferably a C₆-C₃₀ arylene group, more preferably a C₆-C₁₈ arylene group, for example, phenylene, biphenyl, naphthyl, terphenyl or the like.

When L⁴ to L⁶ are a fluorenyl group, L⁴ to L⁶ may be 9,9-dimethyl-9H-fluorene, 9,9-diphenyl-9H-fluorene, 9,9'-spirobifluorene or the like.

Ar⁴~Ar⁶ may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group, a C₈-C₂₀ arylalkenyl group and -L'-N(Rₐ)(R_{b}).

L' may be selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof.

Rₐ and R_{b} may be each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, and a combination thereof.

L⁴~L⁶, R¹, L', Rₐ and R_{b} may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group

Formula 2 may be represented by one of the following Formula 2-A to Formula 2-L.

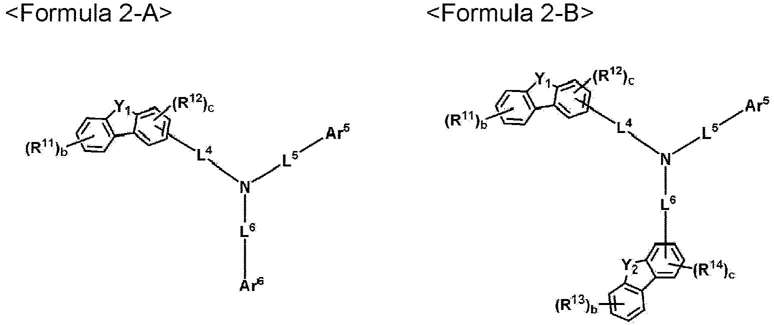

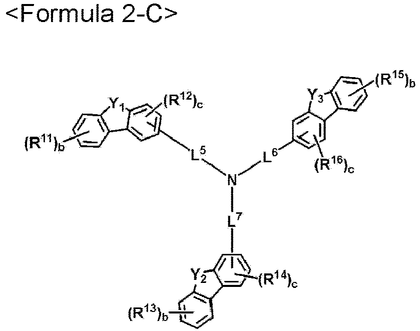

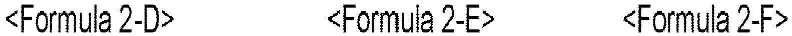

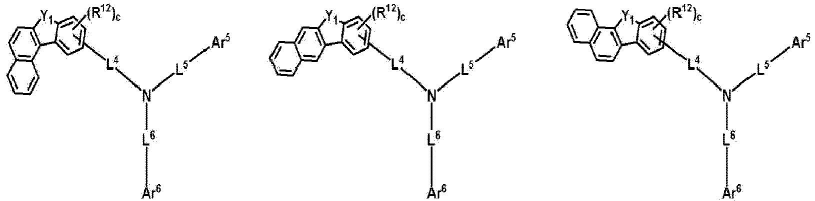

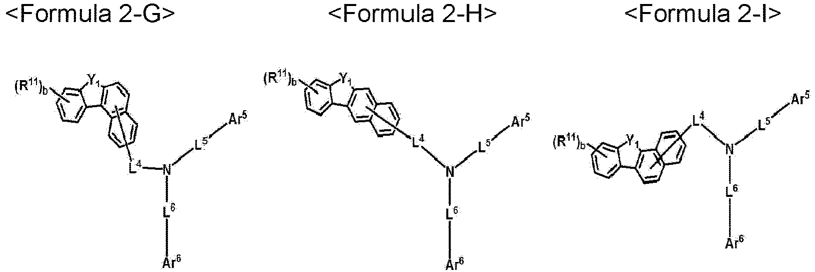

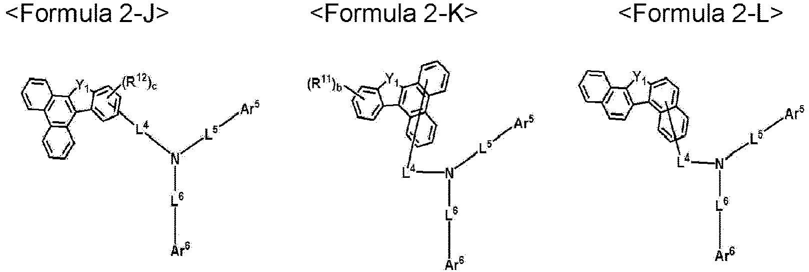

In Formulas 2-A to 2-L, Ar⁵, Ar⁶, and L⁴~L⁶ are the same as defined in Formula 2, and Y₁~Y₃ are each independently O, S, or C(R')(R").

R¹¹~R¹⁶, R' and R" are each independently selected from the group consisting of hydrogen, deuterium, halogen, a silane group substituted or unsubstituted with C₁-C₂₀ alkyl group or C₆-C₂₀ aryl group, a cyano group, a nitro group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and -L'-N(Rₐ)(R_{b}), and adjacent groups may optionally be linked to each other to form a ring. Here, L', Rₐ and R_{b} are the same as defined in Formula 2.

The ring formed by bonding adjacent groups to each other may be a C₆-C₆₀ aromatic ring group, a fluorenyl group, a C₂-C₆₀ heterocyclic group containing at least one heteroatom selected from the group consisting of O, N, S, Si, and P, or a C₃-C₆₀ aliphatic ring or the like. For example, where adjacent R¹¹ groups, adjacent R¹² groups, adjacent R¹³ groups, adjacent R¹⁴ groups, adjacent R¹⁵ groups, adjacent R¹⁶ groups, and/or R' and R" groups, respectively, are linked to each other to form an aromatic ring group, the ring may be preferably a C₆-C₃₀ aromatic ring group, more preferably a C₆-C₁₄ aromatic ring group, for example, benzene, naphthalene, phenanthrene or the like.

b is an integer of 0~4, and where b is an integer of 2 or more, each of a plurality of R¹¹, each of a plurality of R¹³ and each of a plurality of R¹⁵ are the same as or different from each other, c is an integer of 0~3, and where c is an integer of 2 or more, each of a plurality of R¹², each of a plurality of R¹⁴ and each of a plurality of R¹⁶ are the same as or different from each other.

In addition, preferably, Formula 2 may be represented by the following Formula 4. The following Formula 4 may be the case where one of R¹¹s in Formula 2-A is -L'-N(Rₐ)(R_{b}) or the case where Ar⁴ is a 3-fused(condensed) ring comprising Y₁ and the 3-fused ring is further substituted with -L'-N(Rₐ)(R_{b}) in Formula 2.

Therefore, in the following Formula 4, Y₁, R¹¹, R¹², L⁴, L⁵, L⁶, Ar⁵, Ar⁶, c and the like are the same as defined in Formula 2-A, L²¹ is defined to be the samen as L' of Formula 2 or Formula 2-A, R²¹ and R²² are each defined to be the samen as Rₐ and R_{b} of Formula 2 or Formula 2-A, and d is an integer of 0~3. Each of a plurality of R¹¹ is the same as or different from each other and adjacent groups may optionally be linked to each other to form a ring, where d is an integer of 2 or more.

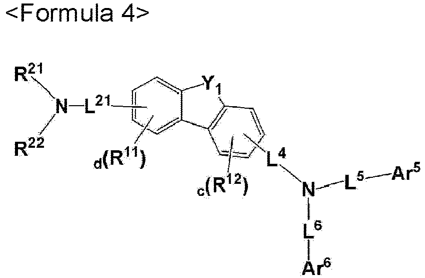

Where R¹¹ and R¹² are an aryl group, R¹¹ and R¹² may be preferably a C₆-C₁₈ aryl group, for example, phenyl, naphthyl, biphenyl, terphenyl, phenanthrene or the like.

Where R¹¹ and R¹² are a heterocyclic group, R¹¹ and R¹² may be preferably a C₂-C₁₂ heterocyclic group, for example, a heterocyclic ring containing N such as pyridine, pyrimidine, triazine, quinazoline, quinoline, isoquinoline and the like, or dibenzothiophene, dibenzofuran and the like.

Where R¹¹ and R¹² are an alkyl group, R¹¹ and R¹² may be preferably a C₁-C₁₀ alkyl group, for example, methyl, t-butyl and the like.

The ring formed by bonding adjacent R¹¹ groups and/or adjacent R¹² groups to each other may be a C₆-C₂₀ aromatic ring group, a fluorenyl group, a C₂-C₂₀ heterocyclic group containing at least one heteroatom selected from the group consisting of O, N, S, Si, and P, or a C₃-C₂₀ aliphatic ring or the like. Where adjacent groups are linked to each other to form an aromatic ring group, the ring may be preferably a C₆-C₁₄ aromatic ring group, for example, benzene, naphthalene, anthracene, phenanthrene or the like.

Where L²¹ is an arylene group, L²¹ may be preferably a C₆-C₃₀ arylene group, more preferably a C₆-C₁₈ arylene group, for example, phenylene, naphthylene, biphenyl, terphenyl or the like.

Where R²¹ and R²² are an aryl group, R²¹ and R²² may be preferably a C₆-C₁₈ aryl group, for example, phenyl, naphthyl, biphenyl, terphenyl, phenanthrene, triphenylene or the like.

Where R²¹ and R²² are a heterocyclic group, R²¹ and R²² may be preferably a C₂-C₁₈ heterocyclic group, for example, benzothiophene, benzofuran, dibenzothiophene, dibenzofuran, benzonaphthothiophene, benzonaphthofuran, carbazole, phenylcarbazole and the like.

When R²¹ and R²² is a fluorenyl group, R²¹ and R²² may be 9,9-dimethylfluorene, 9,9-diphenylfluorene, 9,9'-bispirofluorene or the like.

Preferably, Formula 4 may be represented by one of the following Formulas 4-1 to 4-9.

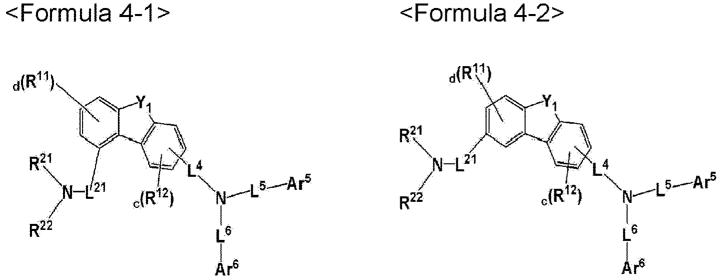

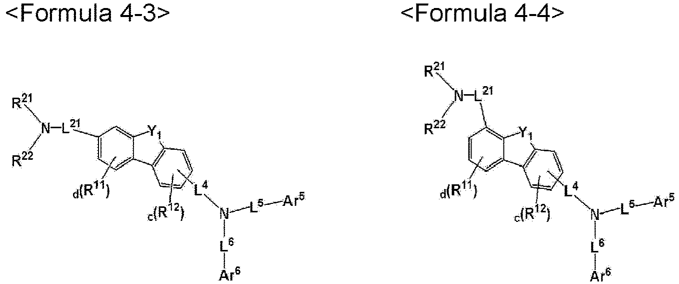

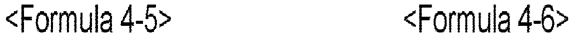

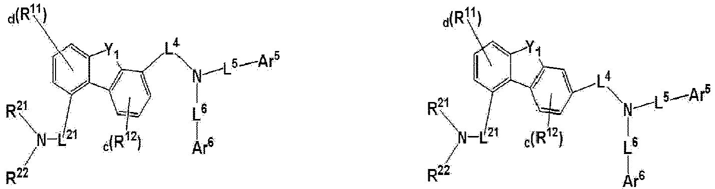

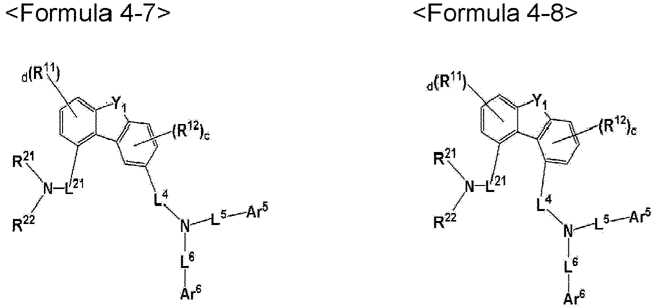

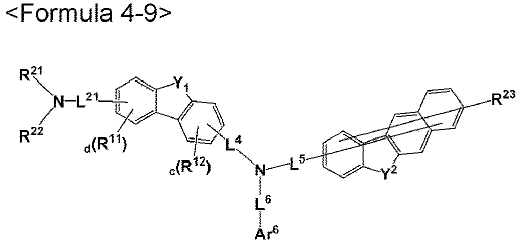

In Formulas 4-1 to 4-9, the symbols such as Y₁, L⁴, L⁵, L⁶, Ar⁵, Ar⁶, R¹¹, R¹², L²¹, R²¹, R²², c, d and the like are the same as defined in Formula 4, Y² is O or S, and R²³ may be defined to be the same as R¹¹.

Specifically, the compound represented by formula 2 may be one of the following compounds, but it is not limited thereto.

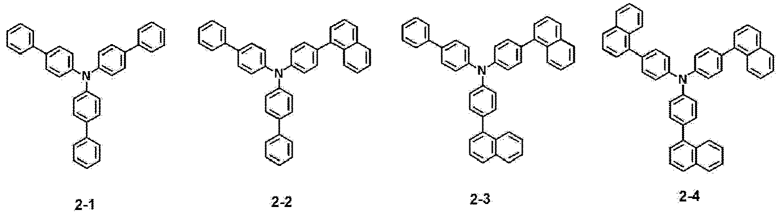

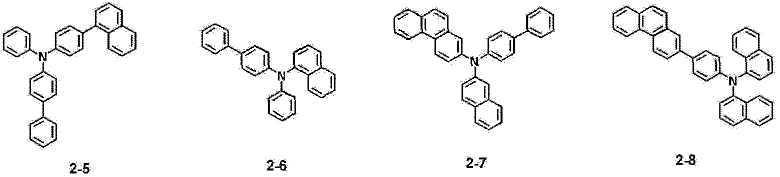

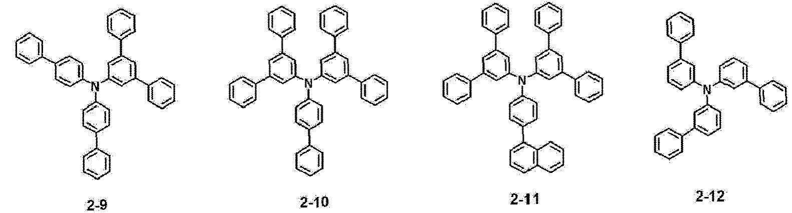

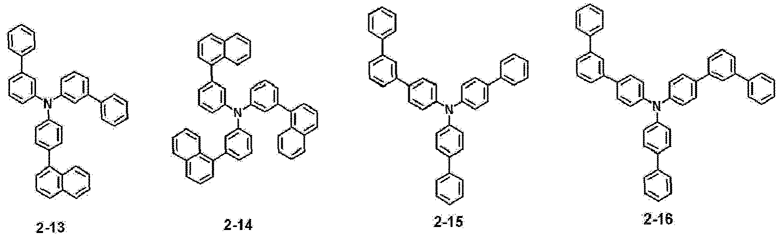

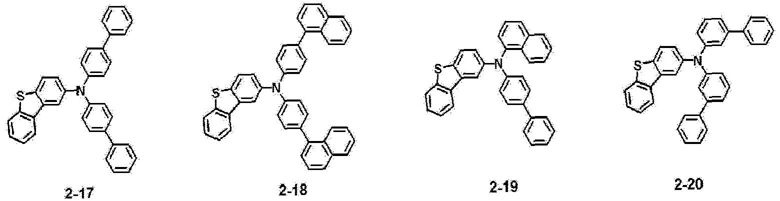

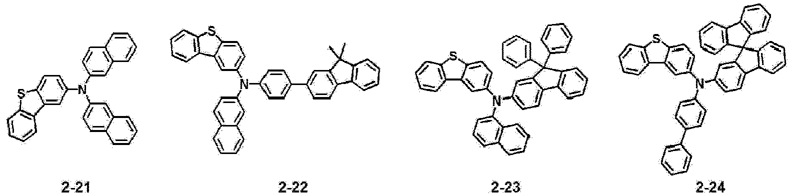

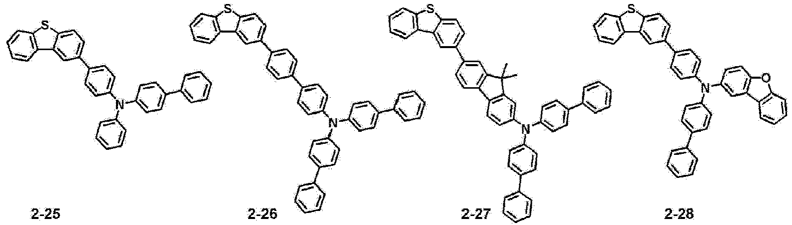

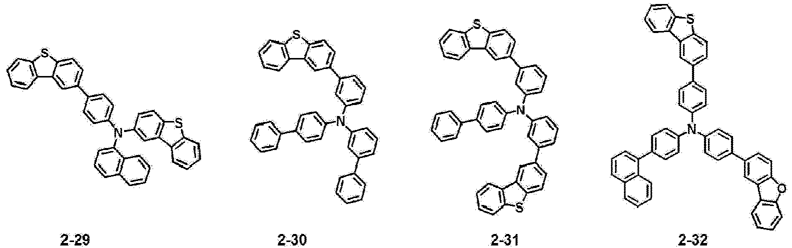

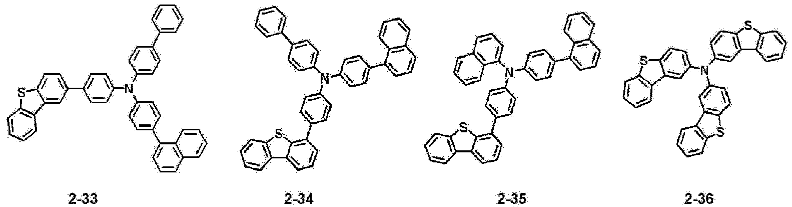

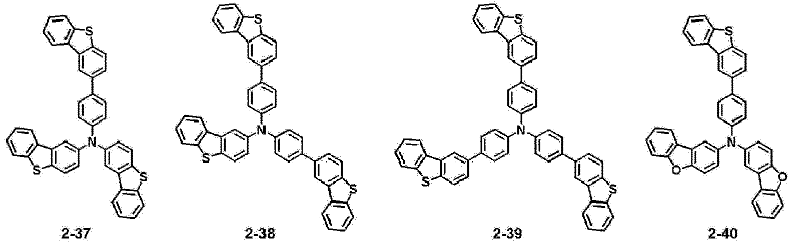

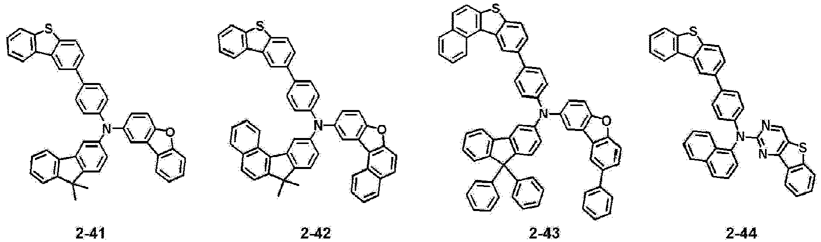

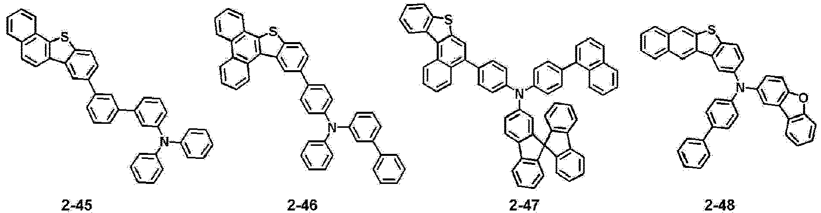

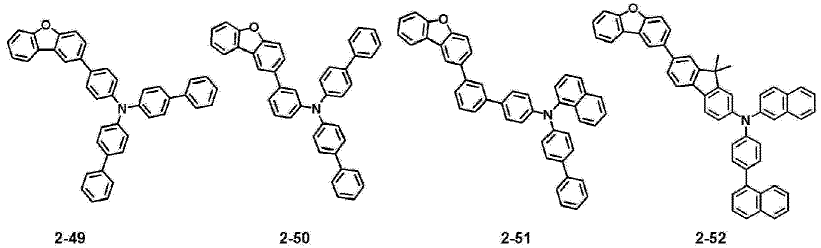

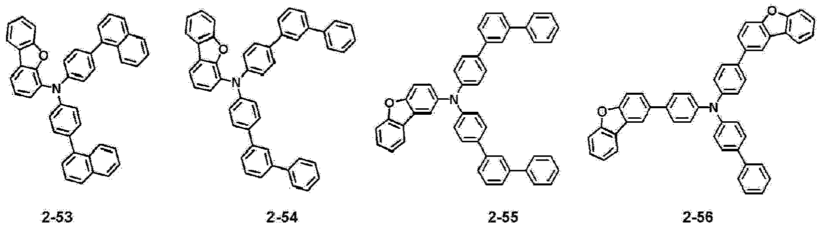

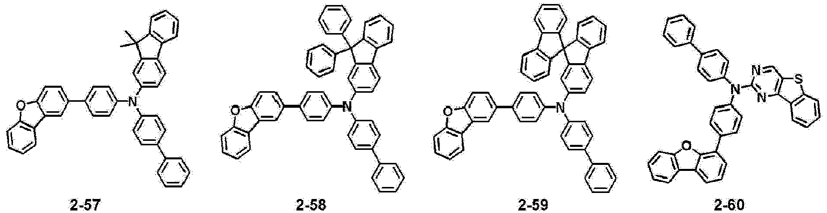

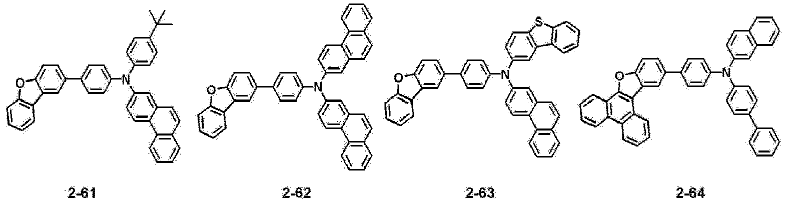

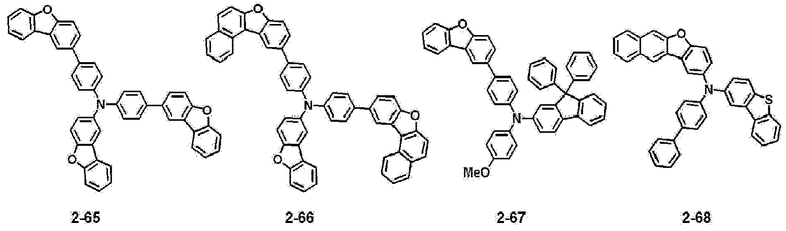

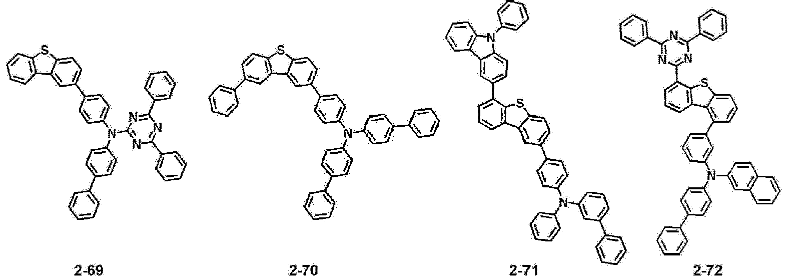

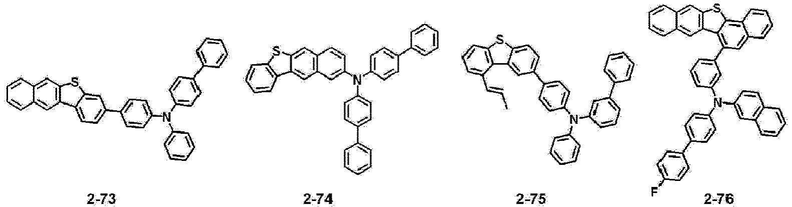

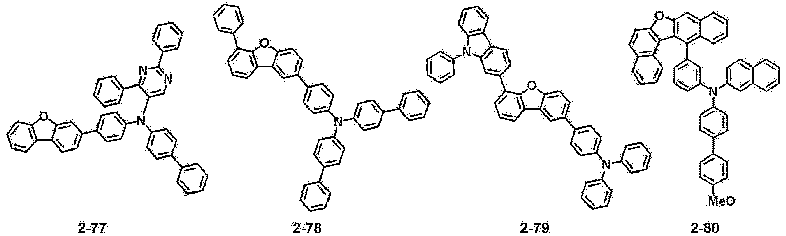

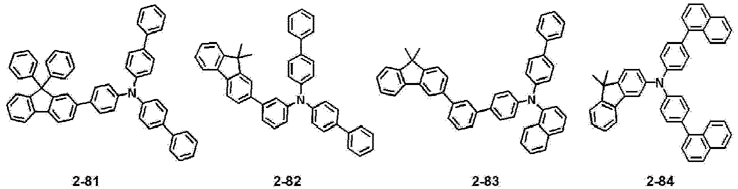

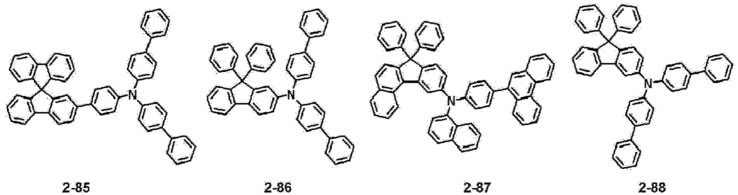

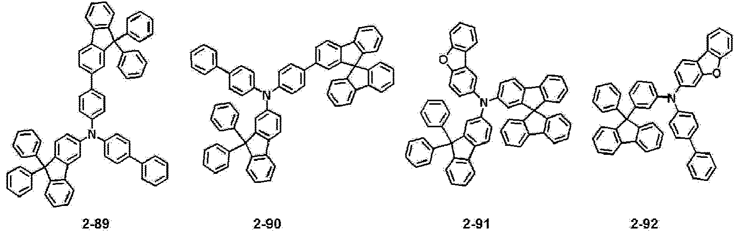

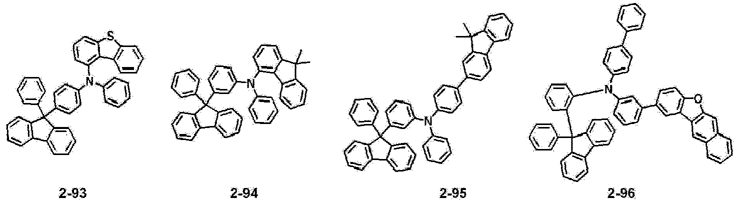

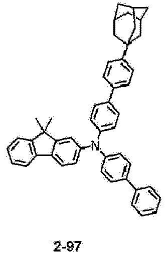

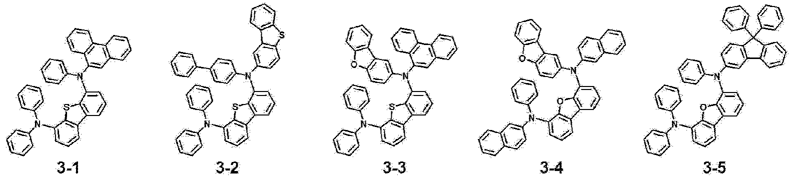

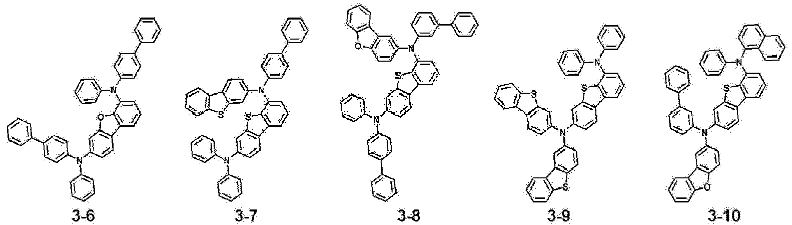

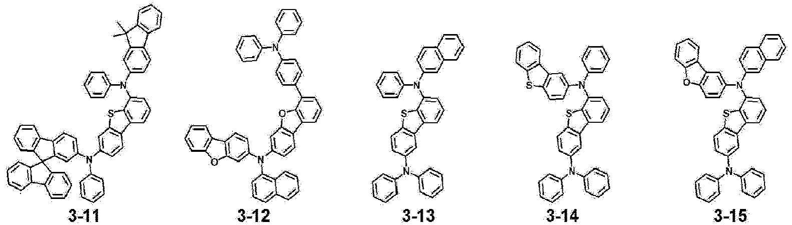

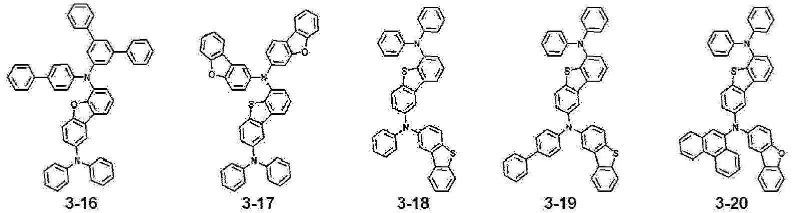

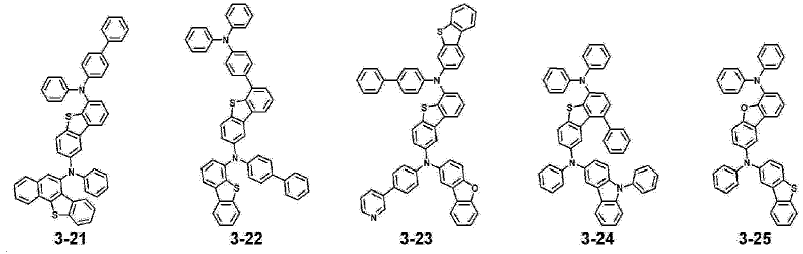

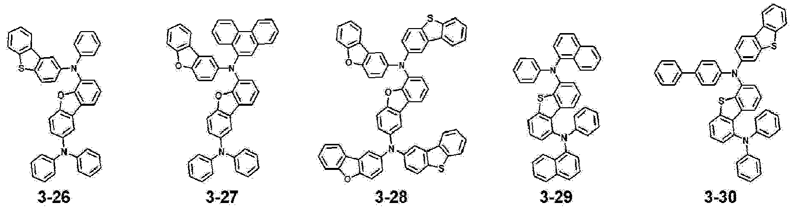

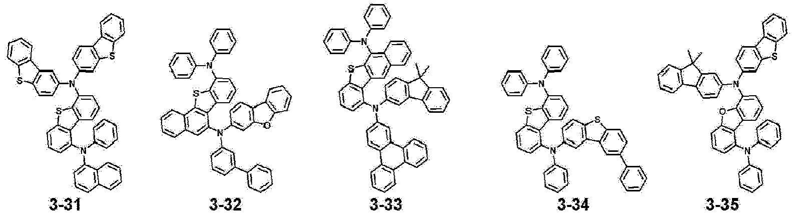

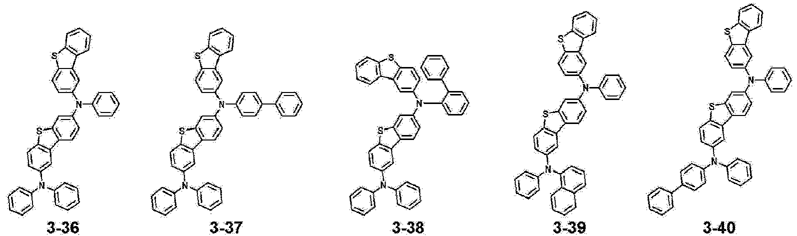

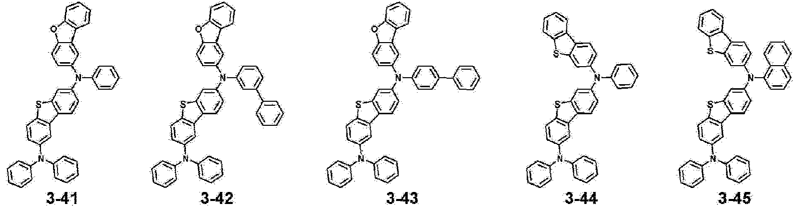

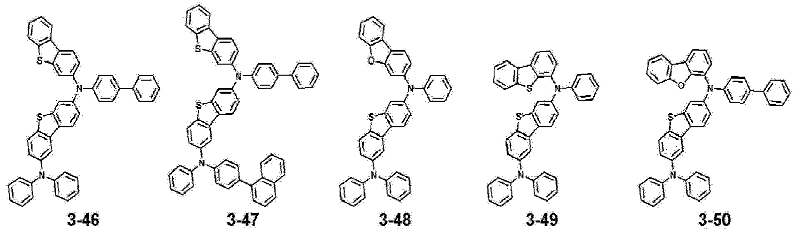

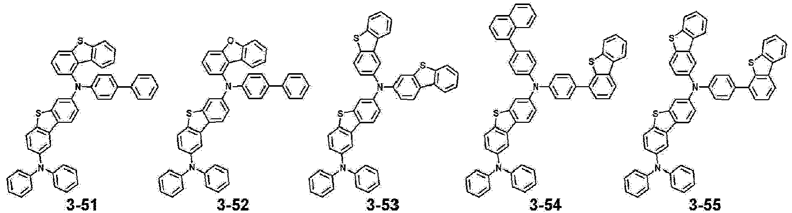

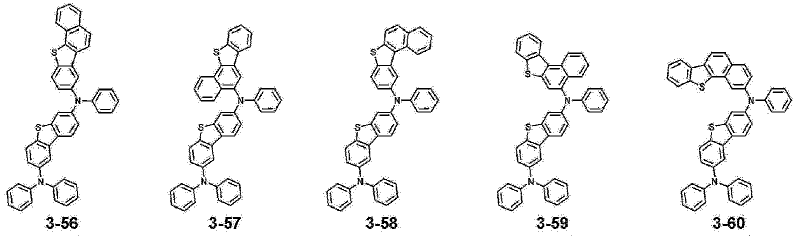

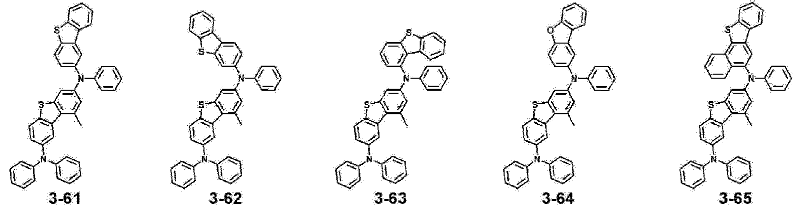

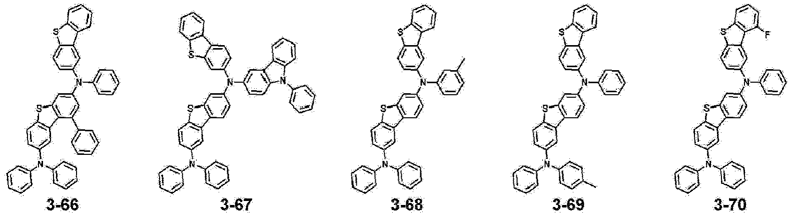

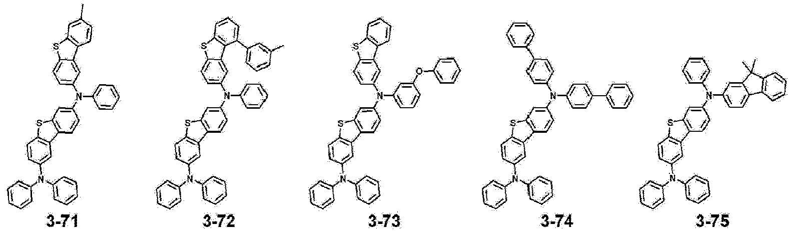

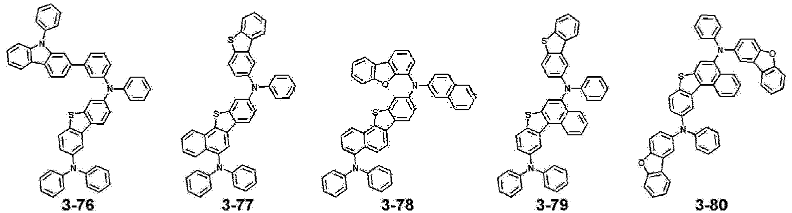

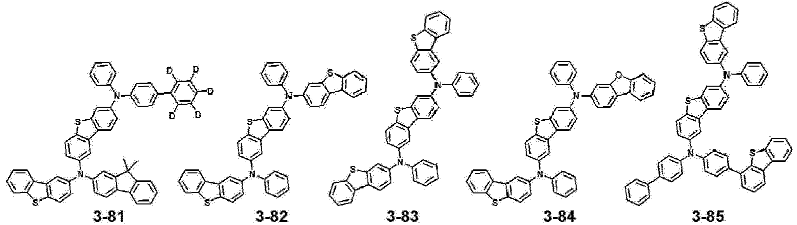

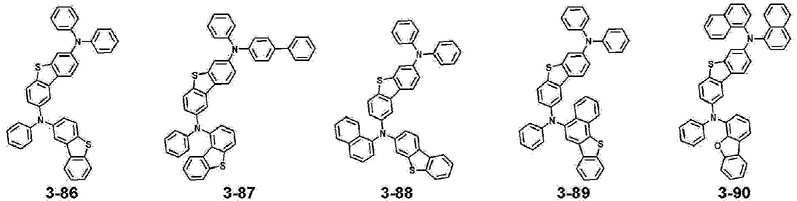

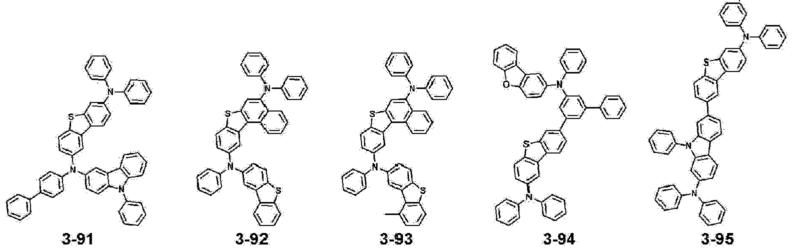

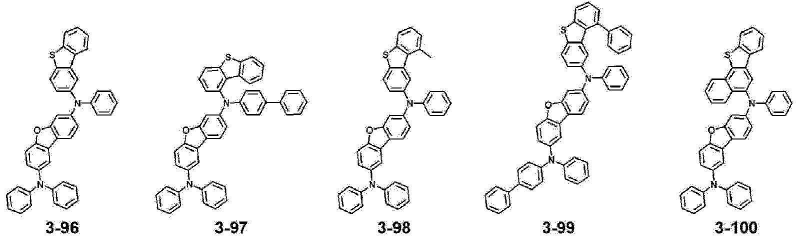

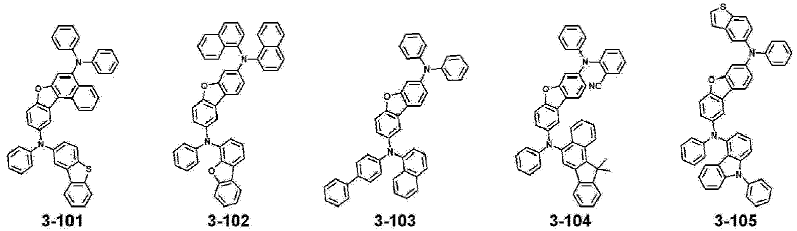

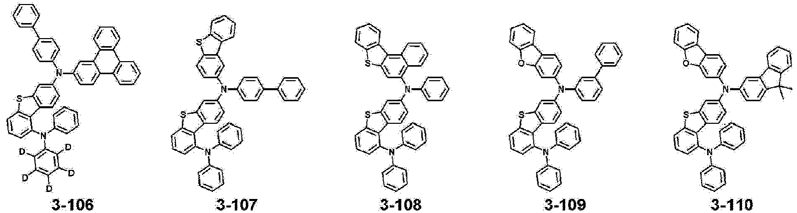

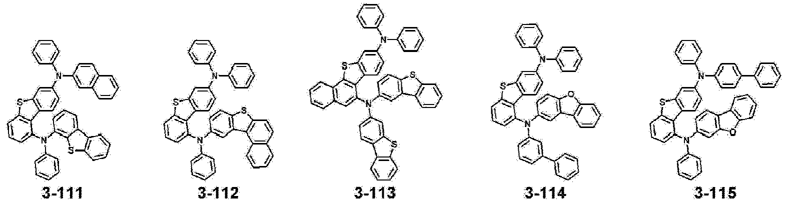

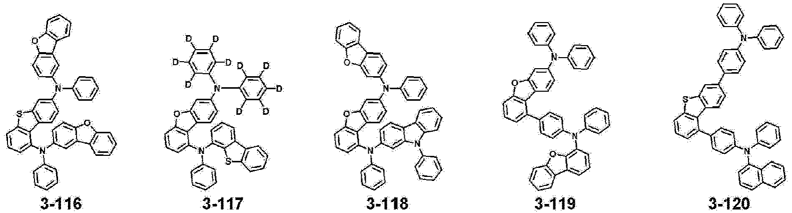

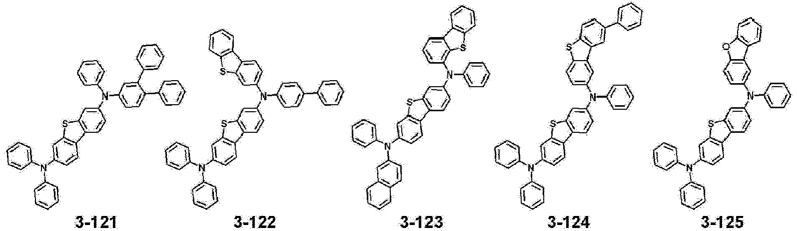

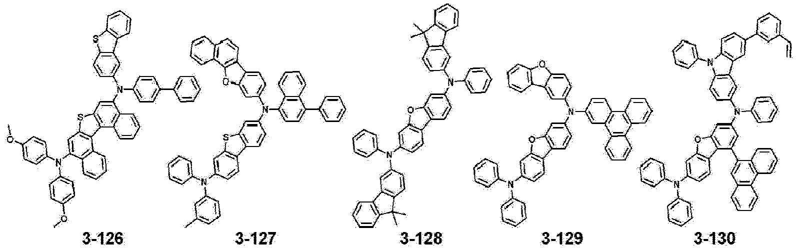

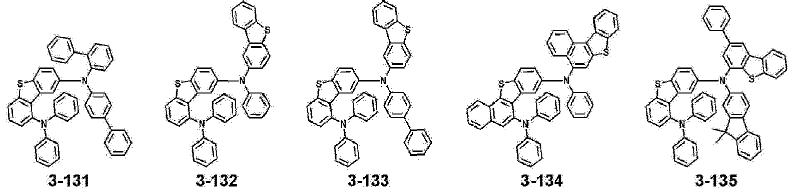

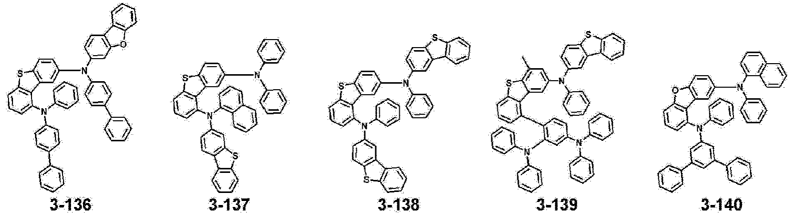

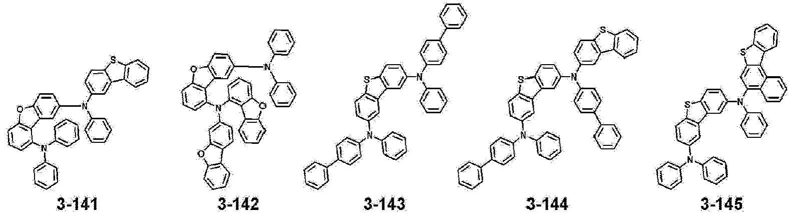

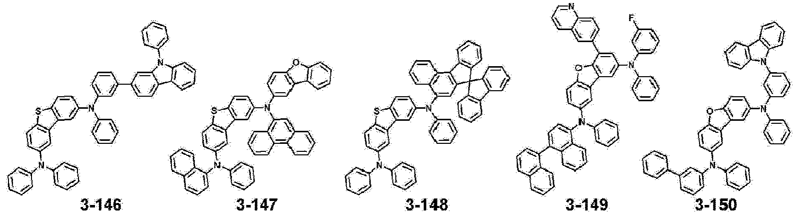

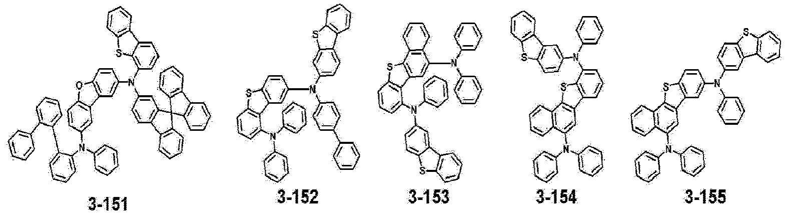

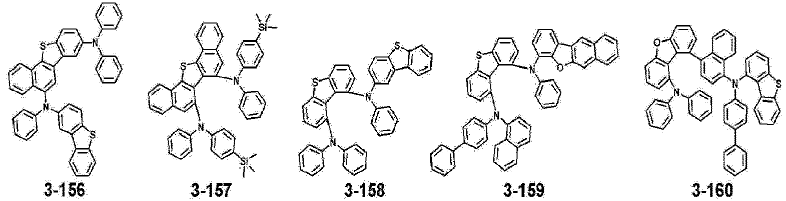

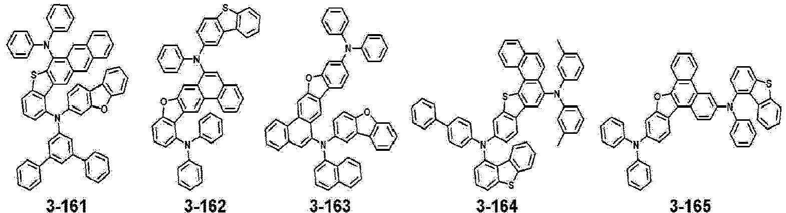

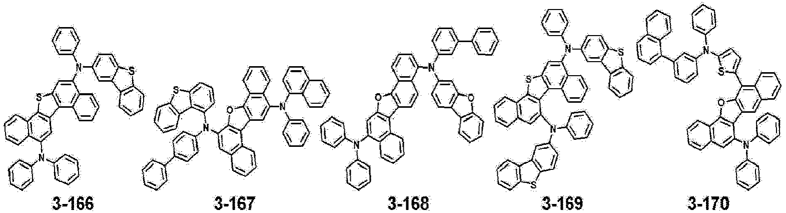

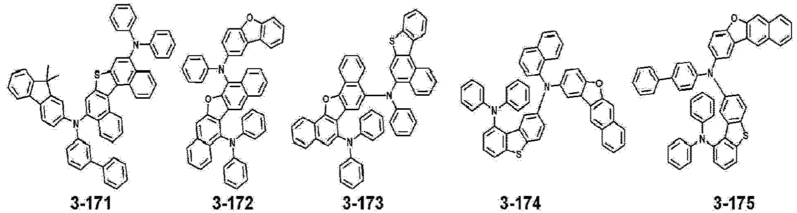

Preferably, in Formulas 1 and 2, L¹ to L⁶ may be each independently one of the following Formulas b-1 to b-13.

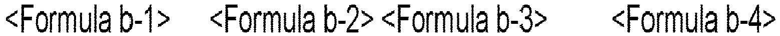

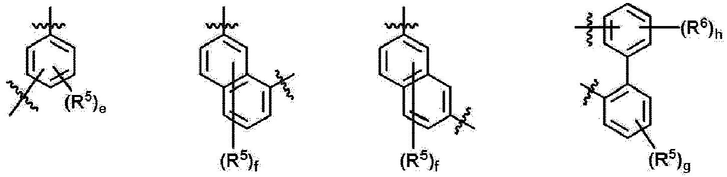

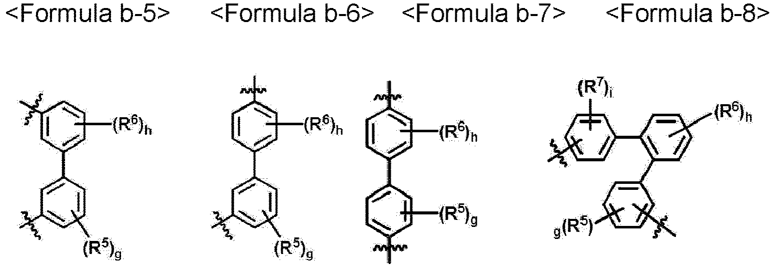

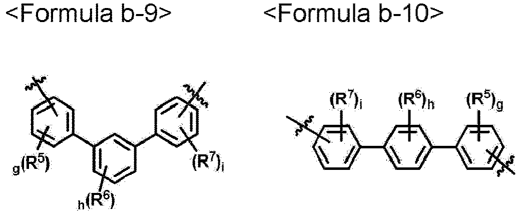

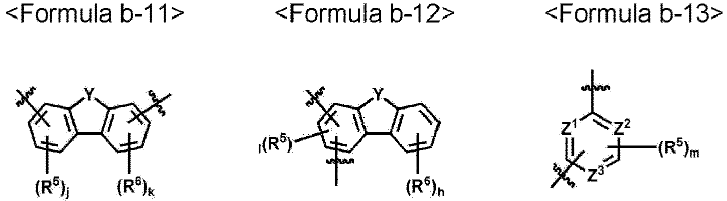

In Formulas b-1 to b-13, each of symbols may be defined as follows.

R⁵ to R⁷ may be each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₂₀ aryloxy group, and adjacent groups may optionally be linked to each other to form a ring.

The ring formed by bonding adjacent groups to each other may be a C₆-C₆₀ aromatic ring group, a fluorenyl group, a C₂-C₆₀ heterocyclic group containing at least one heteroatom selected from the group consisting of O, N, S, Si, and P, or a C₃-C₆₀ aliphatic ring or the like. Where adjacent groups are linked to each other to form an aromatic ring group, the ring may be preferably a C₆-C₃₀ aromatic ring group, more preferably a C₆-C₁₄ aromatic ring group, for example, benzene, naphthalene, phenanthrene or the like.

Y is N-(L^{a}-Ar^{a}), O, S or C(R')(R").

Z¹ to Z³ are each independently C, C(R') or N, and at least one of Z¹ to Z³ is N.

f is an integer of 0~6, e, g, h and i are each an integer of 0~4, j and k are each an integer of 0~3, l is an integer of 0~2, m is an integer of 0~3, and where they are an integer of 2 or more, respectively, each of a plurality of R⁵, each of a plurality of R⁶, and each of a plurality of R⁷ are the same as or different from each other.

R' and R" may be each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, a C₆-C₃₀ aryloxy group, and -L^{a}-N(R^{a})(R^{b}).

R' and R" of C(R')(R") may optionally be linked to each other to form a ring, and adjacent R's of C(R') may optionally be linked to each other to form a ring.

Ar^{a} may be selected from the group consisting of a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and a combination thereof.

L^{a} may be selected from the group consisting of a single bond, a C₆-C₂₀ arylene group, a fluorenylene group, a C₂-C₂₀ heterocyclic group comprising at least one heteroatom selected from the group consisting of O, N, S, Si and P, a C₃-C₂₀ aliphatic ring, and a combination thereof.

R^{a} and R^{b} may be each independently selected from the group consisting of a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and a combination thereof.

R⁵ to R⁷, L^{a}, Ar^{a}, R', R", R^{a}, R^{b}, and the ring formed by bonding adjacent groups to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a C₆-C₂₀ aryl group substituted with deuterium, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group.

In one embodiment of the present invention, it is preferable to use a mixture of the compound represented by Formula 1 and the compound represented by Formula 2 in a weight ratio of 2: 8 to 8: 2 as a host material.

In another embodiment of the present invention, the compound represented by Formula 3 of the present invention may be comprised in a light emitting layer and/or an emission-auxiliary layer, preferably used as a host of a light emitting layer or a material of an emission-auxiliary layer.

In another embodiment of the present invention, a mixture of the compound represented by Formula 3 and the compound represented by Formula 4 of the present invention may be used as a host material of a light emitting layer.

Hereinafter, synthesis examples of the compounds represented by Formulas 1 and 2, respectively, and preparation method of an organic electric element according to one embodiment of the present invention will be described in detail by way of examples. However, the present invention is not limited to the following examples.

### [SYNTHESIS EXAMPLE 11 Formula 1

As shown in Reaction Scheme 1 below, the compound(final product) represented by Formula 1 according to the present invention can be synthesized by reacting Sub 1 with Sub 2 , but there is no limitation thereto.

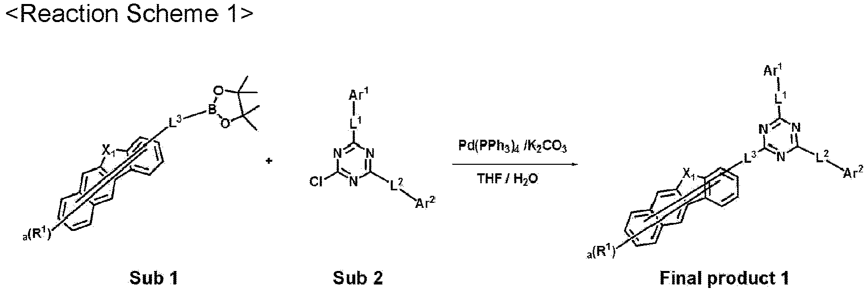

### 1. Exemplary Compounds of Sub 1 and Synthesis Example

The compound belonging to Sub 1 may be, but not limited to, the following compounds.

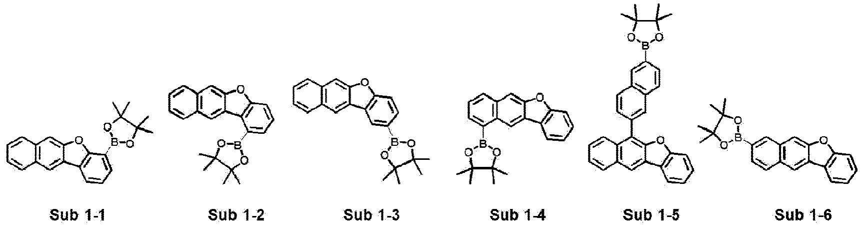

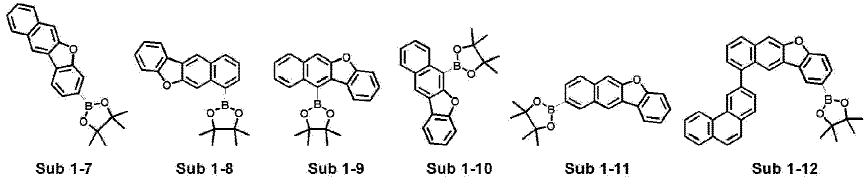

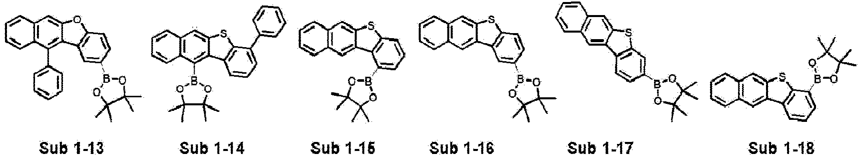

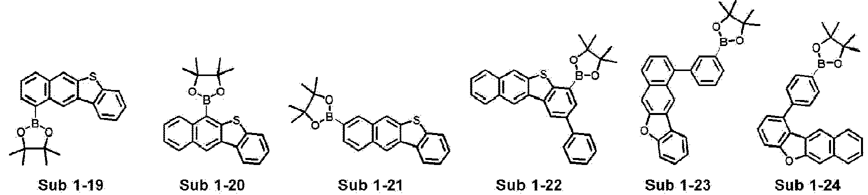

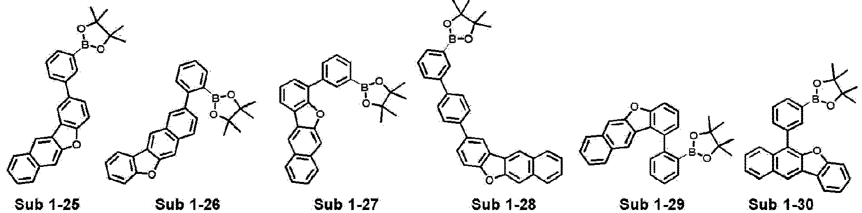

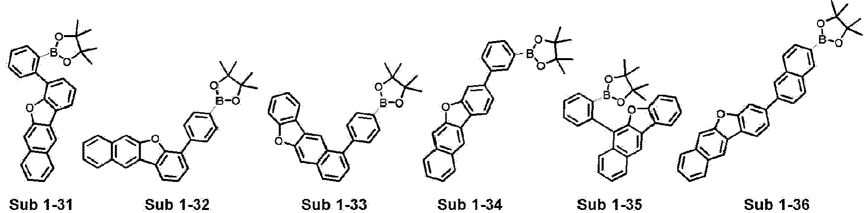

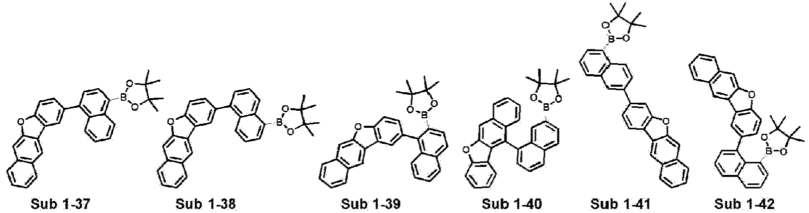

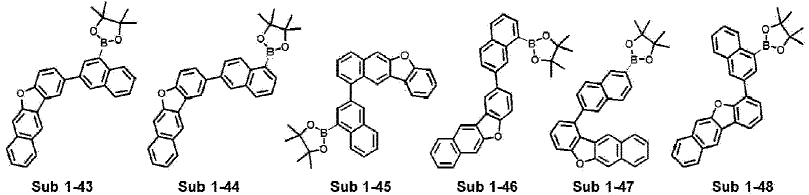

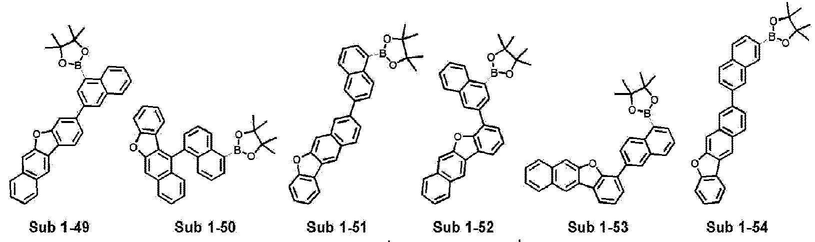

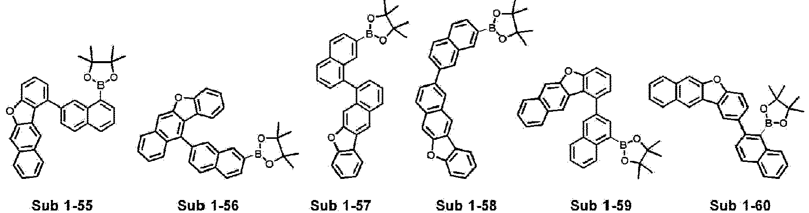

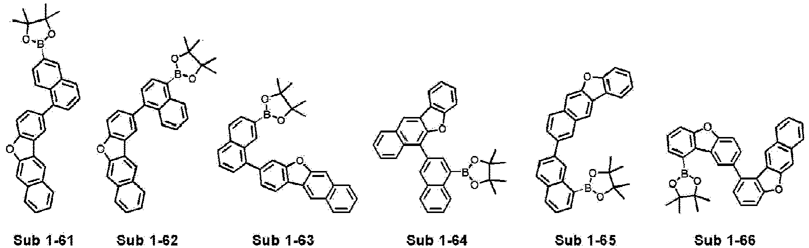

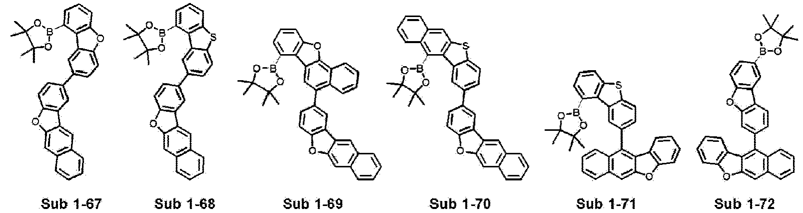

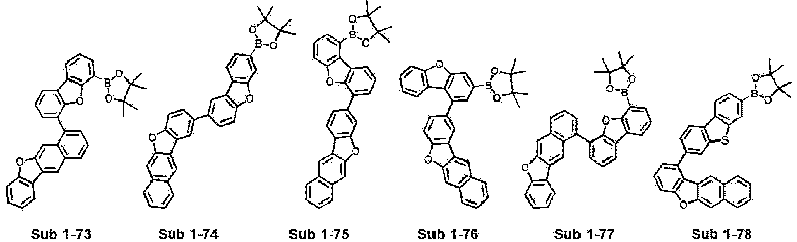

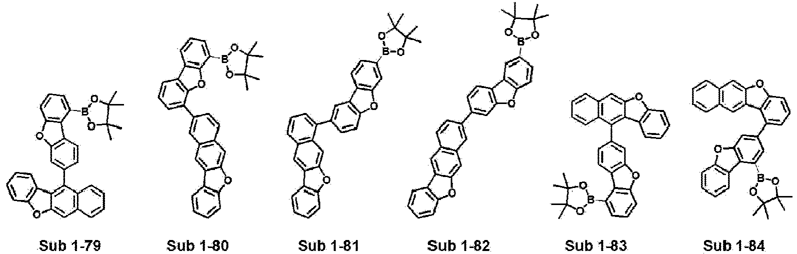

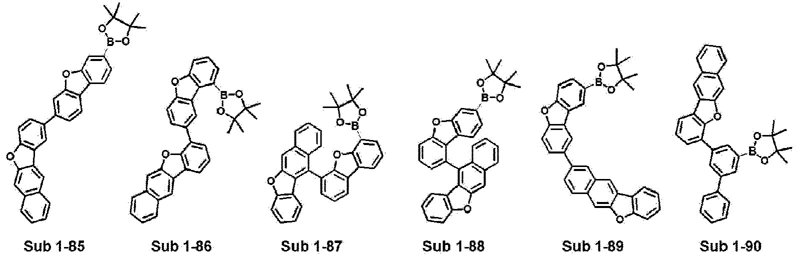

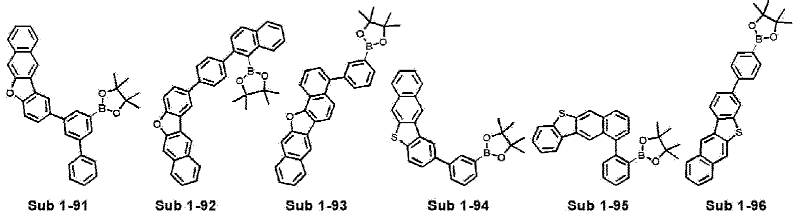

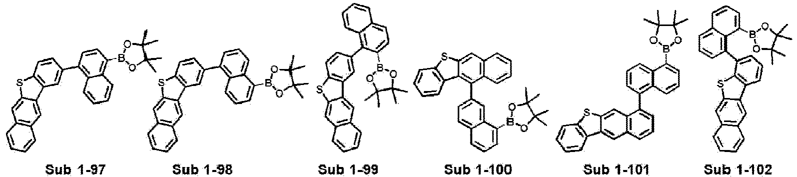

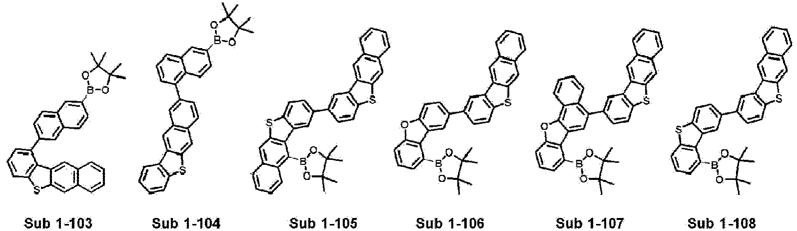

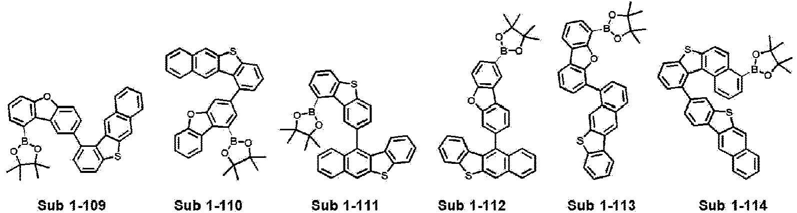

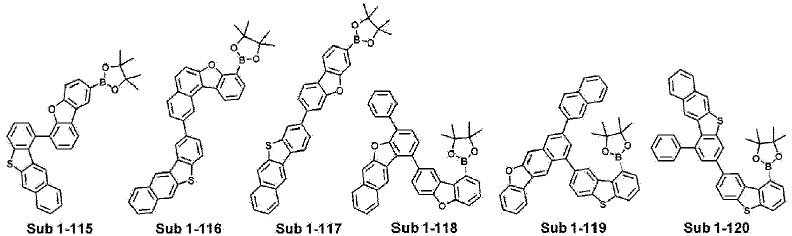

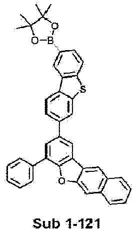

The FD-MS (Field Desorption-Mass Spectrometry) values of the above compounds belonging to Sub 1 are shown in the following Table 1.

**[Table 1]**

| **compound** | **FD-MS** | **compound** | **FD-MS** |
|---|---|---|---|
| **Sub 1-1** | **m/z=344.16(C₂₂H₂₁BO₃=344.22)** | **Sub 1-2** | **m/z=344.16(C₂₂H₂₁BO₃=344.22)** |
| **Sub 1-3** | **m/z=344.16(C₂₂H₂₁BO₃=344.22)** | **Sub 1-4** | **m/z=344.16(C₂₂H₂₁BO₃=344.22)** |
| **Sub 1-5** | **m/z=470.21(C₃₂H₂₇BO₃=470.38)** | **Sub 1-6** | **m/z=344.16(C₂₂H₂₁BO₃=344.22)** |
| **Sub 1-7** | **m/z=344.16(C₂₂H₂₁BO₃=344.22)** | **Sub 1-8** | **m/z=344.16(C₂₂H₂₁BO₃=344.22)** |
| **Sub 1-9** | **m/z=344.16(C₂₂H₂₁BO₃=344.22)** | **Sub 1-10** | **m/z=344.16(C₂₂H₂₁BO₃=344.22)** |
| **Sub 1-11** | **m/z=344.16(C₂₂H₂₁BO₃=344.22)** | **Sub 1-12** | **m/z=520.22(C₃₆H₂₉BO₃=520.44)** |
| **Sub 1-13** | **m/z=420.19(C₂₈H₂₅BO₃=420.32)** | **Sub 1-14** | **m/z=436.17(C₂₈H₂₅BO₂S=436.38)** |
| **Sub 1-15** | **m/z=360.14(C₂₂H₂₁BO₂S=360.28)** | **Sub 1-16** | **m/z=360.14(C₂₂H₂₁BO₂S=360.28)** |
| **Sub 1-17** | **m/z=360.14(C₂₂H₂₁BO₂S=360.28)** | **Sub 1-18** | **m/z=360.14(C₂₂H₂₁BO₂S=360.28)** |
| **Sub 1-28** | **m/z=496.22(C₃₄H₂₉BO₃=496.41)** | **Sub 1-30** | **m/z=420.19(C₂₈H₂₅BO₃=420.32)** |
| **Sub 1-36** | **m/z=470.21(C₃₂H₂₇BO₃=470.38)** | **Sub 1-66** | **m/z=510.2(C₃₄H₂₇BO₄=510.4)** |
| **Sub 1-68** | **m/z=526.18(C₃₄H₂₇BO₃S=526.46)** | **Sub 1-69** | **m/z=560.22(C₃₈H₂₉BO₄=560.46)** |
| **Sub 1-70** | **m/z=576.19(C₃₈H₂₉BO₃S=576.52)** | **Sub 1-90** | **m/z=496.22(C₃₄H₂₉BO₃=496.41)** |
| **Sub 1-92** | **m/z=546.24(C₃₈H₃₁BO₃=546.47)** | **Sub 1-93** | **m/z=470.21(C₃₂H₂₇BO₃=470.38)** |
| **Sub 1-94** | **m/z=436.17(C₂₈H₂₅BO₂S=436.38)** | **Sub 1-97** | **m/z=486.18(C₃₂H₂₇BO₂S=486.44)** |
| **Sub 1-105** | **m/z=592.17(C₃₈H₂₉BO₂S₂=592.58)** | **Sub 1-106** | **m/z=526.18(C₃₄H₂₇BO₃S=526.46)** |
| **Sub 1-107** | **m/z=576.19(C₃₈H₂₉BO₃S=576.52)** | **Sub 1-108** | **m/z=542.15(C₃₄H₂₇BO₂S₂=542.52)** |
| **Sub 1-118** | **m/z=586.23(C₄₀H₃₁BO₄=586.49)** | **Sub 1-119** | **m/z=652.22(C₄₄H₃₃BO₃S=652.62)** |
| **Sub 1-120** | **m/z=618.19(C₄₀H₃₁BO₂S₂=618.62)** | **Sub 1-121** | **m/z=602.21(C₄₉H₃₁BO₃S=602.56)** |

Sub 1 of the Reaction Scheme 1 can be synthesized according to the reaction route of the following Reaction Scheme 2, but there is no limitation thereto.

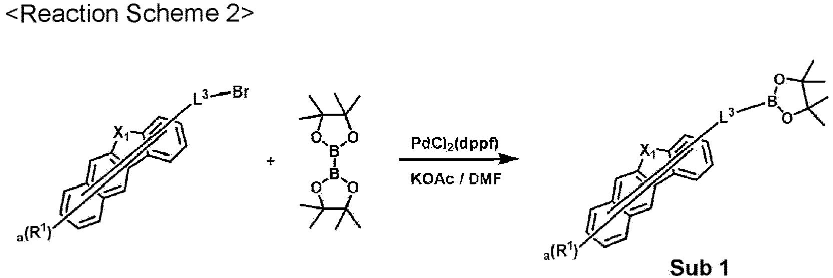

### Synthesis example of Sub 1-3

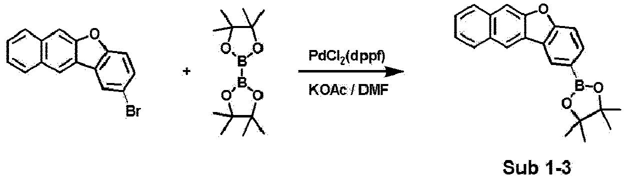

Bis(pinacolato)diboran (CAS Registry Number: 73183-34-3) (33.28g, 131.04mmol), PdCl₂(dppf) (2.92g, 3.57mmol), KOAc (35.07g, 357.40mmol) and DMF (596ml) were added to 2-bromonaphtho[2,3-b]benzofuran (35.4g, 119.13mmol) and the mixture was stirred under reflux. When the reaction was completed, the reaction product was extracted with CH₂Cl₂ and water, and then the organic layer was dried with MgSO₄ and concentrated. Then, the concentrate was applied to silica gel column and recrystallized to obtain 34.86g (yield: 85%) of the product.

### Synthesis example of Sub 1-21

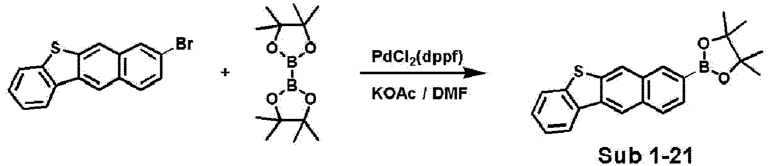

After bis(pinacolato)diboran (CAS Registry Number: 73183-34-3) (31.57g, 124.33mmol), PdCl₂(dppf) (2.77g, 3.39mmol), KOAc (33.28g, 339.07mmol) and DMF (565ml) were added to 8-bromobenzo[b]naphtho[2,3-d]thiophene (35.40g, 113.02mmol), the reaction was carried out in the same manner as in the synthesis method of 1-3 to obtain 33.39 g (yield: 82%) of product.

### Synthesis example of Sub 1-37

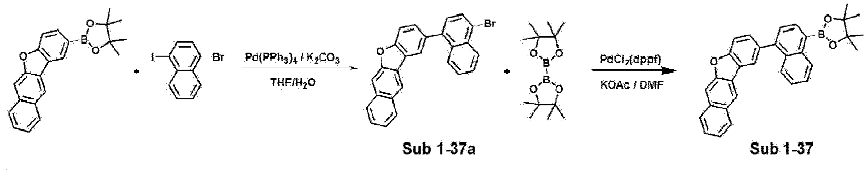

### (1) Synthesis of Sub 1-37a

1-bromo-4-iodonaphthalene (69.65g, 209.17mmol), Pd(PPh₃)₄ (8.06g, 6.97mmol), K₂CO₃ (72.27g, 522.92mmol), THF (639ml) and water (320ml) were added to 4,4,5,5-tetramethyl-2-(naphtho[2,3-b]benzofuran-2-yl)-1,3,2-dioxaborolane (60g, 174.31mmol) and the mixture was stirred under reflux. When the reaction was completed, the reaction product was extracted with ether and water. Then the organic layer was concentrated, dried with MgSO₄ and concentrated. Finally, the concentrate was applied to silica gel column and recrystallized to obtain 45.01g (yield: 61% of the product.

### (2) Synthesis of Sub 1-37

After bis(pinacolato)diboran (CAS Registry Number: 73183-34-3) (29.70g, 116.96mmol), PdCl₂(dppf) (2.60g, 3.19mmol), KOAc (31.31g, 318.99mmol) and DMF (532ml) were added to Sub 1-37a (45.01g, 106.33mmol), the reaction was carried out in the same manner as in the synthesis method of 1-3 to obtain 39.01g (yield: 78%) of product.

### Synthesis example of Sub 1-68

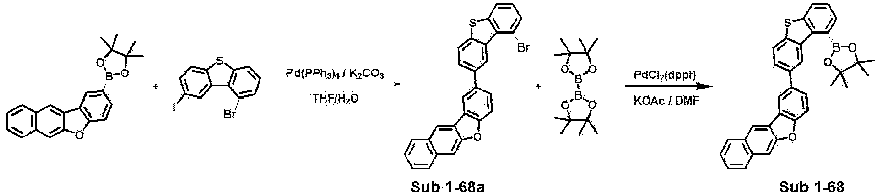

### (1) Synthesis of Sub 1-68a

After 1-bromo-8-iododibenzo[b,d]thiophene (81.38g, 209.17mmol), Pd(PPh₃)₄ (8.06g, 6.97mmol), K₂CO₃ (72.27g, 522.92mmol), THF (639ml) and water(320ml) were added to 4,4,5,5-tetramethyl-2-(naphtho[2,3-b]benzofuran-2-yl)-1,3,2-dioxaborolane (60g, 174.31mmol), the reaction was carried out in the same manner as in the synthesis method of Sub 1-37a to obtain 56.82g (yield: 68%) of product.

### (2) Synthesis of Sub 1-68

After bis(pinacolato)diboran (CAS Registry Number: 73183-34-3) (33.11g, 130.38mmol), PdCl₂(dppf) (2.90g, 3.56mmol), KOAc (34.90g, 355.58mmol) and DMF (593ml) were added to Sub 1-68a (56.82g, 118.53mmol), the reaction was carried out in the same manner as in the synthesis method of 1-3 to obtain 46.80g (yield: 75%) of product.

### Synthesis example of Sub 1-98

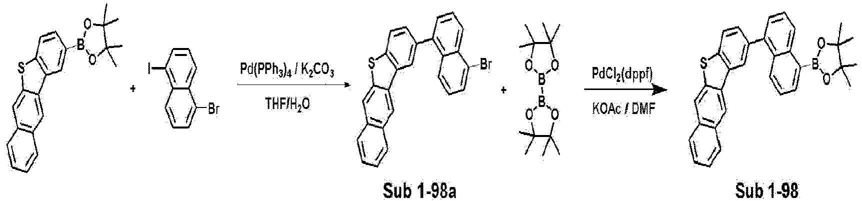

### (1) Synthesis of Sub 1-98a

After 1-bromo-5-iodonaphthalene (66.54g, 199.84mmol), Pd(PPh₃)₄ (7.70g, 6.66mmol), K₂CO₃ (69.05g, 499.61mmol), THF (611ml) and water (305ml) were added to 2-(benzo[b]naphtho[2,3-d]thiophen-2-yl)-4,4,5,5-tetramethyl-1,3,2-dioxaborolane (60g, 166.54mmol), the reaction was carried out in the same manner as in the synthesis method of Sub 1-37a to obtain 46.83g (yield: 64%) of product.

### (2) Synthesis of Sub 1-98

After bis(pinacolato)diboran (CAS Registry Number: 73183-34-3) (29.77g, 117.24mmol), PdCl₂(dppf) (2.61g, 3.20mmol), KOAc (31.38g, 319.75mmol) and DMF (533ml) were added to 1-98a (46.83g, 106.58mmol), the reaction was carried out in the same manner as in the synthesis method of 1-3 to obtain 37.33g (yield: 72%) of product.

### Synthesis example of Sub 1-111

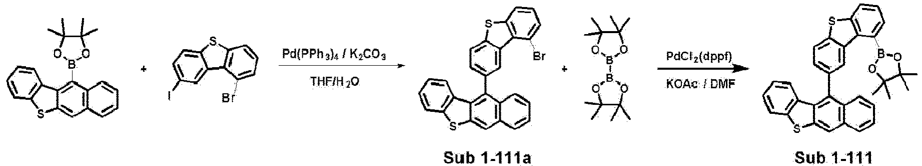

### (1) Synthesis of Sub 1-111a

After 1-bromo-8-iododibenzo[b,d]thiophene (77.75g, 199.84mmol), Pd(PPh₃)₄ (7.70g, 6.66mmol), K₂CO₃ (69.05g, 499.61mmol), THF (611ml) and water (305ml) were added to 2-(benzo[b]naphtho[2,3-d]thiophen-11-yl)-4,4,5,5-tetramethyl-1,3,2-dioxaborolane (60g, 166.54mmol), the reaction was carried out in the same manner as in the synthesis method of Sub 1-37a to obtain 54.46g (yield: 66%) of product.

### (2) Synthesis of Sub 1-111

After bis(pinacolato)diboran (CAS Registry Number: 73183-34-3) (30.70g, 120.91mmol), PdCl₂(dppf) (2.69g, 3.30mmol), KOAc (32.36g, 329.76mmol) and DMF (550ml) were added to Sub 1-111a (54.46g, 109.92mmol), the reaction was carried out in the same manner as in the synthesis method of 1-3 to obtain 41.15g (yield: 69%) of product.

### 2. Exemplary Compounds of Sub 2 and Synthesis Example

The compounds belonging to Sub 2 of Reaction Scheme 1 may be, but not limited to, the following compounds.

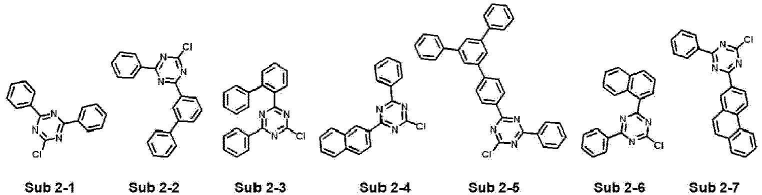

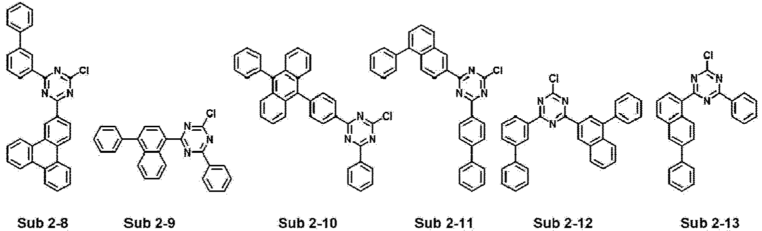

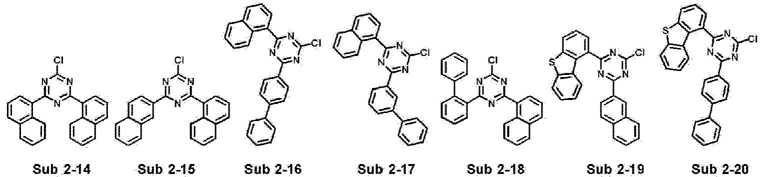

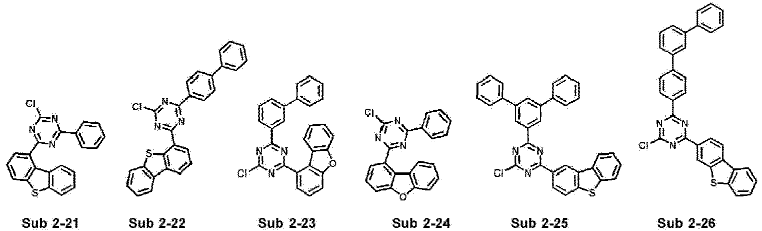

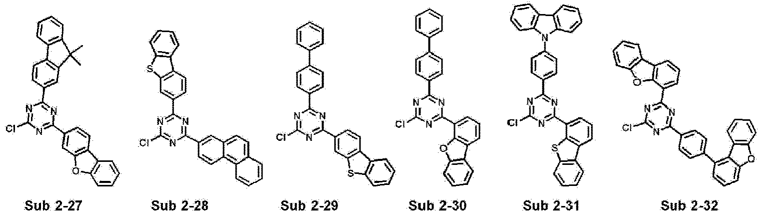

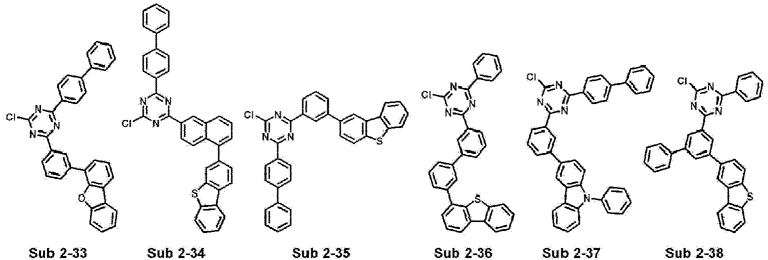

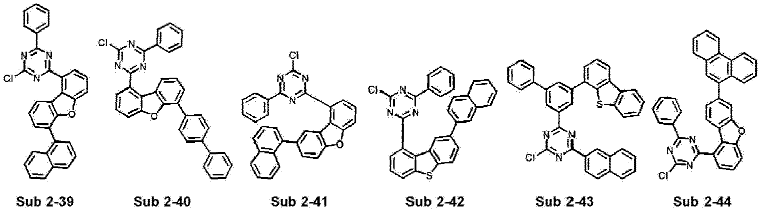

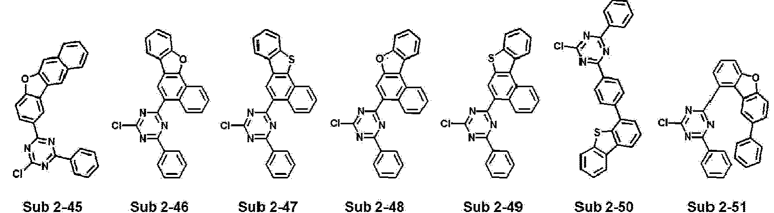

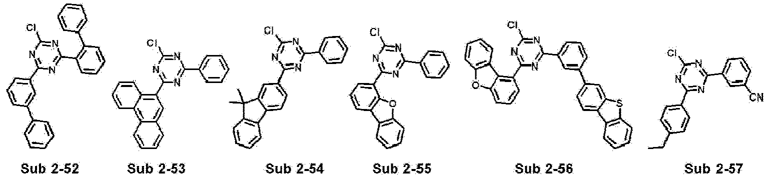

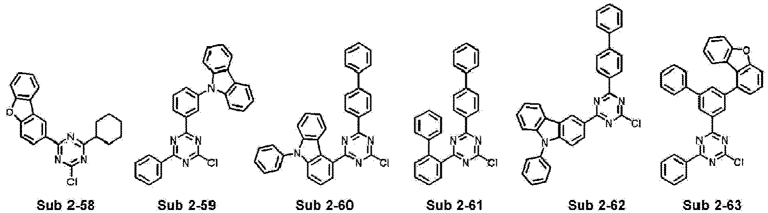

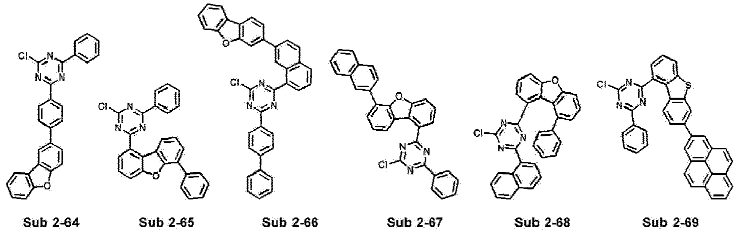

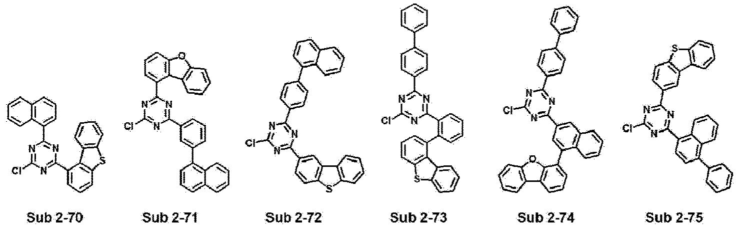

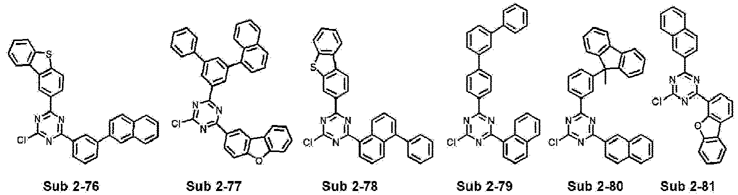

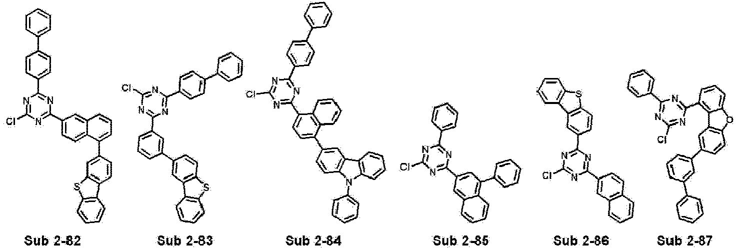

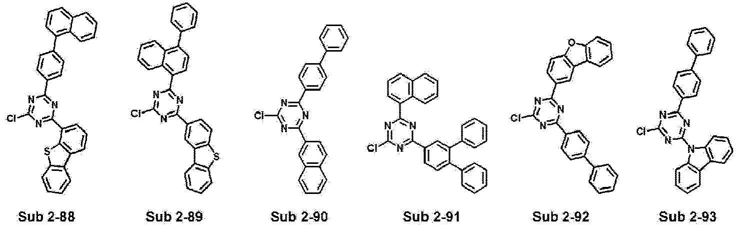

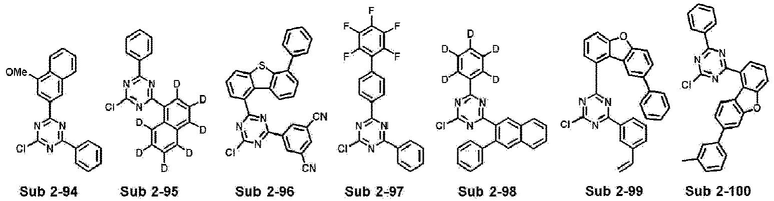

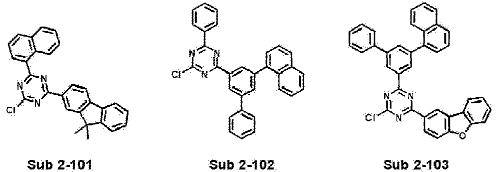

The FD-MS values of the above compounds belonging to Sub 2 are shown in the following Table 2.

**[Table 2]**

| **compound** | **FD-MS** | **compound** | **FD-MS** |
|---|---|---|---|
| **Sub 2-1** | **m/z=267.06(C₁₅H₁₀ClN₃=267.72)** | **Sub 2-2** | **m/z=343.09(C₂₁H₁₄ClN₃=343.81)** |
| **Sub 2-4** | **m/z=317.07(C₁₉H₁₂ClN₃=317.78)** | **Sub 2-5** | **m/z=495.15(C₃₃H₂₂ClN₃=496.01)** |
| **Sub 2-7** | **m/z=367.09(C₂₃H₁₄ClN₃=367.84)** | **Sub 2-8** | **m/z=493.13(C₃₃H₂₀ClN₃=493.99)** |
| **Sub 2-9** | **m/z=393.1 (C₂₅H₁₆ClN₃=393.87)** | **Sub 2-10** | **m/z=519.15(C₃₅H₂₂ClN₃=520.03)** |
| **Sub 2-11** | **m/z=469.13(C₃₁H₂₀ClN₃=469.97)** | **Sub 2-12** | **m/z=393.1(C₂₅H₁₆ClN₃=393.87)** |
| **Sub 2-14** | **m/z=367.09(C₂₃H₁₄ClN₃=367.84)** | **Sub 2-19** | **m/z=423.06(C₂₅H₁₄ClN₃S=423.92)** |
| **Sub 2-20** | **m/z=449.08(C₂₇H₁₆ClN₃S=449.96)** | **Sub 2-21** | **m/z=373.04(C₂₁H₁₂ClN₃S=373.86)** |
| **Sub 2-22** | **m/z=433.1(C₂₇H₁₆ClN₃O=433.9)** | **Sub 2-24** | **m/z=357.07(C₂₁H₁₂ClN₃O=357.8)** |
| **Sub 2-25** | **m/z=525.11(C₃₃H₂₀ClN₃S=526.05)** | **Sub 2-27** | **m/z=473.13(C₃₀H₂₀ClN₃O=473.96)** |
| **Sub 2-28** | **m/z=473.08(C₂₉H₁₆ClN₃S=473.98)** | **Sub 2-31** | **m/z=538.1(C₃₃H₁₉ClN₄S=539.05)** |
| **Sub 2-32** | **m/z=523.11(C₃₃H₁₈ClN₃O₂=523.98)** | **Sub 2-33** | **m/z=509.13(C₃₃H₂₀ClN₃O=509.99)** |
| **Sub 2-34** | **m/z=575.12(C₃₇H₂₂ClN₃S=576.11)** | **Sub 2-37** | **m/z=584.18(C₃₉H₂₅ClN₄=585.11)** |
| **Sub 2-39** | **m/z=483.11(C₃₁H₁₈ClN₃O=483.96)** | **Sub 2-40** | **m/z=509.13(C₃₃H₂₀ClN₃O=509.99)** |
| **Sub 2-42** | **m/z=499.09(C₃₁H₁₈ClN₃S=500.02)** | **Sub 2-43** | **m/z=575.12(C₃₇H₂₂ClN₃S=576.11)** |
| **Sub 2-44** | **m/z=533.13(C₃₅H₂₀ClN₃O=534.02)** | **Sub 2-45** | **m/z=407.08(C₂₅H₁₄ClN₃O=407.86)** |
| **Sub 2-47** | **m/z=423.06(C₂₅H₁₄ClN₃S=423.92)** | **Sub 2-50** | **m/z=449.08(C₂₇H₁₆ClN₃S=449.96)** |
| **Sub 2-51** | **m/z=433.1(C₂₇H₁₆ClN₃O=433.9)** | **Sub 2-52** | **m/z=419.12(C₂₇H₁₈ClN₃=419.91)** |
| **Sub 2-54** | **m/z=383.12(C₂₄H₁₈ClN₃=383.88)** | **Sub 2-55** | **m/z=357.07(C₂₁H₁₂ClN₃O=357.8)** |
| **Sub 2-56** | **m/z=539.09(C₃₃H₁₈ClN₃OS=540.04)** | **Sub 2-57** | **m/z=320.08(C₁₈H₁₃ClN₄=320.78)** |
| **Sub 2-58** | **m/z=363.11(C₂₁H₁₈ClN₃O=363.85)** | **Sub 2-59** | **m/z=432.11(C₂₇H₁₇ClN₄=432.91)** |
| **Sub 2-60** | **m/z=508.15(C₃₃H₂₁ClN₄=509.01)** | **Sub 2-64** | **m/z=433.1(C₂₇H₁₆ClN₃O=433.9)** |
| **Sub 2-66** | **m/z=559.15(C₃₇H₂₂ClN₃O=560.05)** | **Sub 2-67** | **m/z=483.11(C₃₁H₁₈ClN₃O=483.96)** |
| **Sub 2-69** | **m/z=573.11(C₃₇H₂₀ClN₃S=574.1)** | **Sub 2-80** | **m/z=495.15(C₃₃H₂₂ClN₃=496.01)** |
| **Sub 2-84** | **m/z=634.19(C₄₃H₂₇ClN₄=635.17)** | **Sub 2-94** | **m/z=347.08(C₂₀H₁₄ClN₃O=347.8)** |
| **Sub 2-95** | **m/z=324.12(C₁₉H₅D₇ClN₃=324.82)** | **Sub 2-96** | **m/z=499.07(C₂₉H₁₄ClN₅S=499.98)** |
| **Sub 2-97** | **m/z=433.04(C₂₁H₉ClF₅N₃=433.77)** | **Sub 2-98** | **m/z=398.13(C₂₅H₁₁D₅ClN₃=398.9)** |
| **Sub 2-99** | **m/z=459.11(C₂₉H₁₈ClN₃O=459.93)** | **Sub 2-100** | **m/z=447.11(C₂₈H₁₈ClN₃O=447.92)** |
| **Sub 2-101** | **m/z=433.13(C₂₈H₂₀ClN₃=433.94)** | **Sub 2-102** | **m/z=469.13(C₃₁H₂₀ClN₃=469.97)** |
| **Sub 2-103** | **m/z=559.15(C₃₇H₂₂ClN₃O=560.05)** | | |

Sub 2 of the Reaction Scheme 1 can be synthesized according to the reaction route of the following Reaction Scheme 3, but there is no limitation thereto.

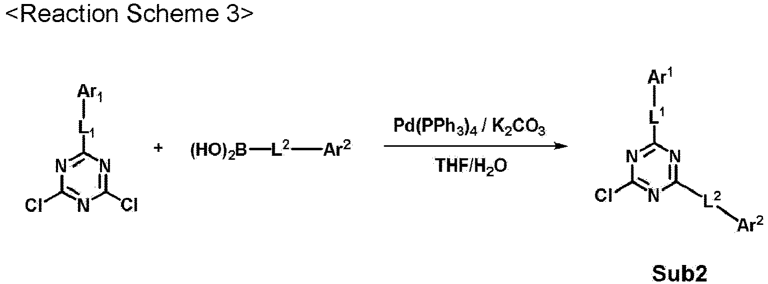

### Synthesis example of Sub 2-2

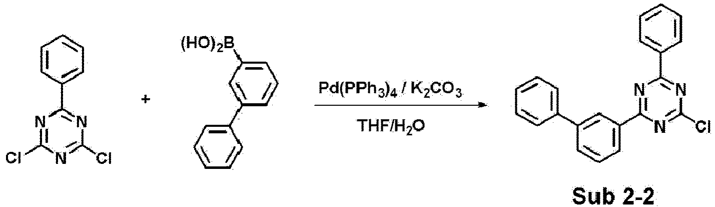

[1,1'-biphenyl]-3-ylboronic acid (31.01g, 156.60mmol), Pd(PPh₃)₄ (7.24g, 6.26mmol), K₂CO₃ (64.93g, 469.79mmol), THF (522ml) and water (261ml) were added to 2,4-dichloro-6-phenyl-1,3,5-triazine (35.4g, 156.60mmol) and the mixture was stirred under reflux. When the reaction was completed, the reaction product was extracted with ether and water. Then the organic layer was concentrated, dried with MgSO₄ and concentrated. Finally, the concentrate was applied to silica gel column and recrystallized to obtain 43.07 g (yield: 80%) of the product.

### Synthesis example of Sub 2-6

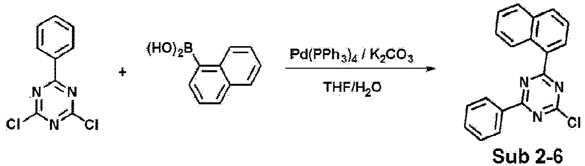

After naphthalen-1-ylboronic acid (26.93g, 156.60mmol), Pd(PPh₃)₄ (7.24g, 6.26mmol), K₂CO₃ (64.93g, 469.79mmol), THF (522ml) and water (261ml) were added to 2,4-dichloro-6-phenyl-1,3,5-triazine (35.4g, 156.60mmol), the reaction was carried out in the same manner as in the synthesis method of Sub 2-2 to obtain 36.82 g (yield: 74%) of product.

### Synthesis example of Sub 2-22

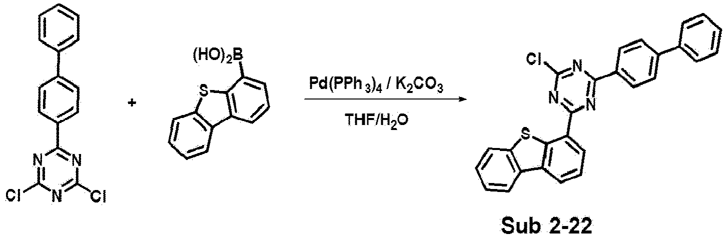

After dibenzo[b,d]thiophen-4-ylboronic acid (26.72g, 117.16mmol), Pd(PPh₃)₄ (5.42g, 4.69mmol), K₂CO₃ (48.58g, 351.47mmol), THF (391ml) and water (195ml) were added to 2-([1,1'-biphenyl]-4-yl)-4,6-dichloro-1,3,5-triazine (35.4g, 117.16mmol), the reaction was carried out in the same manner as in the synthesis method of Sub 2-2 to obtain 40.06 g (yield: 76%) of product.

### Synthesis example of Sub 2-24

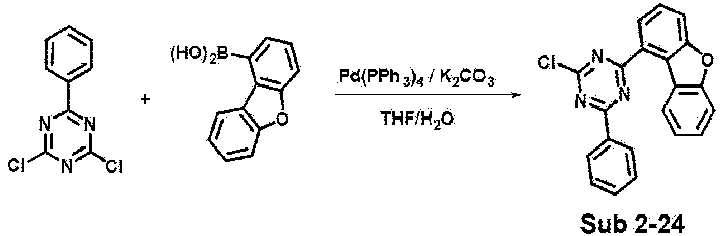

After dibenzo[b,d]furan-1-ylboronic acid (33.20 g, 156.60 mmol), Pd(PPh₃)₄ (7.24 g, 6.26 mmol), K₂CO₃ (64.93 g, 469.79 mmol), THF (522ml) and water (261ml) were added to 2,4-dichloro-6-phenyl-1,3,5-triazine (35.4g, 156.60mmol), the reaction was carried out in the same manner as in the synthesis method of Sub 2-2 to obtain 38.66 g (yield: 69%) of product.

### Synthesis example of Sub 2-47

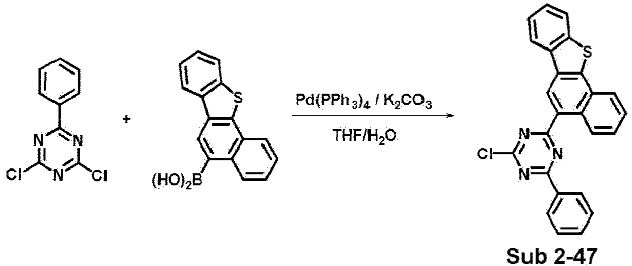

After benzo[b]naphtho[2,1-d]thiophen-5-ylboronic acid (43.55 g, 156.60 mmol), Pd(PPh₃)₄(7.24g, 6.26 mmol), K₂CO₃ (64.93 g, 469.79 mmol), THF (522ml) and water (261ml) were added to 2,4-dichloro-6-phenyl-1,3,5-triazine (35.4g, 156.60mmol), the reaction was carried out in the same manner as in the synthesis method of Sub 2-2 to obtain 19.58 g (yield: 45%) of product.

### 3. Synthesis example of final product

### Synthesis example of 1-6

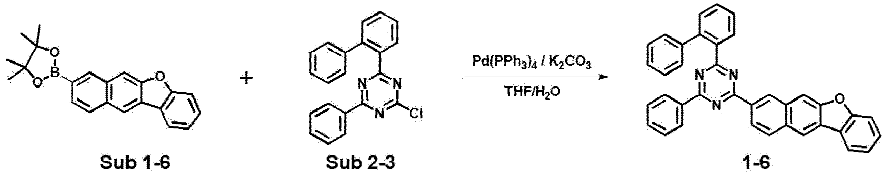

After Sub 1-6 (56.7g, 164.72mmol) was dissolved in THF(604mL) in a round bottom flask, Sub 2-3 (67.96 g, 197.66 mmol), Pd(PPh₃)₄ (7.61 g, 6.59 mmol), K₂CO₃ (68.30 g, 494.16 mmol) and water (302 mL) were added thereto and the mixture was stirred under reflux. When the reaction was completed, the reaction product was extracted with ether and water. Then the organic layer was dried with MgSO₄ and concentrated. Finally, the concentrate was applied to silica gel column and recrystallized to obtain 64.93 (yield: 75%) of the product.

### Synthesis example of 1-46

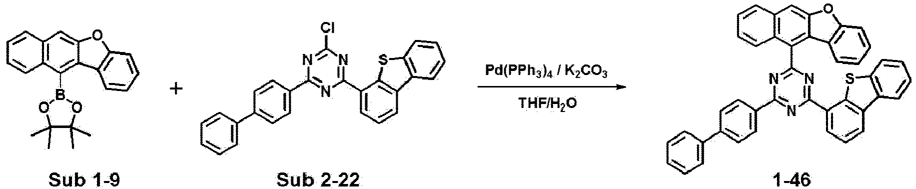

After THF(604 mL), Sub 2-22 (88.94 g, 197.66 mmol), Pd(PPh₃)₄ (7.61 g, 6.59 mmol), K₂CO₃ (68.30 g, 494.16 mmol) and water (302 mL) were added to Sub 1-9 (56.7 g, 164.72 mmol), the reaction was carried out in the same manner as in the synthesis method of 1-6 to obtain 81.17 g (yield: 73%) of product.

### Synthesis example of 1-81

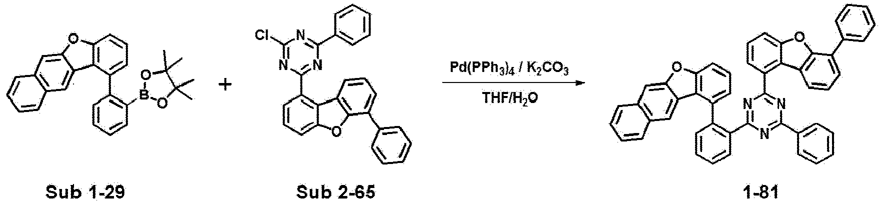

After THF(495 mL), Sub 2-65 (70.24 g, 161.88 mmol), Pd(PPh₃)₄ (6.24 g, 5.40 mmol), K₂CO₃ (55.93 g, 404.69 mmol) and water (247 mL) were added to Sub 1-29 (56.7 g, 134.90 mmol), the reaction was carried out in the same manner as in the synthesis method of 1-6 to obtain 59.73 g (yield: 64%) of product.

### Synthesis example of 1-92

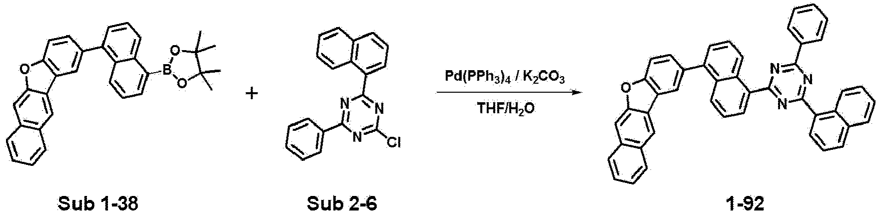

After THF(442 mL), Sub 2-6 (45.97 g, 144.65 mmol), Pd(PPh₃)₄ (5.57 g, 4.82 mmol), K₂CO₃ (49.98 g, 361.62 mmol) and water (221 mL) were added to Sub 1-38 (56.7 g, 120.54 mmol), the reaction was carried out in the same manner as in the synthesis method of 1-6 to obtain 65.62 g (yield: 87%) of product.

### Synthesis example of 1-122

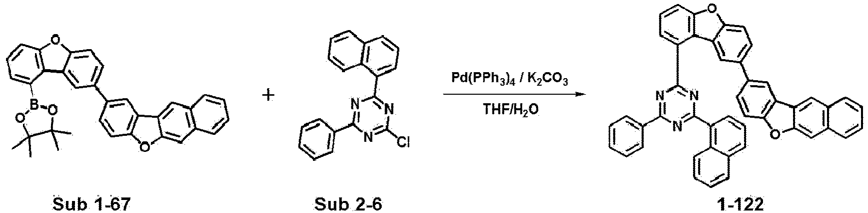

After THF(407 mL), Sub 2-6 (42.36 g, 133.31 mmol), Pd(PPh₃)₄ (5.13 g, 4.44 mmol), K₂CO₃ (46.06 g, 333.27 mmol) and water (204 mL) were added to Sub 1-67 (56.7 g, 111.09 mmol), the reaction was carried out in the same manner as in the synthesis method of 1-6 to obtain 59.17 g (yield: 80%) of product.

### Synthesis example of 1-148

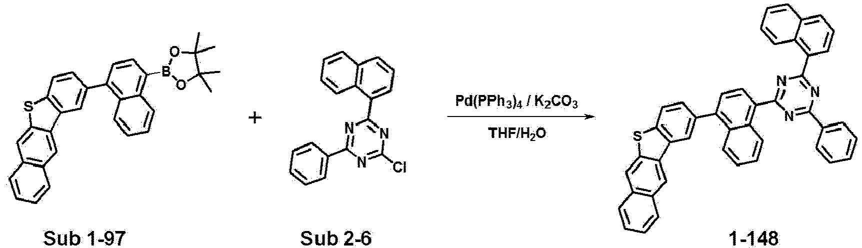

After THF(427 mL), Sub 2-6 (44.45 g, 139.87 mmol), Pd(PPh₃)₄ (5.39 g, 4.66 mmol), K₂CO₃ (48.33 g, 349.68 mmol) and water (214 mL) were added to Sub 1-97 (56.7 g, 116.56 mmol), the reaction was carried out in the same manner as in the synthesis method of 1-6 to obtain 62.84 g (yield: 84%) of product.

### Synthesis example of 1-157

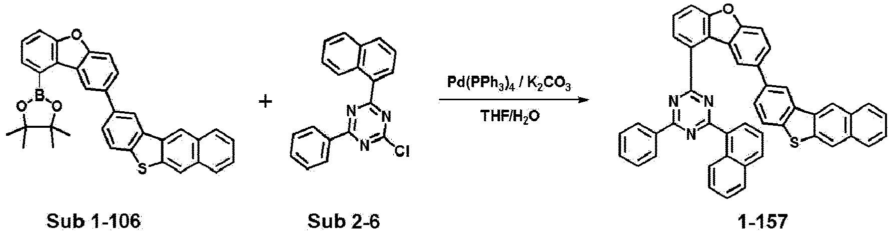

After THF(395 mL), Sub 2-6 (41.07 g, 129.24 mmol), Pd(PPh₃)₄ (4.98 g, 4.31 mmol), K₂CO₃ (44.66 g, 323.10 mmol) and water (197 mL) were added to Sub 1-106 (56.7 g, 107.70 mmol), the reaction was carried out in the same manner as in the synthesis method of 1-6 to obtain 59.48 (yield: 81%) of product.

### Synthesis example of 2-109

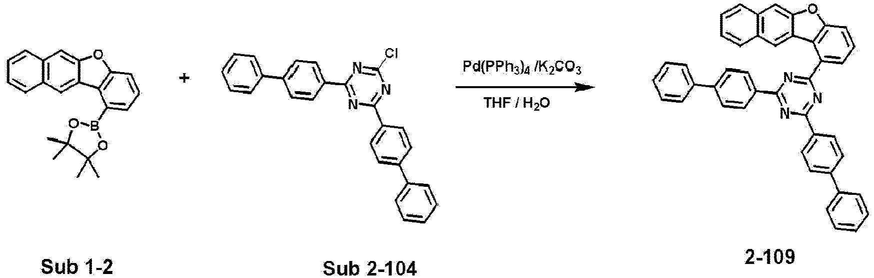

After THF(395 mL), Sub 2-104 (54.3g, 129.24 mmol), Pd(PPh₃)₄ (4.98 g, 4.31 mmol), K₂CO₃ (44.66 g, 323.10 mmol) and water (197 mL) were added to Sub 1-2 (37.1g, 107.7 mmol), the reaction was carried out in the same manner as in the synthesis method of 1-6 to obtain 55.5g (yield: 81%) of product.

### Synthesis example of 2-111

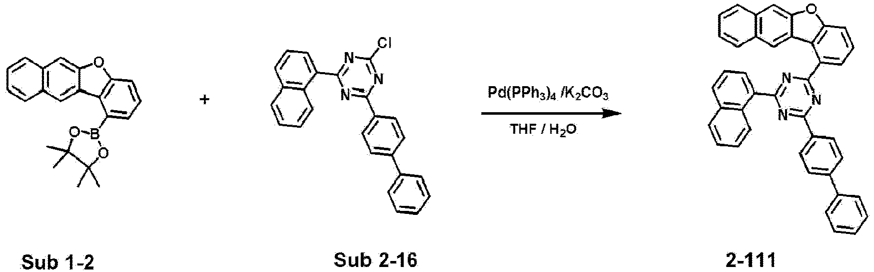

After THF(395 mL), Sub 2-16 (50.9g, 129.24 mmol), Pd(PPh₃)₄ (4.98 g, 4.31 mmol), K₂CO₃ (44.66 g, 323.10 mmol) and water (197 mL) were added to Sub 1-2 (37.1g, 107.7 mmol), the reaction was carried out in the same manner as in the synthesis method of 1-6 to obtain 50.8g (yield: 82%) of product.

The FD-MS values of the compounds 1-1 to 1-176 and compounds 2-101 to 2-124 of the present invention prepared according to the above synthesis examples are shown in Table 3 below.

**[Table 3]**

| compound | FD-MS | compound | FD-MS |
|---|---|---|---|
| 1-1 | m/z=449.15(C₃₁H₁₉N₃O=449.51) | 1-2 | m/z=525.18(C₃₇H₂₃N₃O=525.61) |
| 1-3 | m/z=449.15(C₃₁H₁₉N₃O=449.51) | 1-4 | m/z=449.15(C₃₁H₁₉N₃O=449.51) |
| 1-5 | m/z=575.2(C₄₁H₂₅N₃O=575.67) | 1-6 | m/z=525.18(C₃₇H₂₃N₃O=525.61) |
| 1-7 | m/z=499.17(C₃₅H₂₁N₃O=499.57) | 1-8 | m/z=677.25(C₄₉H₃₁N₃O=677.81) |
| 1-9 | m/z=499.17(C₃₅H₂₁N₃O=499.57) | 1-10 | m/z=549.18(C₃₉H₂₃N₃O=549.63) |
| 1-11 | m/z=675.23(C₄₉H₂₉N₃O=675.79) | 1-12 | m/z=575.2(C₄₁H₂₅N₃O=575.67) |
| 1-13 | m/z=701.25(C₅₁H₃₁N₃O=701.83) | 1-14 | m/z=651.23(C₄₇H₂₉N₃O=651.77) |
| 1-15 | m/z=651.23(C₄₇H₂₉N₃O=651.77) | 1-16 | m/z=575.2(C₄₁H₂₅N₃O=575.67) |
| 1-17 | m/z=549.18(C₃₉H₂₃N₃O=549.63) | 1-18 | m/z=549.18(C₃₉H₂₃N₃O=549.63) |
| 1-19 | m/z=575.2(C₄₁H₂₅N₃O=575.67) | 1-20 | m/z=575.2(C₄₁H₂₅N₃O=575.67) |
| 1-21 | m/z=575.2(C₄₁H₂₅N₃O=575.67) | 1-22 | m/z=555.14(C₃₇H₂₁N₃OS=555.66) |
| 1-23 | m/z=605.16(C₄₁H₂₃N₃OS=605.72) | 1-24 | m/z=631.17(C₄₃H₂₅N₃OS=631.75) |
| 1-25 | m/z=631.17(C₄₃H₂₅N₃OS=631.75) | 1-26 | m/z=615.19(C₄₃H₂₅N₃O₂=615.69) |
| 1-27 | m/z=539.16(C₃₇H₂₁N₃O₂=539.59) | 1-28 | m/z=707.2(C₄₉H₂₉N₃OS=707.85) |
| 1-29 | m/z=707.2(C₄₉H₂₉N₃OS=707.85) | 1-30 | m/z=655.23(C₄₆H₂₉N₃O₂=655.76) |
| 1-31 | m/z=655.17(C₄₅H₂₅N₃OS=655.78) | 1-32 | m/z=631.17(C₄₃H₂₅N₃OS=631.75) |
| 1-33 | m/z=615.19(C₄₃H₂₅N₃O₂=615.69) | 1-34 | m/z=720.2(C₄₉H₂₈N₄OS=720.85) |
| 1-35 | m/z=705.21(C₄₉H₂₇N₃O₃=705.77) | 1-36 | m/z=691.23(C₄₉H₂₉N₃O₂=691.79) |
| 1-37 | m/z=757.22(C₅₃H₃₁N₃OS=757.91) | 1-38 | m/z=707.2(C₄₉H₂₉N₃OS=707.85) |
| 1-39 | m/z=707.2(C₄₉H₂₉N₃OS=707.85) | 1-40 | m/z=766.27(C₅₅H₃₄N₄O=766.9) |
| 1-41 | m/z=631.17(C₄₃H₂₅N₃OS=631.75) | 1-42 | m/z=631.17(C₄₃H₂₅N₃OS=631.75) |
| 1-43 | m/z=631.17(C₄₃H₂₅N₃OS=631.75) | 1-44 | m/z=707.2(C₄₉H₂₉N₃OS=707.85) |
| 1-45 | m/z=631.17(C₄₃H₂₅N₃OS=631.75) | 146 | m/z=631.17(C₄₃H₂₅N₃OS=631.75) |
| 1-47 | m/z=665.21(C₄₇H₂₇N₃O₂=665.75) | 1-48 | m/z=691.23(C₄₉H₂₉N₃O₂=691.79) |
| 1-49 | m/z=665.21(C₄₇H₂₇N₃O₂=665.75) | 1-50 | m/z=681.19(C₄₇H₂₇N₃OS=681.81) |
| 1-51 | m/z=757.22(C₅₃H₃₁N₃OS=757.91) | 1-52 | m/z=715.23(C₅₁H₂₉N₃O₂=715.81) |
| 1-53 | m/z=589.18(C₄₁H₂₃N₃O₂=589.65) | 1-54 | m/z=589.18(C₄₁H₂₃N₃O₂=589.65) |
| 1-55 | m/z=605.16(C₄₁H₂₃N₃OS=605.72) | 1-56 | m/z=589.18(C₄₁H₂₃N₃O₂=589.65) |
| 1-57 | m/z=605.16(C₄₁H₂₃N₃OS=605.72) | 1-58 | m/z=807.23(C₅₇H₃₃N₃OS=807.97) |
| 1-59 | m/z=691.23(C₄₉H₂₉N₃O₂=691.79) | 1-60 | m/z=591.18(C₄₁H₂₅N₃S=591.73) |
| 1-61 | m/z=515.15(C₃₅H₂₁N₃S=515.63) | 1-62 | m/z=617.19(C₄₃H₂₇N₃S=617.77) |
| 1-63 | m/z=565.16(C₃₉H₂₃N₃S=565.69) | 1-64 | m/z=565.16(C₃₉H₂₃N₃S=565.69) |
| 1-65 | m/z=581.19(C₄₀H₂₇N₃S=581.74) | 1-66 | m/z=555.14(C₃₇H₂₁N₃OS=555.66) |
| 1-67 | m/z=737.16(C₄₉H₂₇N₃OS₂=737.9) | 1-68 | m/z=518.16(C₃₄H₂₂N₄S=518.64) |
| 1-69 | m/z=515.15(C₃₅H₂₁N₃S=515.63) | 1-70 | m/z=561.19(C₃₇H₂₇N₃OS=561.7) |
| 1-71 | m/z=630.19(C₄₃H₂₆N₄S=630.77) | 1-72 | m/z=591.18(C₄₁H₂₅N₃S=591.73) |
| 1-73 | m/z=766.27(C₅₅H₃₄N₄O=766.9) | 1-74 | m/z=575.2(C₄₁H₂₅N₃O=575.67) |
| 1-75 | m/z=677.25(C₄₉H₃₁N₃O=677.81) | 1-76 | m/z=707.2(C₄₉H₂₉N₃OS=707.85) |
| 1-77 | m/z=766.27(C₅₅H₃₄N₄O=766.9) | 1-78 | m/z=707.2(C₄₉H₂₉N₃OS=707.85) |
| 1-79 | m/z=767.26(C₅₅H₃₃N₃O₂=767.89) | 1-80 | m/z=767.26(C₅₅H₃₃N₃O₂=767.89) |
| 1-81 | m/z=691.23(C₄₉H₂₉N₃O₂=691.79) | 1-82 | m/z=817.27(C₅₉H₃₅N₃O₂=817.95) |
| 1-83 | m/z=741.24(C₅₃H₃₁N₃O₂=741.85) | 1-84 | m/z=741.24(C₅₃H₃₁N₃O₂=741.85) |
| 1-85 | m/z=831.23(C₅₉H₃₃N₃OS=831.99) | 1-86 | m/z=767.26(C₅₅H₃₃N₃O₂=767.89) |
| 1-87 | m/z=757.19(C₅₁H₂₇N₅OS=757.87) | 1-88 | m/z=601.22(C₄₃H₂₇N₃O=601.71) |
| 1-89 | m/z=757.22(C₅₃H₃₁N₃OS=757.91) | 1-90 | m/z=625.22(C₄₅H₂₇N₃O=625.73) |
| 1-91 | m/z=625.22(C₄₅H₂₇N₃O=625.73) | 1-92 | m/z=625.22(C₄₅H₂₇N₃O=625.73) |
| 1-93 | m/z=625.22(C₄₅H₂₇N₃O=625.73) | 1-94 | m/z=651.23(C₄₇H₂₉N₃O=651.77) |
| 1-95 | m/z=731.2(C₅₁H₂₉N₃OS=731.87) | 1-96 | m/z=833.25(C₅₉H₃₅N₃OS=834.01) |
| 1-97 | m/z=757.22(C₅₃H₃₁N₃OS=757.91) | **1**-98 | m/z=867.29(C₆₃H₃₇N₃O₂=868.01) |
| 1-99 | m/z=757.22(C₅₃H₃₁N₃OS=757.91) | 1-100 | m/z=655.23(C₄₆H₂₉N₃O₂=655.76) |
| **1-101** | m/z=625.22(C₄₅H₂₇N₃O=625.73) | 1-102 | m/z=625.22(C₄₅H₂₇N₃O=625.73) |
| 1-103 | m/z=625.22(C₄₅H₂₇N₃O=625.73) | 1-104 | m/z=807.23(C₅₇H₃₃N₃OS=807.97) |
| 1-105 | m/z=731.2(C₅₁H₂₉N₃OS=731.87) | 1-106 | m/z=731.2(C₅₁H₂₉N₃OS=731.87) |
| 1-107 | m/z=791.26(C₅₇H₃₃N₃O₂=791.91) | 1-108 | m/z=807.23(C₅₇H₃₃N₃OS=807.97) |
| 1-109 | m/z=807.23(C₅₇H₃₃N₃OS=807.97) | 1-110 | m/z=575.20(C₄₁H₂₅N₃O=575.67) |
| 1-111 | m/z=867.29(C₆₃H₃₇N₃O₂=868.01) | 1-112 | m/z=807.23(C₅₇H₃₃N₃OS=807.97) |
| 1-113 | m/z=777.28(C₅₇H₃₅N₃O=777.93) | 1-114 | m/z=803.29(C₅₉H₃₇N₃O=803.97) |
| 1-115 | m/z=632.26(C₄₅H₂₀D₇N₃O=632.77) | 1-116 | m/z=715.23(C₅₁H₂₉N₃O₂=715.81) |
| 1-117 | m/z=883.27(C₆₃H₃₇N₃OS=884.07) | 1-118 | m/z=833.25(C₅₉H₃₅N₃OS=834.01) |
| 1-119 | m/z=942.34(C₆₉H₄₂N₄O=943.12) | 1-120 | m/z=701.25(C₅₁H₃₁N₃O=701.83) |
| 1-121 | m/z=615.19(C₄₃H₂₅N₃O₂=615.69) | 1-122 | m/z=665.21(C₄₇H₂₇N₃O₂=665.75) |
| 1-123 | m/z=681.19(C₄₇H₂₇N₃OS=681.81) | 1-124 | m/z=715.23(C₅₁H₂₉N₃O₂=715.81) |
| 1-125 | m/z=691.23(C₄₉H₂₉N₃O₂=691.79) | 1-126 | m/z=691.23(C₄₉H₂₉N₃O₂=691.79) |
| | | 1-128 | m/z=691.23(C₄₉H₂₉N₃O₂=691.79) |
| 1-129 | m/z=757.22(C₅₃H₃₁N₃OS=757.91) | 1-130 | m/z=691.23(C₄₉H₂₉N₃O₂=691.79) |
| 1-131 | m/z=817.27(C₅₉H₃₅N₃O₂=817.95) | 1-132 | m/z=691.23(C₄₉H₂₉N₃O₂=691.79) |
| 1-133 | m/z=781.24(C₅₅H₃₁N₃O₃=781.87) | 1-134 | m/z=797.21(C₅₅H₃₁N₃O₂S=797.93) |
| | | 1-136 | m/z=691.23(C₄₉H₂₉N₃O₂=691.79) |
| 1-137 | m/z=691.23(C₄₉H₂₉N₃O₂=691.79) | 1-138 | m/z=780.25(C₅₅H₃₂N₄O₂=780.89) |
| 1-139 | m/z=691.23(C₄₉H₂₉N₃O₂=691.79) | 1-140 | m/z=781.18(C₄₉H₂₄F₅N₃O₂=781.74) |
| 1-141 | m/z=732.29(C₅₃H₂₈D₅N₃O=732.9) | 1-142 | m/z=793.27(C₅₇H₃₅N₃O₂=793.93) |
| 1-143 | m/z=651.23(C₄₇H₂₉N₃O=651.77) | 1-144 | m/z=755.26(C₅₄H₃₃N₃O₂=755.88) |
| 1-145 | m/z=591.18(C₄₁H₂₅N₃S=591.73) | 1-146 | m/z=617.19(C₄₃H₂₇N₃S=617.77) |
| 1-147 | m/z=707.24(C₅₀H₃₃N₃S=707.9) | 1-148 | m/z=641.19(C₄₅H₂₇N₃S=641.79) |
| 1-149 | m/z=641.19(C₄₅H₂₇N₃S=641.79) | 1-150 | m/z=641.19(C₄₅H₂₇N₃S=641.79) |
| 1-151 | m/z=793.26(C₅₇H₃₅N₃S=793.99) | 1-152 | m/z=641.19(C₄₅H₂₇N₃S=641.79) |
| 1-153 | m/z=667.21(C₄₇H₂₉N₃S=667.83) | 1-154 | m/z=823.21(C₅₇H₃₃N₃S₂=824.03) |
| 1-155 | m/z=883.27(C₆₃H₃₇N₃OS=884.07) | 1-156 | m/z=747.18(C₅₁H₂₉N₃S₂=747.93) |
| 1-157 | m/z=681.19(C₄₇H₂₇N₃OS=681.81) | 1-158 | m/z=697.16(C₄₇H₂₇N₃S₂=697.87) |
| 1-159 | m/z=731.2(C₅₁H₂₉N₃OS=731.87) | 1-160 | m/z=631.17(C₄₃H₂₅N₃OS=631.75) |
| | | 1-162 | m/z=737.16(C₄₉H₂₇N₃OS₂=737.9) |
| 1-163 | m/z=707.2(C₄₉H₂₉N₃OS=707.85) | 1-164 | m/z=707.2(C₄₉H₂₉N₃OS=707.85) |
| 1-165 | m/z=747.18(C₅₁H₂₉N₃S₂=747.93) | 1-166 | m/z=681.19(C₄₇H₂₇N₃OS=681.81) |
| 1-167 | m/z=731.2(C₅₁H₂₉N₃OS=731.87) | 1-168 | m/z=631.17(C₄₃H₂₅N₃OS=631.75) |
| 1-169 | m/z=731.2(C₅₁H₂₉N₃OS=731.87) | 1-170 | m/z=721.18(C₄₉H₂₇N₃O₂S=721.83) |
| 1-171 | m/z=691.23(C₄₉H₂₉N₃O₂=691.79) | 1-172 | m/z=691.23(C₄₉H₂₉N₃O₂=691.79) |
| 1-173 | m/z=741.24(C₅₃H₃₁N₃O₂=741.85) | 1-174 | m/z=807.23(C₅₇H₃₃N₃OS=807.97) |
| 1-175 | m/z=799.21(C₅₅H₃₃N₃S₂=800.01) | 1-176 | m/z=757.22(C₅₃H₃₁N₃OS=757.91) |
| 2-101 | m/z=449.15(C₃₁H₁₉N₃O=449.51) | 2-102 | m/z=499.17(C₃₅H₂₁N₃O=499.57) |
| 2-103 | m/z=499.17(C₃₅H₂₁N₃O=499.57) | 2-104 | m/z=525.18(C₃₇H₂₃N₃O=525.61) |
| 2-105 | m/z=525.18(C₃₇H₂₃N₃O=525.61) | 2-106 | m/z=525.18(C₃₇H₂₃N₃O=525.61) |
| 2-107 | m/z=575.20(C₄₁H₂₅N₃O=575.67) | 2-108 | m/z=575.20(C₄₁H₂₅N₃O=575.67) |
| 2-109 | m/z=601.22(C₄₃H₂₇N₃O=601.71) | 2-110 | m/z=525.18(C₃₇H₂₃N₃O=525.61) |
| 2-111 | m/z=575.20(C₄₁H₂₅N₃O=575.67) | 2-112 | m/z=575.20(C₄₁H₂₅N₃O=575.67) |
| 2-113 | m/z=449.15(C₃₁H₁₉N₃O=449.51) | 2-114 | m/z=499.17(C₃₅H₂₁N₃O=499.57) |
| 2-115 | m/z=499.17(C₃₅H₂₁N₃O=499.57) | 2-116 | m/z=525.18(C₃₇H₂₃N₃O=525.61) |
| 2-117 | m/z=525.18(C₃₇H₂₃N₃O=525.61) | 2-118 | m/z=525.18(C₃₇H₂₃N₃O=525.61) |
| 2-119 | m/z=575.20(C₄₁H₂₅N₃O=575.67) | 2-120 | m/z=575.20(C₄₁H₂₅N₃O=575.67) |
| 2-121 | m/z=601.22(C₄₃H₂₇N₃O=601.71) | 2-122 | m/z=525.18(C₃₇H₂₃N₃O=525.61) |
| 2-123 | m/z=575.20(C₄₁H₂₅N₃O=575.67) | 2-124 | m/z=575.20(C₄₁H₂₅N₃O=575.67) |

### [SYNTHESIS EXAMPLE 21 Formula 2

As shown in Reaction Scheme 4 below, the compounds(final product 2) represented by Formula 2 according to the present invention can be synthesized by reacting Sub 3 with Sub 4 , but there is no limitation thereto.

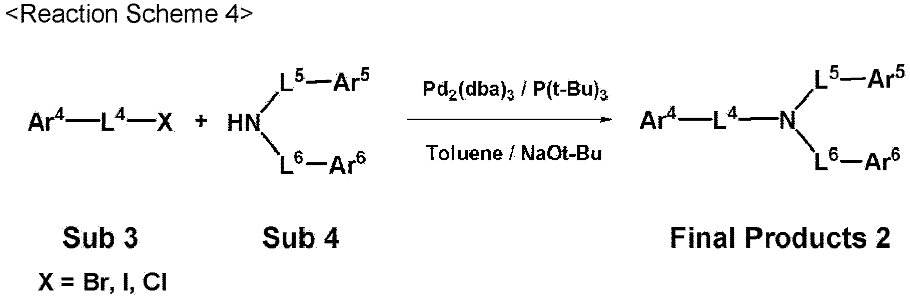

### 1. Exemplary Compounds of Sub 3

Sub 3 of the Reaction Scheme 4 can be synthesized according to the reaction route of the following Reaction Scheme 5, but there is no limitation thereto.

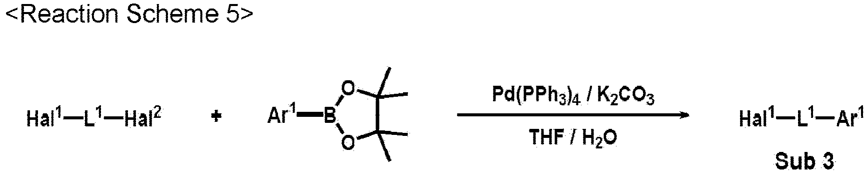

The compounds belonging to Sub 3 of the Reaction Scheme 4 may be, but not limited to, the following compounds.

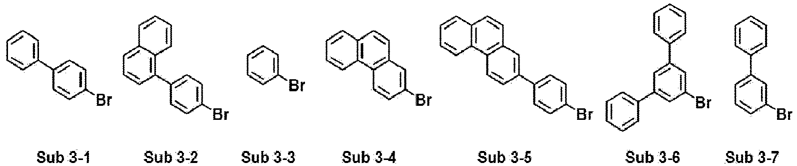

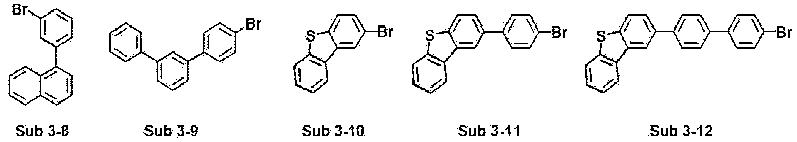

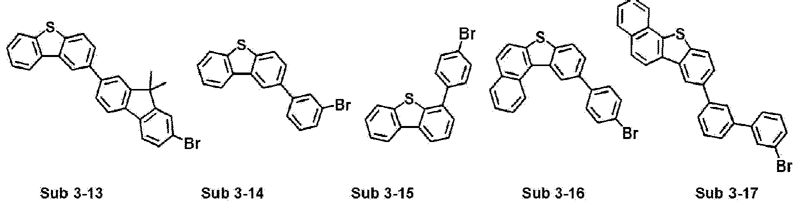

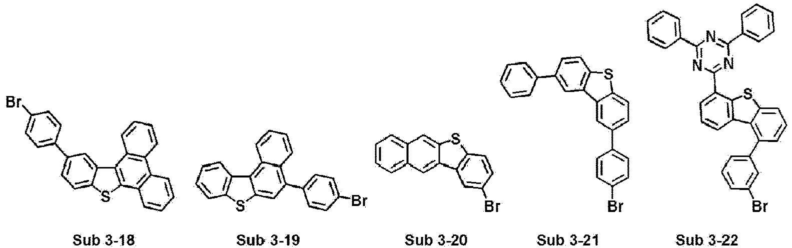

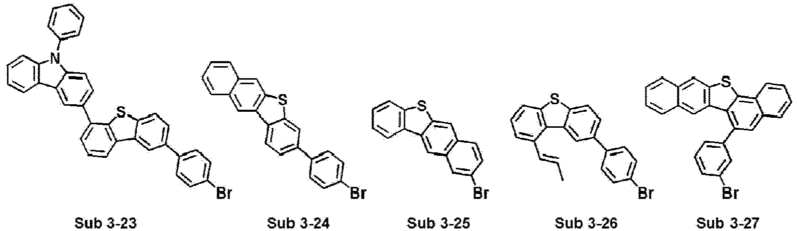

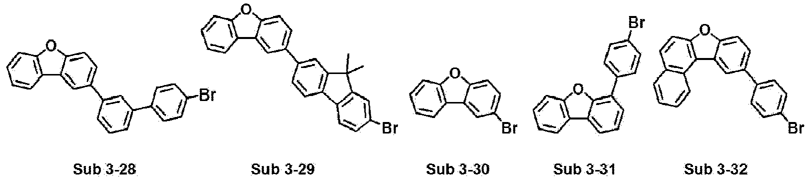

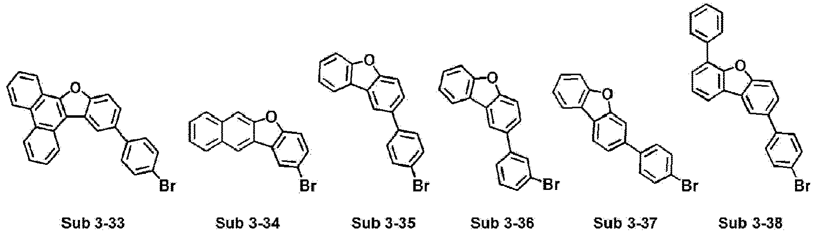

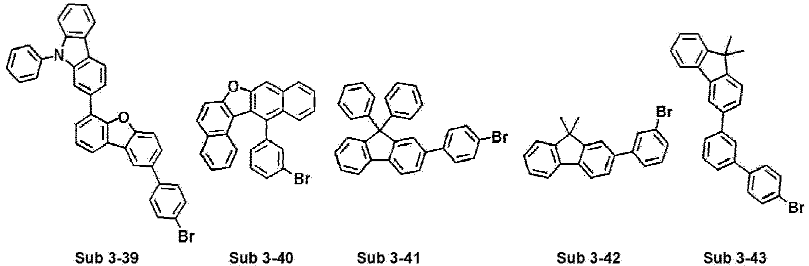

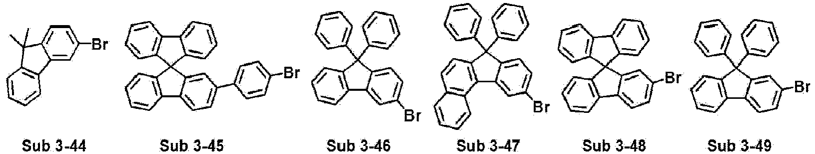

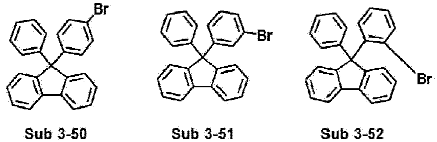

Table 4 shows FD-MS values of the compounds belonging to Sub 3.

**[Table 4]**

| Compound | FD-MS | Compound | FD-MS |
|---|---|---|---|
| Sub 3-1 | m/z:231.99(C₁₂H₉Br=233.11) | Sub 3-2 | m/z=282(C₁₆H₁₁Br=283.17) |
| Sub 3-3 | m/z=155.96(C₅H₅Br=157.01) | Sub 3-4 | m/z=255.99(C₁₄H₉Br=257.13) |
| Sub 3-5 | m/z=332.02(C₂₀H₁₃Br=333.23) | Sub 3-6 | m/z=308.02(C₁₈H₁₃Br=309.21) |
| Sub 3-7 | m/z=231.99(C₁₂-H₉Br=233.11) | Sub 3-8 | m/z=282(C₁₆H₁₁Br=283.17) |
| Sub 3-9 | m/z=308.02(C₁₈H₁₃Br=309.21) | Sub 3-10 | m/z=261.95(C₁₂H₇BrS=263.15) |
| Sub 3-11 | m/z=337.98(C₁₈H₁₁BrS=339.25) | Sub 3-12 | m/z=414.01(C₂₄H₁₅BrS=415.35) |
| Sub 3-1 3 | m/z=454.04(C₂₇H₁₉BrS=455.41) | Sub 3-14 | m/z=337.98(C₁₈H₁₁BrS=339.25) |
| Sub 3-15 | m/z=337.98(C₁₈H₁₁BrS=339.25) | Sub 3-16 | m/z=387.99(C₂₂H₁₃BrS=389.31) |
| Sub 3-17 | m/z=464.02(C₂₈H₁₇BrS=465.41) | Sub 3-18 | m/z=438.01(C₂₆H₁₅BrS=439.37) |
| Sub 3-19 | m/z=387.99(C₂₂H₁₃BrS=389.31) | Sub 3-20 | m/z=311.96(C₁₆H₉BrS=313.21) |
| Sub 3-21 | m/z=414.01(C₂₄H₁₅BrS=415.35) | Sub 3-22 | m/z=569.06(C₃₃H₂₀BrN₃S=570.51) |
| Sub 3-23 | m/z=579.07(C₃₆H₂₂BrNS=580.54) | Sub 3-24 | m/z=387.99(C₂₂H₁₃BrS=389.31) |
| Sub 3-25 | m/z=311.96(C₁₆H₉BrS=313.21) | Sub 3.26 | m/z=378.01(C₂₁H₁₅BrS=379.32) |
| Sub 3-27 | m/z=438.01(C₂₆H₁₅BrS=439.37) | Sub 3-28 | m/z=398.03(C₂₄H₁₅BrO=399.29) |
| Sub 3-29 | m/z=438.06(C₂₇H₁₉BrO=439.35) | Sub 3-30 | m/z=245.97(C₁₂H₇BrO=247.09) |
| Sub 3.31 | m/z=322(C₁₈H₁₁BrO=323.19) | Sub 3.32 | m/z=372.01(C₂₂H₁₃BrO=373.25) |
| Sub 3-33 | m/z=422.03(C₂₅H₁₅BrO=423.31) | Sub 3-34 | m/z=295.98(C₁₆H₉BrO=297.15) |
| Sub 3-35 | m/z=322(C₁₆H₁₁BrO=323.19) | Sub 3-36 | m/z=322(C₁₈H₁₁BrO=323.19) |
| Sub 3-37 | m/z=322(C₁₈H₁₁BrO=323.19) | Sub 3-38 | m/z=398.03(C₂₄H₁₅BrO=399.29) |
| Sub 3-39 | m/z=563.09(C₃₆H₂₂BrNO=564.48) | Sub 3-40 | m/z=422.03(C₂₆H₁₅BrO=423.31) |
| Sub 3-41 | m/z=472.08(C₃₁H₂₁Br=473.41) | Sub 3-42 | m/z=348.05(C₂₁H₁₇Br=349.27) |
| Sub 3-43 | m/z=424.08(C₂₇H₂₁Br=425.37) | Sub 3-44 | m/z=272.0,2(C₁₅H₁₃Br=273.17) |
| Sub 3-45 | m/z=470.07(C₃₁H₁₉Br=471.4) | Sub 3-46 | m/z=396.05(C₂₅H₁₇Br=397.32) |
| Sub 3-47 | m/z=446.07(C₂₉H₁₉Br=447.38) | Sub 3-48 | m/z=394.04(C₂₅H₁₅Br=395.3) |
| Sub 3-49 | m/z=396.05(C₂₅H₁₇Br=397.32) | Sub 3-50 | m/z=396.05(C₂₅H₁₇Br=397.32) |
| Sub 3-51 | m/z=396.05(C₂₅H₁₇Br=397.32) | Sub 3-52 | m/z=396.05(C₂₅H₁₇Br=397.32) |

### 2. Exemplary Compounds of Sub 4 and Synthesis Example

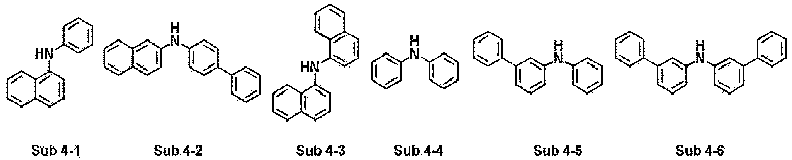

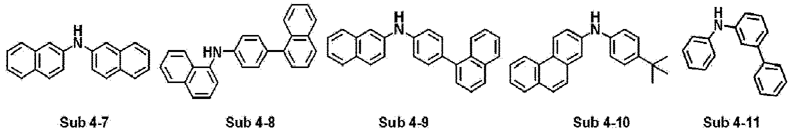

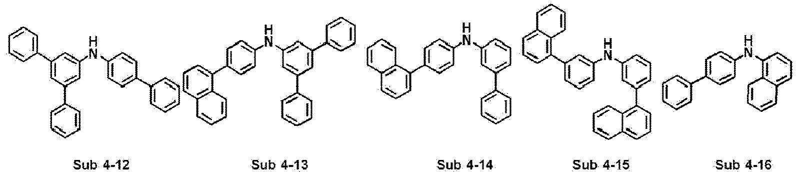

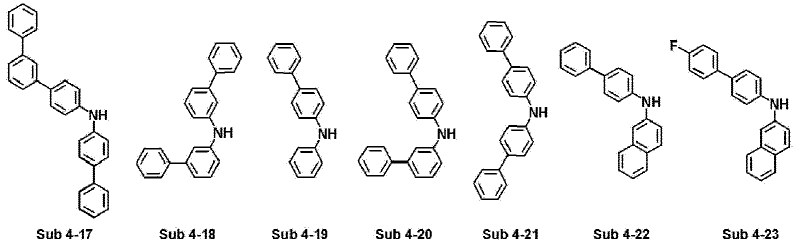

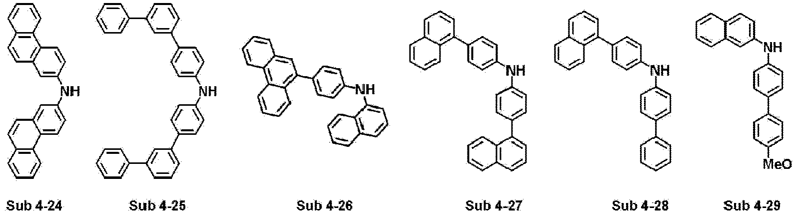

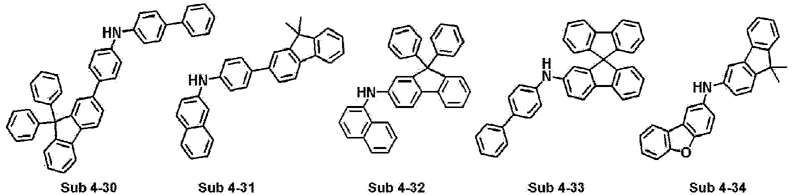

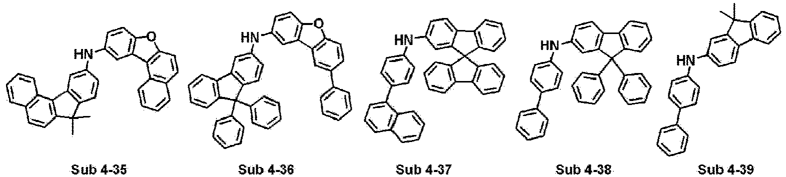

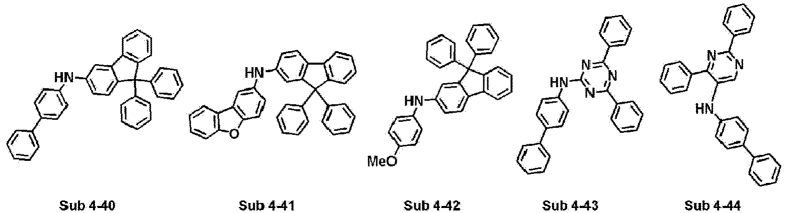

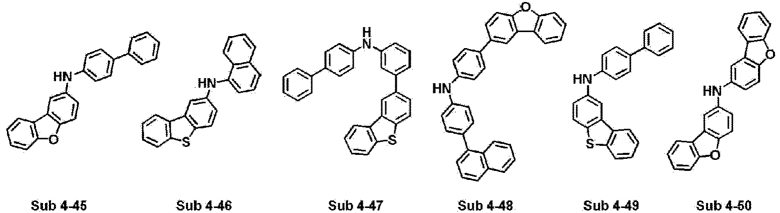

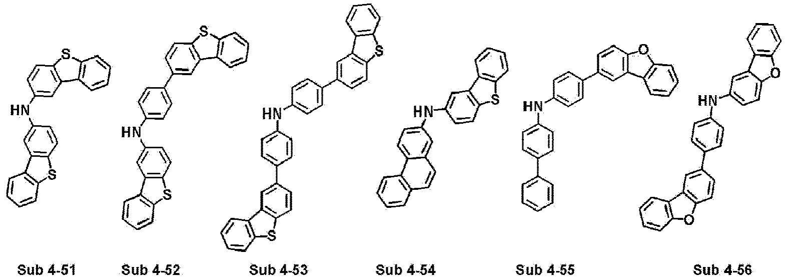

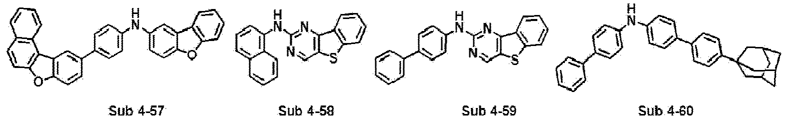

Table 5 shows FD-MS values of the compounds belonging to Sub 4.

**[Table 5]**

| Compound | FD-MS | Compound | FD-MS |
|---|---|---|---|
| Sub 4-1 | m/z=219.1(C16H13N=219.29) | Sub 4-2 | m/z=295.14(C22H17N=295.39) |
| Sub 4-3 | m/z=269.12(C20H15N=269.35) | Sub 4-4 | m/z=169.09(C12H12N=169.23) |
| Sub 4-5 | m/z=245.12(C18H15N=245.33) | Su b 4-6 | m/z=321.15(C24H19N=321.42) |
| Sub 4-7 | m/z=269.12(C20H15N=269.35) | Sub 4-8 | m/z=345.15(C26H19N=345.45) |
| Sub 4-9 | m/z=345.15(C26H19N=345.45) | Sub 4-10 | m/z=325.18(C24H23N=325.46) |
| Sub 4-1 1 | m/z=245.12(C18H15N=245.33) | Sub 4-12 | m/z=397.18(C30H23N=397.52) |
| Sub 4-13 | m/z=447.2(C34H25N=447.58) | Sub 4.14 | m/z=371.17(C28H21N=371.48) |
| Sub 4-15 | m/z=421.18(C32H23N=421.54) | Sub 4-16 | m/z=295.14(C22H17N=295.39) |
| Sub 4.17 | m/z=397.18(C30H23N=397.52) | Sub 4.18 | m/z=321.15(C24H19N=321,42) |
| Sub 4-19 | m/z=245.12(C18H15N=245.33) | Sub 4-20 | m/z=321.15(C24H19N=321.42) |
| Sub 4-21 | m/z=321.15(C24H19N=321.42) | Sub 4-22 | m/z=295.14(C22H17N=295.39) |
| Sub 4-23 | m/z=313.13(C22H16FN=313.38) | Sub 4.24 | m/z=369.15(C28H19N=369.47) |
| Sub 4-25 | m/z=473.21(C36H27N=473.62) | Sub 4.26 | m/z=395.17(C30H21N=395.51) |
| Sub 4-27 | m/z=421.18(C32H23N=421.54) | Sub 4-28 | m/z=371.17(C28H21N=371.48) |
| Sub 4-29 | m/z=325.15(C23H19NO=325.41) | Sub 4.30 | m/z=561.25(C43H31N=561.73) |
| Sub 4-31 | m/z=411.2(C31H25N=411.55) | Sub 4-32 | m/z=459.2(C35H25N=459.59) |
| Sub 4-33 | m/z=483.2(C37H25N=483.61) | Sub 4-34 | m/z=375.16(C27H21NO=375.47) |
| Sub 4-35 | m/z=475.19(C35H25NO=475.59) | Sub 4-36 | m/z=575.22(C43H29NO=575.71) |
| Sub 4-37 | m/z=533.21(C41H27N=533.67) | Sub 4.38 | m/z=485.21(C37H27N=485.63) |
| Sub 4-39 | m/z=361.18(C27H23N=361.49) | Sub 4-40 | m/z=485.21(C37H27N=485.63) |
| Sub 4-41 | m/z=499.19(C37H25NO=499.61) | Sub 4-42 | m/z=439.19(C32H25NO=439.56) |
| Sub 4-43 | m/z=400.17(C27H20N4=400.49) | Sub 4.44 | m/z=399.17(C28H21N3=399.5) |
| Sub 4-45 | m/z=335.13(C24H17NO=335.41) | Sub 4-46 | m/z=325.09(C22H1SNS=325.43) |
| Sub 4-47 | m/z=427.1(C30H21NS=427.57) | Sub 4-48 | m/z=461-18(C34H23NO=461.56) |
| Sub 4-49 | m/z=351.11(C24H17NS=351.47) | Sub 4-50 | m/z=349.11(C24H15NO2=349.39) |
| Sub 4-51 | m/z=381.06(C24H15NS2=381.51) | Sub 4-52 | m/z=457.1(C30H19NS2=457.61) |
| Sub 4.53 | m/z=533.13(C36H23NS2=533.71) | Sub 4.54 | m/z=375.11(C26H17NS=375.49) |
| Sub 4-55 | m/z=411.16(C30H21NO=411.5) | Sub 4-56 | m/z=425.14(C30H19NO2=425.49) |
| Sub 4-57 | m/z=475.16(C34H21NO2=475.55) | Sub 4-58 | m/z=327.08(C20H13N3S=327.41) |
| Sub 4-59 | m/z=353,1(C22H15N3S=353.44) | Sub 4-60 | m/z=455.26(C₃₄H₃₃N=455.65) |

Sub 4 of the Reaction Scheme 2 can be synthesized according to the reaction routes of the following Reaction Scheme 6, but it is not limited thereto.

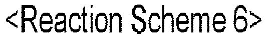

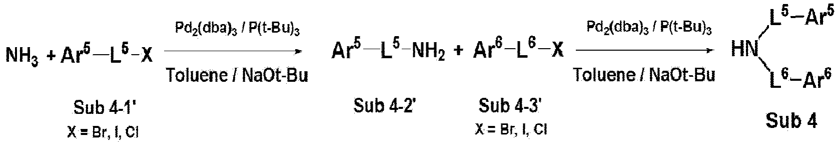

### Synthesis Example of Sub 4-1

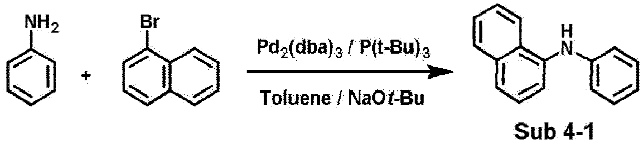

Aniline (15 g, 161.1 mmol), 1-bromonaphthalene (36.7 g, 177.2 mmol), Pd₂(dba)₃ (7.37 g, 8.05 mmol), P(t-Bu)₃ (3.26 g, 16.1 mmol), NaOt-Bu (51.08 g, 531.5 mmol) and toluene (1690 mL) were placed in a round bottom flask and the reaction proceeded at 100°C. When the reaction was completed, the reaction product was extracted with CH₂Cl₂ and water, and then the organic layer was dried with MgSO₄ and concentrated. Then, the concentrate was applied to silica gel column and recrystallized to obtain 25.4 g (yield: 72%) of Sub 4-1.

### Synthesis Example of Sub 4-30

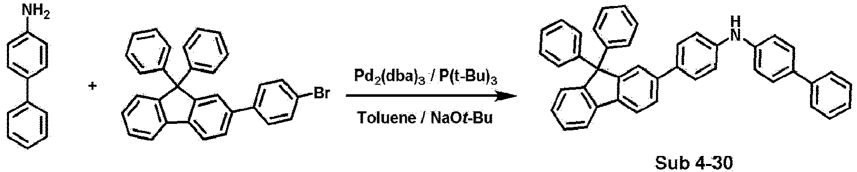

After 1,1'-biphenyl]-4-amine (15 g, 88.6 mmol), 2-(4-bromophenyl)-9,9-diphenyl-9H-fluorene (46.2 g, 97.5 mmol), Pd₂(dba)₃ (4.06 g, 4.43 mmol), P(t-Bu)₃ (1.8 g, 8.86 mmol), NaOt-Bu (28.1 g, 292.5 mmol) and toluene (931 mL) were placed in a round bottom flask, the reaction was carried out in the same manner as in the synthesis method of Sub 4-1 to obtain 34.9 (yield : 70%) of Sub 4-30.

### Synthesis Example of Sub 4-46

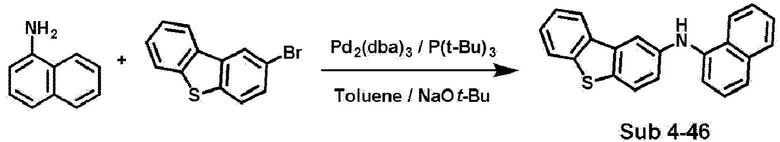

After naphthalen-1-amine (15 g, 104.8 mmol), 2-bromodibenzo[b,d]thiophene (30.3 g, 115.2 mmol), Pd₂(dba)₃ (4.8 g, 5.24 mmol), P(t-Bu)₃ (2.12 g, 10.48 mmol), NaOt-Bu (33.22 g, 345.7 mmol) and toluene (1100 mL) were placed in a round bottom flask, the reaction was carried out in the same manner as in the synthesis method of Sub 4-1 to obtain 24.9 g (yield : 73%) of Sub 4-46.

### Synthesis Example of Sub 4-49

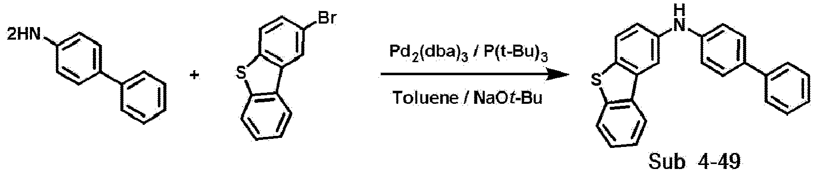

After [1,1'-biphenyl]-4-amine (15 g, 88.64 mmol), 2-bromodibenzo[b,d]thiophene (23.32 g, 88.64 mmol), Pd₂(dba)₃ (2.4 g, 2.66 mmol), P(t-Bu)₃ (17.93 g, 88.64 mmol), NaOt-Bu (17.04 g, 177.27 mmol) and toluene (886 mL) were placed in a round bottom flask, the reaction was carried out in the same manner as in the synthesis method of Sub 4-1 to obtain 24.61 g (yield : 79%) of Sub 4-49.

### Synthesis Example of Sub 4-56

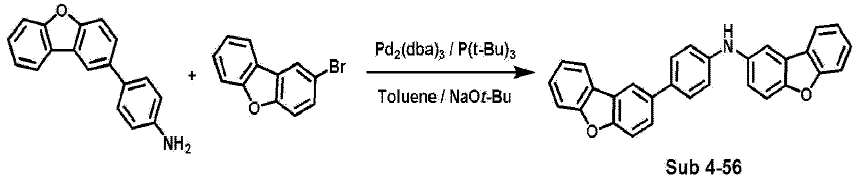

After 4-(dibenzo[b,d]furan-2-yl)aniline (15 g, 57.85 mmol), 2-bromodibenzo[b,d]furan (15.7 g, 63.63 mmol), Pd₂(dba)₃ (2.65 g, 2.89 mmol), P(t-Bu)₃ (1.17 g, 5.78 mmol), NaOt-Bu (18.35 g, 190.9 mmol) and toluene (607 mL) were placed in a round bottom flask, the reaction was carried out in the same manner as in the synthesis method of Sub 4-1 to obtain 17.2 g (yield : 70%) of Sub Sub 4-56.

### 3. Synthesis Example of a final product

### Synthesis of 2-1

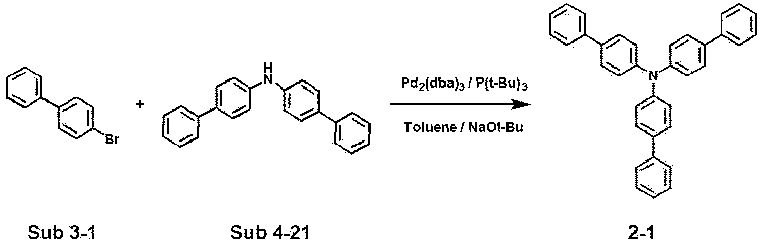

Sub 3-1 (10 g, 42.9 mmol), Sub 4-21 (13.79 g, 42.9 mmol), Pd₂(dba)₃ (1.18 g, 1.29 mmol), P(t-Bu)₃ (8.68 g, 42.9 mmol), NaOt-Bu (8.25 g, 85.80 mmol) and toluene (429 mL) were placed in a round bottom flask and the reaction proceeded at 100°C. When the reaction was completed, the reaction product was extracted with CH₂Cl₂ and water, and then the organic layer was dried with MgSO₄ and concentrated. Then, the concentrate was applied to silica gel column and recrystallized to obtain 15.6 g (yield: 77%) of the product 2-1.

### Synthesis of 2-4

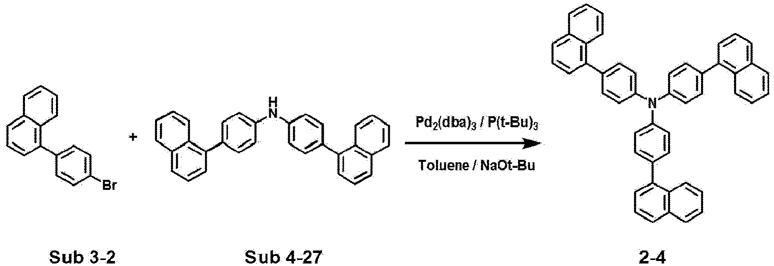

After Sub 3-2 (10 g, 35.3 mmol), Sub 4-27 (14.8 g, 35.31 mmol), Pd₂(dba)₃ (0.97 g, 1.06 mmol), P(t-Bu)₃ (7.14 g, 35.31 mmol), NaOt-Bu (6.79 g, 70.63 mmol) and toluene (353 mL) were placed in a round bottom flask, the reaction was carried out in the same manner as in the synthesis method of 2-1 to obtain 16.9 g (yield : 78%) of the product 2-4.

### Synthesis Example of 2-9

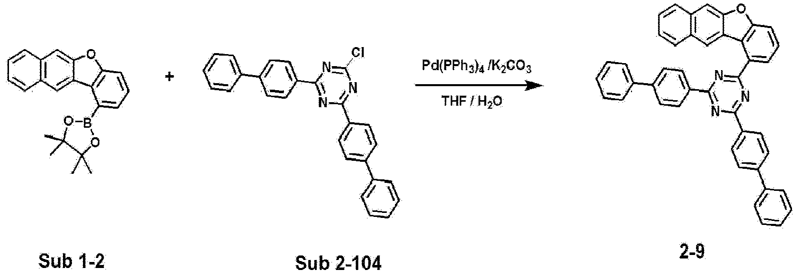

After adding THF(395 mL), Sub 2-104 (54.3g, 129.24 mmol), Pd(PPh₃)₄ (4.98 g, 4.31 mmol), K₂CO₃ (44.66 g, 323.10 mmol) and water(197 mL) to Sub 1-2 (37.1g, 107.7 mmol), the reaction was carried out in the same manner as in the synthesis method of 1-6 to obtain the product (55.5g, 81%).

### Synthesis of 2-10

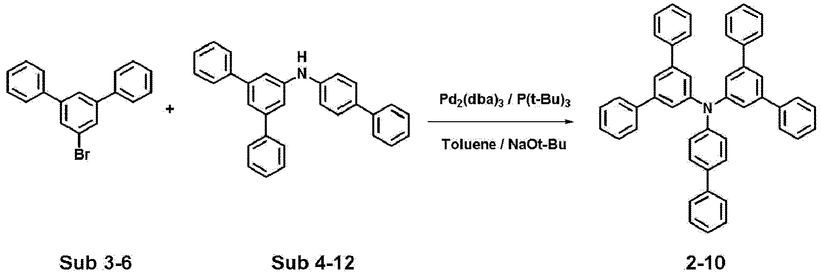

After Sub 3-6 (10 g, 32.34 mmol), Sub 4-12 (12.86 g, 32.34 mmol), Pd₂(dba)₃ (0.89 g, 0.97 mmol), P(t-Bu)₃ (6.54 g, 32.34 mmol), NaOt-Bu (6.22 g, 64.68 mmol) and toluene (323 mL) were placed in a round bottom flask, the reaction was carried out in the same manner as in the synthesis method of 2-1 to obtain 15.1 g (yield : 75%) of the product 2-10.

### Synthesis Example of 2-11

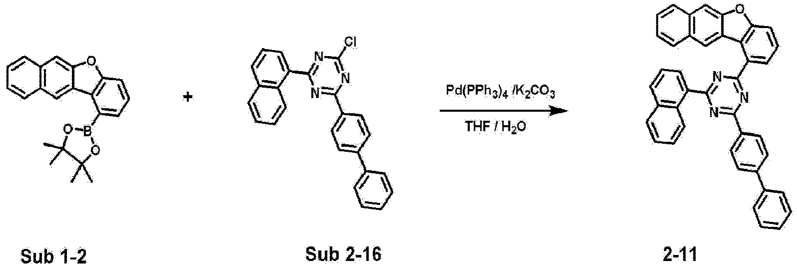

After adding THF(395 mL), Sub 2-16 (50.9g, 129.24 mmol), Pd(PPh₃)₄ (4.98 g, 4.31 mmol), K₂CO₃ (44.66 g, 323.10 mmol) and water (197 mL) to Sub 1-2 (37.1g, 107.7 mmol), the reaction was carried out in the same manner as in the synthesis method of 1-6 to obtain the product (50.8g, 82%).

### Synthesis of 2-19

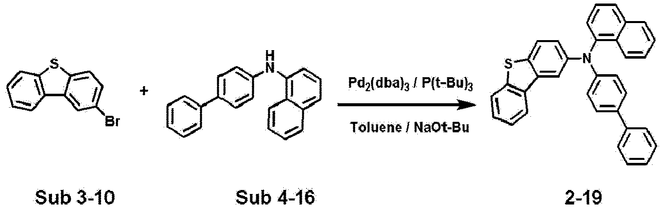

After Sub 3-10 (10 g, 38 mmol), Sub 4-16 (11.23 g, 38 mmol), Pd₂(dba)₃ (1.04 g, 1.14 mmol), P(t-Bu)₃ (7.69 g, 38 mmol), NaOt-Bu (7.3 g, 76 mmol) and toluene (380 mL) were placed in a round bottom flask, the reaction was carried out in the same manner as in the synthesis method of 2-1 to obtain 13.7 g (yield : 76%) of the product 2-19.

### Synthesis of 2-68

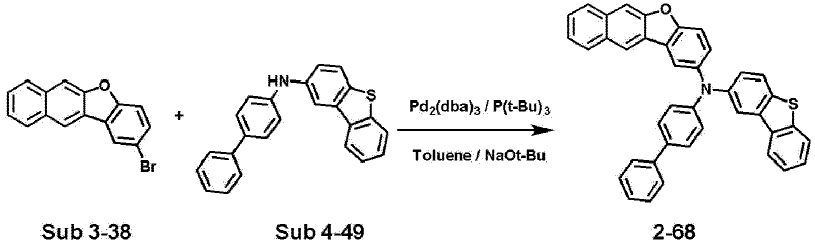

After Sub 3-38 (10 g, 33.65 mmol), Sub 4-49 (11.83 g, 33.65 mmol), Pd₂(dba)₃ (0.92 g, 1.01 mmol), P(t-Bu)₃ (6.81 g, 33.65 mmol), NaOt-Bu (6.47 g, 67.31 mmol) and toluene (337 mL) were placed in a round bottom flask, the reaction was carried out in the same manner as in the synthesis method of 2-1 to obtain 15.28 g (yield : 80%) of the product 2-68.

### Synthesis of 2-24

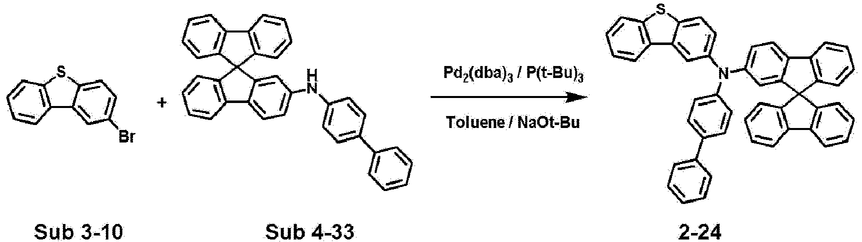

After Sub 3-10 (10 g, 38 mmol), Sub 4-33 (18.38 g, 38 mmol), Pd₂(dba)₃ (1.04 g, 1.14 mmol), P(t-Bu)₃ (7.69 g, 38 mmol), NaOt-Bu (7.3 g, 76 mmol) and toluene (380 mL) were placed in a round bottom flask, the reaction was carried out in the same manner as in the synthesis method of 2-1 to obtain 18.72 g (yield : 74%) of the product.

### Synthesis of 2-29

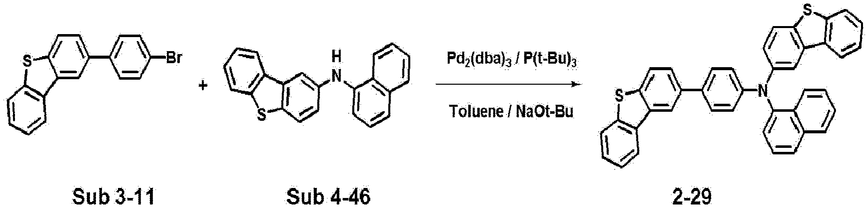

After Sub 3-11 (10 g, 29.48 mmol), Sub 4-46 (9.59 g, 29.48 mmol), Pd₂(dba)₃ (0.81 g, 0.88 mmol), P(t-Bu)₃ (5.96 g, 29.48 mmol), NaOt-Bu (5.67 g, 58.95 mmol) and toluene (295 mL) were placed in a round bottom flask, the reaction was carried out in the same manner as in the synthesis method of 2-1 to obtain 12.39 g (yield : 72%) of the product 2-29.

### Synthesis of 2-30

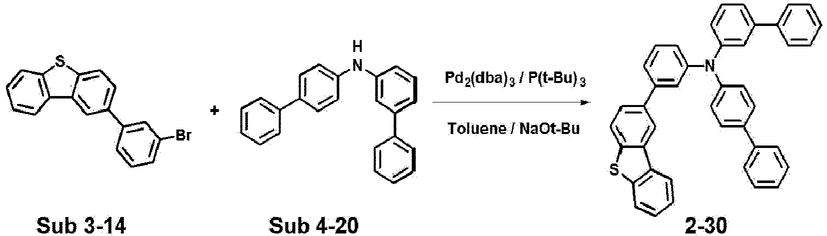

After Sub 3-14 (10 g, 29.48 mmol), Sub 4-20 (9.47 g, 29.48 mmol), Pd₂(dba)₃ (0.81 g, 0.88 mmol), P(t-Bu)₃ (5.96 g, 29.48 mmol), NaOt-Bu (5.67 g, 58.95 mmol) and toluene (295 mL) were placed in a round bottom flask, the reaction was carried out in the same manner as in the synthesis method of 2-1 to obtain 12.13 g (yield : 71%) of the product 2-30.

### Synthesis of 2-36

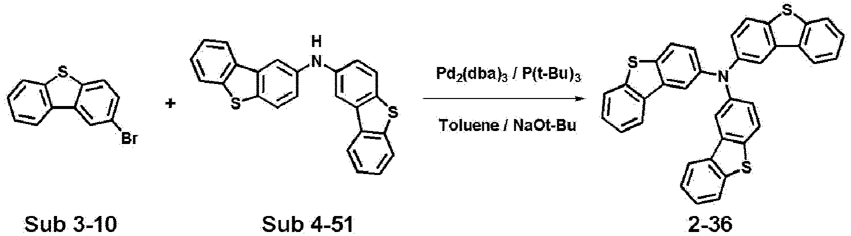

After Sub 3-10 (10 g, 38 mmol), Sub 4-51 (14.5 g, 38 mmol), Pd₂(dba)₃ (1.04 g, 1.14 mmol), P(t-Bu)₃ (7.69 g, 38 mmol), NaOt-Bu (7.30 g, 76 mmol) and toluene (380 mL) were placed in a round bottom flask, the reaction was carried out in the same manner as in the synthesis method of 2-1 to obtain 16.5 g (yield : 77%) of the product 2-36.

### Synthesis of 2-50

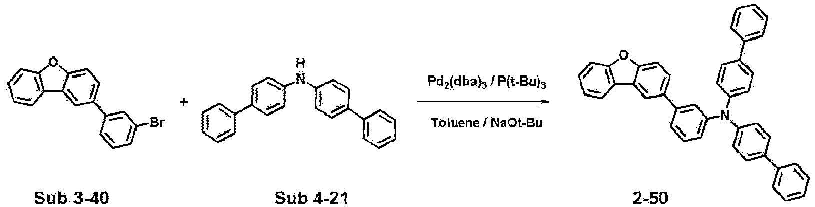

After Sub 3-40 (10 g, 30.94 mmol), Sub 4-21 (9.95 g, 30.94 mmol), Pd₂(dba)₃ (0.85 g, 0.93 mmol), P(t-Bu)₃ (6.26 g, 30.94 mmol), NaOt-Bu (5.95 g, 61.88 mmol) and toluene (309 mL) were placed in a round bottom flask, the reaction was carried out in the same manner as in the synthesis method of 2-1 to obtain 13.26 g (yield : 76%) of the product 2-50.

### Synthesis of 2-52

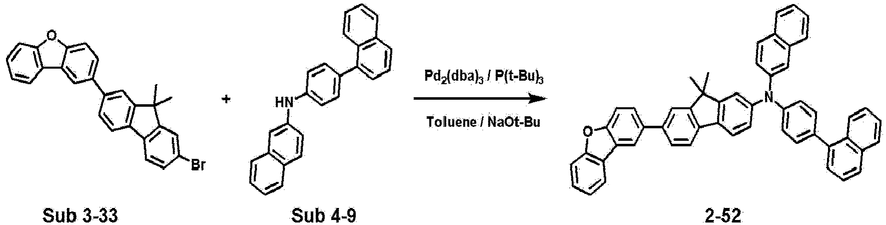

After Sub 3-33 (10 g, 22.76 mmol), Sub 4-9 (7.86 g, 22.76 mmol), Pd₂(dba)₃ (0.63 g, 0.68 mmol), P(t-Bu)₃ (4.60 g, 22.76 mmol), NaOt-Bu (4.38 g, 45.52 mmol) and toluene (228 mL) were placed in a round bottom flask, the reaction was carried out in the same manner as in the synthesis method of 2-1 to obtain 11.38 g (yield : 71%) of the product 2-52.

### Synthesis of 2-60

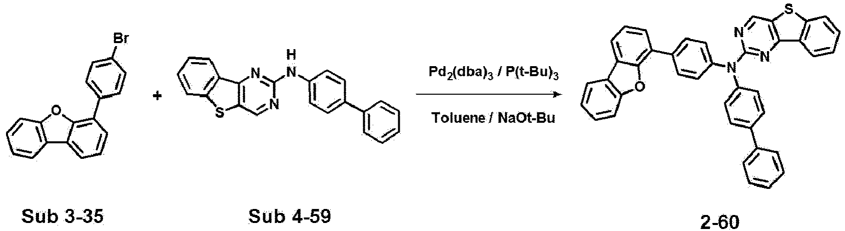

After Sub 3-35 (10 g, 30.94 mmol), Sub 4-59 (10.94 g, 30.94 mmol), Pd₂(dba)₃ (0.85 g, 0.93 mmol), P(t-Bu)₃ (6.26 g, 30.94 mmol), NaOt-Bu (5.95 g, 61.88 mmol) and toluene (309 mL) were placed in a round bottom flask, the reaction was carried out in the same manner as in the synthesis method of 2-1 to obtain 13.46 g (yield : 73%) of the product 2-60.

### Synthesis of 2-85

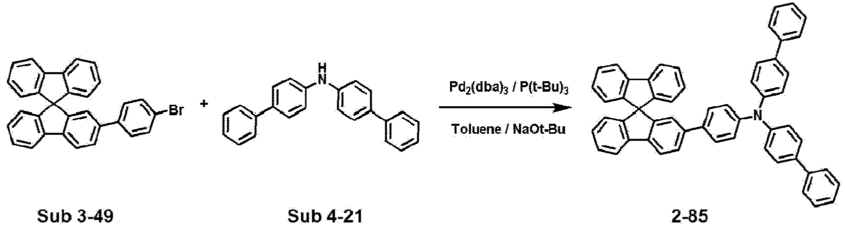

After Sub 3-49 (10 g, 21.21 mmol), Sub 4-21 (6.82 g, 21.21 mmol), Pd₂(dba)₃ (0.58 g, 0.64 mmol), P(t-Bu)₃ (4.29 g, 21.21 mmol), NaOt-Bu (4.08 g, 42.43 mmol) and toluene (212 mL) were placed in a round bottom flask, the reaction was carried out in the same manner as in the synthesis method of 2-1 to obtain 10.57 g (yield : 70%) of the product 2-85.

### Synthesis of 2-89

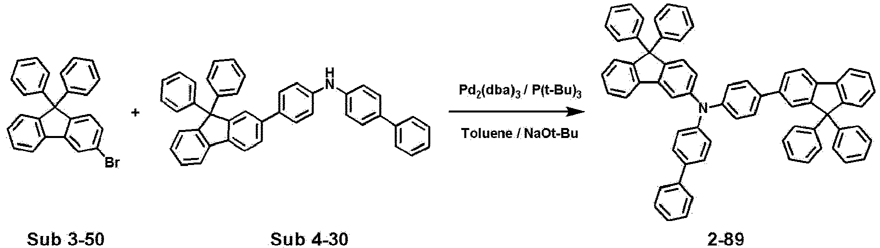

After Sub 3-50 (10 g, 25.17 mmol), Sub 4-30 (14.14 g, 25.17 mmol), Pd₂(dba)₃ (0.69 g, 0.76 mmol), P(t-Bu)₃ (5.09 g, 25.17 mmol), NaOt-Bu (4.84 g, 50.34 mmol) and toluene (252 mL) were placed in a round bottom flask, the reaction was carried out in the same manner as in the synthesis method of 2-1 to obtain 15.91 g (yield : 72%) of the product 2-89.

### Synthesis of 3-1

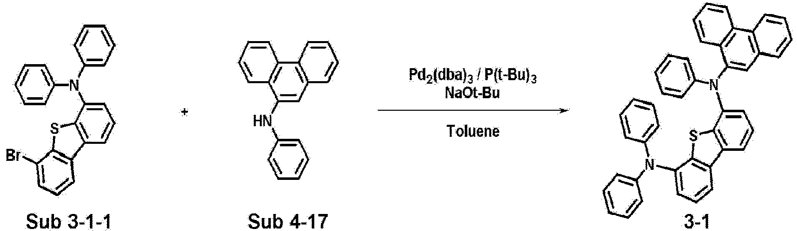

After dissolving Sub 3-3-1 (5.97 g, 13.87 mmol) in toluene (140ml), Sub 4-17 (3.74 g, 13.87 mmol), Pd₂(dba)₃ (0.38 g, 0.42 mmol), P(*t*-Bu)₃ (0.28 g, 1.39 mmol) and NaOt-Bu (2.67 g, 27.74 mmol) were added to the solution and the mixture was stirred at 100°C. When the reaction was completed, the reaction product was extracted with CH₂Cl₂ and water, and then the organic layer was dried with MgSO₄. Then, the concentrate was applied to silica gel column and recrystallized to obtain 6.09 g (yield: 71%) of the product.

### Synthesis of 3-2

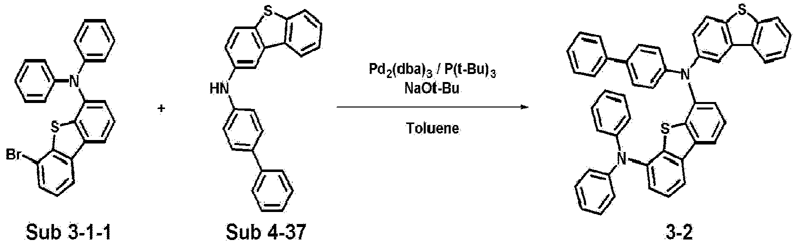

After adding Sub 4-37 (4.32 g, 12.29 mmol), Pd₂(dba)₃ (0.34 g, 0.37 mmol) P(t-Bu)₃ (0.25 g, 1.23 mmol), NaOt-Bu (2.36 g, 24.58 mmol) and toluene (125ml) to Sub 3-3-1 (5.29 g, 12.29 mmol), the reaction was carried out in the same manner as in the synthesis method of 3-1 to obtain 6.81 g (yield: 79%) of the product.

### Synthesis of 3-4

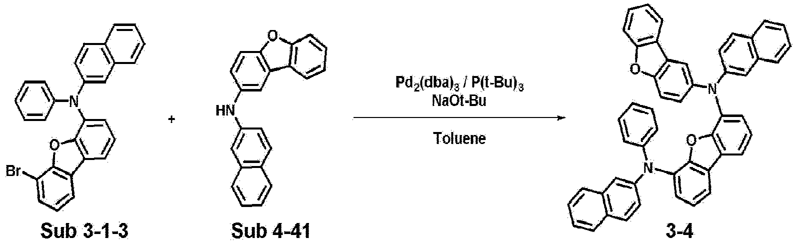

After adding Sub 4-41 (4.08 g, 13.20 mmol), Pd₂(dba)₃ (0.36 g, 0.40 mmol) P(t-Bu)₃ (0.27 g, 1.32 mmol), NaOt-Bu (2.54 g, 26.40 mmol) and toluene (130ml) to Sub 3-3-3 (6.13 g, 13.20 mmol), the reaction was carried out in the same manner as in the synthesis method of 3-1 to obtain 7.32 g (yield: 80%) of the product.

### Synthesis of 3-10

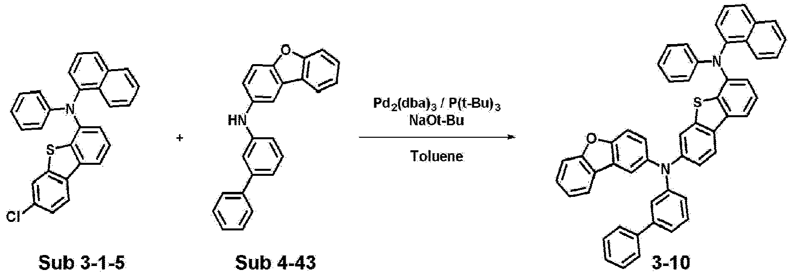

After adding Sub 4-43 (4.28 g, 12.75 mmol), Pd₂(dba)₃ (0.35 g, 0.38 mmol) P(*t-*Bu)₃ (0.26 g, 1.28 mmol), NaOt-Bu (2.45 g, 25.51 mmol) and toluene (130ml) to Sub 3-3-5 (5.56 g, 12.75 mmol), the reaction was carried out in the same manner as in the synthesis method of 3-1 to obtain 8.06 g (yield: 86%) of the product.

### Synthesis of 3-24

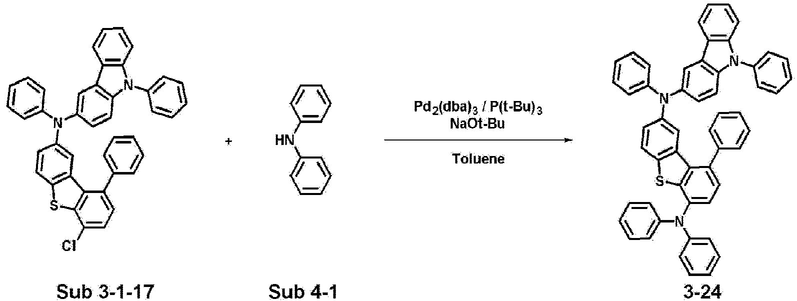

After adding Sub 4-1 (1.67 g, 9.85 mmol), Pd₂(dba)₃ (0.27 g, 0.30 mmol) P(*t*-Bu)₃ (0.20 g, 0.99 mmol), NaOt-Bu (1.89 g, 19.71 mmol) and toluene (100ml) to Sub 3-3-17 (6.18 g, 9.85 mmol), the reaction was carried out in the same manner as in the synthesis method of 3-1 to obtain 5.62 g (yield: 75%) of the product.

### Synthesis of 3-122

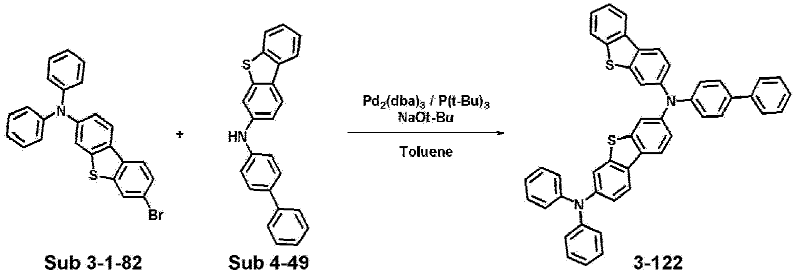

After adding Sub 4-49 (4.12 g, 11.73 mmol), Pd₂(dba)₃ (0.32 g, 0.35 mmol) P(*t-*Bu)₃ (0.24 g, 1.17 mmol), NaO*t*-Bu (2.26 g, 23.47 mmol) and toluene (120ml) to Sub 3-3-82 (5.05 g, 11.73 mmol), the reaction was carried out in the same manner as in the synthesis method of 3-1 to obtain 7.65 g (yield: 93%) of the product.

### Synthesis of 3-125

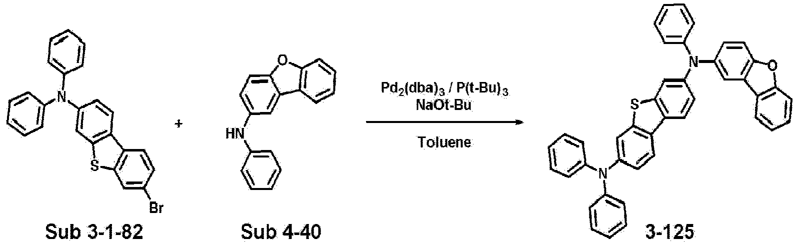

After adding Sub 4-40 (3.13 g, 12.08 mmol), Pd₂(dba)₃ (0.33 g, 0.36 mmol) P(*t-*Bu)₃ (0.24 g, 1.21 mmol), NaOt-Bu (2.32 g, 24.17 mmol) and toluene (120ml) to Sub 3-3-82 (5.20 g, 12.08 mmol), the reaction was carried out in the same manner as in the synthesis method of 3-1 to obtain 6.69 g (yield: 91%) of the product.

### Synthesis of 3-141

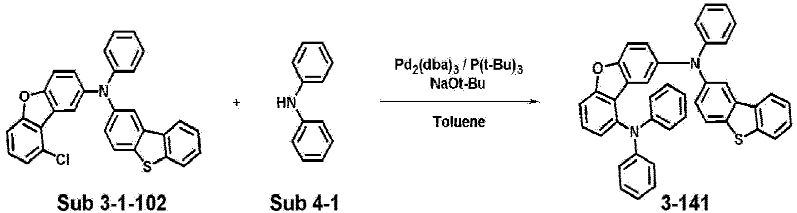

After adding Sub 4-1 (2.23 g, 13.15 mmol), Pd₂(dba)₃ (0.36 g, 0.39 mmol) P(*t*-Bu)₃ (0.27 g, 1.32 mmol), NaOt-Bu (2.53 g, 26.30 mmol) and toluene (130ml) to Sub 3-3-102 (6.26 g, 13.15 mmol), the reaction was carried out in the same manner as in the synthesis method of 3-1 to obtain 6.57 g (yield: 82%) of the product.

### Synthesis of 3-146

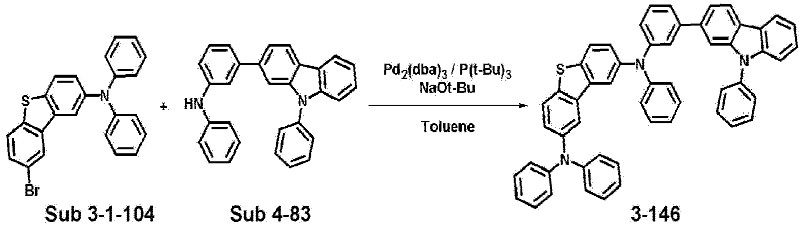

After adding Sub 4-83 (5.84 g, 14.22 mmol), Pd₂(dba)₃ (0.39 g, 0.43 mmol) P(*t-*Bu)₃ (0.29 g, 1.42 mmol), NaO*t*-Bu (2.73 g, 28.43 mmol) and toluene (140ml) to Sub 3-3-104 (6.12 g, 14.22 mmol), the reaction was carried out in the same manner as in the synthesis method of 3-1 to obtain 8.00 g (yield: 74%) of the product.

### Synthesis of 3-158

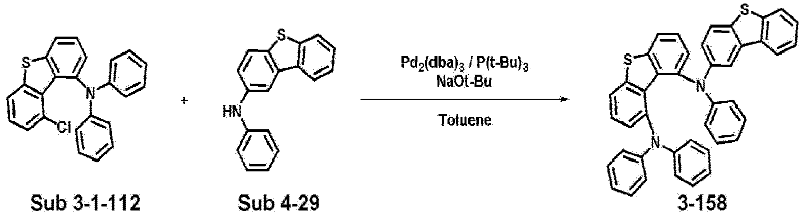

After adding Sub 4-29 (4.10 g, 14.90 mmol), Pd₂(dba)₃ (0.41 g, 0.45 mmol) P(*t-*Bu)₃ (0.30 g, 1.49 mmol), NaO*t*-Bu (2.86 g, 29.80 mmol) and toluene (150ml) to Sub 3-3-112 (5.75 g, 14.90 mmol), the reaction was carried out in the same manner as in the synthesis method of 3-1 to obtain 6.24 g (yield: 67%) of the product.

### Synthesis of 3-175

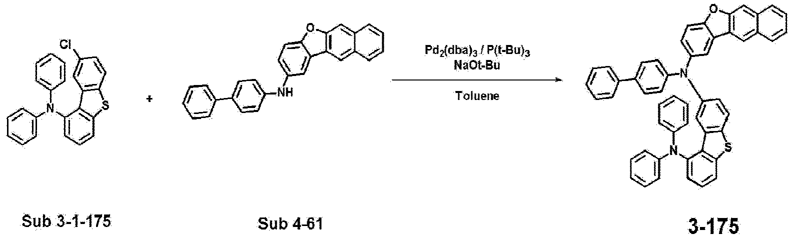

After adding Sub 4-61 (5.74 g, 14.90 mmol), Pd₂(dba)₃ (0.41 g, 0.45 mmol) P(*t-*Bu)₃ (0.30 g, 1.49 mmol), NaO*t*-Bu (2.86 g, 29.80 mmol) and toluene (150ml) to Sub 3-3-175 (5.75 g, 14.90 mmol), the reaction was carried out in the same manner as in the synthesis method of 3-1 to obtain 7.67 g (yield: 67%) of the product.

The FD-MS values of the compounds 2-1 to 2-96 and 3-1 to 3-175 prepared according to the above synthesis examples are shown in the following Tables 6 and 7, respectively.

**[Table 6]**

| Compound | FD-MS | Compound | FD-MS |
|---|---|---|---|
| 2-1 | m/z=473.21(C₃₄H₂₇N=473.61) | 2-2 | m/z=521.23(C₄₀H₂₉N=523.66) |
| 2-3 | m/z=573.25(C₁₄H₃₁N=573.72) | 2-4 | m/z=623.26(C₄₈H₃₃N=623.78) |
| 2-5 | m/z=447.20(C₃₄H₂₅N=447.57) | 2-6 | m/z=371.17(C₂₈H₂₁N=371.47) |
| 2.7 | m/z=471.20(C₃₅H₂₅N=471.59) | 2-8 | m/z=521.21(C₄₀H₂₇N=521.65) |
| 2-9 | m/z=549.25(C₄₂H₃₁N=549.70) | 2-10 | m/z=625.28(C₄₈H₃₅N=625.80) |
| 2-11 | m/z=675.29(C₅₂H₃₇N=675.86) | 2-12 | m/z=473.21(C₃₆H₂₇N=473.61) |
| 2-13 | m/z=523.23(C₄₀H₂₉N=523.66) | 2-14 | m/z=623.26(C₄₈H₃₃N=623.78) |
| 2-15 | m/z=549.25(C₄₂H₃₁N=549.70) | 2-16 | m/z=625.28(C₄₈H₃₅N=625.80) |
| 2-17 | m/z=503.17(C₃₆H₂₅NS=503.66) | 2-18 | m/z=603.20(C₄₄H₂₉NS=603.77) |
| 2-19 | m/z=477.16(C₃₄H₂₃NS=477.62) | 2-20 | m/z=503.17(C₃₆H₂₅NS=503.66) |
| 2-21 | m/z=451,14(C₃₂H₂₁NS=451.58) | 2-22 | m/z=593.22(C₄₃H₃₁NS=593.78) |
| 2-23 | m/z=641.22(C₄₇H₃₁NS=641.82) | 2-24 | m/z=665.22(C₄₉H₃₁NS=665.84) |
| 2-25 | m/z=503.17(C₃₆H₂₅NS=503.66) | 2-26 | m/z=655.23(C₄₈H₃₃NS=655.85) |
| 2-27 | m/z=695.26(C₅₁H₃₇NS=695.91) | 2-28 | m/z=593.18(C₄₂H₂₇NOS=593.73) |
| 2-29 | m/z=583.14(C₄₀H₂₅NS₂=583.76) | 2-30 | m/z=579.20(C₄₂H₂₉NS=579.75) |
| 2-31 | m/z=685.19(C₄₈H₃₁NS₂=685.90) | 2-32 | m/z=719.23(C₅₂H₃₃NOS=719.89) |
| 2-33 | m/z=629.22(C₄₆H₃₁NS=629.81) | 2-34 | m/z=629.22(C₄₆H₃₁NS=629.81) |
| 2-35 | m/z=603.20(C₄₄H₂₉NS=603.77) | 2-36 | m/z=563.08(C₃₆H₂₁NS₃=563.75) |
| 2-37 | m/z=639.11(C₄₂H₂₅NS₃=639.85) | 2-38 | m/z=715.15(C₄₆H₂₉NS₃=715.95) |
| 2-39 | m/z=791.18(C₅₄H₃₃NS₃=792.04) | 2-40 | m/z=607.16(C₄₂H₂₅NO₂S=607.72) |
| 2-41 | m/z=633.21(C₄₅H₃₁NOS=633.80) | 2-42 | m/z=733.24(C₅₃H₃₅NOS=733.92) |
| 2-43 | m/z=883.29(C₆₅H₄₁NOS=884.09) | 2-44 | m/z=585.13(C₃₇H₂₃N₃S₂=585.74) |
| 2-45 | m/z=553.19(C₄₀H₂₇NS=553.71) | 2-46 | m/z=603.20(C₄₀H₂₉NS=603.77) |
| 2-47 | m/z=841.28(C₆₃H₃₉NS=842.06) | 2-48 | m/z=567.17(C₄₀H₂₅NOS=567.71) |
| 2-49 | m/z=563.22(C₄₂H₂₉NO=563.69) | 2-50 | m/z=563.22(C₂H₂₉NO=563.69) |
| 2-51 | m/z=613.24(C₄₆H₃₁NO=613.74) | 2-52 | m/z=703.29(C₅₃H₃₇NO=703.87) |
| 2-53 | m/z=587.22(C₄₄H₂₉NO=587.71) | 2-54 | m/z=639.26(C₄₆H₃₃NO=639.78) |
| 2-55 | m/z=639.26(C₄₈H₃₃NO=639.78) | 2-56 | m/z=653.24(C₄₈H₃₁NO₂=653.77) |
| 2-57 | m/z=603.26(C₄₅H₃₃NO=603.75) | 2-58 | m/z=727.29(C₅₅H₃₇NO=727.89) |
| 2-59 | m/z=725.27(C₅₅H₃₅NO=725.87) | 2-60 | m/z=595.17(C₄₀H₂₅N₃OS=595.71) |
| 2-61 | m/z=567.26(C₄₂H₃₃NO=567.72) | 2-62 | m/z=611.22(C₄₆H₂₉NO=611.73) |
| 2-63 | m/z=617.18(C₄₄H₂₇NOS=617.76) | 2-64 | m/z=637.24(C₄₈H₃₁NO=637.77) |
| 2-65 | m/z=667.21(C₄₈H₂₉NO₃=667.75) | 2-66 | m/z=767.25(C₅₆H₃₃NO₃=767.87) |
| 2-67 | m/z=681.27(C₅₀H₃₅NO₂=681.82) | 2-68 | m/z=567.17(C₄₀H₂₅NOS=567.71 |
| 2-69 | m/z=658.22(C₄₅H₃₀N₄S=658.82) | 2-70 | m/z=655.23(C₄₈H₃₃NS=655.86) |
| 2-71 | m/z=744.26(C₅₄H₃₆N₂S=744.96) | 2-72 | m/z=784.27(C₅₅H₃₆N₄S=784.98) |
| 2-73 | m/z=553.19(C₄₀H₂₇NS=553.72) | 2-74 | m/z=553.19(C₄₀H₂₇NS=553.72) |
| 2-75 | m/z=543.2(C₃₉H₂₉NS=543.73) | 2-76 | m/z=671.21(C₄₈H₃₀FNS=671.83) |
| 2-77 | m/z=641.25(C₄₆H₃₁N₃O=641.77) | 2-78 | m/z=639.26(C₄₈H₃₃NO=639.8) |
| 2-79 | m/z=652.25(C₄₈H₃₂N₂O=652.8) | 2-80 | m/z=667.25(C₄₉H₃₃NO₂=667.81) |
| 2-81 | m/z=713.31(C₅₅H₃₉N=713.90) | 2-82 | m/z=589.28(C₄₅H₃₅N=589.77) |
| 2-83 | m/z=639.29(C₄₉H₃₇N=639.82) | 2-84 | m/z=613.28(C₄₇H₃₅N=613.79) |
| 2-85 | m/z=711.29(C₅₆H₃₇N=711.89) | 2-86 | m/z=637.28(C₄₉H₃₅N=637.81) |
| 2-87 | m/z=761.31(C₅₉H₃₉N=761.95) | 2-88 | m/z=637.28(C₄₉H₃₅N=637.81) |
| 2-89 | m/z=877.37(C₆₈H₄₇N=878.11) | 2-90 | m/z=875.36(C₆₈H₄₅N=876.09) |
| 2-91 | m/z=813.30(C₆₂H₃₉NO=813.98) | 2-92 | m/z=651.26(C₄₉H₃₃NO=651.81) |
| 2-93 | m/z=591.2(C₄₃H₂₉NS=591.77) | 2-94 | m/z=601.28(C₄₆H₃₅N=601.79) |
| 2-95 | m/z=677.31(C₅₂H₃₉N=677.89) | 2-96 | m/z=777.3(C₅₉H₃₉NO=777.97) |
| 2-97 | m/z=647.36(C₄₉H₄₅N=647.91) | | |

**[Table 7]**

| Com. | FD-MS | Com. | FD-MS |
|---|---|---|---|
| 3-1 | m/z=618.21(C₄₄H₃₀N₂S=618.80) | 3-2 | m/z=700.20(C₄₈H₃₂N₂S₂=700.92) |
| 3-3 | m/z=708.22(C₅₀H₃₂N₂OS=708.88) | 3-4 | m/z=692.25(C₅₀H₃₂N₂O₂=692.82) |
| 3-5 | m/z=742.30(C₅₅H₃₈N₂O=742.92) | 3-6 | m/z-654.27(C₄₈H₃₄N₂O=654.81) |
| 3-7 | m/z=700.20(C₄₈H₃₂N₂S₂=700.92) | 3-8 | m/z=760.25(C₅₄H₃₆N₂OS=760.96) |
| 3-9 | m/z=730.16(C₄₈H₃₀N₂S₃=750.96) | 3-10 | m/z=734.24(C₅₂H₃₄N₂OS=734.92) |
| 3-11 | m/z=872.32(C₆₄H₄₄N₂S=873.13) | 3-12 | m/z=718.26(C₅₂H₃₄N₃O₂=718.86) |
| 3-13 | m/z=568.20(C₄₀H₂₈N₂S=568.74) | 3-14 | m/z=624.17(C₄₂H₂₈N₂S₂=624.82) |
| 3-15 | m/z=658.21(C₄₆H₃₀N₂OS=658.82) | 3-16 | m/z=730.30(C₅₄H₃₈N₂O=730.91) |
| 3-17 | m/z=698.20(C₄₈H₃₀N₂O₂S=698.84) | 3-18 | m/z=624.17(C₄₂H₂₈N₂S₂=624.82) |
| 3-19 | m/z=700.20(C₄₈H₃₂N₂S₂=700.92) | 3-20 | m/z=708.22(C₅₀H₃₂N₂OS=708.88) |
| 3-21 | m/z=750.22(C₅₂H₃₄N₂S₂=750.98) | 3-22 | m/z=776.23(C₅₄H₃₆N₂S₂=777.02) |
| 3-23 | m/z=867.24(C₃₉H₃₇N₃OS₂=868.09) | 3-24 | m/z=759.27(C₅₄H₃₇N₃S=759.97) |
| 3-25 | m/z=608.19(C₄₂N₂₈OS=608.76) | 3-26 | m/z=608.19(C₄₂H₂₈N₂OS=608.76) |
| 3-21 | m/z=692.25(C₅₀H₃₂N₂O₂=692.82) | 3-28 | m/z=894.20(C₆₀H₃₄N₂O₃S₂=895.06) |
| 3-29 | m/z=618.21(C₄₄H₃₀N₂S=618.80) | 3-30 | m/z=700.20(C₄₈H₃₂N₂S₂=700.92) |
| 3-31 | m/z=780.17(C₅₂H₃₂N₂S₃=781.02) | 3-32 | m/z=734.24(C₅₂H₃₄N₂OS=734.92) |
| 3-33 | m/z=834.31(C₆₁H₄₂N₂S=835.08) | 3-34 | m/z=700.20(G₄₈H₃₂N₂S₂=700.92) |
| 3-35 | m/z=724.5(C₅₁H₃₆N₂OS=724.92) | 3-36 | m/z=624.17(C₄₂H₂₈N₂S₂=624.82) |
| 3-37 | m/z=700.20(C₄₈H₃₂N₂S₂=700.92) | 3-38 | m/z=700.20(G₄₈H₃₂N₂S₂=700.92) |
| 3-39 | m/z=674.19(C₄₆H₃₀N₂S₂=674.88) | 3-40 | m/z=700.20(C₄₈H₃₂N₂S₂=700.92) |
| 3-41 | m/z=608.19(C₄₂H₂₈N₂OS=608.76) | 3-42 | m/z=684.22(C₄₈H₃₂N₂OS=684.86) |
| 3-43 | m/z=684.22(C₄₈H₃₂N₂OS=684.86) | 3-44 | m/z=624.17(C₄₂H₂₈N₂S₂=624.82) |
| 3-45 | m/z=674.19(C₄₆H₃₀N₂S₂=674.88) | 3-46 | m/z=700.20(C₄₈H₃₂N₂S₂=700.92) |
| 3-47 | m/z=826.25(C₅₈H₃₈N₂S₂=827.08) | 3-48 | m/z=608.19(C₄₂H₂₈N₂OS=608.76) |
| 3-49 | m/z=624.17(C₄₂H₂₈N₂S₂=624.82) | 3-50 | m/z=684.22(C₄₈H₃₂N₂OS=684.86) |
| 3-51 | m/z=700.20(C₄₈H₃₂N₂S₂=700.92) | 3-52 | m/z=684.22(C₄₈H₃₂N₂OS=684.86) |
| 3-53 | m/z=730.16(C₄₈H₃₀N₂S₃=730.96) | 3-54 | m/z=826.25(C₅₈H₃₈N₂S₂=827.08) |
| 3-55 | m/z=806.19(C₅₄H₃₄N₂S₃=807.06) | 3-56 | m/z=674.19(C₄₆H₃₀N₂S₂=674.88) |
| 3-57 | m/z=674.19(C₄₆H₃₀N₂S₂=674.88) | 3-58 | m/z=674.19(C₄₆H₃₀N₂S₂=674.88) |
| 3-59 | m/z=674.19(C₄₆H₂₀N₂S₂=674.88) | 3.60 | m/z=674.19(C₄₆H₃₀N₂S₂=674.88) |
| 3-61 | m/z=638.19(C₄₃H₃₀N₂S₂=638.85) | 3-62 | m/z=638.19(C₄₃H₃₀N₂S₂=638.85) |
| 3-63 | m/z=638.19(C₄₃H₃₀N₂S₂=638.85) | 3-64 | m/z=622.21(C₄₃H₃₀N₂OS=622.79) |
| 3-65 | m/z=688.20(C₄₇H₃₂N₂S₂=688.91) | 3-66 | m/z=700.20(C₄₈H₃₂N₂S₂=700.92) |
| 3-67 | m/z=789.23(C₅₄H₃₅N₃S₂=790.02) | 3-68 | m/z=638.19(C₄₃H₃₀N₂S₂=638.85) |
| 3-69 | m/z=638.19(C₄₃H₃₀N₂S₂=638.85) | 3-70 | m/z=642.16(C₄₂H₂₇FN₂S₂=642.81) |
| 3-71 | m/z=638.19(C₄₃H₃₀N₂S₂=638.85) | 3-72 | m/z=714.22(C₄₉H₃₄N₂S₂=714.95) |
| 3-73 | m/z=716.20(C₄₈H₃₂N₂OS₂=716.92) | 3-74 | m/z=670.24(C₄₈H₃₄N₂S=670.87) |
| 3-75 | m/z=634.24(C₄₅H₃₄N₂S=634.84) | 3-76 | m/z=759.27(C₅₄H₃₇N₃S=759.97) |
| 3-77 | m/z=674.19(C₄₆H₃₀N₂S₂=674.88) | 3-78 | m/z=708.22(C₅₀H₃₂N₂OS=708.88) |
| 3-79 | m/z=674.19(C₄₆H₃₀N₃S₂=674.88) | 3-80 | m/z=748.22(C₅₂H₃₂N₂O₂S=748.90) |
| 3-81 | m/z=821.29(C₅₇H₃₅D₅N₂S₂=822.11) | 3-82 | m/z=730.16(C₄₈H₃₀N₂S₃=730.96) |
| 3-83 | m/z=730.16(C₄₈H₃₀N₂S₃=730.96) | 3-84 | m/z=714.18(C₄₈H₃₀N₂OS₂=714.90) |
| 3-85 | m/z=882.22(C₆₀H₃₈N₂S₃=883.16) | 3-86 | m/z=624.17(C₄₂H₂₈N₂S₂=624.82) |
| 3-87 | m/z=700.20(C₄₈H₃₂N₂S₂=700.92) | 3-88 | m/z=674.19(C₄₆H₃₀N₂S₂=674.88) |
| 3-89 | m/z=674.19(C₄₆H₃₀N₂S₂=674.88) | 3-90 | m/z=708.22(C₅₀H₃₂N₂OS=708.88) |
| 3-91 | m/z=759.27(C₅₄H₃₇N₃S=759.97) | 3-92 | m/z=674.19(C₄₆H₃₀N₂S₂=674.88) |
| 3-93 | m/z=688.20(C₄₇H₃₂N₂S₂=688.91) | 3-94 | m/z=760.25(C₅₄H₃₆N₂OS=760.96) |
| 3-95 | m/z=759.27(C₅₄H₃₇N₃S=759.97) | 3-96 | m/z=608.19(C₄₂H₂₈N₂OS=608.76) |
| 3-97 | m/z=684.22(C₄₈H₃₂N₂OS=684.86) | 3-98 | m/z=622.21(C₄₃H₃₀N₂OS=622.79) |
| 3-99 | m/z=760.25(C₅₄H₃₈N₂OS=760.96) | 3-100 | m/z=658.21(C₄₆H₃₀N₂OS=658.82) |
| 3-101 | m/z=658.21(C₄₆H₃₀N₂OS=658.82) | 3-102 | m/z=692.25(C₅₀H₃₂N₂O₂=692.82) |
| 3-103 | m/z=628.25(C₄₆H₃₂N₂O=628.78) | 3-104 | m/z=693.28(C₅₀H₃₅N₃O=693.85) |
| 3-105 | m/z=723.23(C₅₀H₃₃N₃OS=723.89) | 3-106 | m/z=749.29(C₅₄H₃₁D₅N₂S=749.99) |
| 3-107 | m/z=700.20(C₄₈H₃₂N₂S₂=700.92) | 3-108 | m/z=674.19(C₄₈H₃₀N₂S₂=674.88) |
| 3-109 | m/z=684.22(C₄₈H₃₂N₂OS=684.86) | 3-110 | m/z=724.25(C₅₁H₃₆N₂OS=724.92) |
| 3-111 | m/z=674.19(C₄₆H₃₀N₂S₂=674.88) | 3-112 | m/z=674.19(C₄₆H₃₀N₂S₂=674.88) |
| 3-113 | m/z=780.17(C₅₂H₃₃N₂S₃=781.02) | 3-114 | m/z=684.22(C₄₈H₃₂N₂OS=684.86) |
| 3-115 | m/z=684.22(C₄₈H₃₂N₂OS=684.86) | 3-116 | m/z=698.20(C₄₈H₃₀N₂O₂S=698.84) |
| 3-117 | m/z=618.26(C₄₂H₁₈D₁₀N₂OS=618.82) | 3-118 | m/z=757.27(C₅₄H₃₅N₃O₂=757.89) |
| 3-119 | m/z=668.25(C₄₈H₃₂N₂O₂=668.80) | 3-120 | m/z=720.26(C₅₂H₃₆N₂S=720.93) |
| 3-121 | m/z=670.24(C₄₈H₃₄N₂S=670.87) | 3-122 | m/z=700.20(C₄₈H₃₂N₂S₂=700.92) |
| 3-123 | m/z=674.19(C₄₆H₃₀N₂S₂=674.88) | 3-124 | m/z=700.20(C₄₈H₃₂N₂S₂=700.92) |
| 3-125 | m/z=608.19(C₄₂H₂₈N₂OS=608.76) | 3-126 | m/z=860.25(C₅₈H₄₀N₂O₂S₂=861.09) |
| 3-127 | m/z=798.27(C₅₇H₃₈N₂OS=799.00) | 3-128 | m/z=734.33(C₅₄H₄₂N₂O=734.94) |
| 3-129 | m/z=742.26(C₅₄H₃₄N₂O₂=742.88) | 3-130 | m/z=945.37(C₇₀H₄₇N₃O=946.17) |
| 3-131 | m/z=670.24(C₄₈H₃₄N₂S=670.87) | 3-132 | m/z=624.17(C₄₂H₂₈N₂S₂=624.82) |
| 3-133 | m/z=700.20(C₄₈H₃₂N₂S₂=700.92) | 3-134 | m/z=724.20(C₅₀H₃₂N₂S₂=724.94) |
| 3-135 | m/z=816.26(C₅₇H₄₀N₂S₂=817.08) | 3-136 | m/z=760.25(C₅₄H₃₆N₂OS=760.96) |
| 3-137 | m/z=674.19(C₄₆H₃₀N₂S₂=674.88) | 3-138 | m/z=730.16(C₄₈H₃₀N₂S₃=730.96) |
| 3-139 | m/z=881.29(C₆₁H₄₃N₃S₂=882.16) | 3-140 | m/z=704.28(C₅₂H₃₆N₂O=704.87) |
| 3-141 | m/z=608.19(C₄₂H₂₈N₂OS=608.76) | 3-142 | m/z=682.23(C₄₈H₃₀N₂O₃=682.78) |
| 3-143 | m/z=670.24(C₄₈H₃₄N₂S=670.87) | 3-144 | m/z=776.23(C₅₄H₃₆N₂S₂=777.02) |
| 3-145 | m/z=674.19(C₄₆H₃₀N₂S₂=674.88) | 3-146 | m/z=759.27(C₅₄H₃₇N₃S=759.97) |
| 3-147 | m/z=758.24(C₅₄H₃₄N₂OS=758.94) | 3-148 | m/z=806.28(C₅₉H₃₈N₂S=807.03) |
| 3-149 | m/z=823.30(C₅₉H₃₈FN₃O=823.97) | 3-150 | m/z=743.29(C₅₄H₃₇N₃O=743.91) |
| 3-151 | m/z=998.33(C₇₃H₄₆N₂OS=999.24) | 3-152 | m/z=700.20(C₄₈H₃₂N₂5₂=700.92) |
| 3-153 | m/z=674.19(C₄₆H₃₀N₂S₂=674.88) | 3-154 | m/z=674.19(C₄₆H₃₀N₂S₂=674.88) |
| 3-155 | m/z=674.19(C₄₆H₃₀N₂S₂=674.88) | 3-156 | m/z=674.19(C₄₆H₃₀N₂S₂=674.88) |
| 3-157 | m/z=762.29(C₅₀H₄₆N₂SSi₂=763.16) | 3-158 | m/z=624.17(C₄₂H₂₈N₂S₂=624.82) |
| 3-159 | m/z=784.25(C₅₆H₃₆N₂OS=784.98) | 3-160 | m/z=810.27(C₅₈H₃₈N₂OS=811.02) |
| 3-161 | m/z=860.29(C₆₂H₄₀N₂OS=861.08) | 3-162 | m/z=708.22(C₅₀H₃₂N₂OS=708.88) |
| 3-163 | m/z=742.26(C₅₄H₃₄N₂O₂=742.88) | 3-164 | m/z=828.26(C₅₈H₄₀N₂S₂=829.09) |
| 3-165 | m/z=708.22(C₅₀H₃₂N₂OS=708.88) | 3-166 | m/z=724.20(C₅₀H₃₂N₂S₂=724.94) |
| 3-167 | m/z=834.27(C₆₀H₃₈N₂OS=835.04) | 3-168 | m/z=768.28(C₅₆H₃₆N₂O₂=768.92) |
| 3-169 | m/z=830.19(C₅₆H₃₄N₂S₃=831.08) | 3-170 | m/z=810.27(C₅₈H₃₈N₂OS=811.02) |
| 3-171 | m/z=810.31(C₅₉H₄₂N₂S=811.06) | 3-172 | m/z=692.25(C₅₀H₃₂N₂O₂=692.82) |
| 3-173 | m/z=758.24(C₅₄H₃₄N₂OS=758.94) | 3-174 | m/z=708.22(C₅₀H₃₂N₃OS=708.88) |
| 3-175 | m/z=732.24(C₅₂H₃₄N₂OS=734.92) | | |

### Fabrication and Evaluation of Organic Electronic Element

### [Example 1] to [Example 102] Red OLED (Mixed phosphorescent host of a light emitting layer

After vacuum depositing 4,4',4"-tris[2-naphthyl(phenyl)amino]triphenylamine (abbreviated as "2-TNATA") film on an ITO layer(anode) to form a hole injection layer having a thickness of 60nm, wherein the ITO layer was formed on a glass substrate, 4,4'-bis[*N*-(1-napthyl)-*N*-phenylamino]biphenyl (abbreviated as "NPD") film was vacuum-deposited with a thickness of 55 nm on the hole injection layer to form a hole transport layer.

Next, a light emitting layer with a thickness of 30 nm was formed on the hole transport layer. As shown in Table 8, a mixture of a compound represented by Formula 1 of the present invention (host 1) and a compound represented by Formula 2 of the present invention (host 2) in a weight ratio of 3: 7 was used as a host and bis-(1-phenylisoquinolyl)iridium(III) acetylacetonate (abbreviated as "(piq)₂Ir (acac)") was used as a dopant, where the host and dopant were used in a 95: 5 weight ratio.

Next, a film of (1,1'-biphenyl-4-olato)bis(2-methyl-8-quinolinolato)aluminum (abbreviated as "BAlq") was vacuum-deposited with a thickness of 10 nm on the light emitting layer to form a hole blocking layer.

Subsequently, after mixing tris-(8-hydroxyquinoline)aluminum (abbreviated as "Alq₃") and bis(10-hydroxybenzo[h]quinolinato)beryllium (abbreviated as "BeBq₂") in 1: 1, the mixture was deposited on the hole blocking layer to form an electron transport layer having a thickness of 45 nm
Next, LiF on the electron transport layer was deposited to a thickness of 0.2 nm and then Al was deposited to a thickness of 150 nm to form a cathode. In this way, the OLED was completed.

### [Comparative Example 1] to [Comparative Example 4]

The OLEDs were fabricated in the same manner as described in Example 1 except that a single compound represented by Formula 1 of the present invention as listed in the following Table 8 was used as a host of a light emitting layer.

### [Comparative Example 5] and [Comparative Example 6]

The OLEDs were fabricated in the same manner as described in Example 1 except that the the mixture of Comparative compounds 1 and 2, the mixture of Comparative compounds 1 and 3 as listed in the following Table 8 were uses as a host of a light emitting layer, respectively.

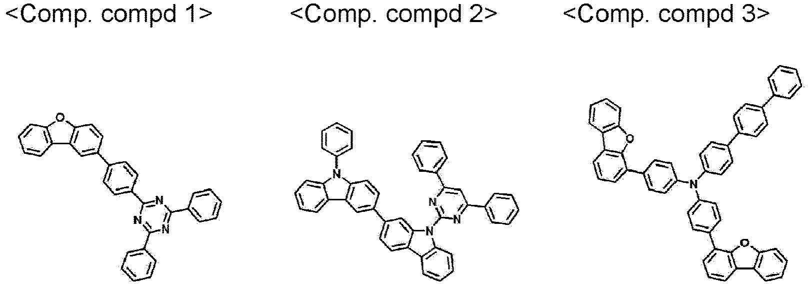

Electroluminescence (EL) characteristics were measured with a PR-650 (Photoresearch) by applying a forward bias DC voltage to the OLEDs prepared in Examples 1 to 102 of the present invention and Comparative Examples 1 to 6. And, the T95 life time was measured using a life time measuring apparatus manufactured by Macscience Inc. at reference brightness of 2500 cd/m². The measurement results are shown in Tables 8 below.

**[Table 8]**

| | Host 1 | Host 2 | Voltage (V) | Current Density (mA/cm²) | Brightness (cd/m²) | Efficiency (cd/A) | Lifetime T(95) |
|---|---|---|---|---|---|---|---|
| comp.Ex(1) | Com. 1-61 | | 6.5 | 20.8 | 2500 | 12.0 | 92.5 |
| comp.Ex(2) | Com. 1-92 | | 5.9 | 13.6 | 2500 | 18.4 | 121.2 |
| comp.Ex(3) | Com. 1-145 | | 6.4 | 18.9 | 2500 | 13.2 | 95.4 |
| comp.Ex(4) | Com. 1-160 | | 6.0 | 14.6 | 2500 | 17.1 | 110.4 |
| comp.Ex(5) | ref 1 | ref 2 | 5.8 | 12.3 | 2500 | 20.3 | 123.4 |
| comp.Ex(6) | ref 1 | ref 3 | 5.7 | 12.1 | 2500 | 20.6 | 125.0 |
| Ex.(1) | Com. 1-61 | Com. 2-4 | 4.8 | 9.1 | 2500 | 27.5 | 142.0 |
| Ex.(2) | Com. 1-91 | | 4.9 | 8.5 | 2500 | 29.3 | 142.7 |
| Ex.(3) | Com. 1-92 | | 4.9 | 8.5 | 2500 | 29.5 | 142.9 |
| Ex.(4) | Com. 1-103 | | 4.9 | 8.6 | 2500 | 29.1 | 142.3 |
| Ex.(5) | Com. 1-121 | | 4.8 | 8.9 | 2500 | 28.1 | 143.4 |
| Ex.(6) | Com. 1-122 | | 4.9 | 9.0 | 2500 | 27.8 | 143.0 |
| Ex.(7) | Com. 1-145 | | 4.8 | 9.1 | 2500 | 27.6 | 142.1 |
| Ex.(8) | Com. 1-148 | | 4.9 | 8.4 | 2500 | 29.6 | 142.4 |
| Ex.(9) | Com. 1-149 | | 4.9 | 8.4 | 2500 | 29.9 | 142.6 |
| Ex.(10) | Com. 1-151 | | 4.8 | 8.6 | 2500 | 29.1 | 142.3 |
| Ex.(11) | Com. 1-158 | | 4.9 | 8.8 | 2500 | 28.3 | 143.3 |
| Ex.(12) | Com. 1-160 | | 4.9 | 8.7 | 2500 | 28.6 | 143.6 |
| Ex.(13) | Com. 1-172 | | 4.9 | 8.9 | 2500 | 28.0 | 143.1 |
| Ex.(14) | Com. 1-173 | | 4.8 | 8.7 | 2500 | 28.7 | 143.7 |
| Ex.(15) | Com. 1-174 | | 4.8 | 8.7 | 2500 | 28.9 | 144.0 |
| Ex.(16) | Com. 1-61 | Com. 2-45 | 4.8 | 8.3 | 2500 | 30.0 | 143.8 |
| Ex.(17) | Com. 1-91 | | 4.9 | 7.9 | 2500 | 31.8 | 144.5 |
| Ex.(18) | Com. 1-92 | | 4.8 | 7.8 | 2500 | 32.1 | 144.7 |
| Ex.(19) | Com. 1-103 | | 4.8 | 7.9 | 2500 | 31.7 | 144.0 |
| Ex.(20) | Com. 1-121 | | 4.9 | 8.1 | 2500 | 30.8 | 145.3 |
| Ex.(21) | Com. 1-122 | | 4.8 | 8.2 | 2500 | 30.5 | 144.9 |
| Ex.(22) | Com. 1-145 | | 4.9 | 8.3 | 2500 | 30.2 | 143.9 |
| Ex.(23) | Com. 1-148 | | 4.8 | 7.7 | 2500 | 32.3 | 144.2 |
| Ex.(24) | Com. 1-149 | | 4.8 | 7.7 | 2500 | 32.4 | 144.3 |
| Ex.(25) | Com. 1-151 | | 4.8 | 7.9 | 2500 | 31.5 | 143.9 |
| Ex.(26) | Com. 1-158 | | 4.8 | 8.1 | 2500 | 30.9 | 145.2 |
| Ex.(27) | Com. 1-160 | | 4.9 | 8.0 | 2500 | 31.2 | 145.5 |
| Ex.(28) | Com. 1-172 | | 4.9 | 8.1 | 2500 | 30.8 | 145.0 |
| Ex.(29) | Com. 1-173 | | 4.8 | 8.0 | 2500 | 31.3 | 145.7 |
| Ex.(30) | Com. 1-174 | | 4.9 | 7.9 | 2500 | 31.5 | 145.8 |
| Ex.(31) | Com. 1-61 | Com. 2-48 | 4.7 | 6.7 | 2500 | 37.5 | 149.2 |
| Ex.(32) | Com. 1-91 | | 4.6 | 6.4 | 2500 | 39.2 | 150.1 |
| Ex.(33) | Com. 1-92 | | 4.7 | 6.3 | 2500 | 39.4 | 150.3 |
| Ex.(34) | Com. 1-103 | | 4.7 | 6.4 | 2500 | 39.1 | 149.7 |
| Ex.(35) | Com. 1-121 | | 4.7 | 6.6 | 2500 | 38.1 | 150.9 |
| Ex.(36) | Com. 1-122 | | 4.7 | 6.6 | 2500 | 37.8 | 150.4 |
| Ex.(37) | Com. 1-145 | | 4.7 | 6.6 | 2500 | 37.7 | 149.4 |
| Ex.(38) | Com. 1-148 | | 4.6 | 6.3 | 2500 | 39.6 | 149.9 |
| Ex.(39) | Com. 1-149 | | 4.7 | 6.3 | 2500 | 39.9 | 150.0 |
| Ex.(40) | Com. 1-151 | | 4.6 | 6.4 | 2500 | 39.0 | 149.5 |
| Ex.(41) | Com. 1-158 | | 4.6 | 6.5 | 2500 | 38.3 | 150.8 |
| Ex.(42) | Com. 1-160 | | 4.6 | 6.5 | 2500 | 38.5 | 150.9 |
| Ex.(43) | Com. 1-172 | | 4.6 | 6.6 | 2500 | 38.0 | 150.6 |
| Ex.(44) | Com. 1-173 | | 4.7 | 6.5 | 2500 | 38.7 | 151.0 |
| Ex.(45) | Com. 1-174 | | 4.7 | 6.4 | 2500 | 38.8 | 151.2 |
| Ex.(46) | Com. 1-61 | Com. 2-49 | 4.6 | 7.7 | 2500 | 32.5 | 145.6 |
| Ex.(47) | Com. 1-91 | | 4.7 | 7.3 | 2500 | 34.3 | 146.5 |
| Ex.(48) | Com. 1-92 | | 4.7 | 7.2 | 2500 | 34.5 | 146.7 |
| Ex.(49) | Com. 1-103 | | 4.7 | 7.3 | 2500 | 34.2 | 146.1 |
| Ex.(50) | Com. 1-121 | | 4.6 | 7.6 | 2500 | 33.1 | 147.3 |
| Ex.(51) | Com. 1-122 | | 4.6 | 7.6 | 2500 | 32.8 | 146.8 |
| Ex.(52) | Com. 1-145 | | 4.7 | 7.6 | 2500 | 32.7 | 145.8 |
| Ex.(53) | Com. 1-148 | | 4.6 | 7.2 | 2500 | 34.6 | 146.2 |
| Ex.(54) | Com. 1-149 | | 4.7 | 7.2 | 2500 | 34.9 | 146.4 |
| Ex.(55) | Com. 1-151 | | 4.7 | 7.4 | 2500 | 34.0 | 145.9 |
| Ex.(56) | Com. 1-158 | | 4.7 | 7.5 | 2500 | 33.3 | 147.1 |
| Ex.(57) | Com. 1-160 | | 4.6 | 7.5 | 2500 | 33.4 | 147.3 |
| Ex.(58) | Com. 1-172 | | 4.6 | 7.6 | 2500 | 33.0 | 147.0 |
| Ex.(59) | Com. 1-173 | | 4.7 | 7.5 | 2500 | 33.5 | 147.4 |
| Ex.(60) | Com. 1-174 | | 4.7 | 7.4 | 2500 | 33.8 | 147.6 |
| Ex.(61) | Com. 1-61 | Com. 2-57 | 4.7 | 7.1 | 2500 | 35.0 | 147.4 |
| Ex.(62) | Com. 1-91 | | 4.8 | 6.8 | 2500 | 36.9 | 148.2 |
| Ex.(63) | Com. 1-92 | | 4.7 | 6.8 | 2500 | 37.0 | 148.4 |
| Ex.(64) | Com. 1-103 | | 4.8 | 6.8 | 2500 | 36.8 | 147.8 |
| Ex.(65) | Com. 1-121 | | 4.7 | 7.0 | 2500 | 35.8 | 149.0 |
| Ex.(66) | Com. 1-122 | | 4.7 | 7.0 | 2500 | 35.5 | 148.5 |
| Ex.(67) | Com. 1-145 | | 4.8 | 7.1 | 2500 | 35.2 | 147.5 |
| Ex.(68) | Com. 1-148 | | 4.8 | 6.7 | 2500 | 37.2 | 147.9 |
| Ex.(69) | Com. 1-149 | | 4.7 | 6.7 | 2500 | 37.4 | 148.0 |
| Ex.(70) | Com. 1-151 | | 4.8 | 6.8 | 2500 | 36.5 | 147.6 |
| Ex.(71) | Com. 1-158 | | 4.8 | 7.0 | 2500 | 35.8 | 148.8 |
| Ex.(72) | Com. 1-160 | | 4.8 | 7.0 | 2500 | 35.9 | 149.0 |
| Ex.(73) | Com. 1-172 | | 4.8 | 7.0 | 2500 | 35.7 | 148.7 |
| Ex.(74) | Com. 1-173 | | 4.8 | 6.9 | 2500 | 36.1 | 149.1 |
| Ex.(75) | Com. 1-174 | | 4.7 | 6.9 | 2500 | 36.3 | 149.4 |
| Ex.(76) | Com. 1-61 | Com. 2-68 | 4.7 | 6.3 | 2500 | 39.7 | 151.0 |
| Ex.(77) | Com. 1-91 | | 4.6 | 6.0 | 2500 | 41.5 | 151.9 |
| Ex.(78) | Com. 1-92 | | 4.6 | 6.0 | 2500 | 41.7 | 152.1 |
| Ex.(19) | Com. 1-103 | | 4.7 | 6.0 | 2500 | 41.4 | 151.6 |
| Ex.(80) | Com. 1-121 | | 4.7 | 6.2 | 2500 | 40.5 | 152.5 |
| Ex.(81) | Com. 1-122 | | 4.7 | 6.2 | 2500 | 40.1 | 152.2 |
| Ex.(82) | Com. 1-145 | | 4.6 | 6.3 | 2500 | 39.9 | 151.1 |
| Ex.(83) | Com. 1-148 | | 4.6 | 6.0 | 2500 | 41.8 | 151.7 |
| Ex.(84) | Com. 1-149 | | 4.6 | 6.0 | 2500 | 42.0 | 151.9 |
| Ex.(85) | Com. 1-151 | | 4.7 | 6.1 | 2500 | 41.3 | 151.4 |
| Ex.(86) | Com. 1-158 | | 4.6 | 6.2 | 2500 | 40.5 | 152.5 |
| Ex.(87) | Com. 1-160 | | 4.6 | 6.1 | 2500 | 40.8 | 152.6 |
| Ex.(88) | Com. 1-172 | | 4.7 | 6.2 | 2500 | 40.3 | 152.4 |
| Ex.(89) | Com. 1-173 | | 4.6 | 6.1 | 2500 | 41.0 | 152.7 |
| Ex.(90) | Com. 1-174 | | 4.6 | 6.1 | 2500 | 41.1 | 153.0 |
| Ex.(91) | Com. (2-101) | Com.(3-36) | 4.4 | 5.7 | 2500.0 | 43.6 | 156.7 |
| Ex.(92) | Com. (2-107) | | 4.5 | 5.7 | 2500.0 | 43.7 | 155.8 |
| Ex.(93) | Com. (2-109) | | 4.5 | 5.8 | 2500.0 | 43.0 | 155.9 |
| Ex.(94) | Com. (2-121) | | 4.4 | 5.7 | 2500.0 | 43.7 | 155.2 |
| Ex.(95) | Com. (2-101) | Com.(3-174) | 4.1 | 5.4 | 2500.0 | 46.0 | 158.8 |
| Ex.(96) | Com. (2-107) | | 3.7 | 5.1 | 2500.0 | 49.1 | 158.9 |
| Ex.(97) | Com. (2-109) | | 4.1 | 5.4 | 2500.0 | 45.9 | 159.6 |
| Ex.(98) | Com. (2-121) | | 4.1 | 5.4 | 2500.0 | 46.1 | 159.3 |
| Ex.(99) | Com. (2-101) | Com.(3-175) | 4.0 | 5.3 | 2500.0 | 47.0 | 159.3 |
| Ex.(100) | Com. (2-107) | | 4.1 | 5.5 | 2500.0 | 45.2 | 159.8 |
| Ex.(101) | Com. (2-109) | | 3.9 | 5.2 | 2500.0 | 48.3 | 162.4 |
| Ex.(102) | Com. (2-121) | | 4.1 | 5.5 | 2500.0 | 45.7 | 158.6 |

From Table 8, it can be seen that the driving voltage, efficiency and lifetime of the element are remarkably improved when the mixture of the compound represented by Formula 1 and the compound represented by Formula 2 of the present invention is used as a phosphorescent host (Examples 1 to 90), compared with the case of using the compound represented by the formula 1 alone (Comparative Examples 1 to 4) or using a mixture of comparative compounds (Comparative Examples 5 to 6).

Comparing Comparative Examples 1 to 6, the characteristics of the element are further improved when the mixture of two kinds of comparative compounds is used (Comparative Examples 5 to 6) than when the compound of the present invention is used alone (Comparative Examples 1 to 4).

In addition, comparing Comparative Examples 5-6 and Examples of the present invention, the driving voltage is lower and the efficiency and lifetime are remarkably improved when the mixture of the compound represented by Formula 1 and the compound represented by Formula 2 of the present invention is used as a host, compared with the case of using the mixture of two kinds of comparative compounds.

From these results, the inventors of the present invention determined that a mixture of the mixture of the compound represented by Formula 1 and the compound represented by Formula 2 had new properties other than those of each compound, and thus, PL lifetime of each of the compounds and the mixture was measured, respectively. As a result, in the case of the mixture of the compound represented by Formula 1 and the compound represented by Formula 2 of the present invention, it was confirmed that a new PL wavelength was formed unlike the case of a single compound.

This is because when the mixture of the compounds of the present invention is used, not only electrons and holes are moved according to the energy levels of the respective materials, but also electrons and holes are moved or energy is transferred according to a novel region with a new energy level formed by mixing and thus the efficiency and lifetime is increased. This is an important example of showing the energy transfer and the emitting process by exciplex of the mixed thin film when the mixture of the compounds of the present invention are used.

In addition, when the polycyclic compound represented by the formula 1 with the high stability for holes as well as electrons and the compound represented by the formula 2 with the strong hole characteristics are mixed, the electrons blocking ability is improved and more holes move quickly and easily into the emitting layer due to the high T1 and LUMO energy values. As a result, as the charge balance increases in the light emitting layer, light is emitted well inside the light emitting layer, not at the hole transport layer interface, and this also reduces the degradation of the HTL interface, thereby the driving voltage, efficiency and lifetime of the element are maximized. That is, when the mixture of the compounds represented by Formulas 1 and 2 is used as a host, the performance of the entire device is greatly improved by electrochemical synergy.

### [Example 103] to [Example 106]

An organic electroluminescent element was manufactured in the same manner as in Example 1 except that host 1 and host 2 were mixed at a predetermined ratio as described in Table 8.

Electroluminescence (EL) characteristics were measured with a PR-650 (Photoresearch) by applying a forward bias DC voltage to the OLEDs prepared in Examples 103 to 106. And, the T95 life time was measured using a life time measuring apparatus manufactured by Macscience Inc. at reference brightness of 2500 cd/m². The measurement results are shown in Tables 9 below. Examples 3 and 80 are the case where the mixture of host 1 and host 2 in a ration of 3: 7 like Table 8 is used as host.

**[Table 9]**

| | **Host 1** | **Host 2** | **Mixing ration (Host 1: Host 2)** | **Voltage (V)** | **Current Density (mA/cm²)** | **Brightness (cd/m²)** | **Efficiency (cd/A)** | **Lifetime T(95)** |
|---|---|---|---|---|---|---|---|---|
| **Ex.(103)** | **1-92** | **2-4** | **7:3** | **5.1** | **8.9** | **2500** | **28.2** | **142.1** |
| **Ex.(104)** | | | **5:5** | **4.9** | **8.6** | **2500** | **29.1** | **142.6** |
| **Ex.(3)** | | | **3:7** | **4.9** | **8.5** | **2500** | **29.5** | **142.9** |
| **Ex.(105)** | **1-121** | **2-68** | **7:3** | **5.0** | **7.2** | **2500** | **34.6** | **151.8** |
| **Ex.(106)** | | | **5:5** | **4.8** | **6.5** | **2500** | **38.4** | **152.2** |
| **Ex.(80)** | | | **3:7** | **4.7** | **6.2** | **2500** | **40.5** | **152.5** |

Referring to Table 9 above, it can be seen when the first host and the second host are mixed at 3: 7, the driving voltage, efficiency, and lifespan are excellent, and as the ratio of the first host increases, the characteristics of the element decrease. This is because the charge balance in the light emitting layer is maximized as the amount of the compound represented by the formula 2 having a stronger hole transport property than the compound represented by the formula 1 is increased.

### [Example 107] to [Example 118] Red OLED

After vacuum depositing 2-TNATA film on an ITO layer(anode) to form a hole injection layer having a thickness of 60nm, wherein the ITO layer was formed on a glass substrate, NPD film was vacuum-deposited with a thickness of 60 nm on the hole injection layer to form a hole transport layer.

Next, a light emitting layer with a thickness of 30 nm was formed on the hole transport layer. As shown in Table 10, the compound represented by Formula 3 of the present invention was used as a host and (piq)₂Ir (acac) was used as a dopant, and dopant was doped so that their weight ratio was 95: 5.

Next, BAIq was vacuum-deposited with a thickness of 10 nm on the light emitting layer to form a hole blocking layer. Subsequently, after mixing Alq₃ was deposited on the hole blocking layer to form an electron transport layer having a thickness of 40 nm.

Next, LiF on the electron transport layer was deposited to a thickness of 0.2 nm and then Al was deposited to a thickness of 150 nm to form a cathode.

### [Comparative Example 7]

The OLED was fabricated in the same manner as described in Example 107 except that the Comparative compound 4 was used as host of a light emitting layer instead of the compound of the present invention.

### <Comparative compound 4>

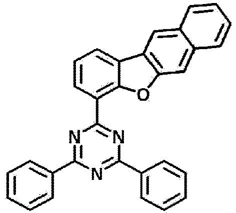

Electroluminescence (EL) characteristics were measured with a PR-650 (Photoresearch) by applying a forward bias DC voltage to the OLEDs prepared in Examples 107 to 118 of the present invention and Comparative Example 7. And, the T95 life time was measured using a life time measuring apparatus manufactured by Macscience Inc. at reference brightness of 2500 cd/m². The measurement results are shown in Tables10 below.

**[Table 10]**

| | **Compound** | **Voltage (V)** | **Current Density (mA/cm²)** | **Brightness (cd/m²)** | **Efficiency (cd/A)** | **Lifetime T(95)** | **CIE** | |
|---|---|---|---|---|---|---|---|---|
| | | | | | | | **x** | **y** |
| **comp.Ex(7)** | **comp.Com 4** | **6.3** | **15.3** | **2500.0** | **16.3** | **100.2** | **0.62** | **0.34** |
| **Ex.(107)** | **2-101** | **5.4** | **12.3** | **2500.0** | **20.4** | **110.6** | **0.63** | **0.32** |
| **Ex.(108)** | **2-102** | **5.2** | **10.4** | **2500.0** | **24.1** | **117.0** | **0.62** | **0.33** |
| **Ex.(109)** | **2-103** | **5.1** | **10.0** | **2500.0** | **25.0** | **116.8** | **0.60** | **0.34** |
| **Ex.(110)** | **2-104** | **5.4** | **11.3** | **2500.0** | **22.1** | **125.0** | **0.61** | **0.32** |
| **Ex.(111)** | **2-105** | **5.4** | **11.2** | **2500.0** | **22.3** | **120.2** | **0.64** | **0.33** |
| **Ex.(112)** | **2-106** | **5.4** | **11.3** | **2500.0** | **22.2** | **120.5** | **0.65** | **0.32** |
| **Ex.(113)** | **2-107** | **5.2** | **10.1** | **2500.0** | **24.8** | **115.4** | **0.61** | **0.34** |
| **Ex.(114)** | **2-108** | **5.2** | **10.4** | **2500.0** | **24.0** | **119.4** | **0.62** | **0.31** |
| **Ex.(115)** | **2-109** | **5.3** | **11.2** | **2500.0** | **22.4** | **121.0** | **0.61** | **0.33** |
| **Ex.(116)** | **2-110** | **5.5** | **11.3** | **2500.0** | **22.0** | **121.1** | **0.62** | **0.33** |
| **Ex.(117)** | **2-111** | **5.1** | **10.3** | **2500.0** | **24.3** | **115.9** | **0.63** | **0.31** |
| **Ex.(118)** | **2-112** | **5.2** | **10.3** | **2500.0** | **24.2** | **115.5** | **0.62** | **0.33** |

As can be seen in Table 10, the organic electroluminescent element using the compound of the present invention as a phosphorescent host has improved the driving voltage, efficiency and lifetime compared to the case of using the comparative compound 4.

Comparative compound 4 and the compound of the present invention is different in the bonding position of the triazine substituent bonded to naphthobenzofuran.

When comprehensively judging the device data of Table 10, the driving voltage, efficiency and lifetime of the element are greatly improved when using a compound of the present invention as a host material rather than the comparative compound 4, wherein in the case of Comparative Compound 4, the triazine substituent is substituted at position 4 of naphthobenzofuran, and in the case of the present invention, it is substituted at position 1 of naphthobenzofuran. In particular, when the compound of the present invention comprising a substituent containing naphthyl in the triazine substituent moiety is used as a host material, the driving voltage and efficiency are greatly improved, and when the compound having a biphenyl as a substituent is used, the lifetime is greatly improved.

The above results can be seen to be due to the torsion angle. Table 11 is a photograph taken from various aspects of the twist angle of 4-naphthobenzofuran such as Comparative Compound 4 and 1-naphthobenzofuran derivative such as Compound 2-109 of the present invention.

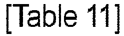

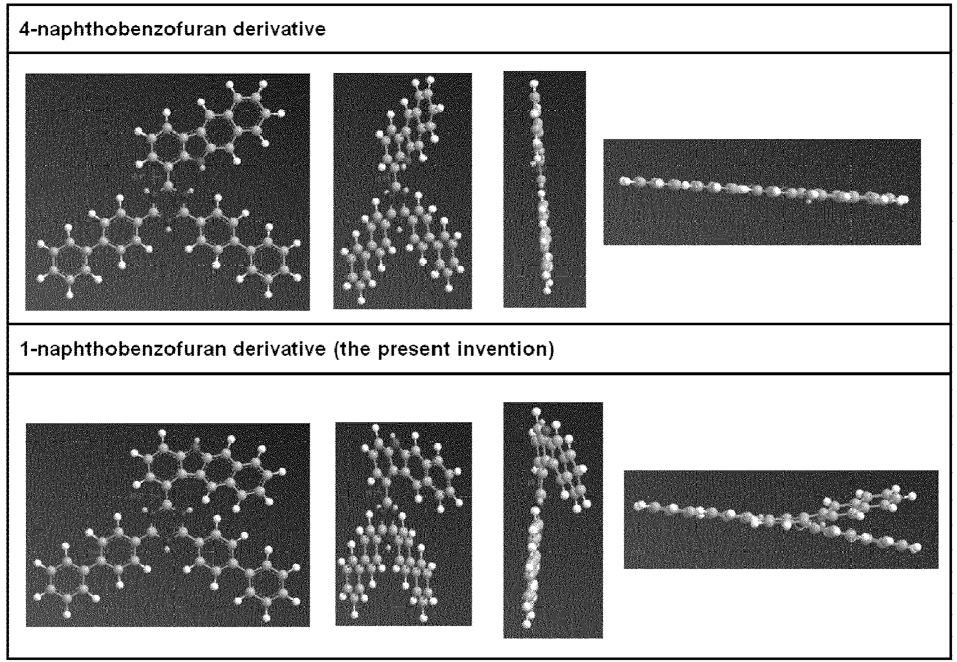

In Table 11, each drawing shows the image of a three-dimensional structure of Comparative Compound 4 and the compound 2-109 of the present invention in front, horizontal axis 60°, horizontal axis 90°, vertical axis 90° using the Chem3D program MM2 minimize energy method of Perkin Elmer, respectively.

Referring to 11, 4-naphthobenzofuran derivatives having a torsional angle of 0.9° with triazine, such as Comparative Compound 4, are nearly torsionless and have very high planarity. On the other hand, 1-naphthobenzofuran derivatives such as the present invention have a planarity that is significantly different from that of Comparative Compound 4 since 1-naphthobenzofuran derivatives are more twisted with triazine, wherein a torsion angle is10.2°.

Therefore, the compound of the present invention which is a relatively steric structure can reduce the degree of electrical polarization by weakening the intermolecular van der Waals force and lower the crystallinity of the thin film by weakening the intermolecular interaction. Moreover, when used as a light emitting layer material, this steric property can cause a certain distance from the electrochemically unstable dopant, which can ultimately increase the stability of the light emitting layer.

These comprehensive characteristics appear to improve the lifetime and characteristics of the element when the material is deposited.

Although exemplary embodiments of the present invention have been described for illustrative purposes, those skilled in the art will appreciate that various modifications, additions and substitutions are possible, without departing from the scope of the invention as disclosed in the accompanying claims. Therefore, the embodiment disclosed in the present invention is intended to illustrate the scope of the technical idea of the present invention, and the scope of the present invention is not limited by the embodiment. The scope of the present invention shall be construed on the basis of the accompanying claims, and it shall be construed that all of the technical ideas included within the scope equivalent to the claims belong to the present invention.

The invention is summarized by the following items:
1. An organic electric element comprising a first electrode, a second electrode, and an organic material layer formed between the first electrode and the second electrode, wherein the organic material layer comprises a phosphorescent light emitting layer, and the host of the phosphorescent light emitting layer comprises a first compound represented by the following Formula 1 and a second compound represented by the following Formula 2: 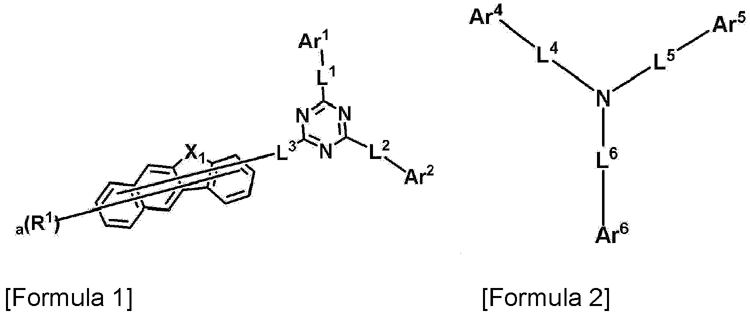 wherein,
   X₁ is O or S,
   Ar¹, Ar², and Ar⁴ to Ar⁶ are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₃₀ alkyl group, a C₂-C₃₀ alkenyl group, a C₂-C₃₀ alkynyl group, a C₁-C₃₀ alkoxyl group and a C₆-C₃₀ aryloxy group,
   L¹ to L⁶ are each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
   R¹ is selected from the group consisting of hydrogen, deuterium, halogen, a cyano group, a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₅₀ alkyl group, a C₂-C₂₀ alkenyl group, a C₂-C₂₀ alkynyl group, a C₁-C₃₀ alkoxyl group, a C₆-C₃₀ aryloxy group and -L'-N(Rₐ)(R_{b}), and adjacent R¹s may optionally be linked to each other to form a ring,
   a is an integer of 0-9, and where a is an integer of 2 or more, each of a plurality of R¹ is the same as or different from each other,
   L' is selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
   Rₐ and R_{b} are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, and a combination thereof,
   Ar¹~Ar², L¹~L⁶, R¹, L', Rₐ, R_{b}, and the ring formed by bonding adjacent R¹s to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group, and
   Ar⁴~Ar⁶ may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group, a C₈-C₂₀ arylalkenyl group and -L'-N(Rₐ)(R_{b}).
2. The organic electric element of item 1, wherein L¹ to L⁶ are each independently represented by one of the following Formulas b-1 to b-13: 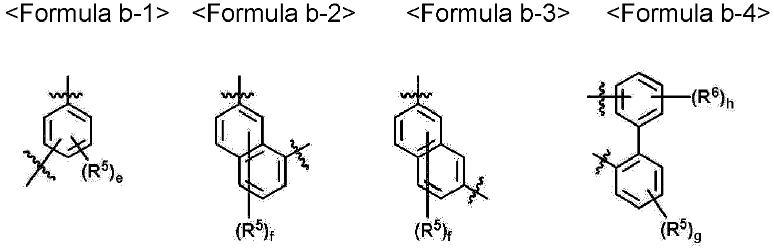 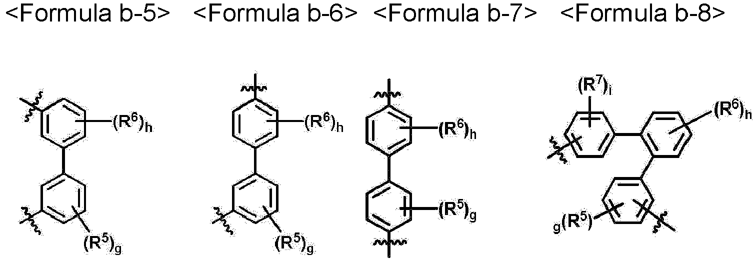 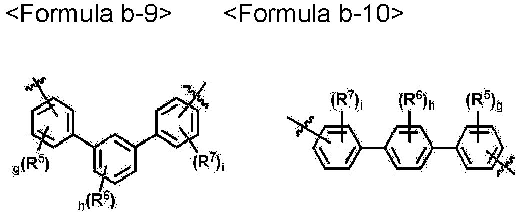 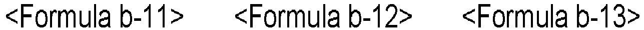 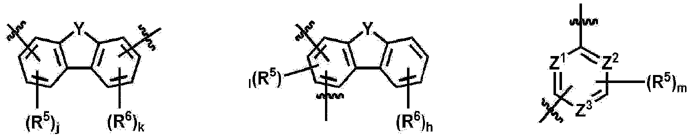 wherein,
   R⁵ to R⁷ are each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₂₀ aryloxy group,
   Y is N-(L^{a}-Ar^{a}), O, S or C(R')(R"),
   Z¹ to Z³ are each independently C, C(R') or N, and at least one of Z¹ to Z³ is N,
   f is an integer of 0-6, e, g, h and i are each an integer of 0-4, j and k are each an integer of 0-3, I is an integer of 0-2, m is an integer of 0-3, and where they are an integer of 2 or more, respectively, each of a plurality of R⁵, each of a plurality of R⁶, and each of a plurality of R⁷ are the same as or different from each other,
   R' and R" are each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, a C₆-C₃₀ aryloxy group, and -L^{a}-N(R^{a})(R^{b}),
   R' and R" of C(R')(R") may optionally be linked to each other to form a ring, and adjacent R's of C(R') may optionally be linked to each other to form a ring,
   Ar^{a} is selected from the group consisting of a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and a combination thereof,
   L^{a} is selected from the group consisting of a single bond, a C₆-C₂₀ arylene group, a fluorenylene group, a C₂-C₂₀ heterocyclic group comprising at least one heteroatom selected from the group consisting of O, N, S, Si and P, a C₃-C₂₀ aliphatic ring, and a combination thereof,
   R^{a} and R^{b} are each independently selected from the group consisting of a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and a combination thereof.
3. The organic electric element of any of items 1 or 2, wherein Formula 1 is represented by one of the following Formula 1-A to Formula 1-G: 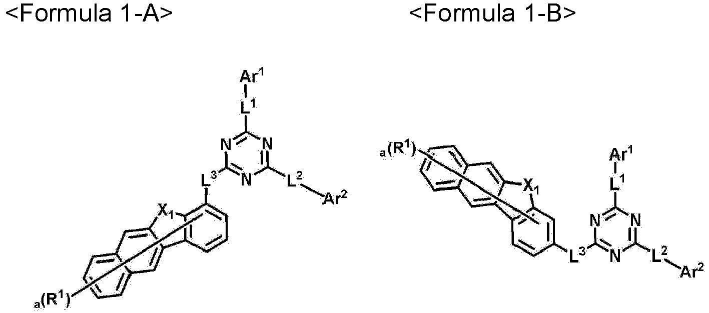 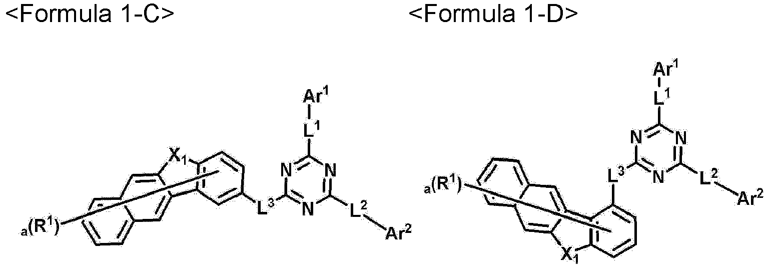 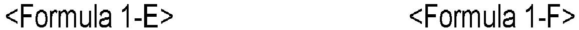 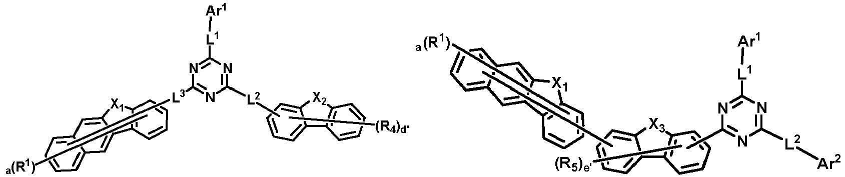 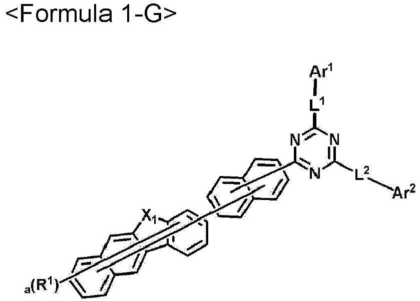 wherein, Ar¹, Ar², L¹~L³, X₁, R¹ and a are the same as defined in item 1,
   X₂ and X₃ are each independently O or S,
   R₄ and R₅ are each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₃₀ aryloxy group, and adjacent groups may optionally be linked to each other to form a ring,
   d' is an integer of 0-7, e' is an integer of 0-6, and where they are an integer of 2 or more, respectively, each of a plurality of R₄ and each of a plurality of R₅ are the same as or different from each other.
4. The organic electric element of any of items 1 to 3, wherein Formula 2 is represented by one of the following Formula 2-A to Formula 2-L: 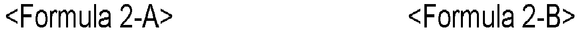 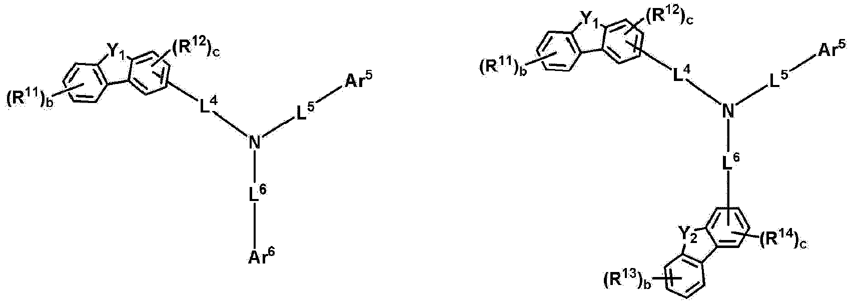 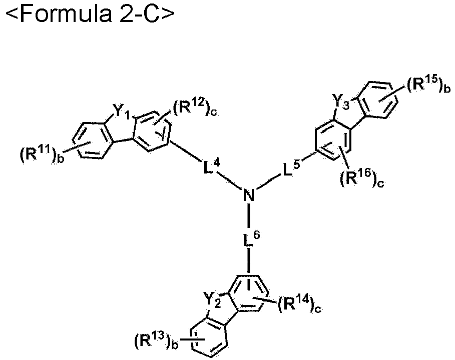 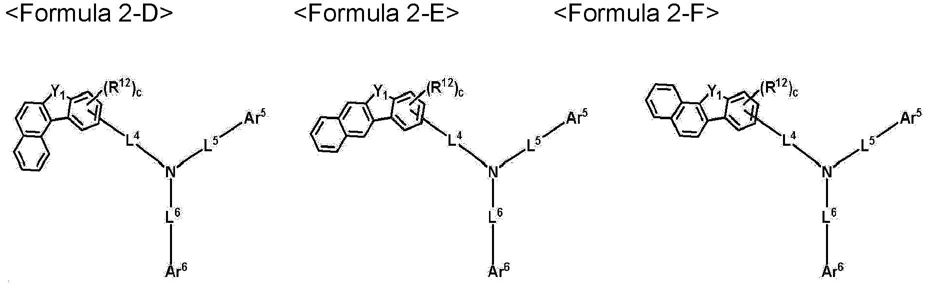 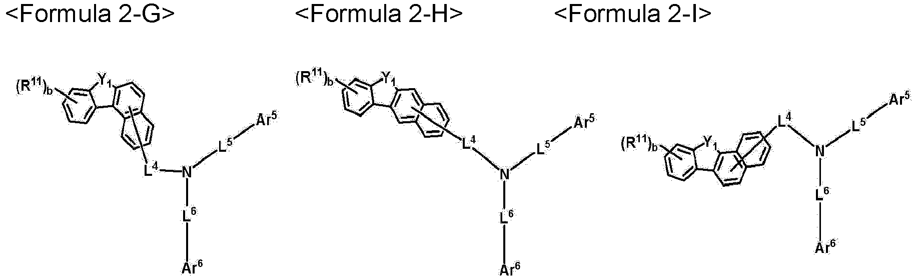 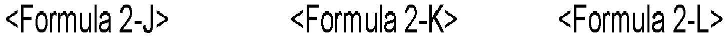 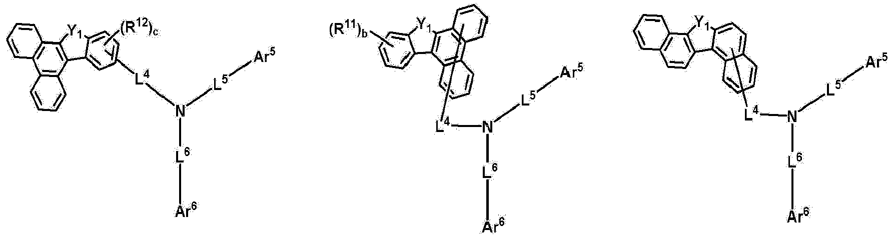 wherein, Ar⁵, Ar⁶, and L⁴~L⁶ are the same as defined in item 1,
   Y₁~Y₃ are each independently O, S, or C(R')(R"),
   R¹¹~R¹⁶, R' and R" are each independently selected from the group consisting of hydrogen, deuterium, halogen, a silane group substituted or unsubstituted with C₁-C₂₀ alkyl group or C₆-C₂₀ aryl group, a cyano group, a nitro group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and -L'-N(Rₐ)(R_{b}), and adjacent groups may optionally be linked to each other to form a ring,
   b is an integer of 0-4, and where b is an integer of 2 or more, each of a plurality of R¹¹, each of a plurality of R¹³, and each of a plurality of R¹⁵ are the same as or different from each other,
   c is an integer of 0-3, and where c is an integer of 2 or more, each of a plurality of R¹², each of a plurality of R¹⁴, and each of a plurality of R¹⁶ are the same as or different from each other,
   L', Rₐ and R_{b} are the same as defined in item 1.
5. The organic electric element of any of items 1 to 4, wherein the compound represented by Formula 1 is one of the following compounds: 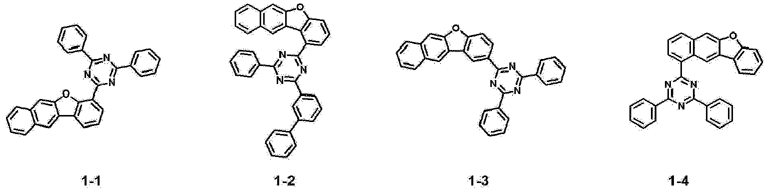 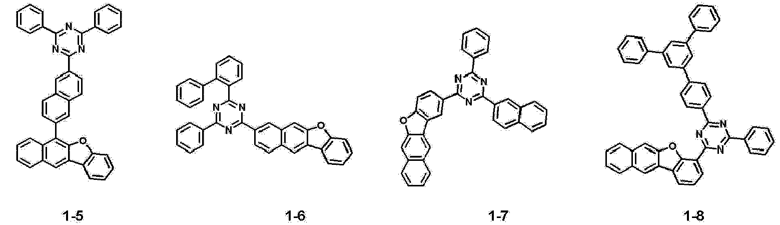 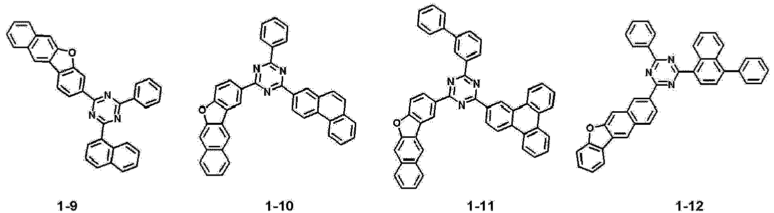 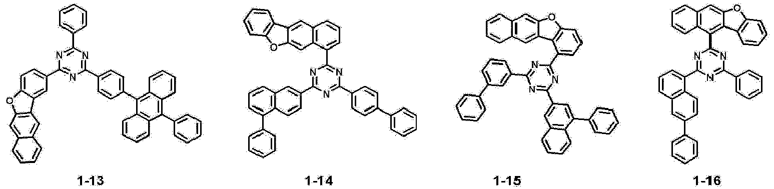 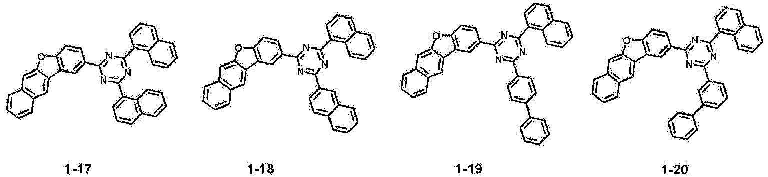 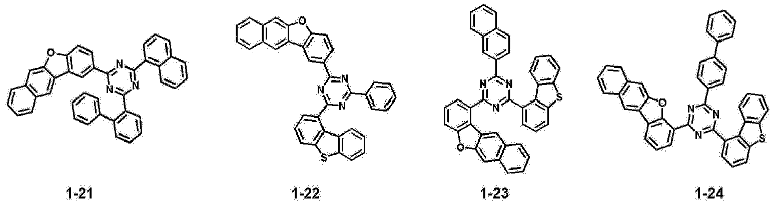 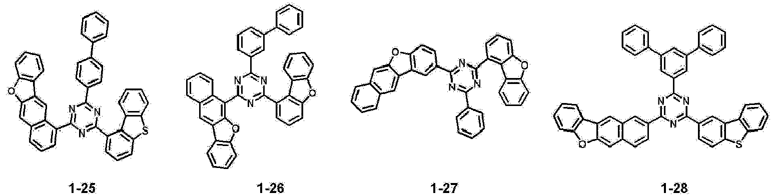 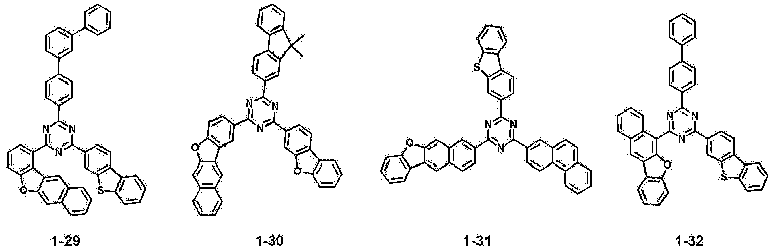 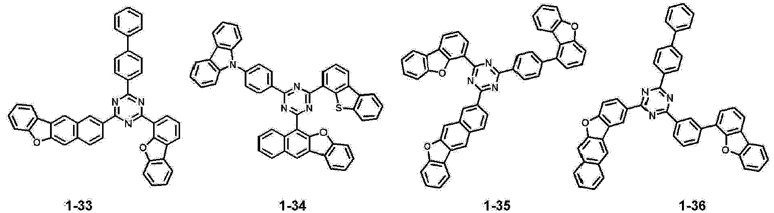 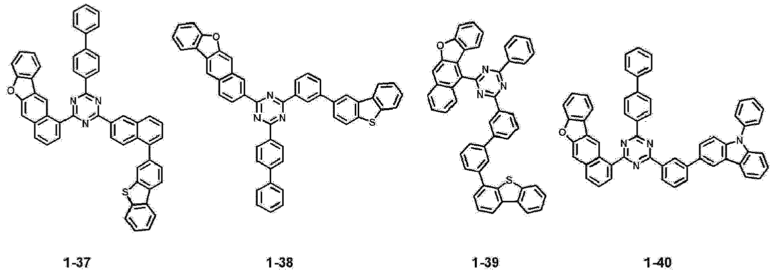 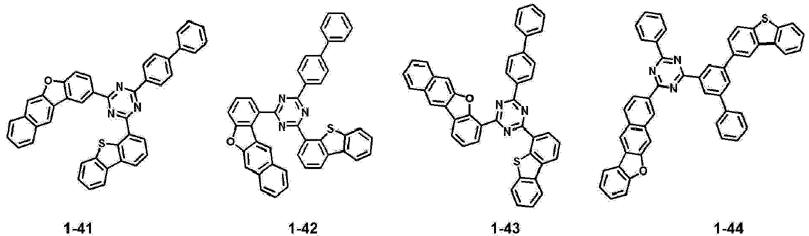 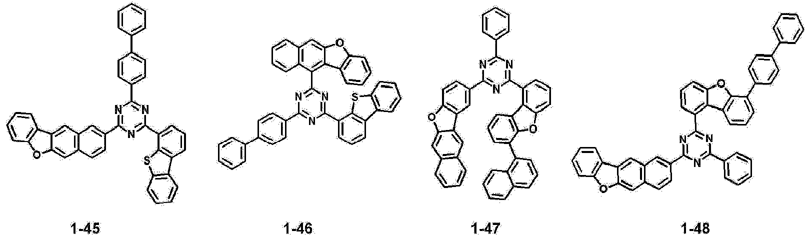 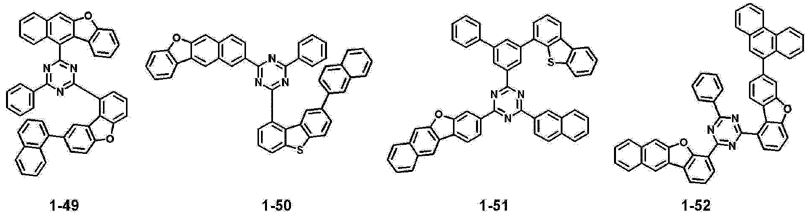 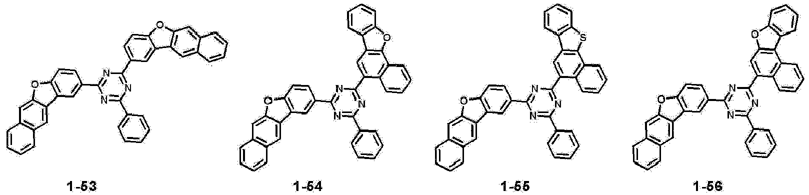 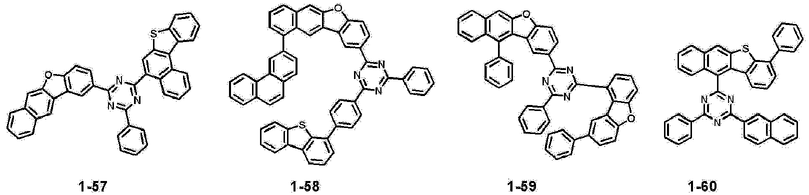 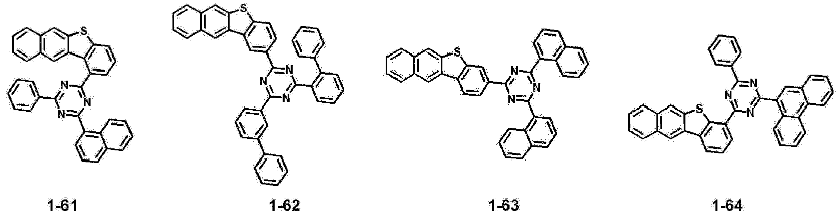 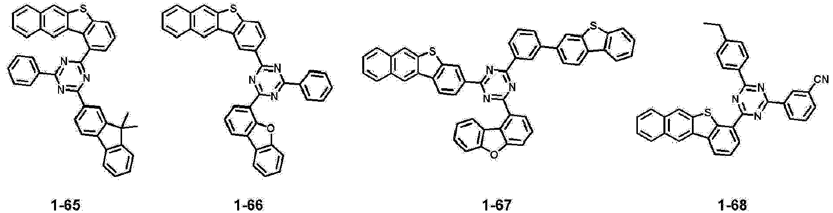 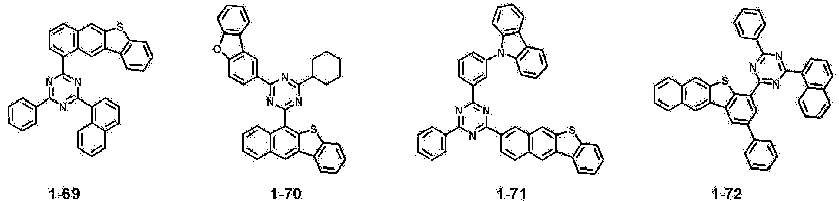 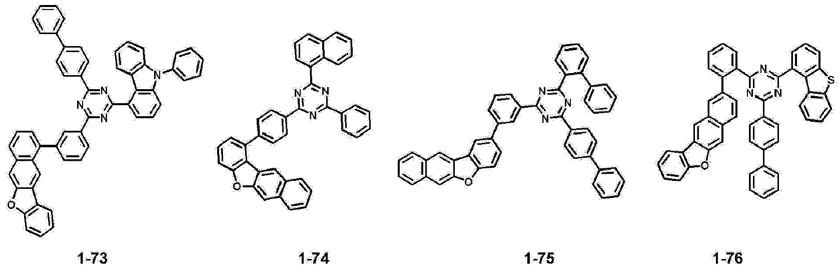 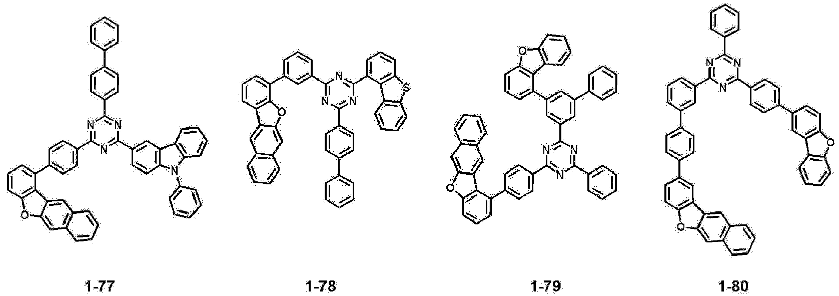 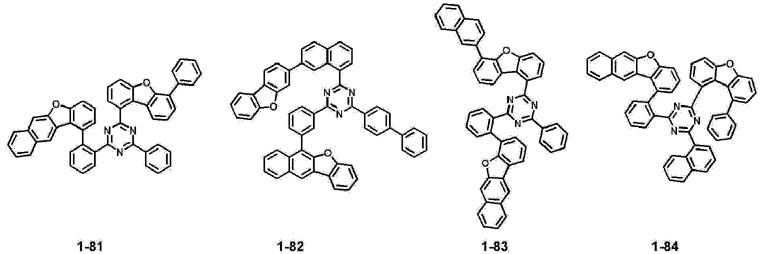 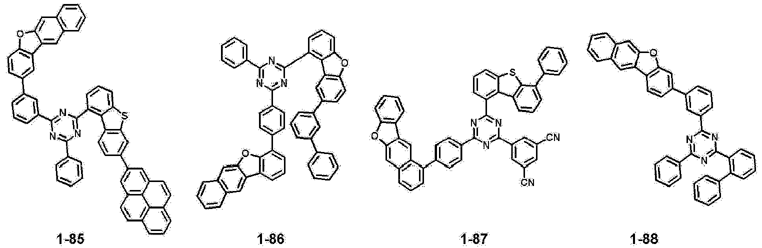 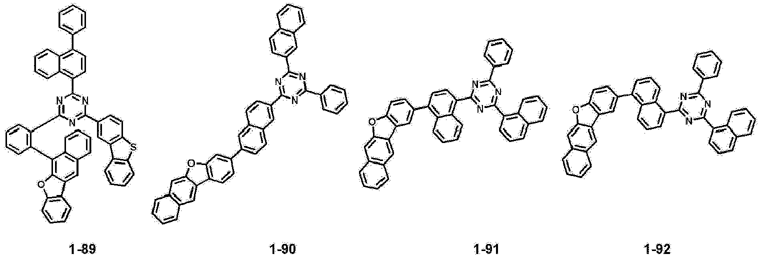 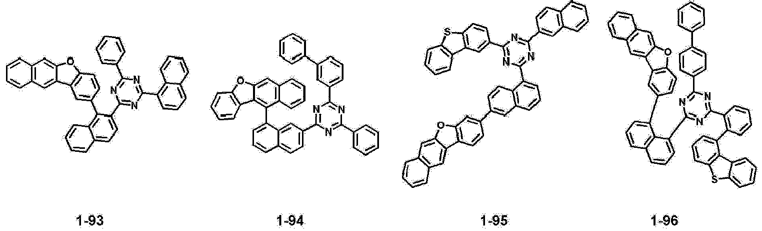 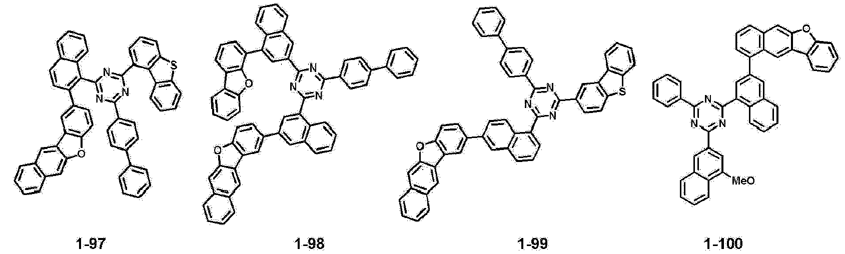 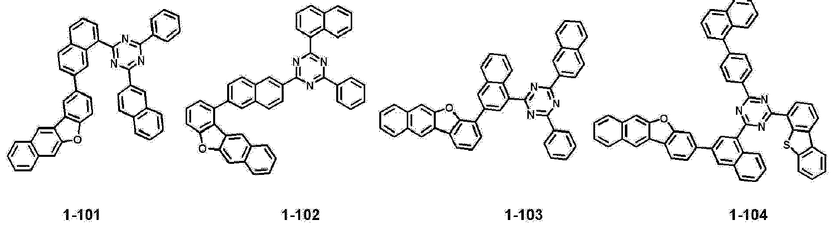 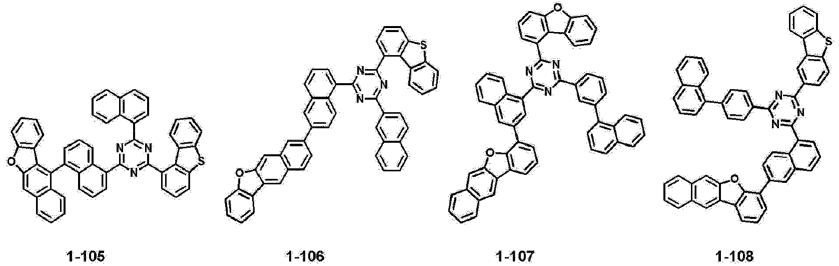 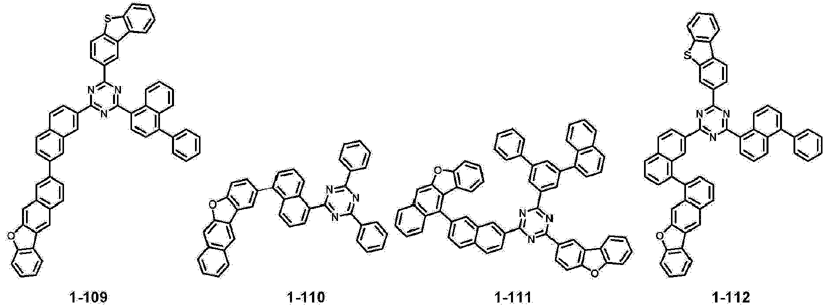 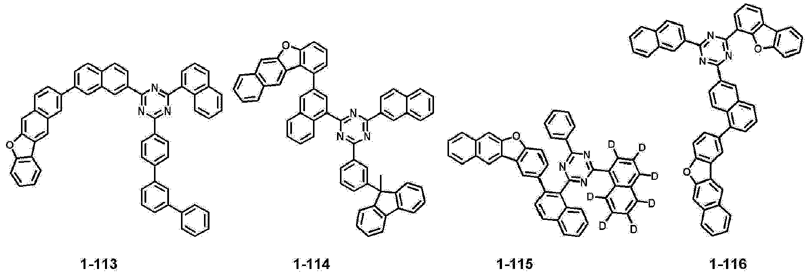 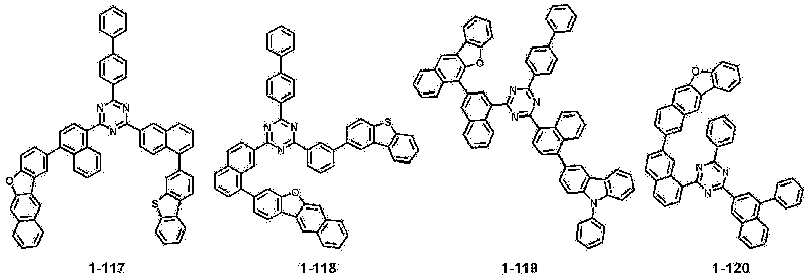 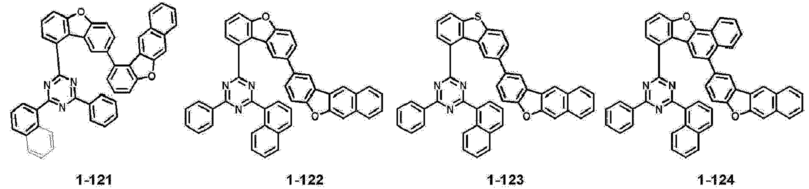 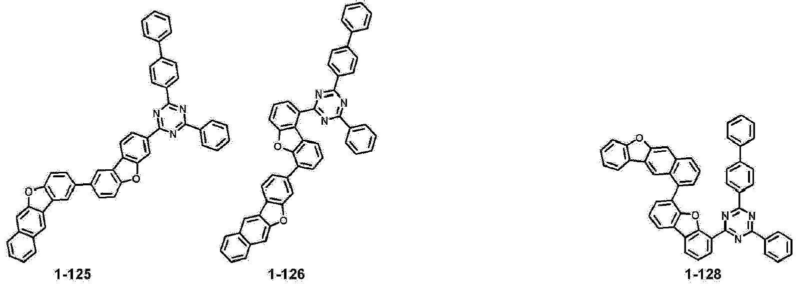 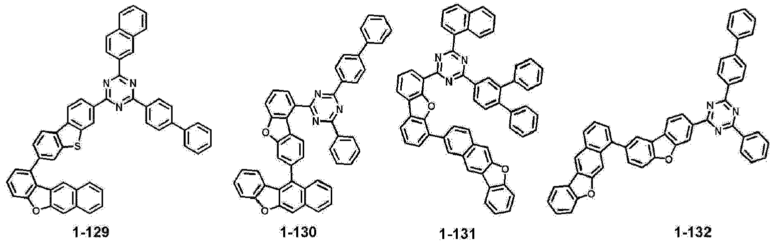 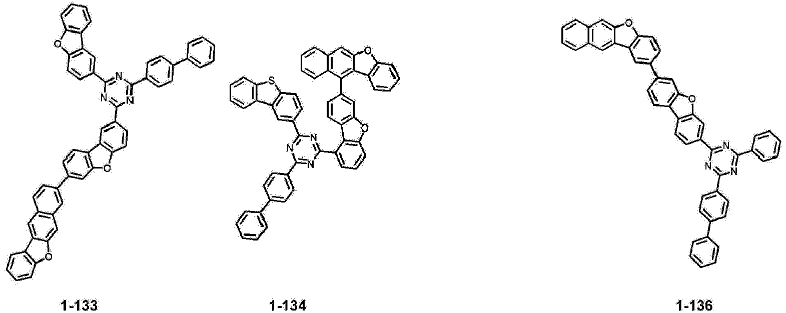 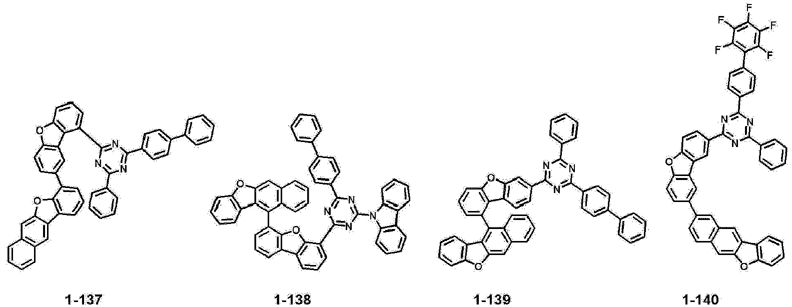 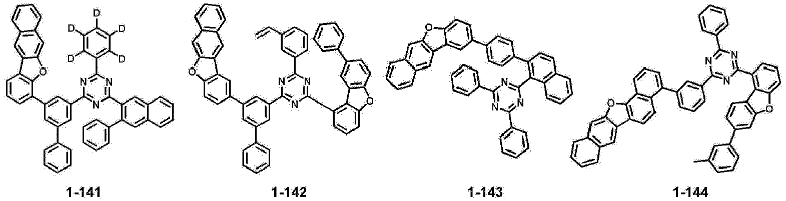 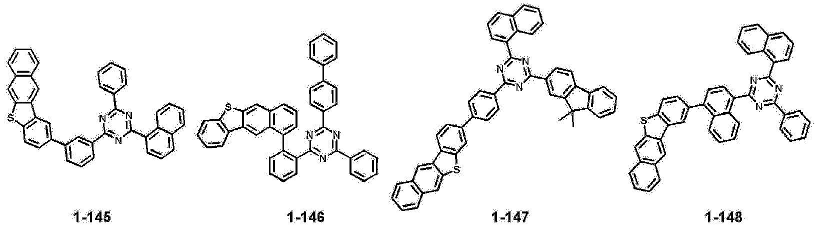 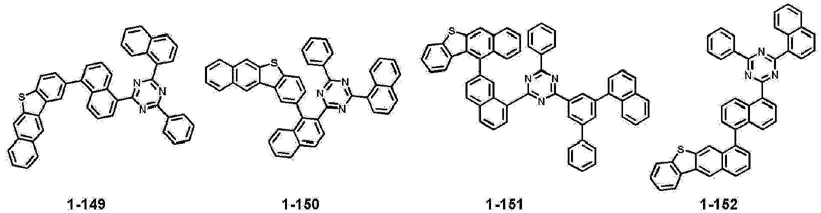 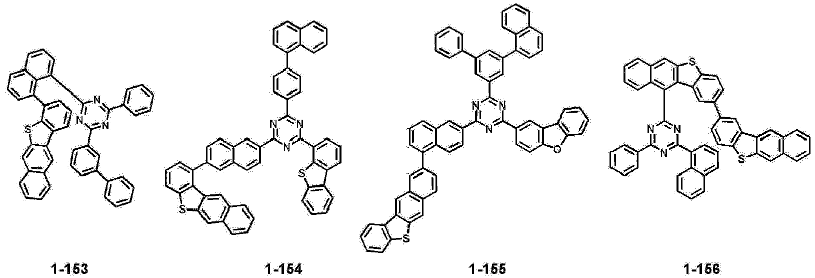 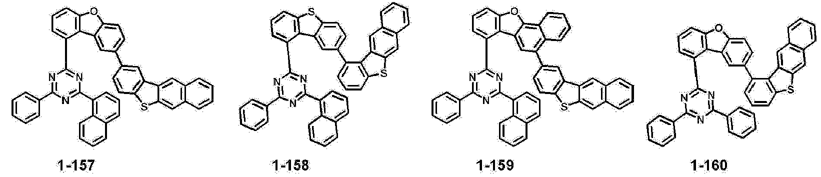 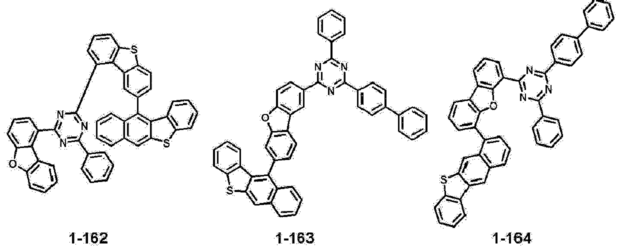 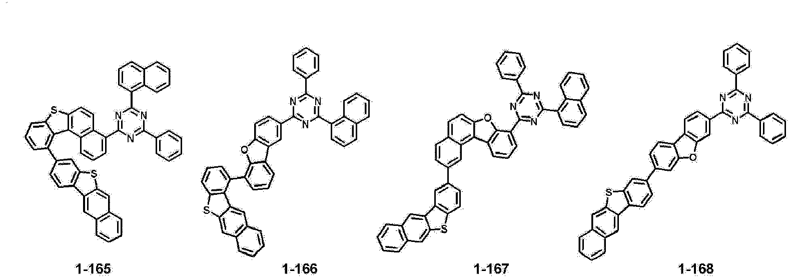 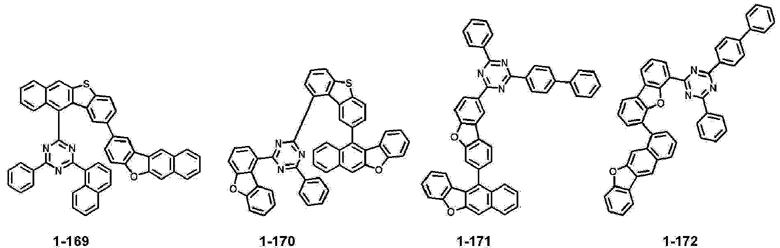 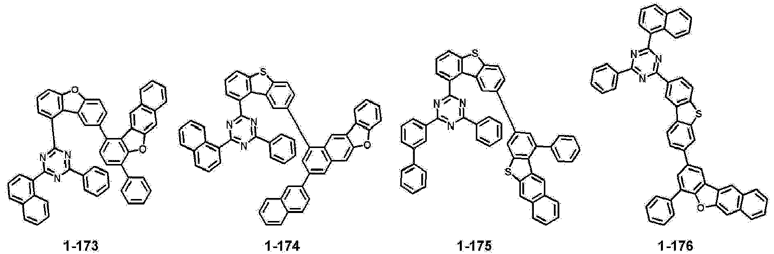 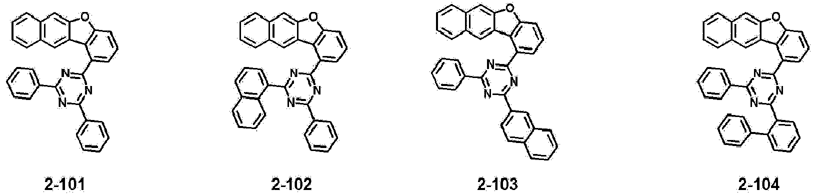 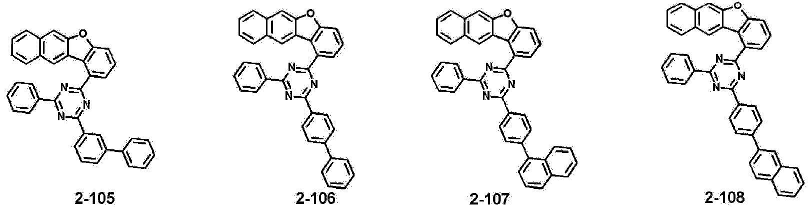 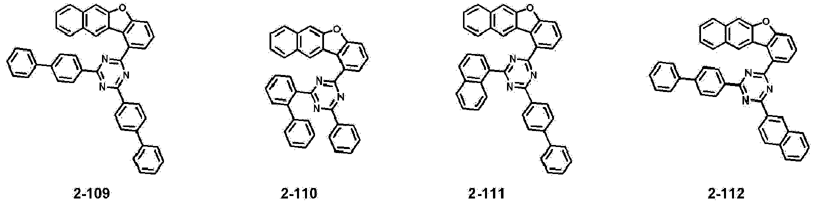 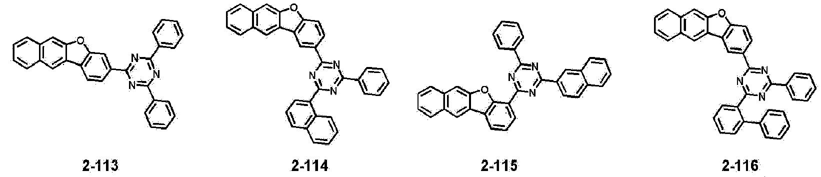 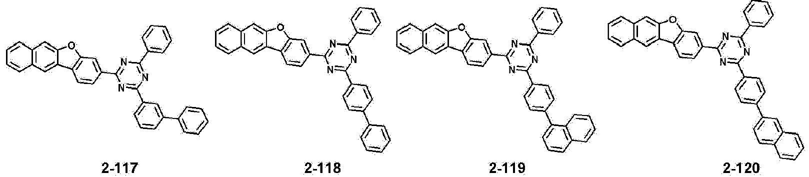 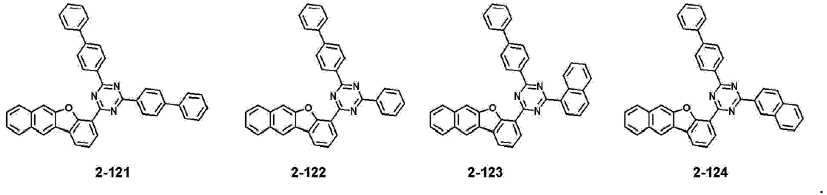
6. The organic electric element of any of items 1 to 5, wherein the compound represented by Formula 2 is one of the following compounds: 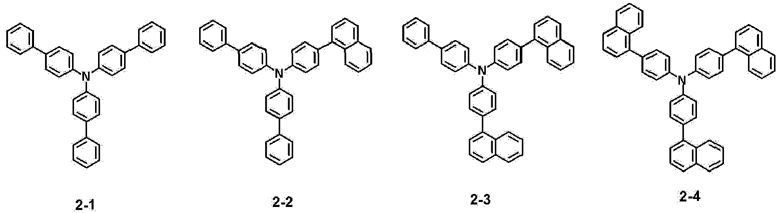 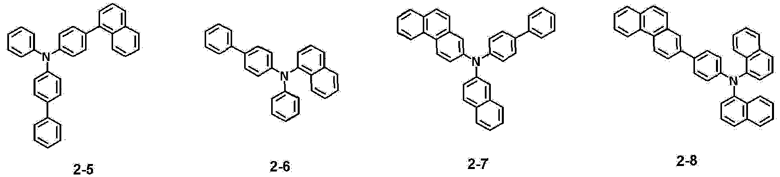 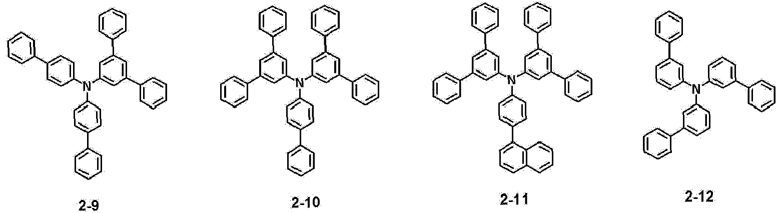 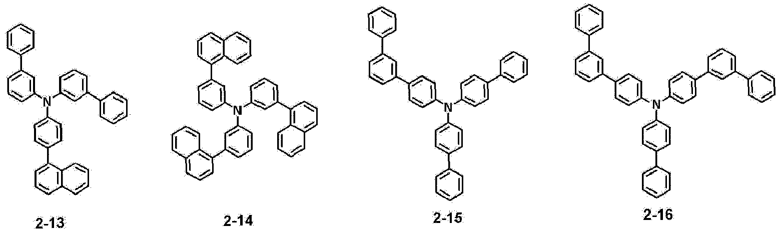 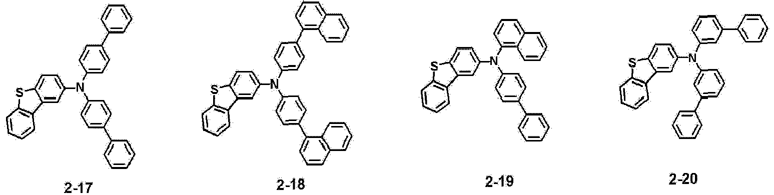 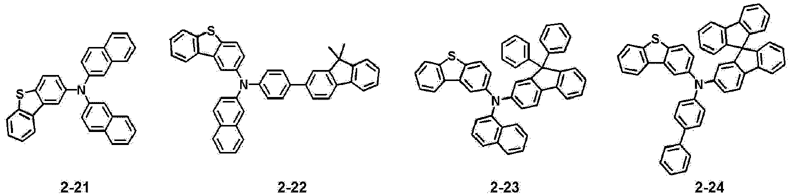 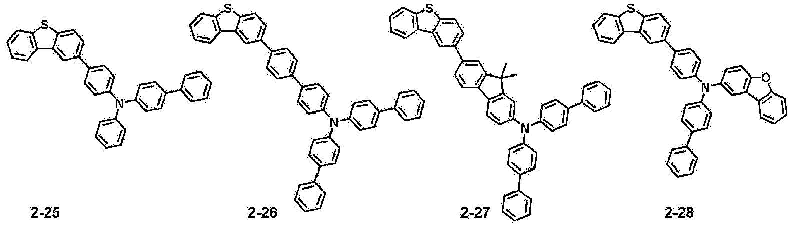 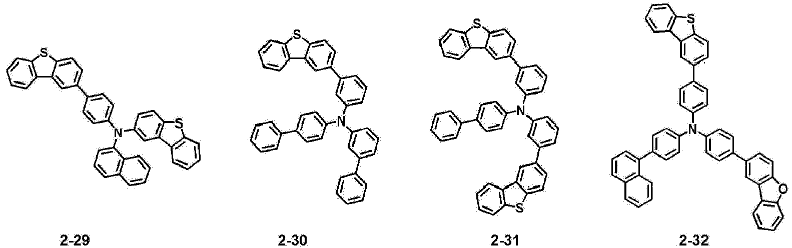 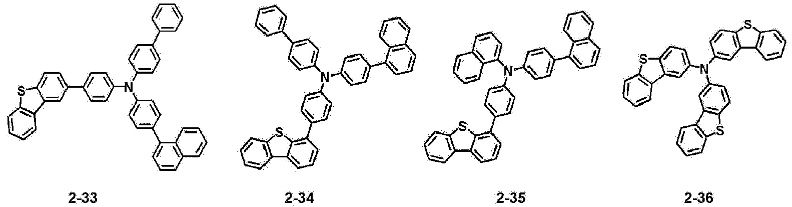 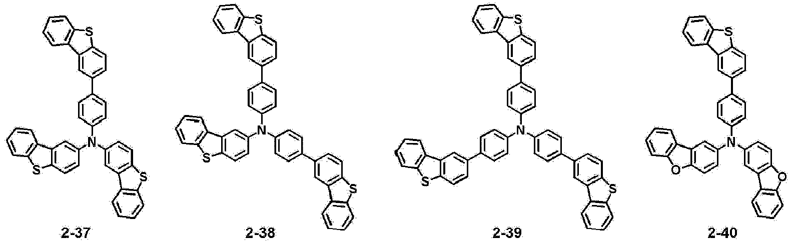 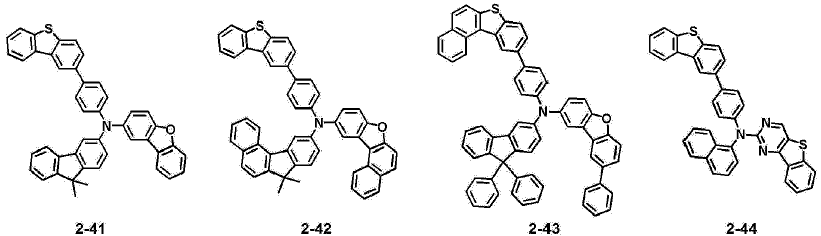 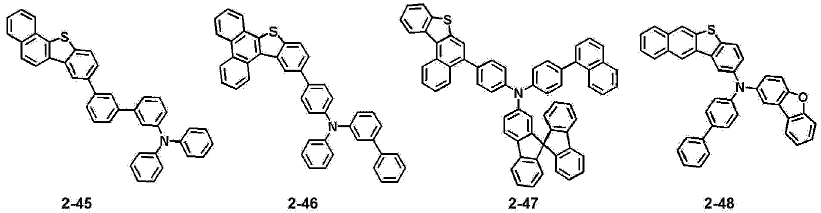 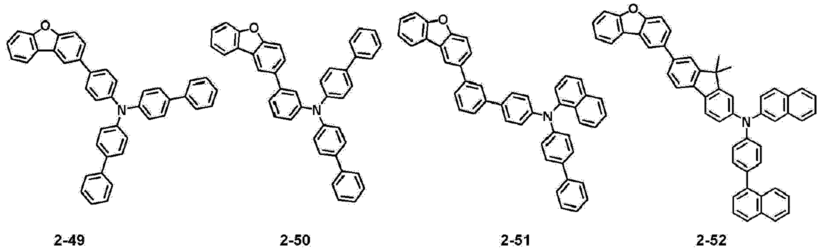 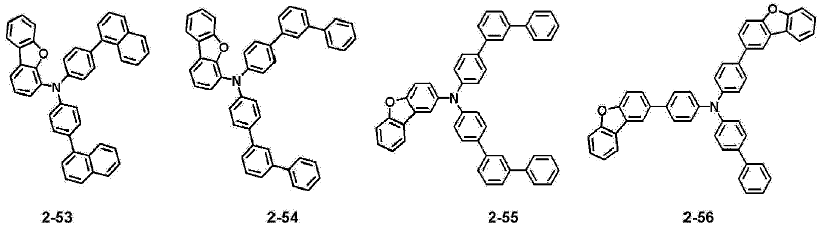 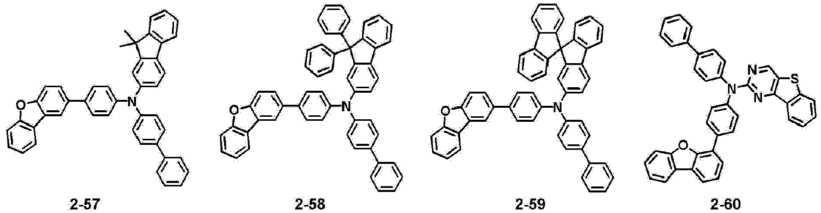 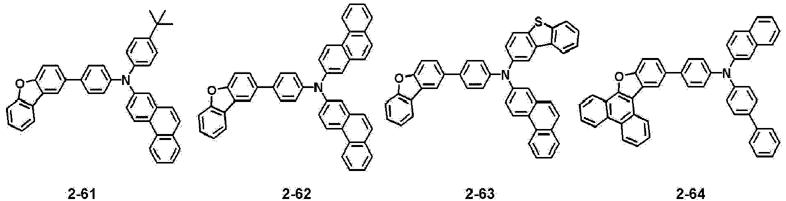 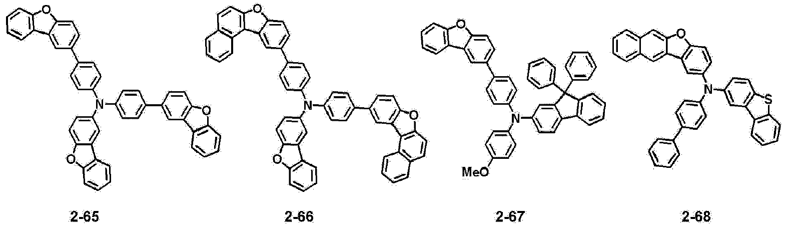 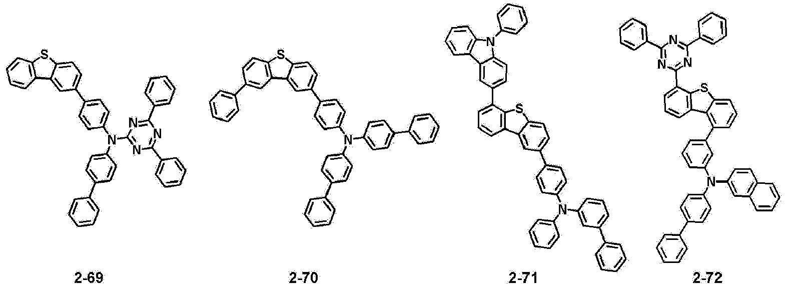 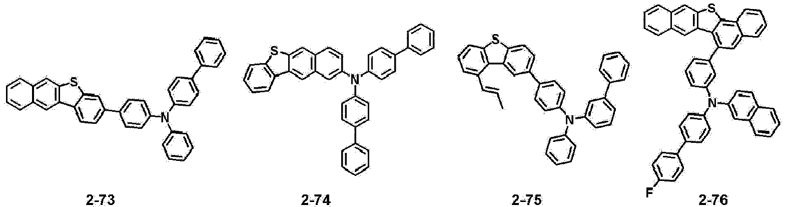 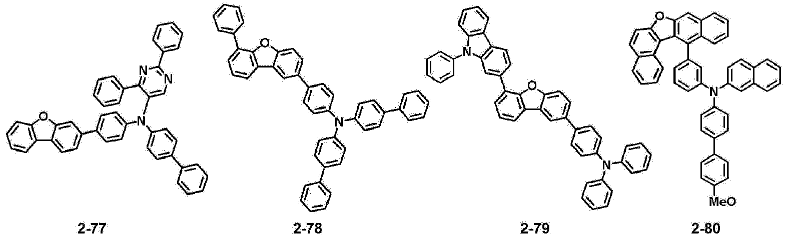 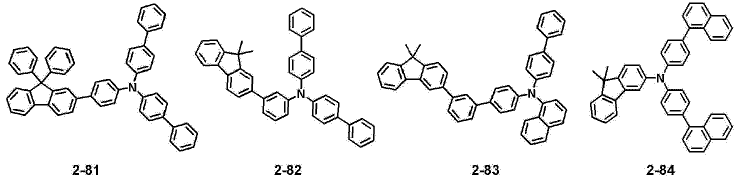 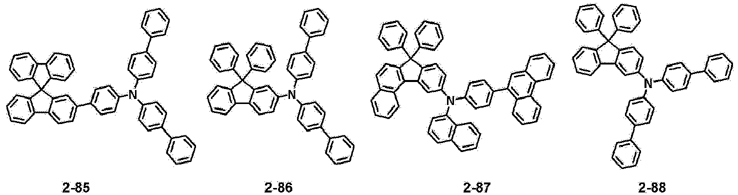 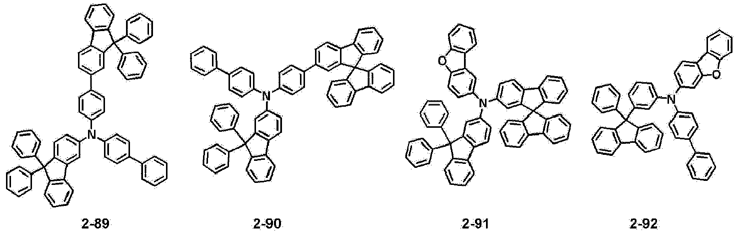 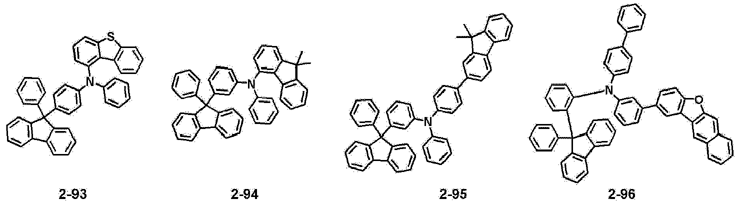 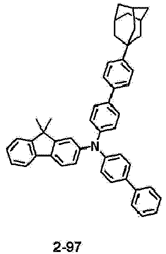 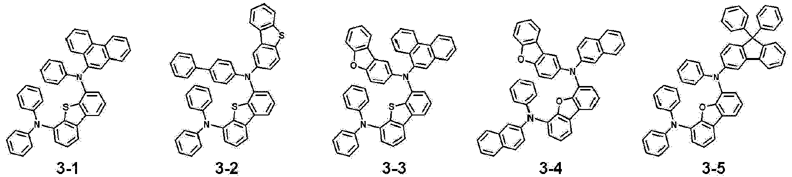 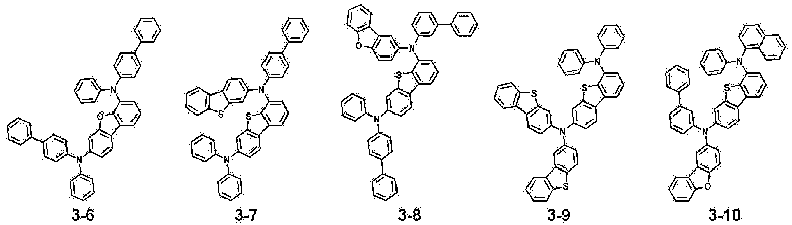 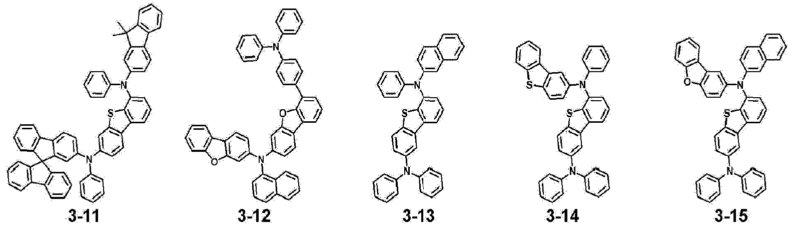 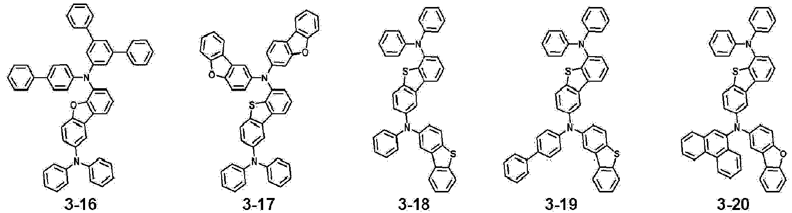 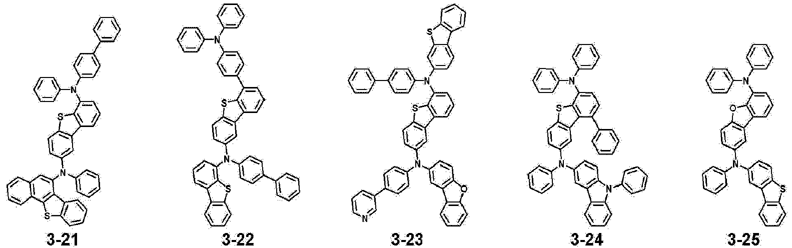 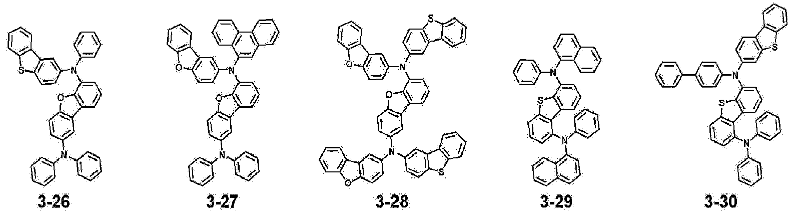 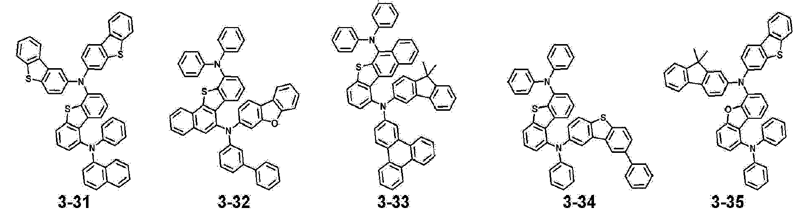 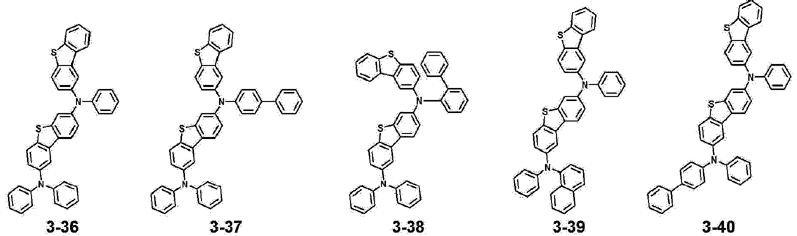 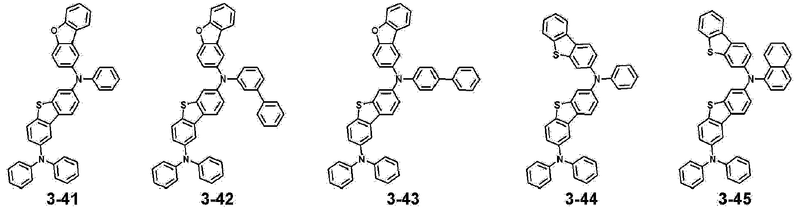 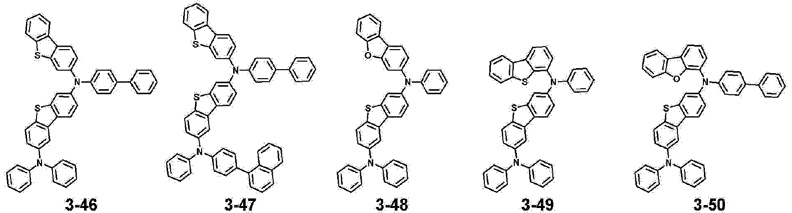 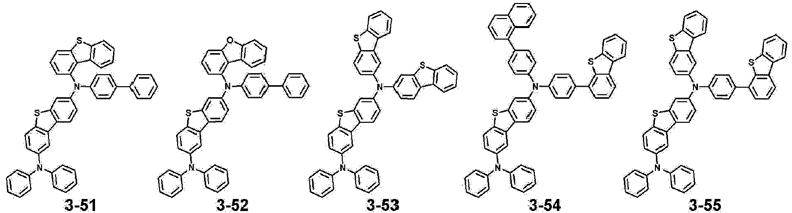 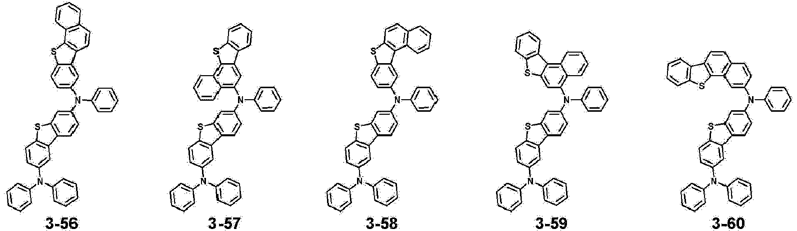 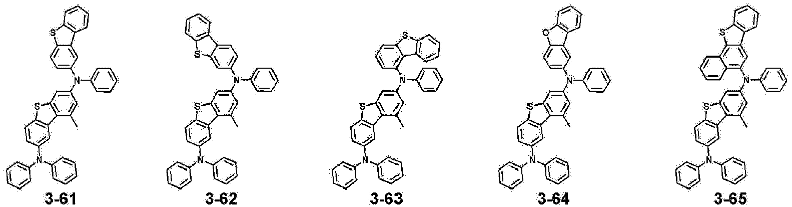 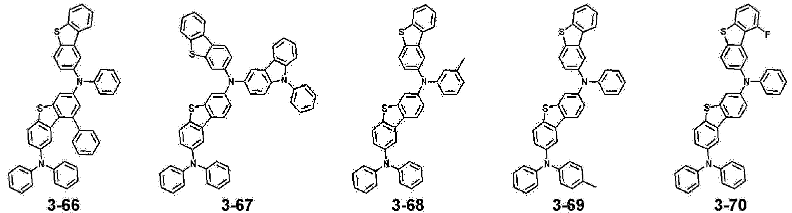 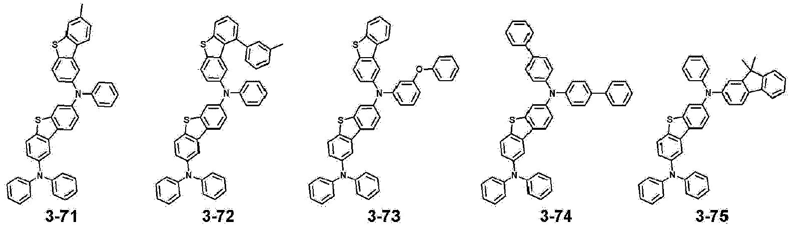 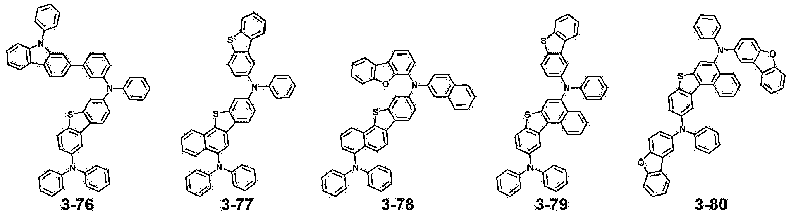 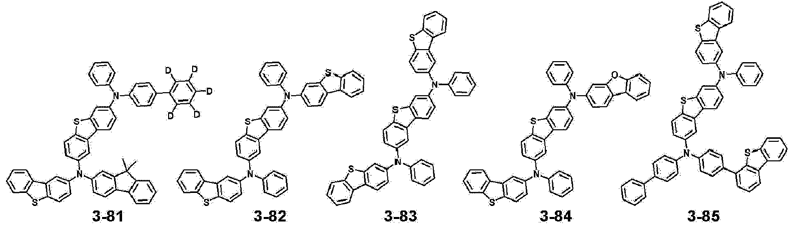 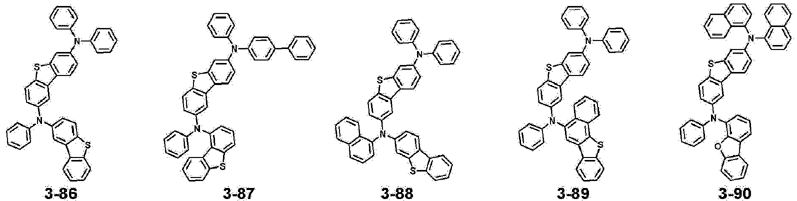 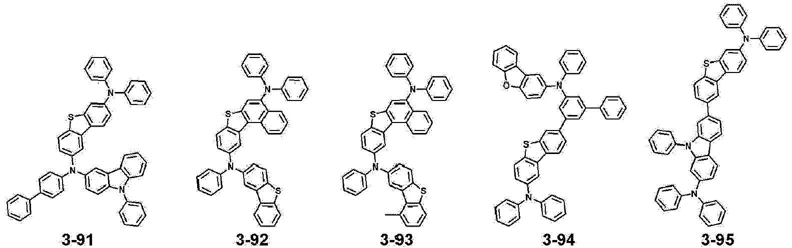 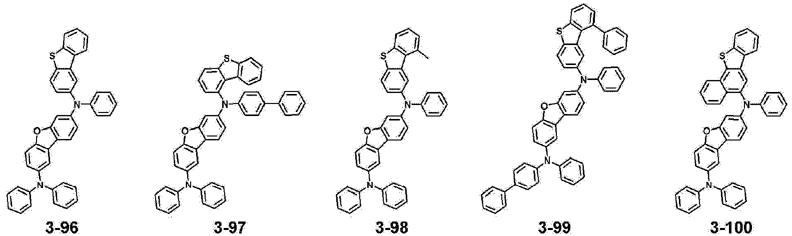 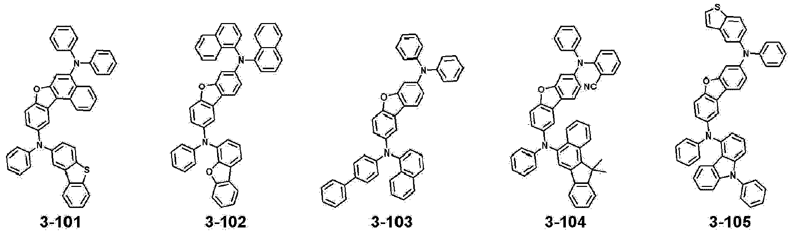 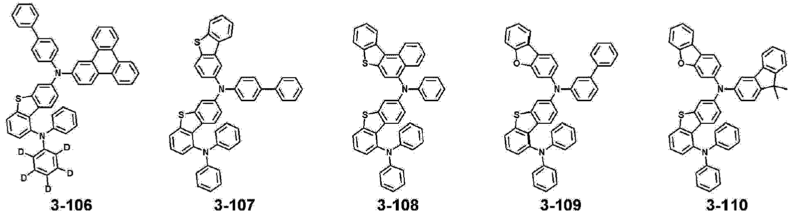 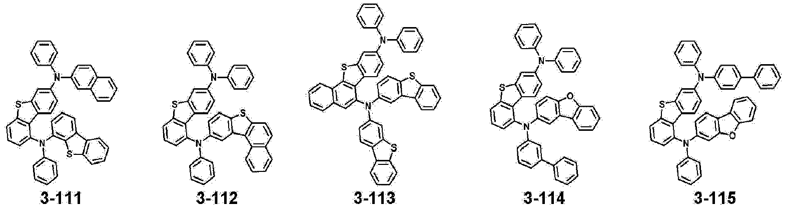 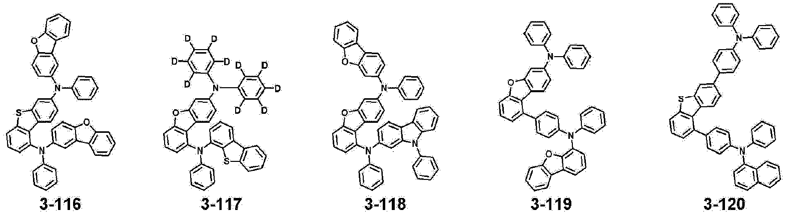 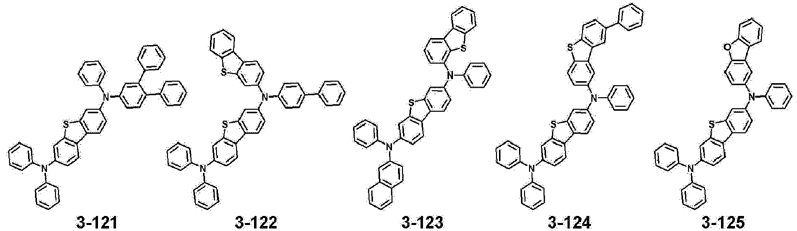 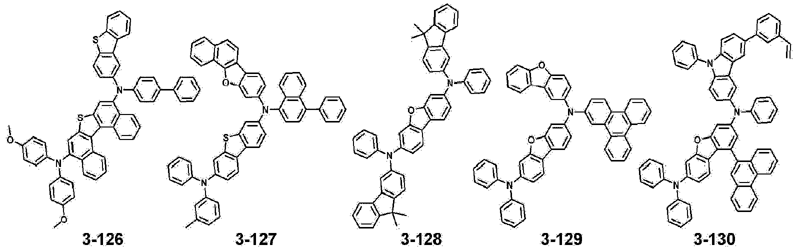 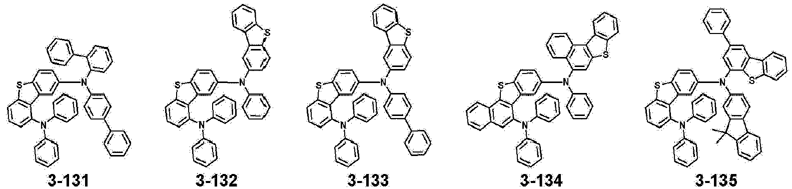 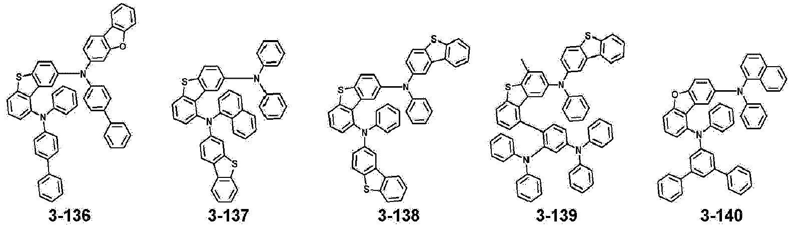 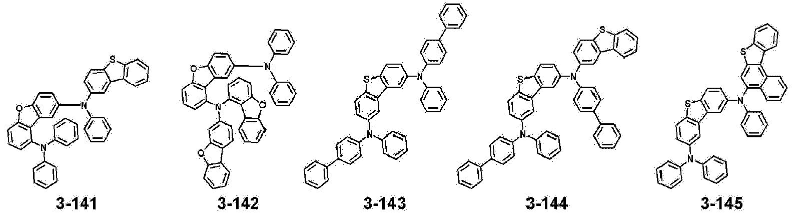 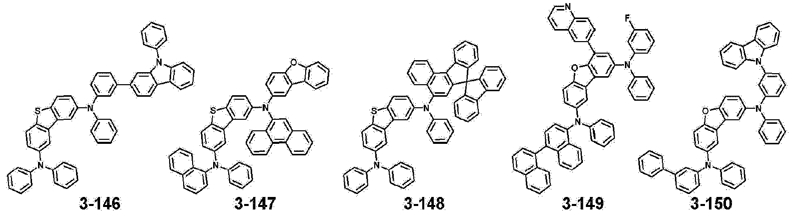 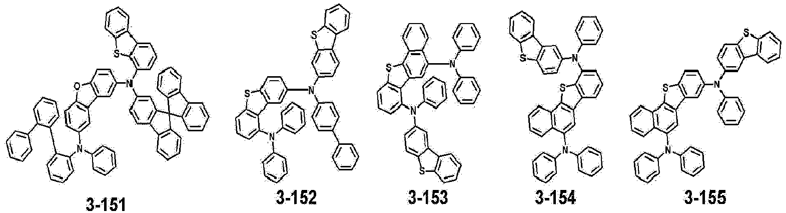 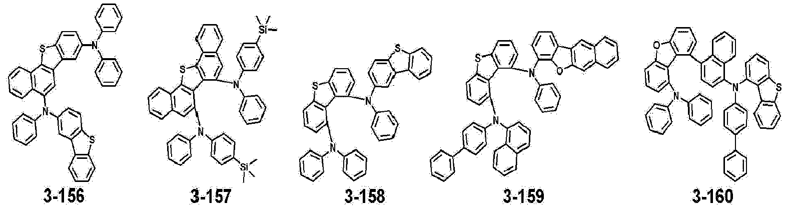 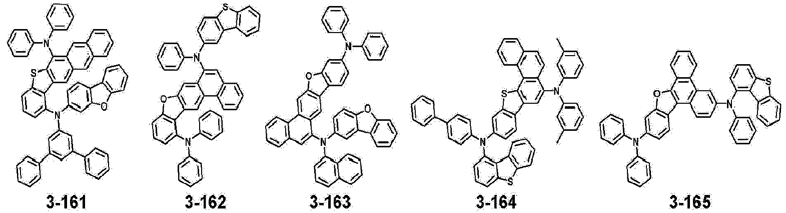 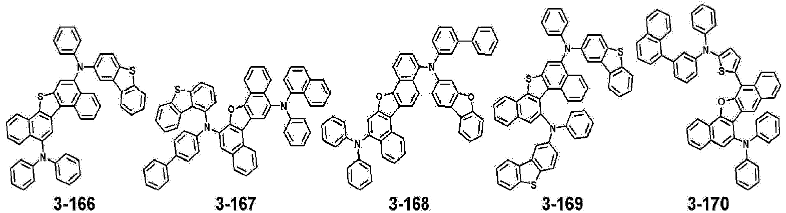 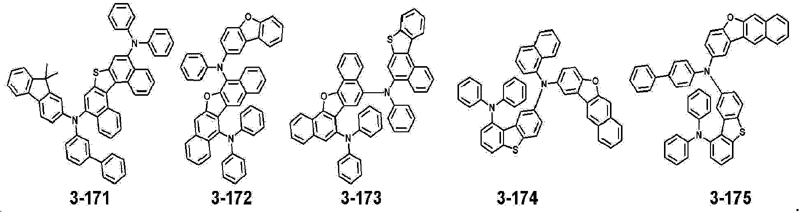
7. A compound of Formula 1: 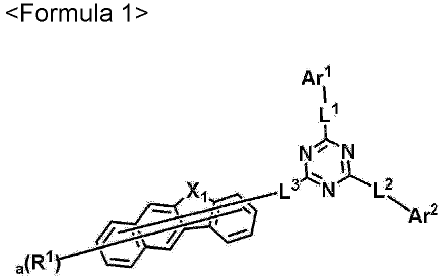 wherein,
   X₁ is O or S,
   Ar¹ and Ar² are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₃₀ alkyl group, a C₂-C₃₀ alkenyl group, a C₂-C₃₀ alkynyl group, a C₁-C₃₀ alkoxyl group and a C₆-C₃₀ aryloxy group,
   L¹ to L³ are each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
   R¹ is selected from the group consisting of hydrogen, deuterium, halogen, a cyano group, a C₆-C₆₀ aryl group, a fluorenyl group, a C₂-C₆₀ heterocyclic group comprising at least one heteroatom selected from the group consisting of O, N, S, Si and P, a C₃-C₃₀ aliphatic ring, a C₁-C₅₀ alkyl group, a C₂-C₂₀ alkenyl group, a C₂-C₂₀ alkynyl group, a C₁-C₃₀ alkoxyl group, a C₆-C₃₀ aryloxy group, and -L'-N(Rₐ)(R_{b}), and adjacent R¹s may optionally be linked to each other to form a ring,
   a is an integer of 0-9, and where a is an integer of 2 or more, each of a plurality of R¹ is the same as or different from each other,
   L' is selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
   Rₐ and R_{b} are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, and a combination thereof,
   Ar¹~Ar², L¹~L³, R¹, L', Rₐ, R_{b}, and the ring formed by bonding adjacent R¹s to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group.
8. The compound of item 7, wherein Formula 1 is represented by the following Formula 3: 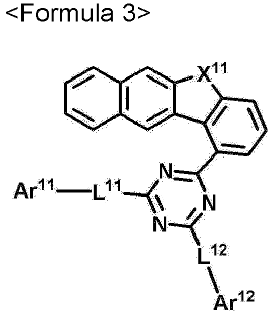 wherein, X¹¹ is O or S, L¹¹ and L¹² are each independently a single bond or a C₆-C₃₀ arylene group , and Ar¹¹ and Ar¹² are each independently C₆-C₃₀ aryl group.
9. The compound of item 8, wherein L¹¹ and L¹² are each independently a single bond or a C₆-C₁₅ arylene group, and Ar¹¹ and Ar¹² are each independently C₆-C₁₅ aryl group.
10. The compound of item 8, wherein Formula 3 is represented by one of the following Formulas 3-1, 3-A or 3-B: 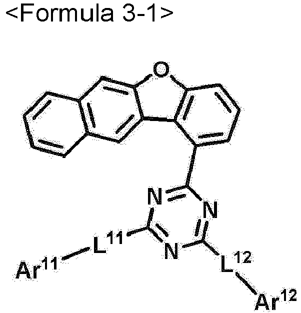 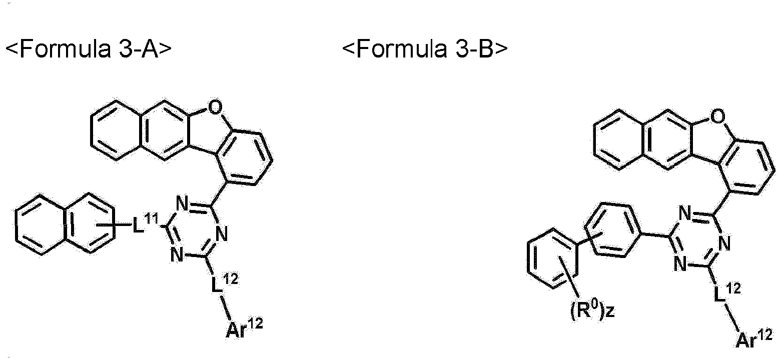 wherein, Ar¹¹, Ar¹², L¹¹ and L¹² are the same as defined in claim 8, R° is defined to be the samen as Ar¹¹, z is an integer of 0∼5, and where z is an integer of 2 or more, each of a plurality of R° is the same as or different from each other.
11. The compound of item 8, wherein the compound represented by Formula 3 is one of the following compounds: 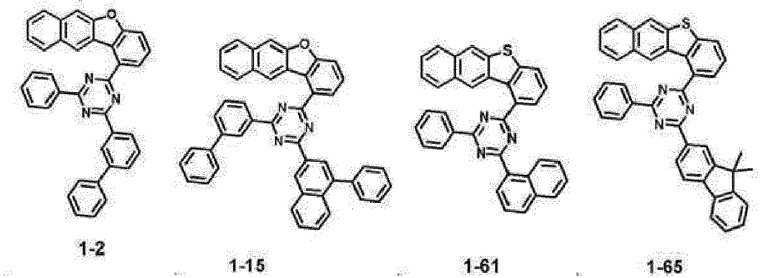 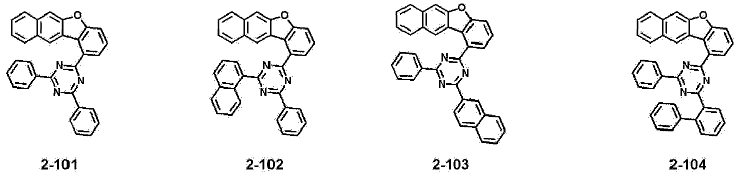 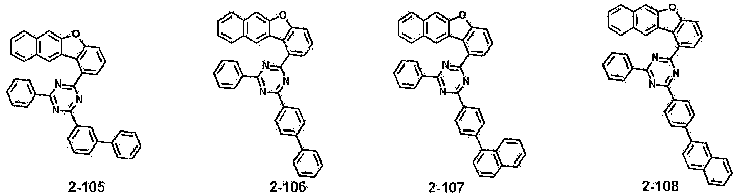 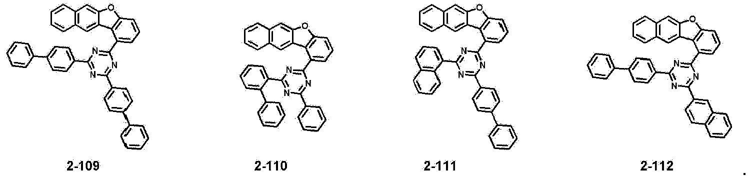
12. An organic electric element comprising a first electrode, a second electrode, and an organic material layer formed between the first electrode and the second electrode, wherein the organic material layer comprises the compound represented by Formula 3 of item 8.
13. The organic electric element of item 12, wherein the organic material layer comprises a light emitting layer or an emission-auxiliary layer, and the compound represented by Formula 3 is comprised in the light emitting layer or the emission-auxiliary layer.
14. The organic electric element of any of items 12 or 13, wherein the organic material layer comprises a light emitting layer, and the light emitting layer comprises the compound represented by Formula 3 and the compound represented by the following Formula 4: 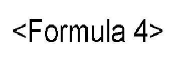 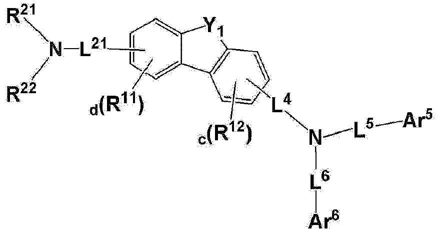 wherein,
   Y₁ is O, S or C(R')(R"),
   Ar⁵ and Ar⁶ are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₃₀ alkyl group, a C₂-C₃₀ alkenyl group, a C₂-C₃₀ alkynyl group, a C₁-C₃₀ alkoxyl group and a C₆-C₃₀ aryloxy group,
   L⁴ to L⁶, and L²¹ are each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring and a combination thereof,
   R¹¹, R¹², R²¹, R²², R' and R" are each independently selected from the group consisting of hydrogen, deuterium, halogen, a silane group substituted or unsubstituted with C₁-C₂₀ alkyl group or C₆-C₂₀ aryl group, a cyano group, a nitro group, a C₁-C₂₀ alkoxyl group, a C₆-C₃₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring and -L'-N(Rₐ)(R_{b}), and adjacent groups may optionally be linked to each other to form a ring,
   c and d are each an integer of 0∼3, and where c or d is an integer of 2 or more, each of a plurality of R¹² is the same as or different from each other.
   L' is selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
   Rₐ and R_{b} are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, and a combination thereof,
   Ar⁵, Ar⁶, L⁴ to L⁶, L²¹, R¹¹, R¹², R²¹, R²², R', R", L', Rₐ, R_{b} and the ring formed by bonding adjacent groups to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group.
15. The organic electric element of item 14, wherein Formula 4 is represented by one of the following Formulas 4-1 to 4-9: 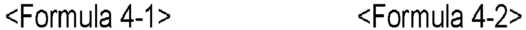 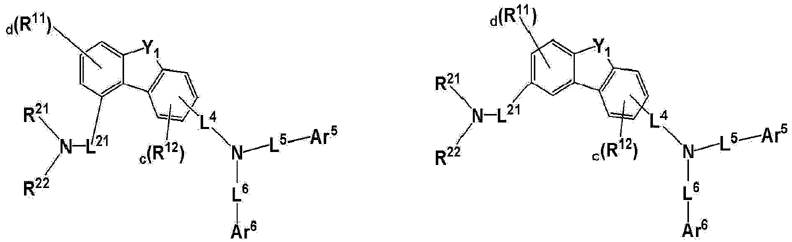 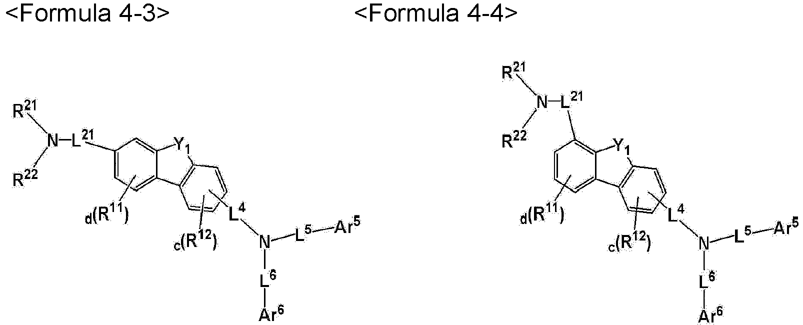 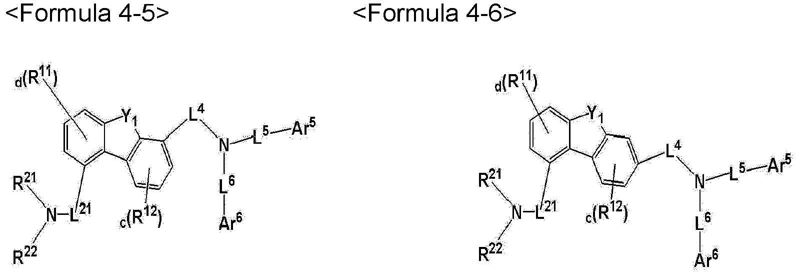 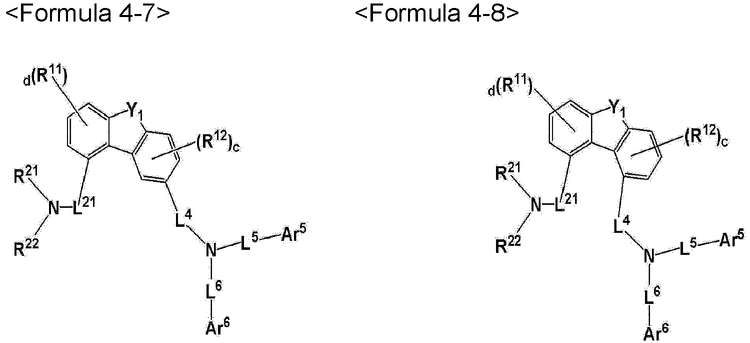 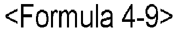 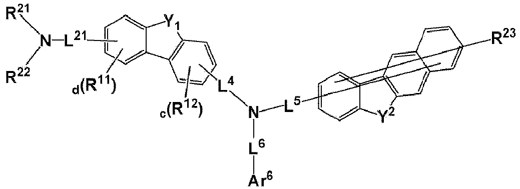 wherein, Y₁, Ar⁵, Ar⁶, L⁴ to L⁶, L²¹, R¹¹, R¹², R²¹, R²², c and d are the same as defined in item 14, Y² is O or S, and R²³ is defined to be the same as R¹¹.
16. The organic electric element of item 14, wherein the organic material layer comprises an emission-auxiliary layer, and the emission-auxiliary layer comprises the compound represented by Formula 4.
17. The organic element of item 14, wherein the compound represented by Formula 4 is one of the following compounds: 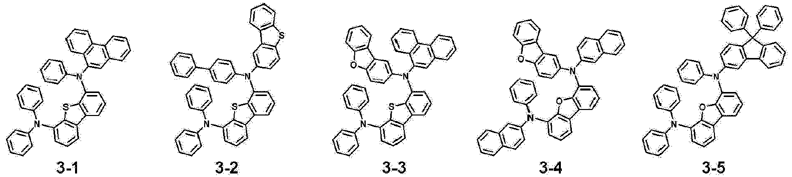 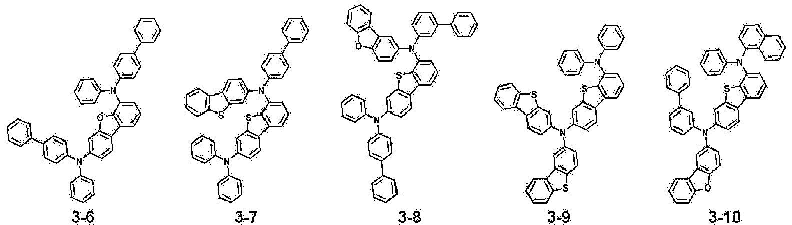 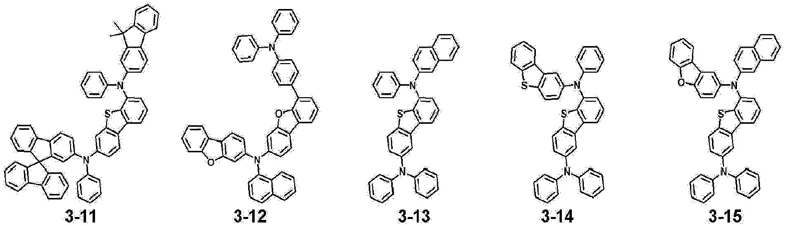 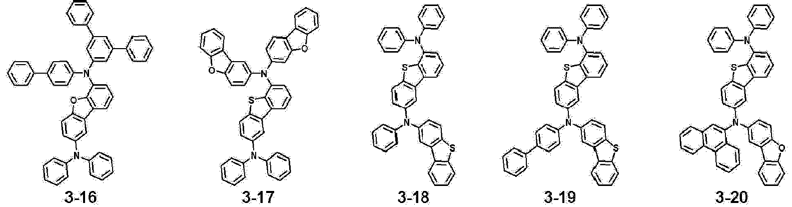 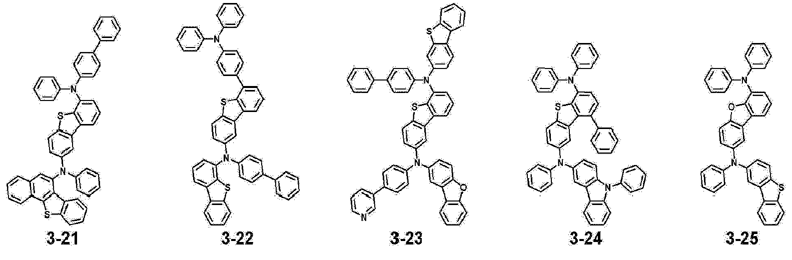 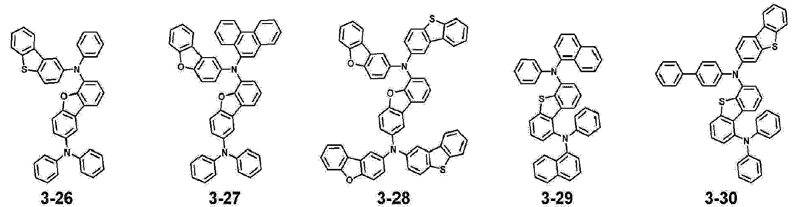 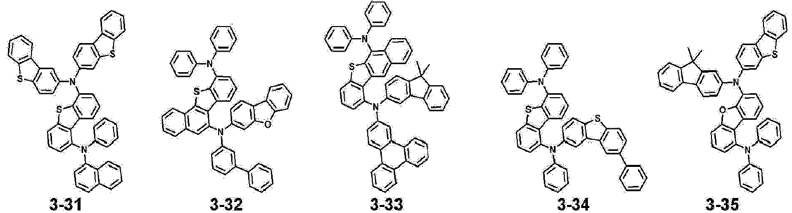 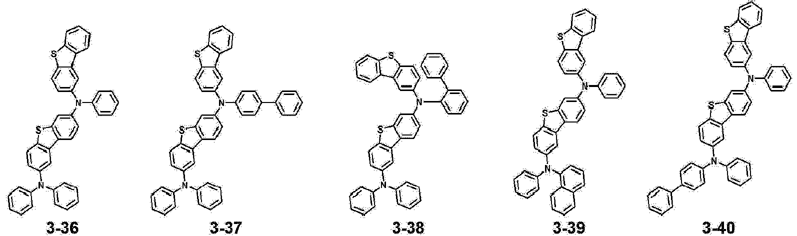 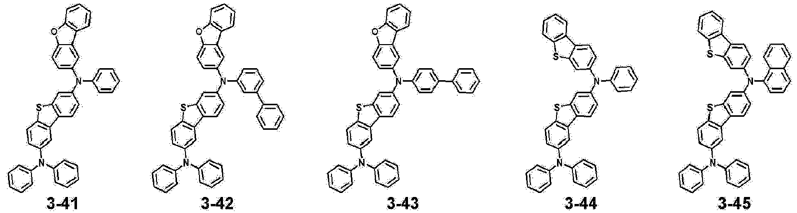 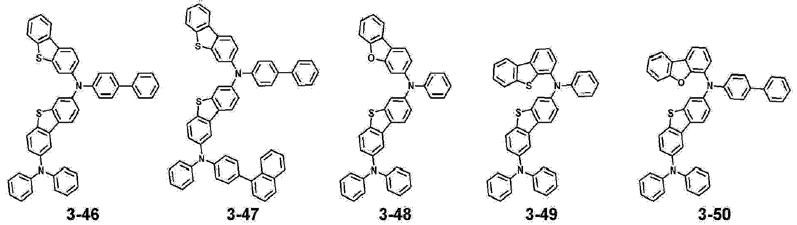 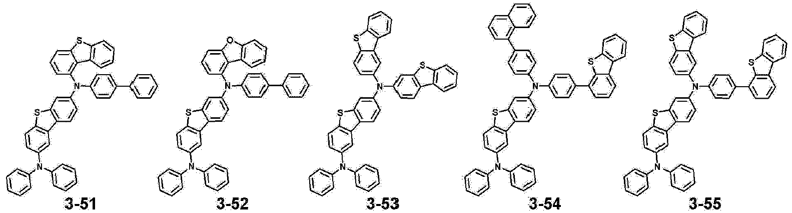 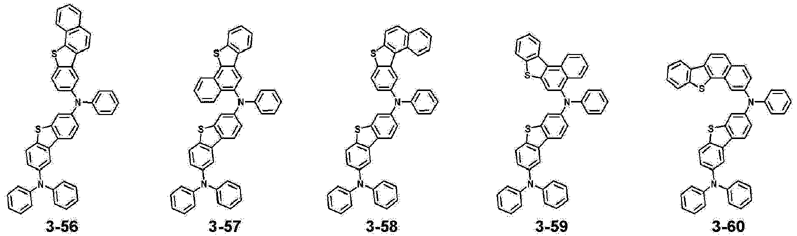 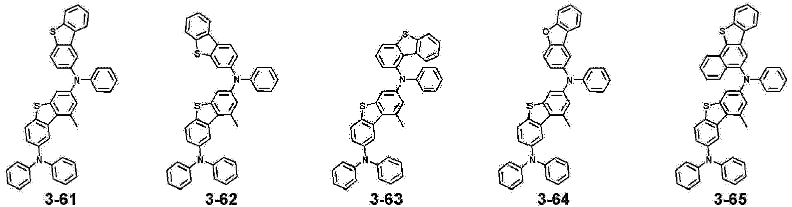 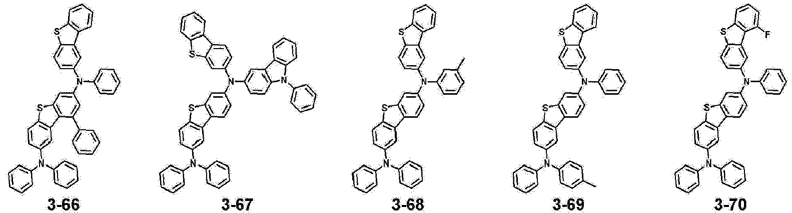 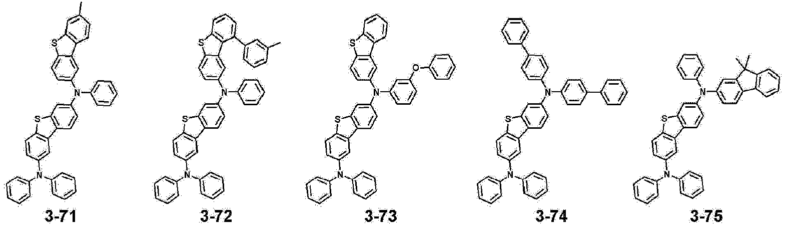 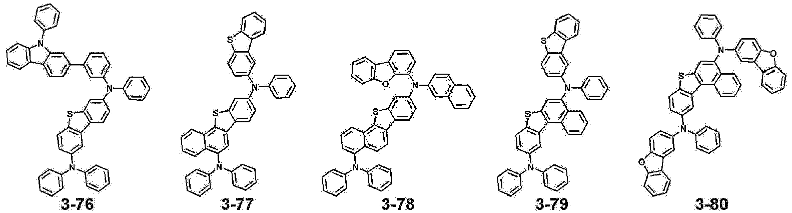 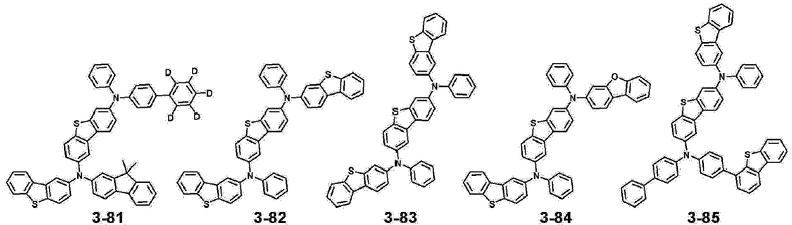 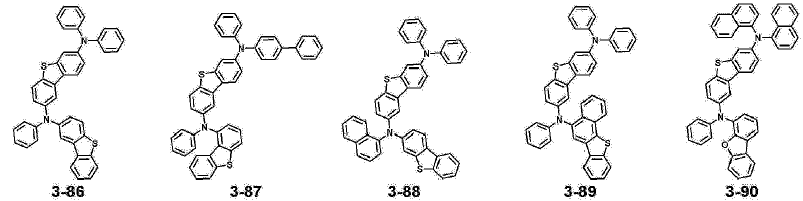 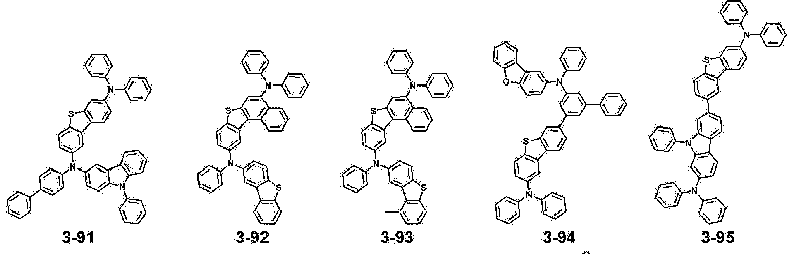 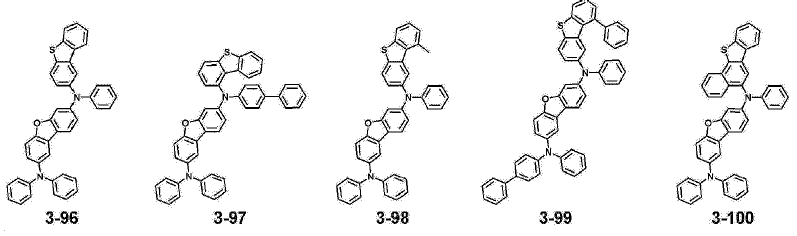 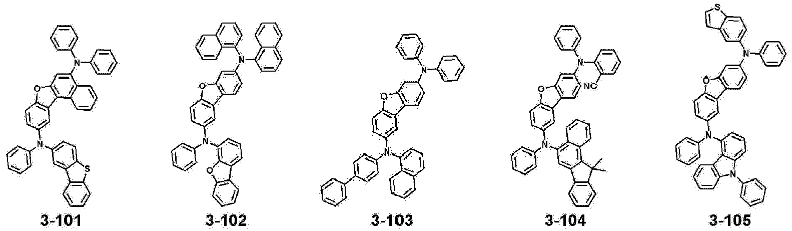 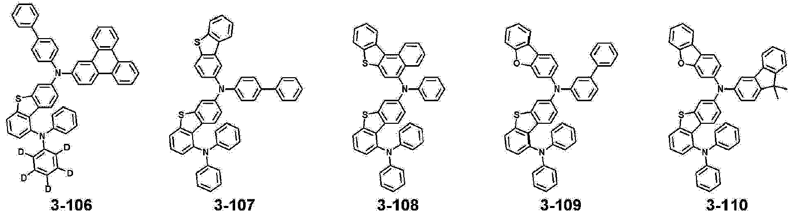 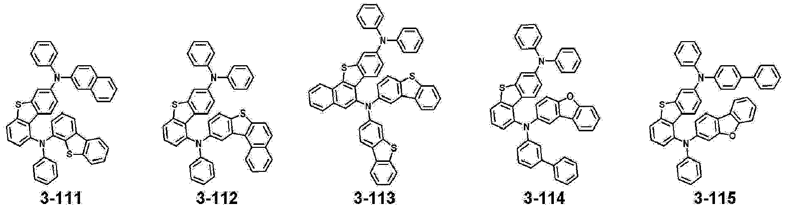 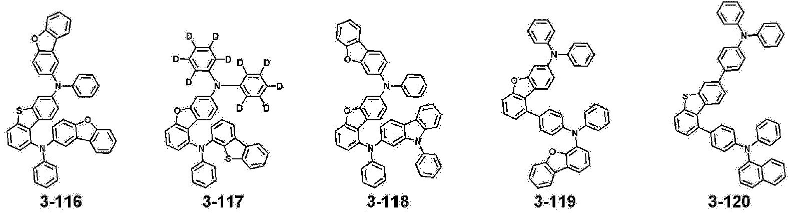 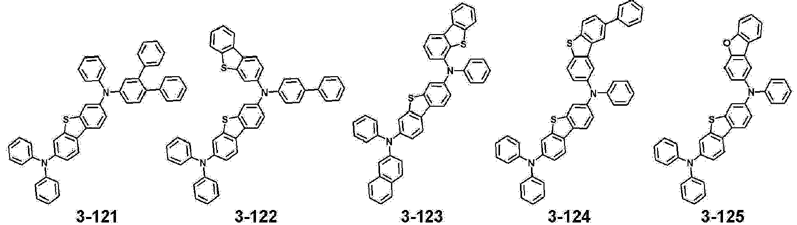 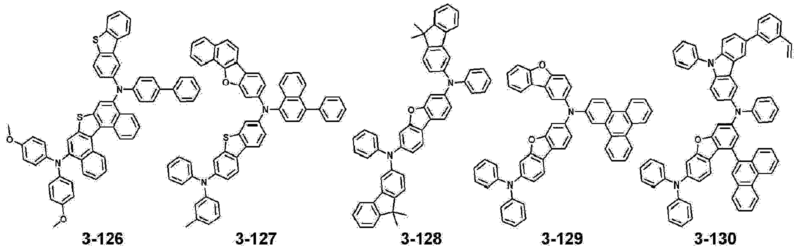 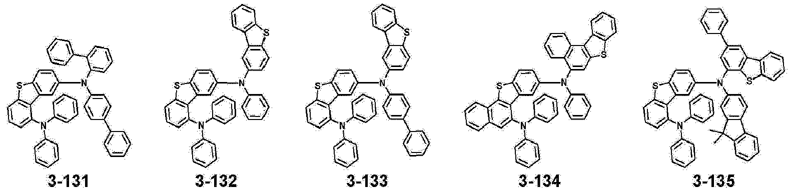 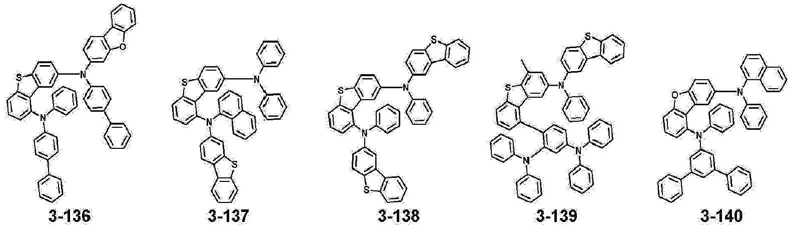 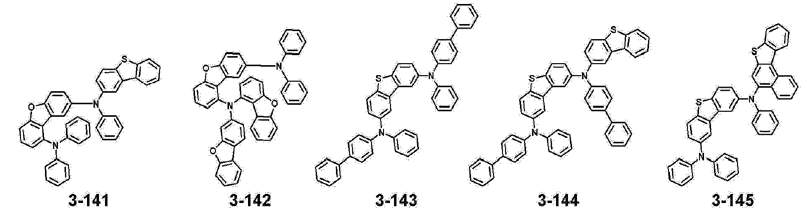 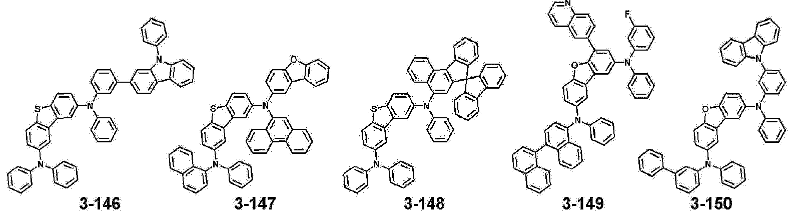 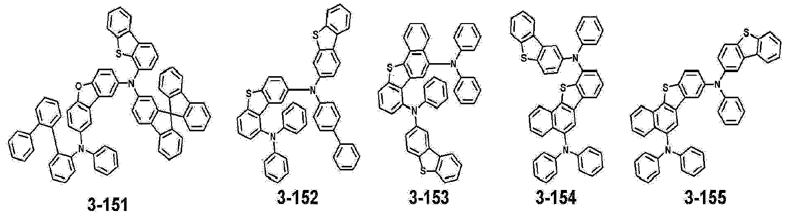 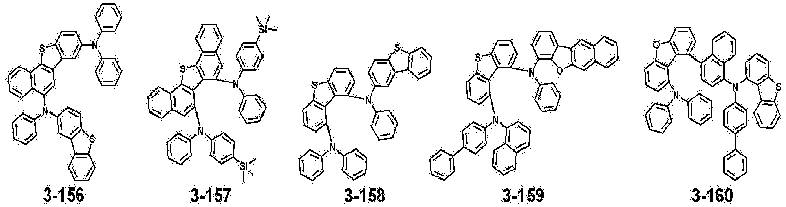 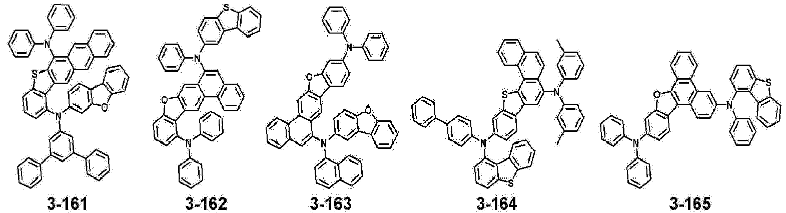 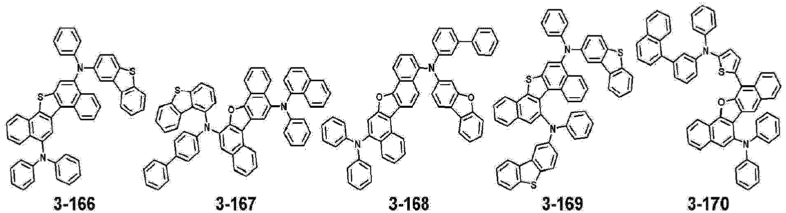 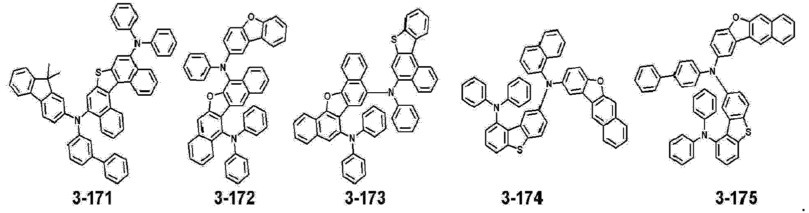
18. The organic electric element of item 1 or 12, further comprising a layer for improving luminous efficiency formed on one side of the first electrode or one side of the second electrode, the side not facing the organic material layer.
19. The organic electric element of item 1 or 12, wherein the organic material layer comprises two or more stacks, the stacks comprising a hole transport layer, a light emitting layer, and an electron transport layer in sequence formed on the anode.
20. The organic electric element of item 19, wherein the organic material layer further comprises a charge generation layer formed between the two or more stacks.
21. An electronic device comprising a display device and a control unit for driving the display device, wherein the display device comprises the organic electric element of item 1 or 12.
22. The electronic device of item 21, wherein the organic electric element is are selected from the group consisting of an organic light emitting diode, an organic solar cell, an organic photo conductor, an organic transistor, an element for monochromatic illumination and a quantum dot display.

## Claims

1. An organic electric element comprising a first electrode, a second electrode, and an organic material layer formed between the first electrode and the second electrode, wherein the organic material layer comprises a phosphorescent light emitting layer, and the host of the phosphorescent light emitting layer comprises a first compound represented by the following Formula 1 and a second compound represented by the following Formula 2: 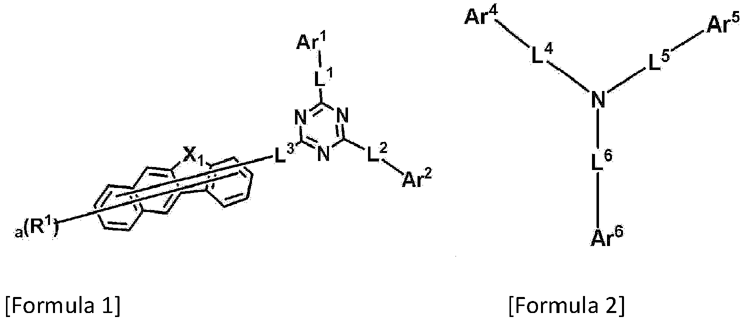 wherein,
X₁ is O or S,
Ar¹, Ar², and Ar⁴ to Ar⁶ are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₃₀ alkyl group, a C₂-C₃₀ alkenyl group, a C₂-C₃₀ alkynyl group, a C₁-C₃₀ alkoxyl group and a C₆-C₃₀ aryloxy group,
L¹ to L⁶ are each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
R¹ is selected from the group consisting of hydrogen, deuterium, halogen, a cyano group, a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₅₀ alkyl group, a C₂-C₂₀ alkenyl group, a C₂-C₂₀ alkynyl group, a C₁-C₃₀ alkoxyl group, a C₆-C₃₀ aryloxy group and -L'-N(Rₐ)(R_{b}), and adjacent R¹s may optionally be linked to each other to form a ring,
a is an integer of 0 to 9, and where a is an integer of 2 or more, each of a plurality of R¹ is the same as or different from each other,
L' is selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
Rₐ and R_{b} are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, and a combination thereof,
Ar¹ to Ar², L¹ to L⁶, R¹, L', Rₐ, R_{b}, and the ring formed by bonding adjacent R¹s to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group, and
Ar⁴ to Ar⁶ may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group, a C₈-C₂₀ arylalkenyl group and -L'-N(Rₐ)(R_{b}).

2. The organic electric element of claim 1, wherein L¹ to L⁶ are each independently represented by one of the following Formulas b-1 to b-13: 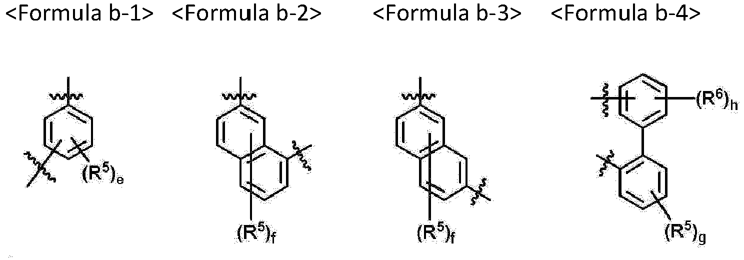 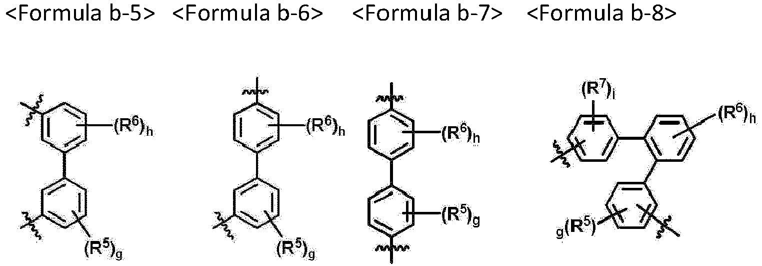 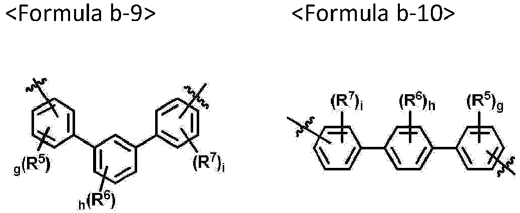 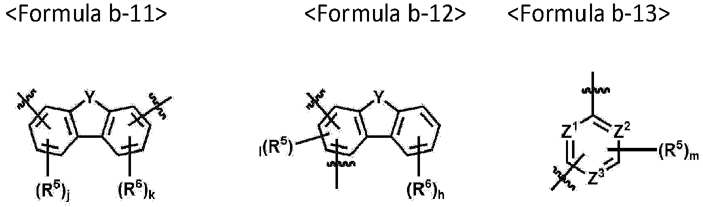 wherein,
R⁵ to R⁷ are each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₂₀ aryloxy group,
Y is N-(L^{a}-Ar^{a}), O, S or C(R')(R"),
Z¹ to Z³ are each independently C, C(R') or N, and at least one of Z¹ to Z³ is N,
f is an integer of 0 to 6, e, g, h and i are each an integer of 0 to 4, j and k are each an integer of 0 to 3, I is an integer of 0 to 2, m is an integer of 0 to 3, and where they are an integer of 2 or more, respectively, each of a plurality of R⁵, each of a plurality of R⁶, and each of a plurality of R⁷ are the same as or different from each other,
R' and R" are each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, a C₆-C₃₀ aryloxy group, and -L^{a}-N(R^{a})(R^{b}),
R' and R" of C(R')(R") may optionally be linked to each other to form a ring, and adjacent R's of C(R') may optionally be linked to each other to form a ring,
Ar^{a} is selected from the group consisting of a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and a combination thereof,
L^{a} is selected from the group consisting of a single bond, a C₆-C₂₀ arylene group, a fluorenylene group, a C₂-C₂₀ heterocyclic group comprising at least one heteroatom selected from the group consisting of O, N, S, Si and P, a C₃-C₂₀ aliphatic ring, and a combination thereof,
R^{a} and R^{b} are each independently selected from the group consisting of a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and a combination thereof.

3. The organic electric element of claim 1, wherein Formula 1 is represented by one of the following Formula 1-A to Formula 1-G: 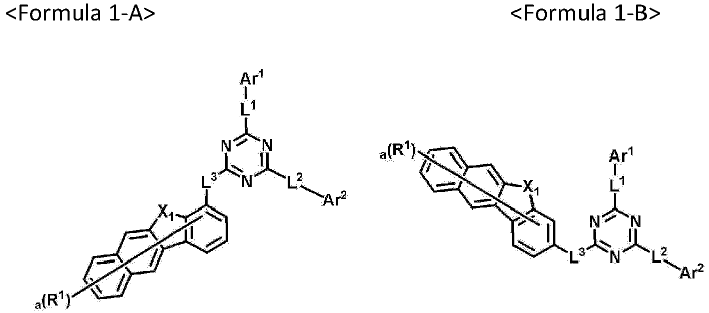 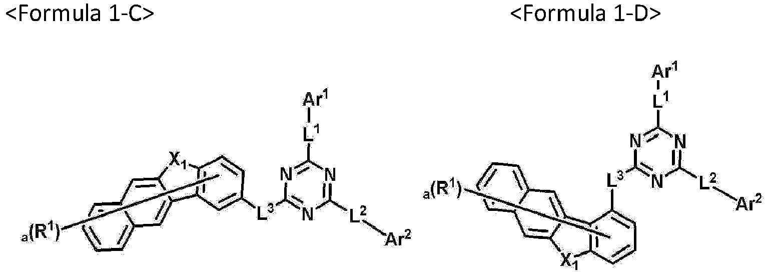 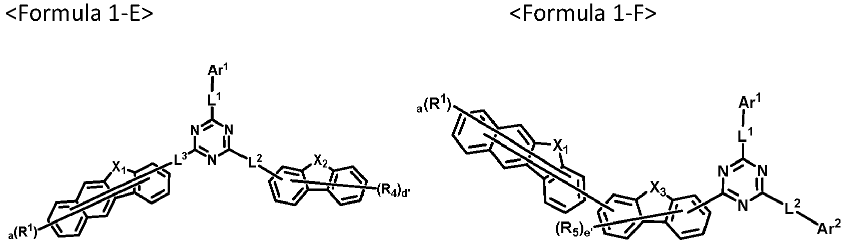 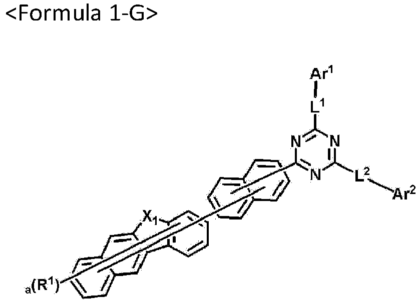
wherein, Ar¹, Ar², L¹ to L³, X₁, R¹ and a are the same as defined in claim 1,
X₂ and X₃ are each independently O or S,
R₄ and R₅ are each independently selected from the group consisting of hydrogen, deuterium, halogen, a cyano group, a nitro group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₃₀ aryloxy group, and adjacent groups may optionally be linked to each other to form a ring,
d' is an integer of 0 to 7, e' is an integer of 0 to 6, and where they are an integer of 2 or more, respectively, each of a plurality of R₄ and each of a plurality of R₅ are the same as or different from each other.

4. The organic electric element of claim 1, wherein Formula 2 is represented by one of the following Formula 2-A to Formula 2-L: 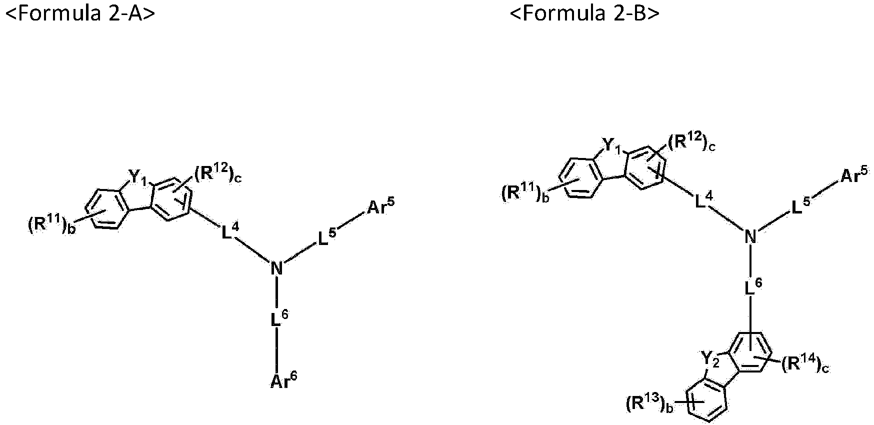 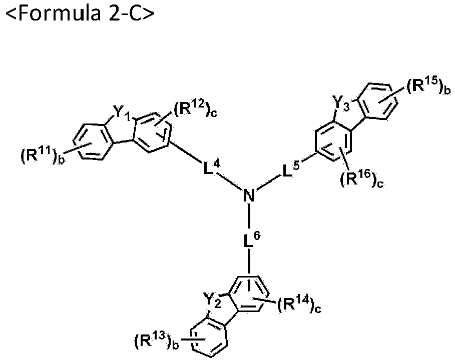 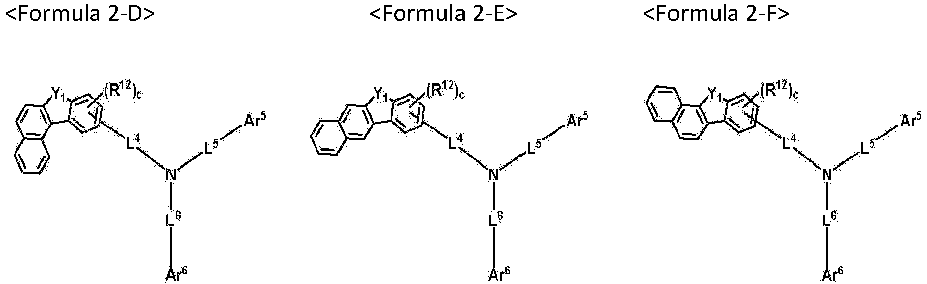 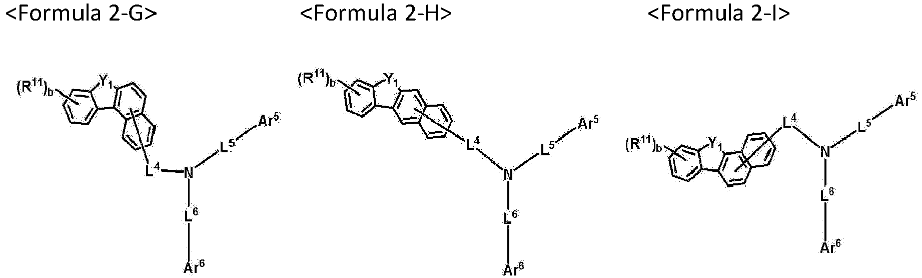 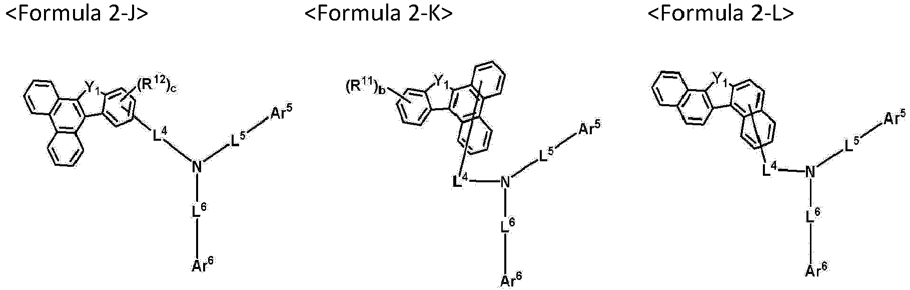
wherein, Ar⁵, Ar⁶, and L⁴ to L⁶ are the same as defined in claim 1,
Y₁ to Y₃ are each independently O, S, or C(R')(R"),
R¹¹ to R¹⁶, R' and R" are each independently selected from the group consisting of hydrogen, deuterium, halogen, a silane group substituted or unsubstituted with C₁-C₂₀ alkyl group or C₆-C₂₀ aryl group, a cyano group, a nitro group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, and -L'-N(Rₐ)(R_{b}), and adjacent groups may optionally be linked to each other to form a ring,
b is an integer of 0 to 4, and where b is an integer of 2 or more, each of a plurality of R¹¹, each of a plurality of R¹³, and each of a plurality of R¹⁵ are the same as or different from each other,
c is an integer of 0 to 3, and where c is an integer of 2 or more, each of a plurality of R¹², each of a plurality of R¹⁴, and each of a plurality of R¹⁶ are the same as or different from each other,
L', Rₐ and R_{b} are the same as defined in claim 1.

5. The organic electric element of claim 1, wherein the compound represented by Formula 1 is one of the following compounds:

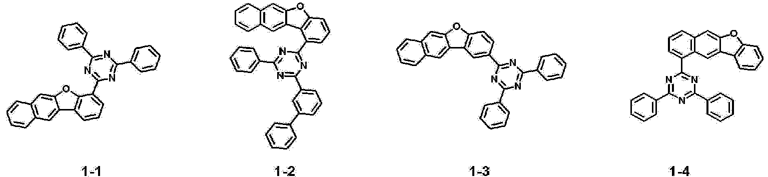

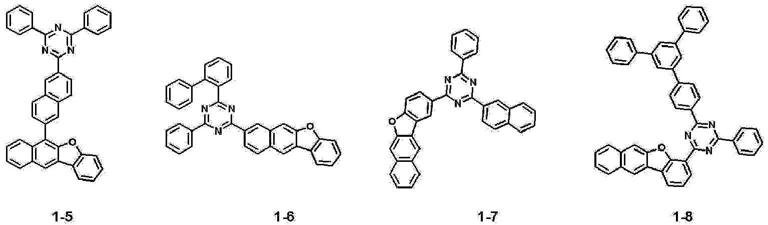

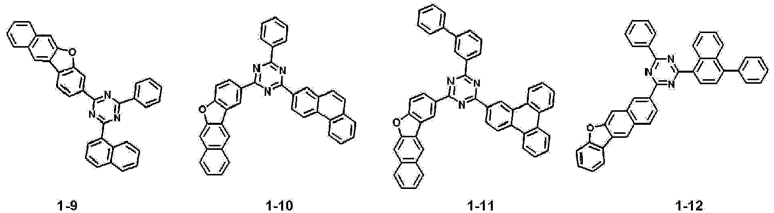

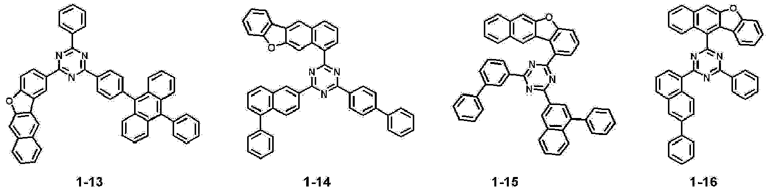

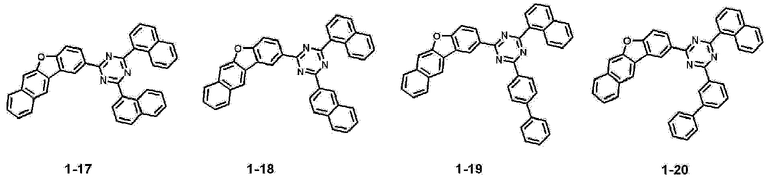

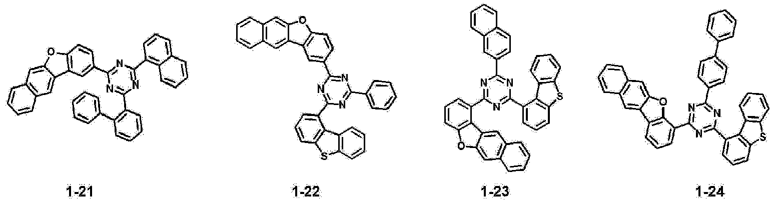

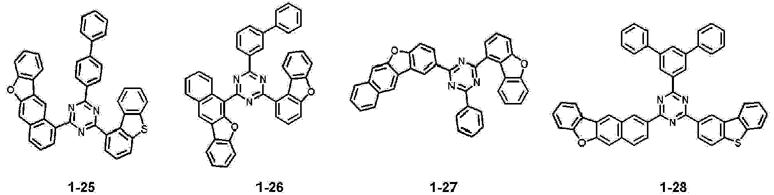

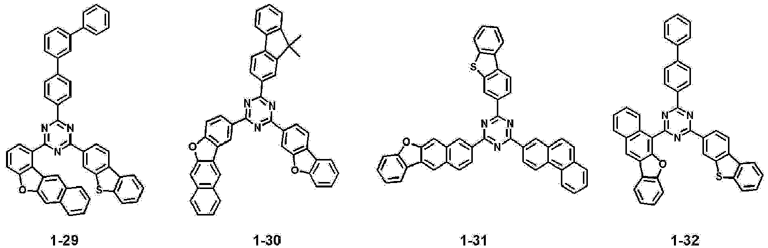

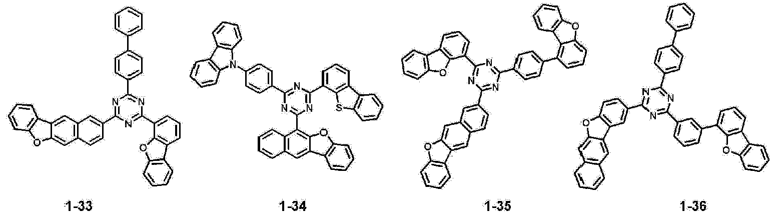

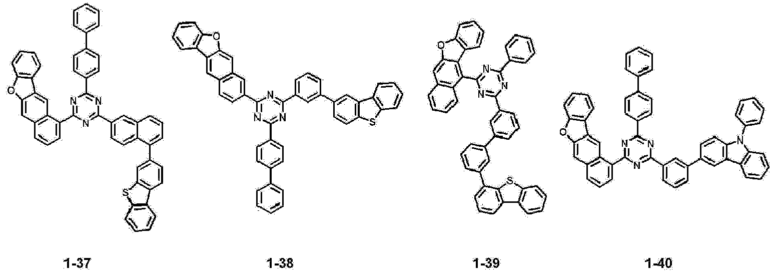

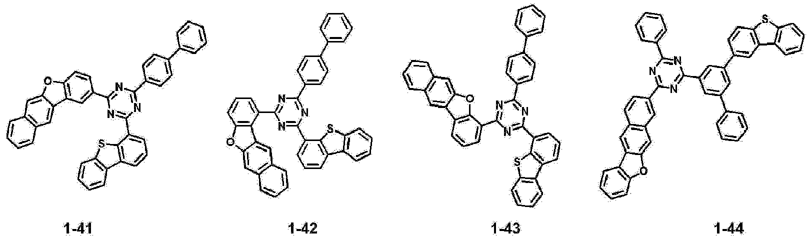

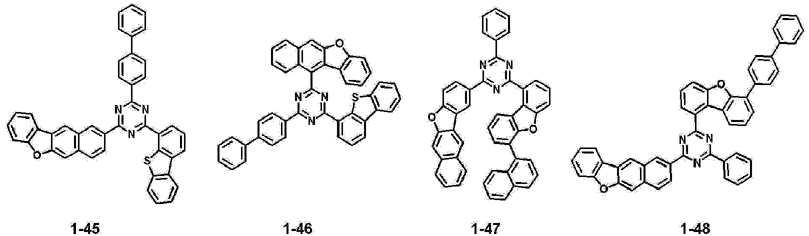

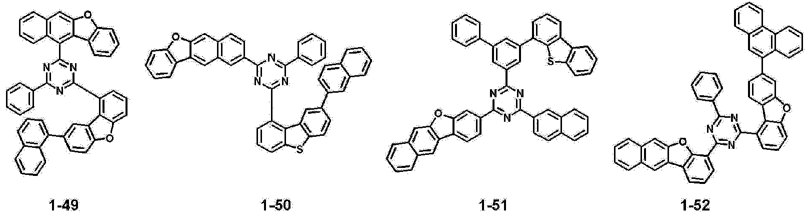

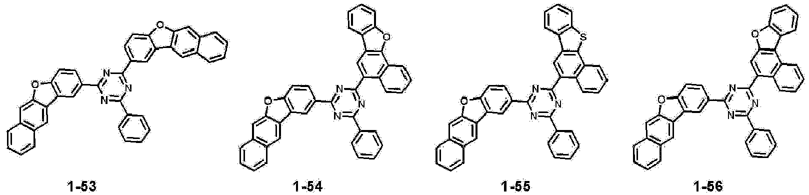

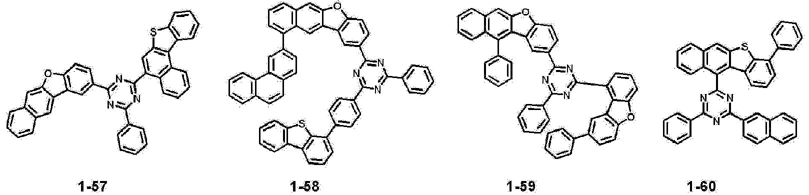

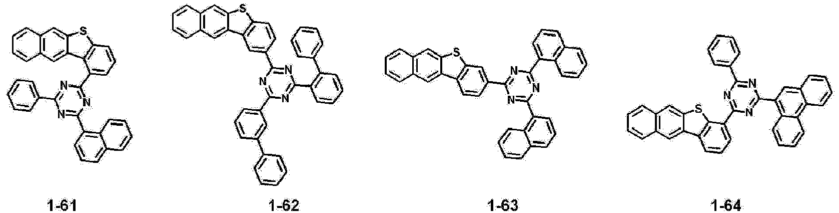

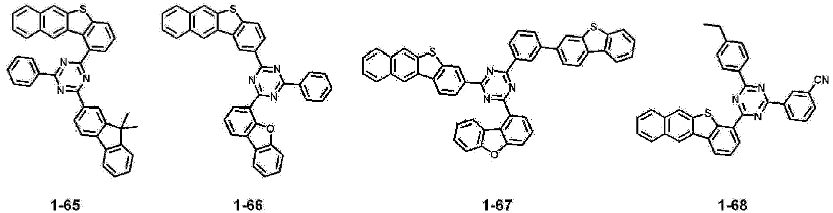

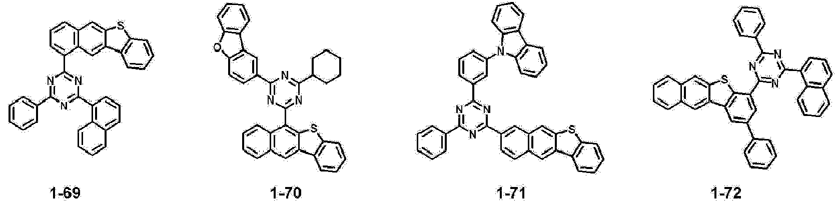

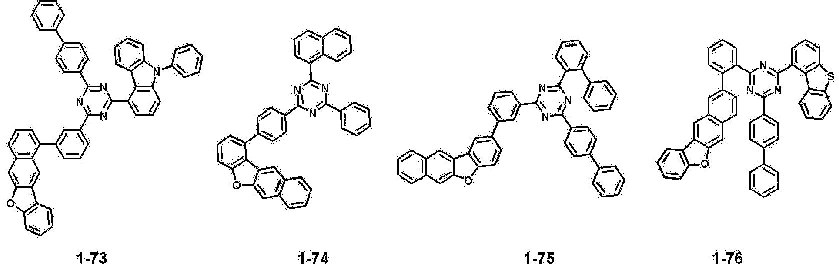

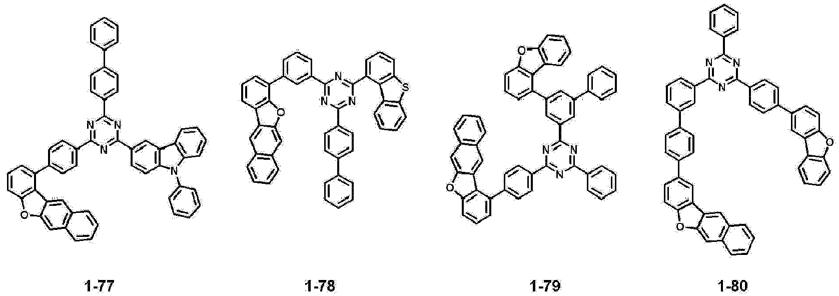

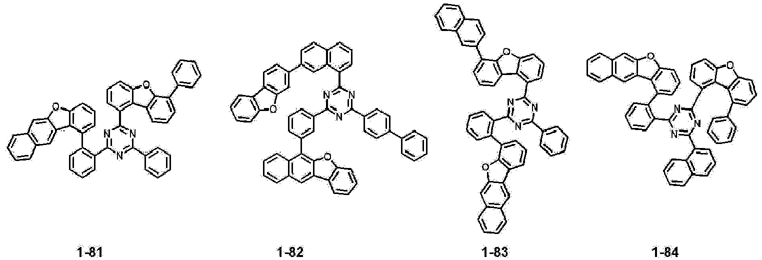

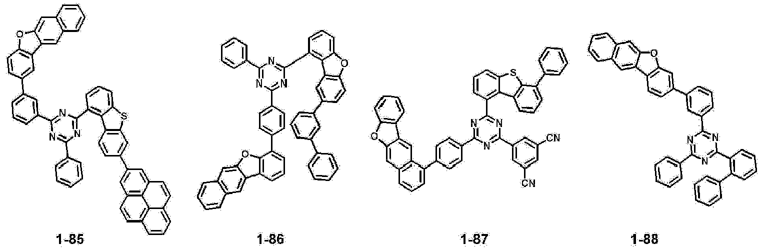

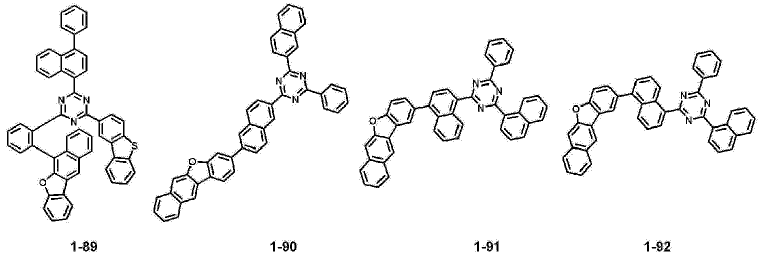

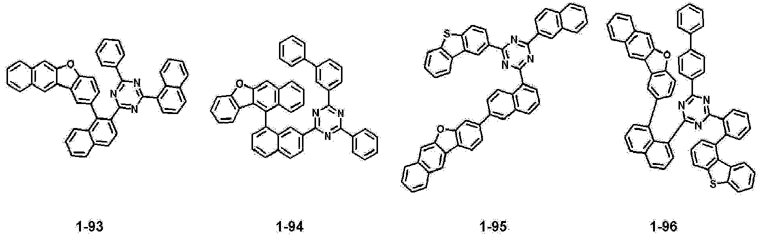

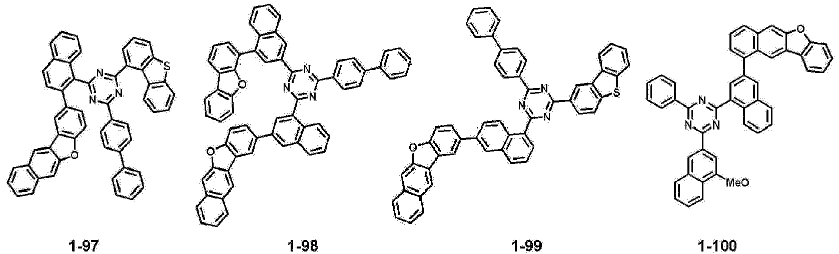

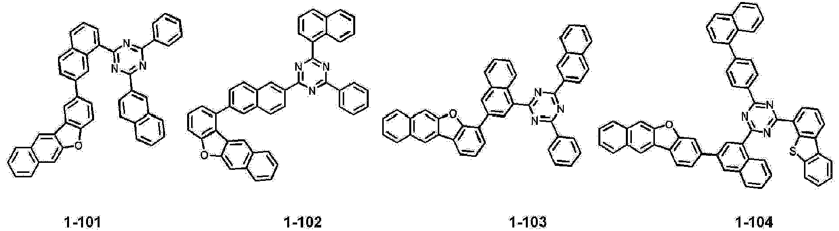

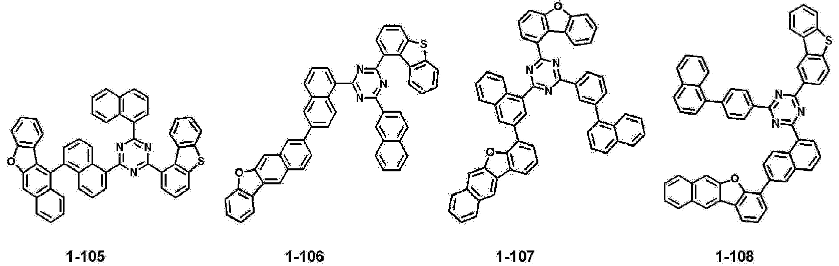

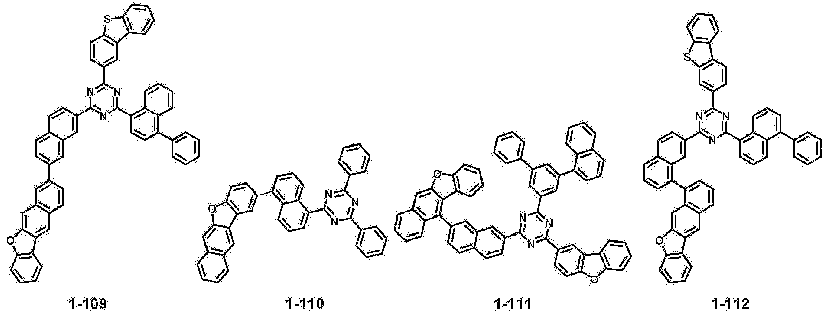

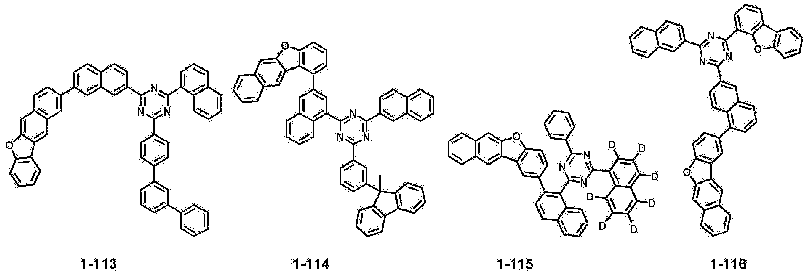

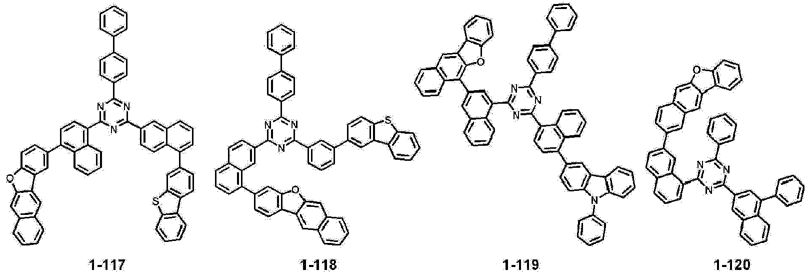

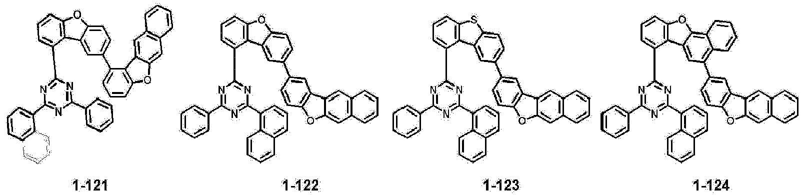

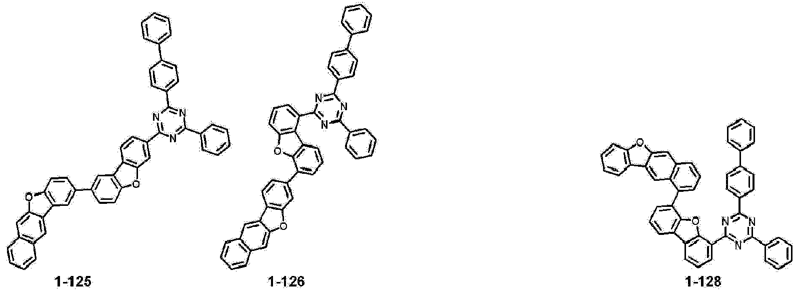

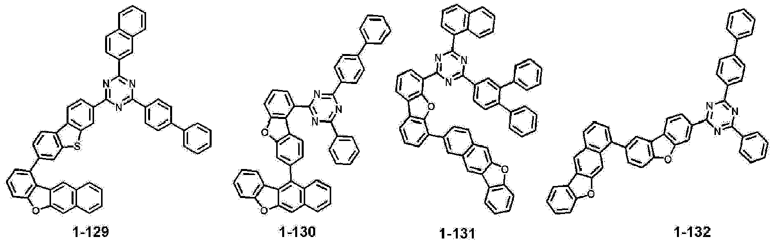

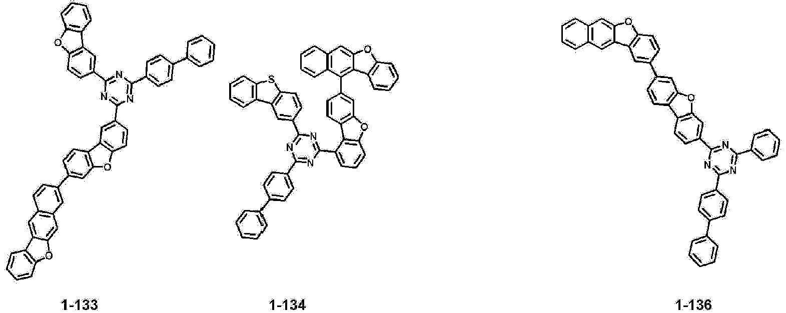

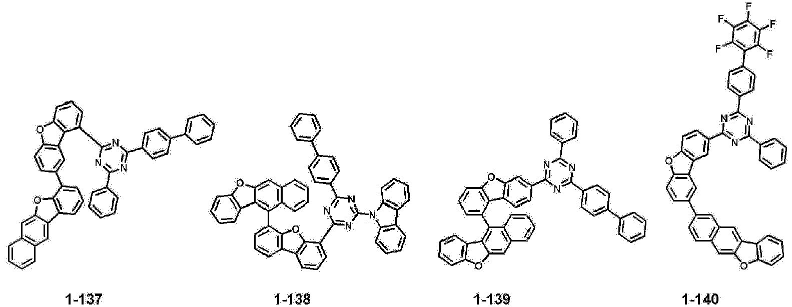

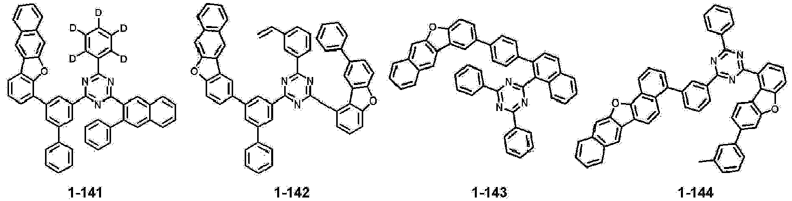

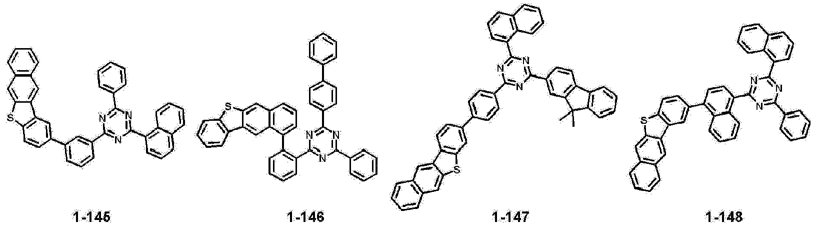

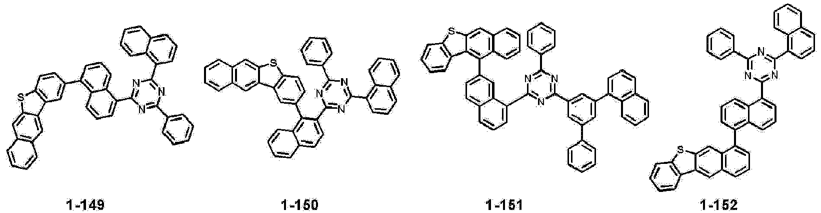

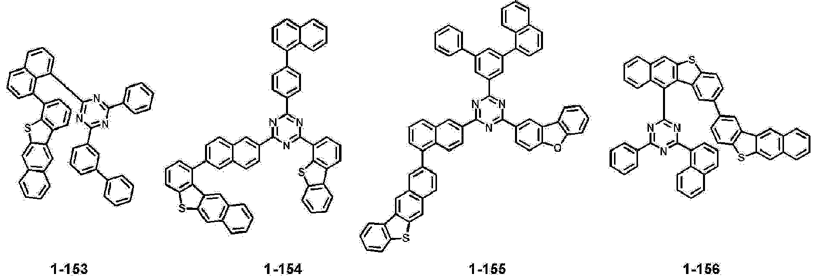

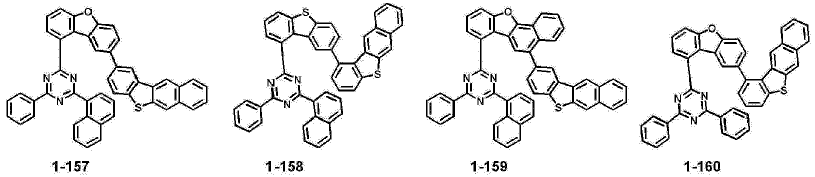

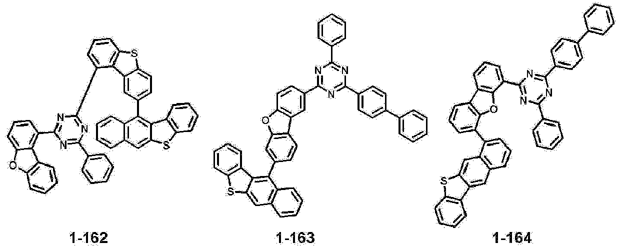

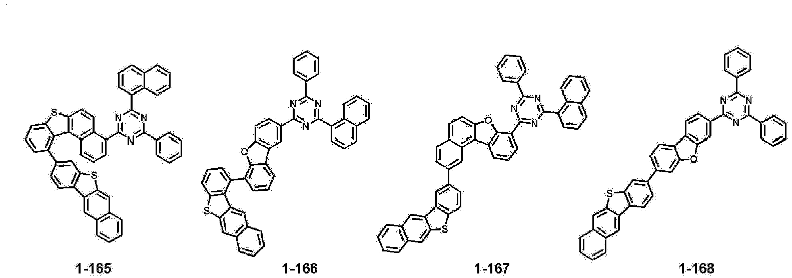

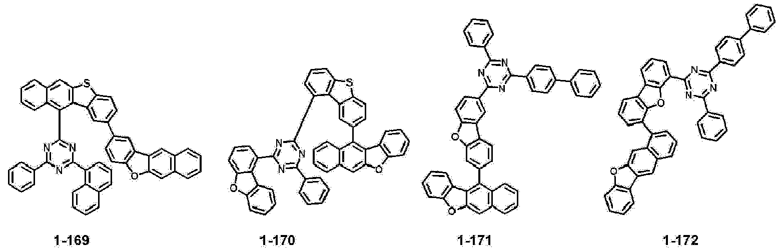

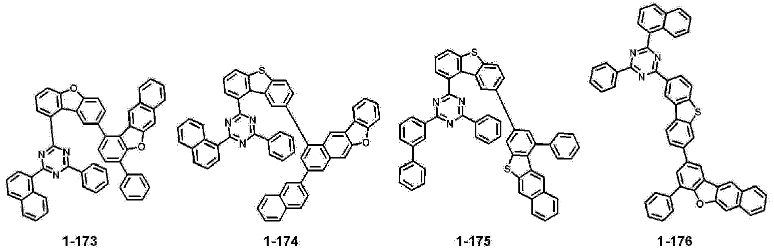

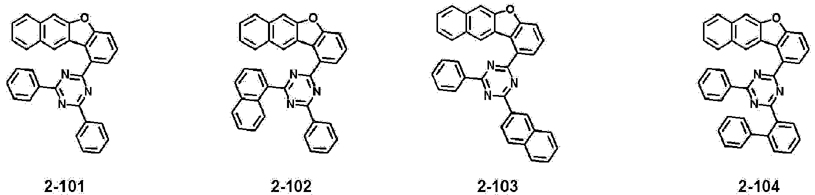

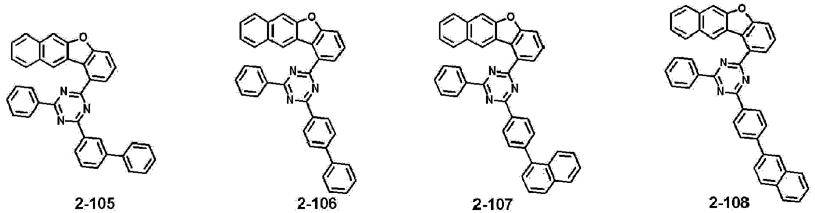

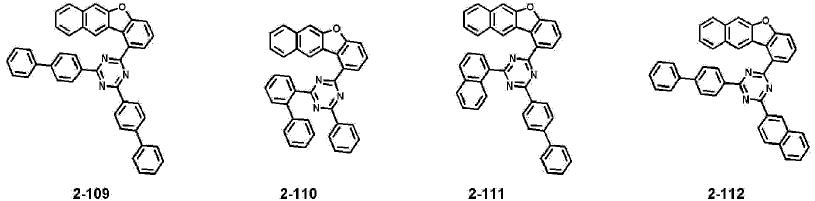

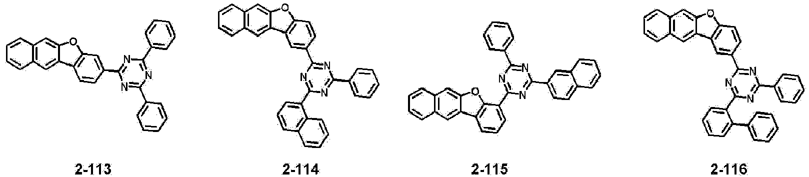

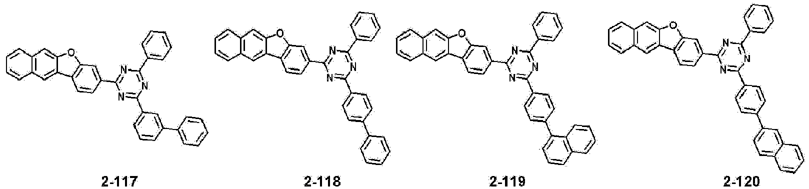

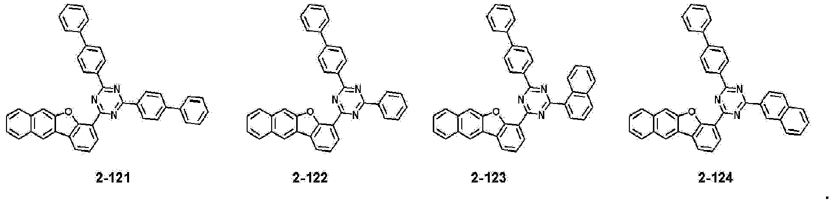

6. The organic electric element of claim 1, wherein the compound represented by Formula 2 is one of the following compounds:

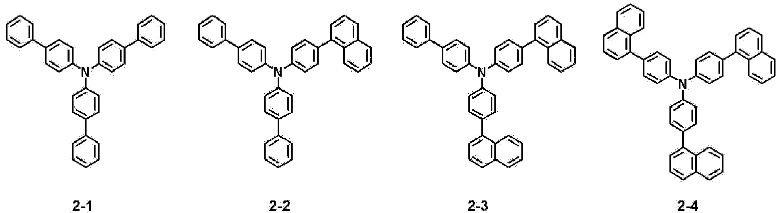

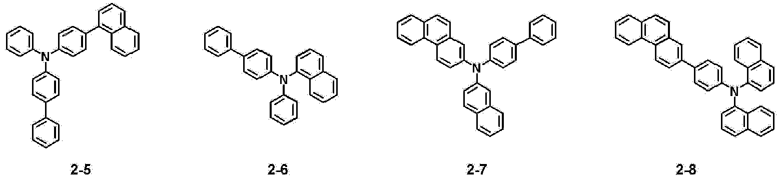

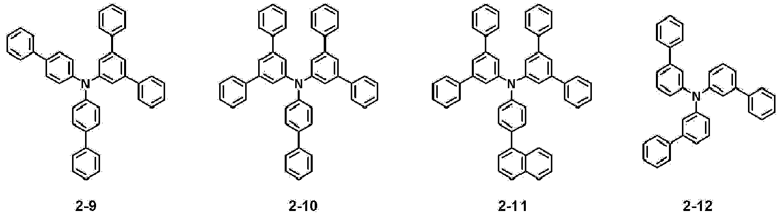

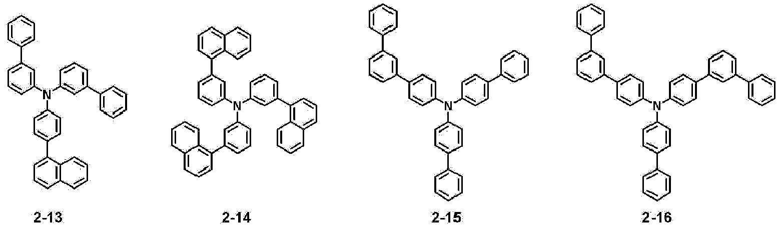

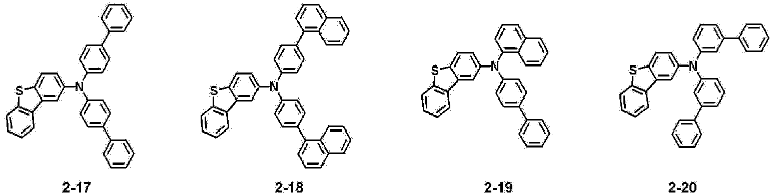

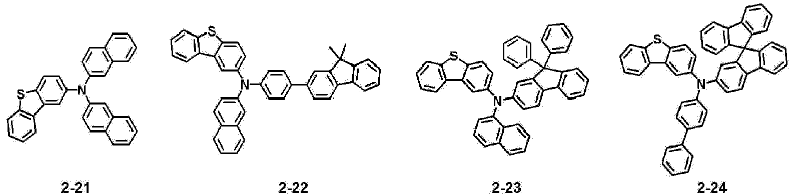

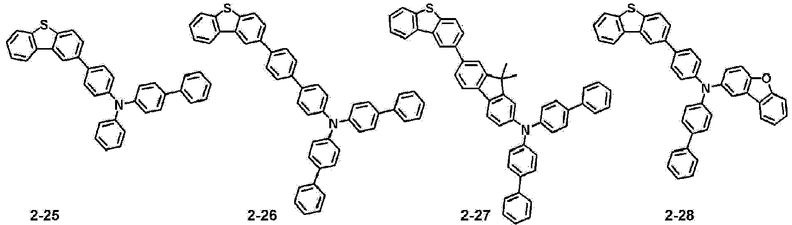

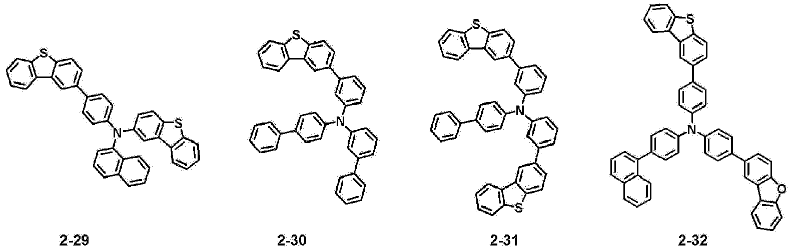

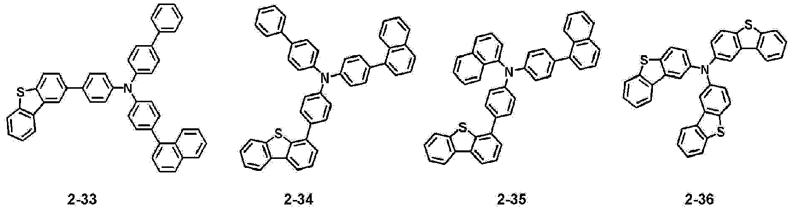

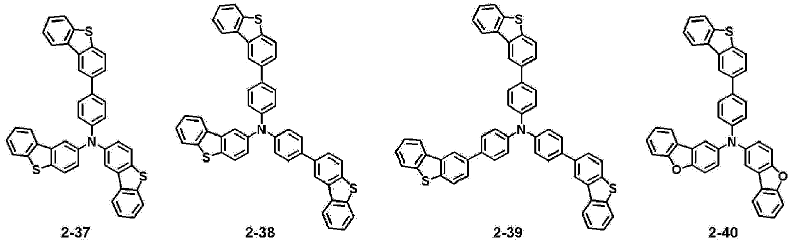

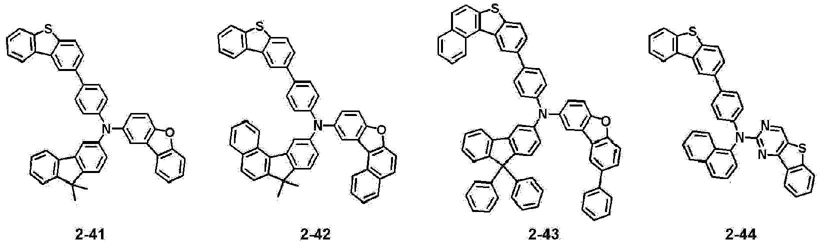

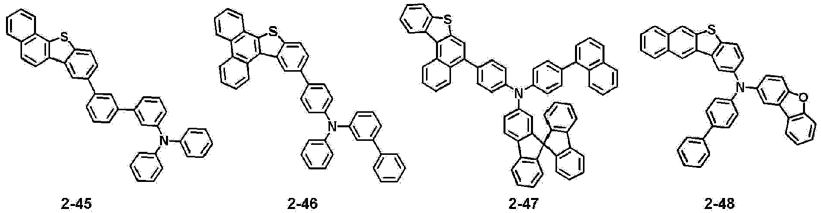

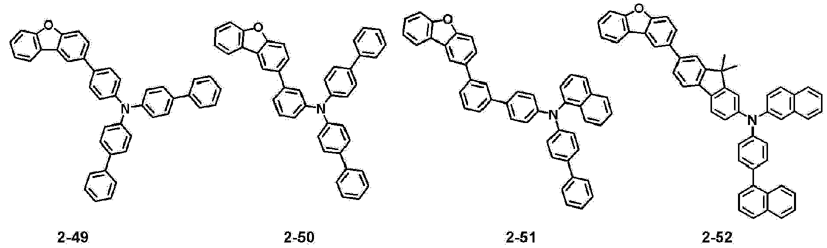

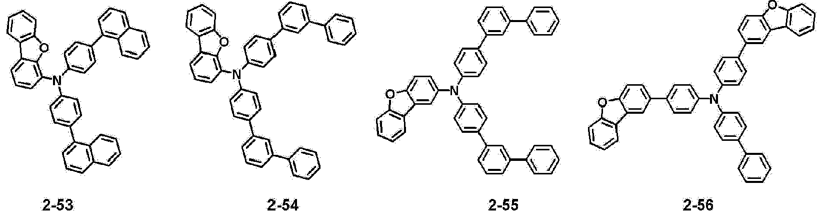

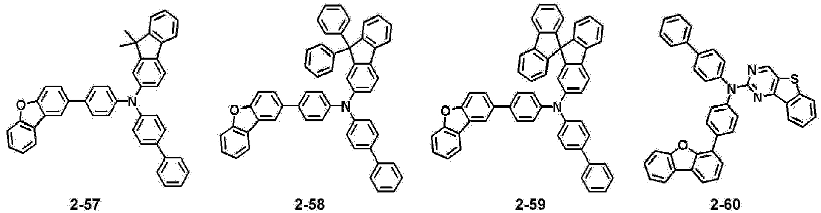

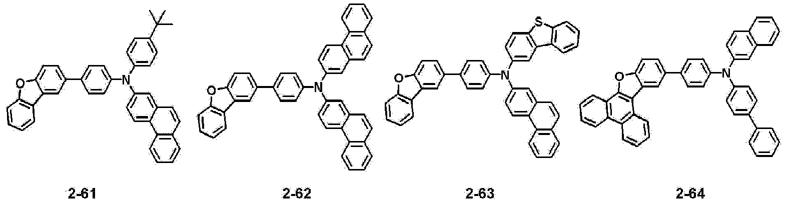

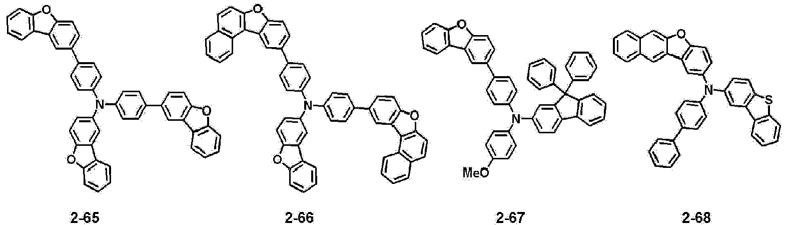

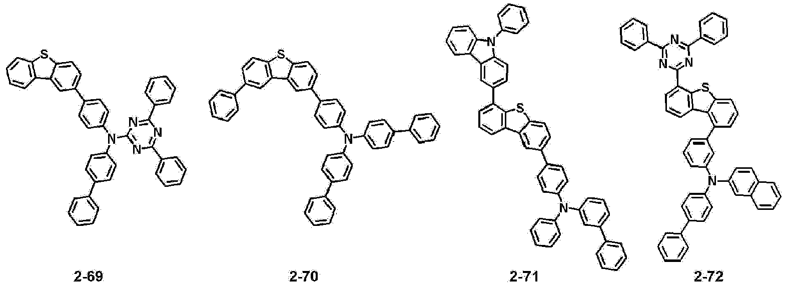

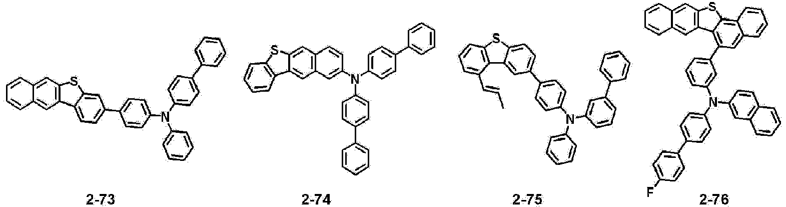

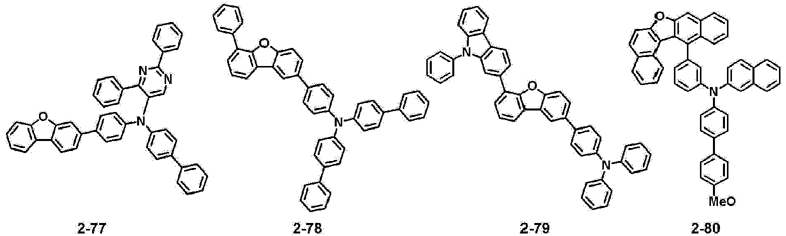

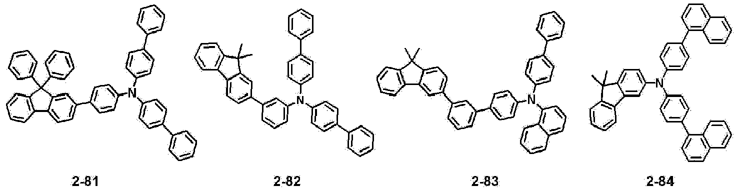

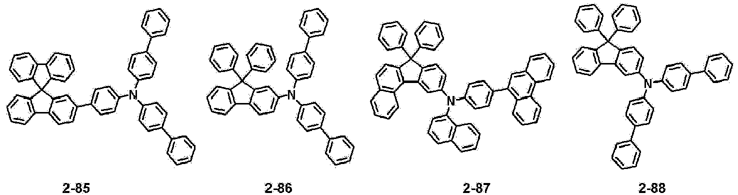

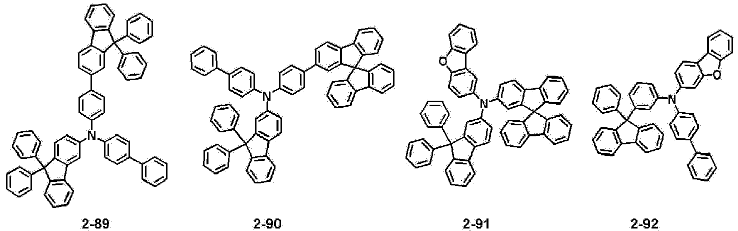

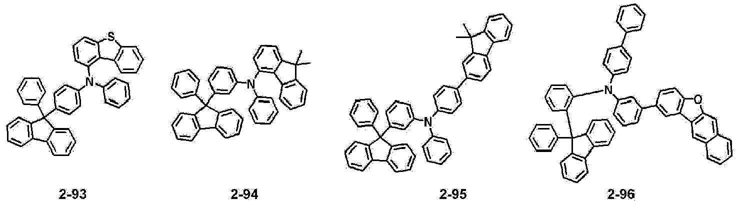

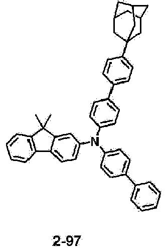

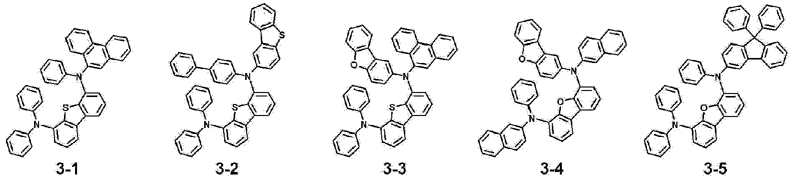

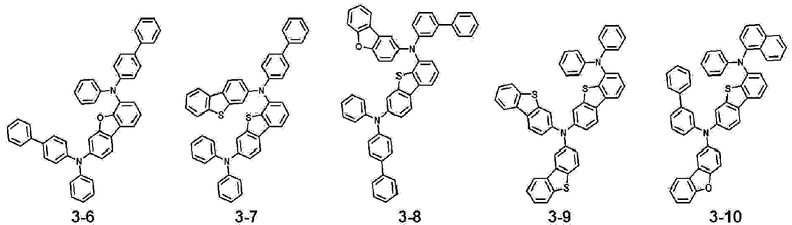

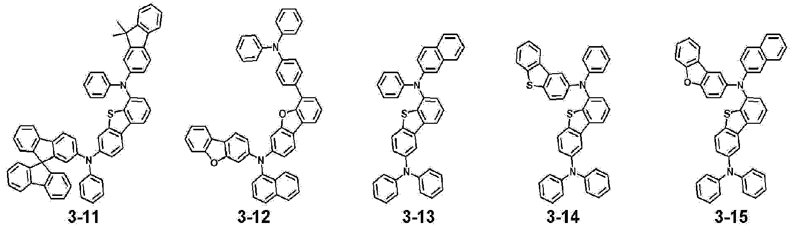

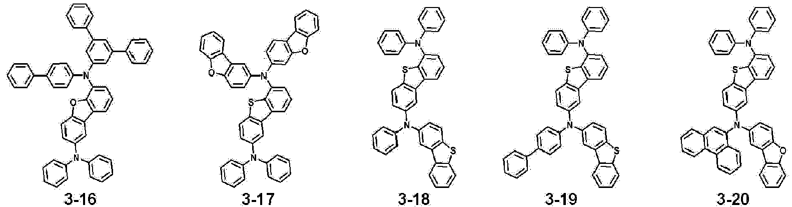

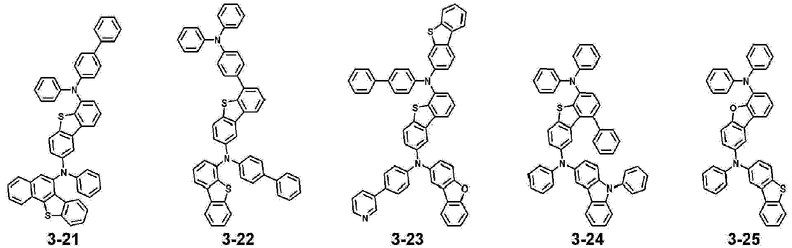

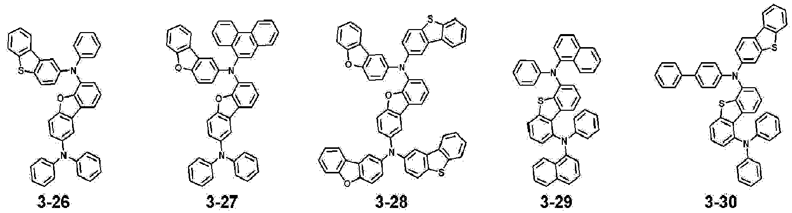

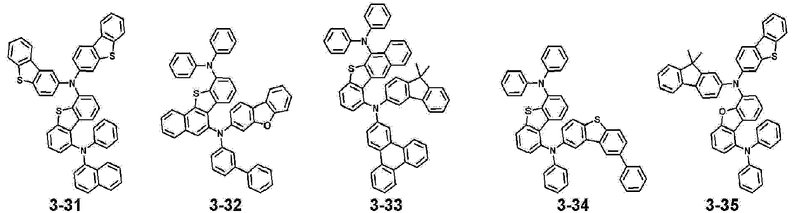

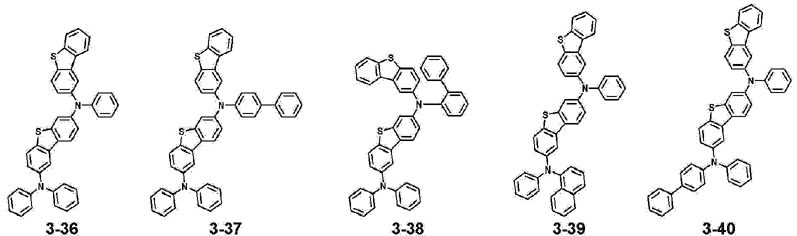

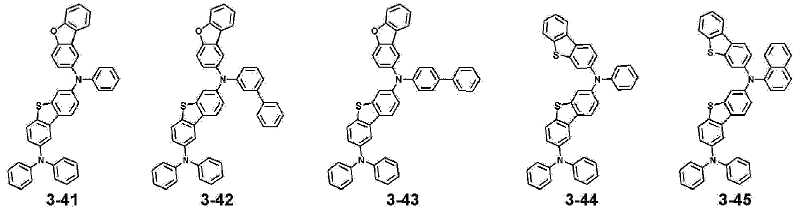

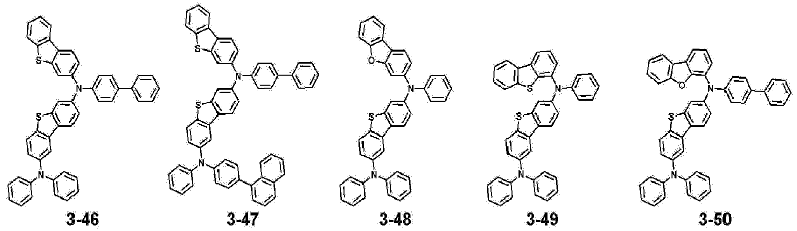

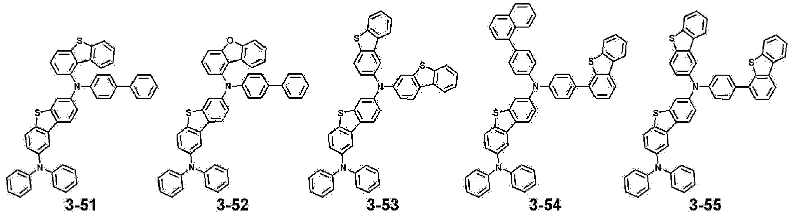

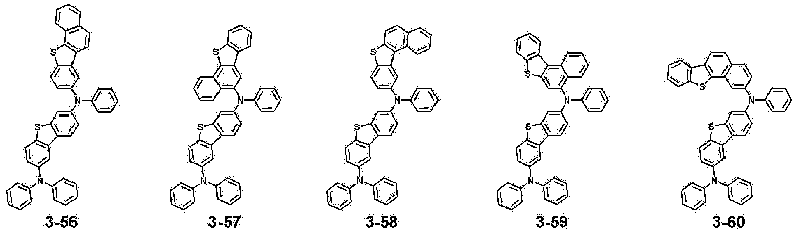

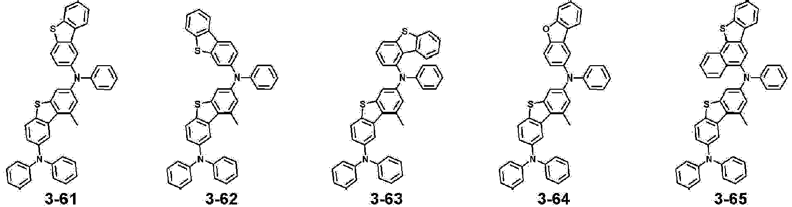

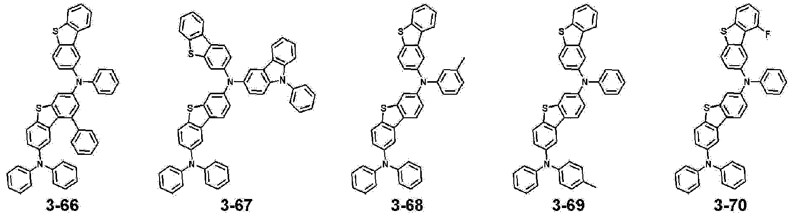

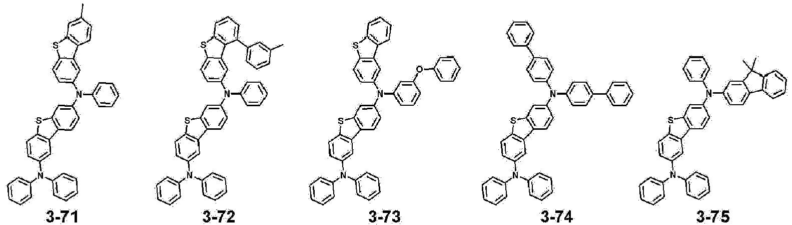

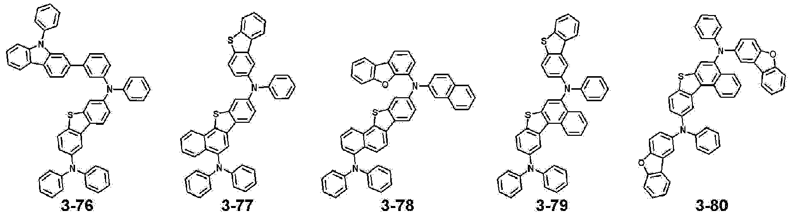

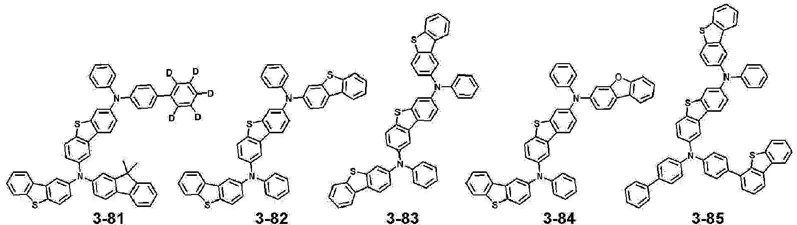

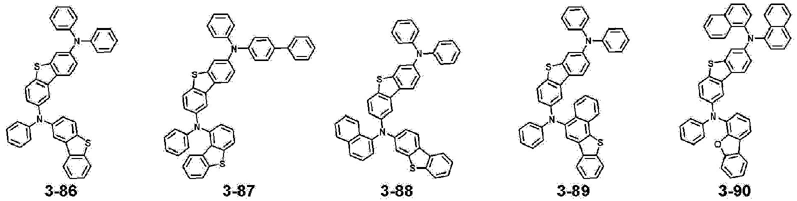

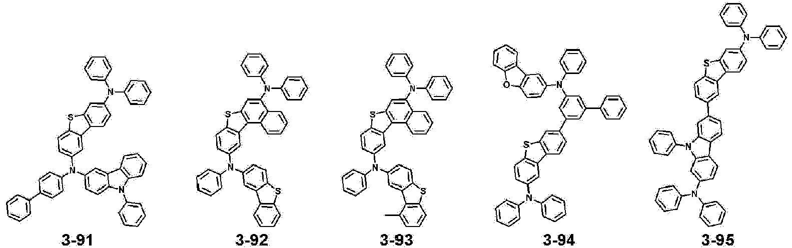

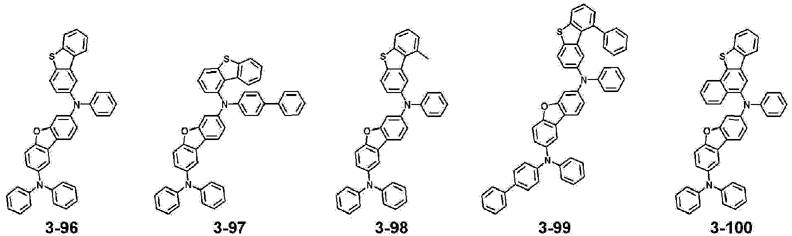

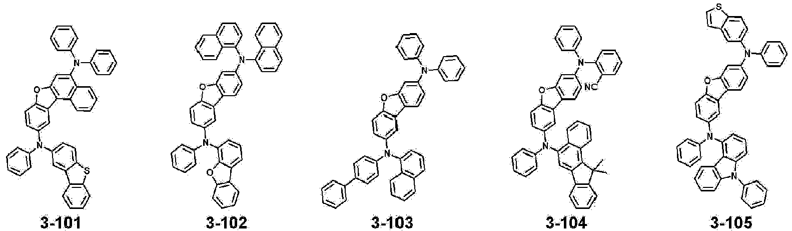

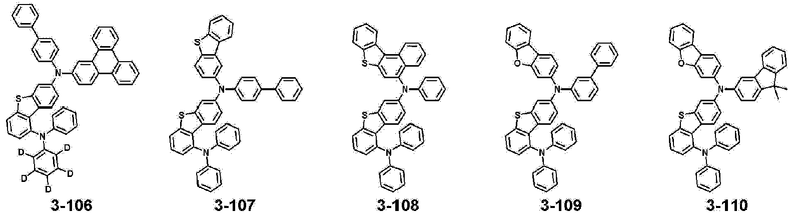

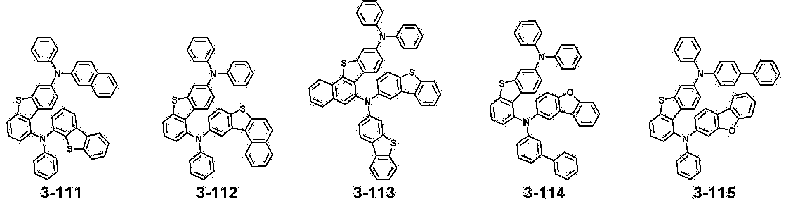

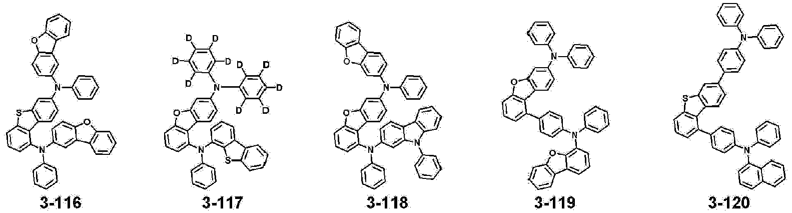

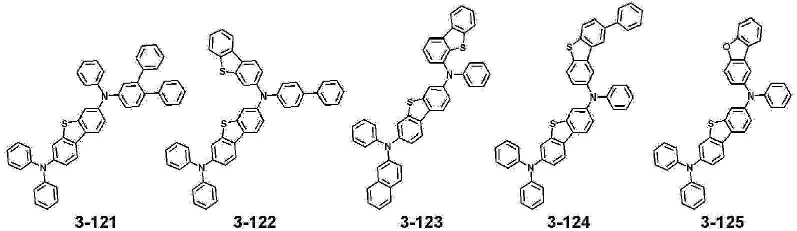

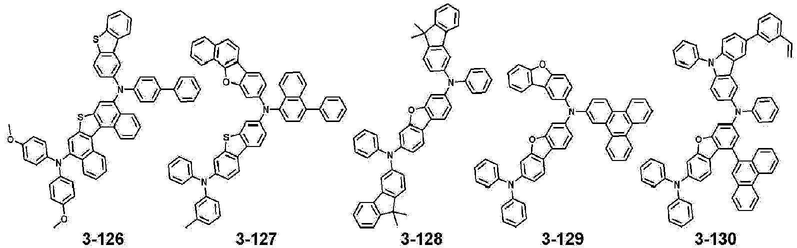

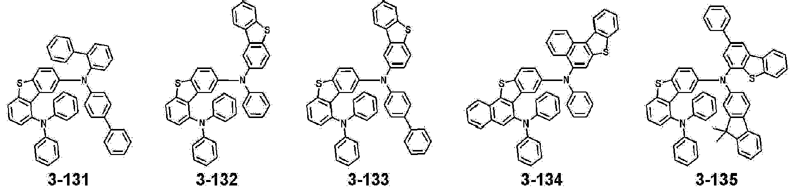

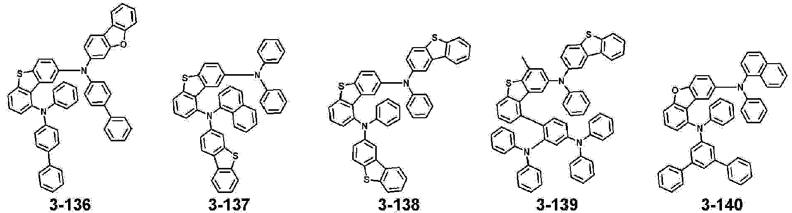

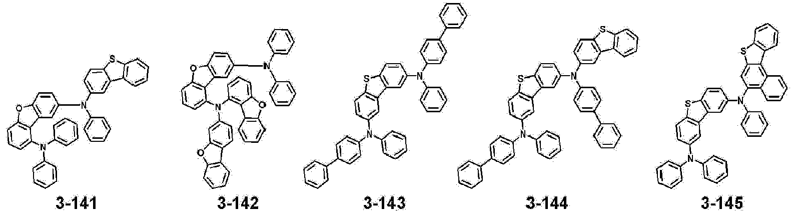

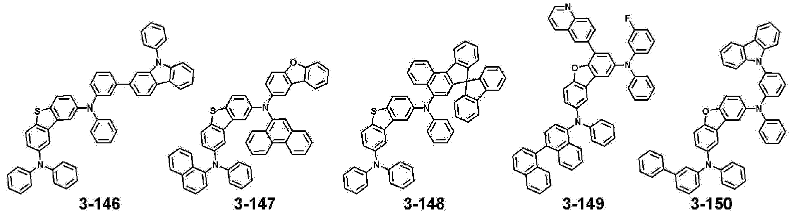

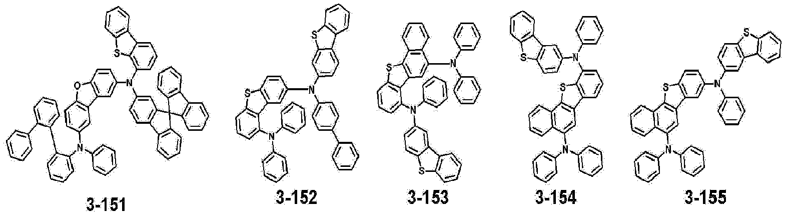

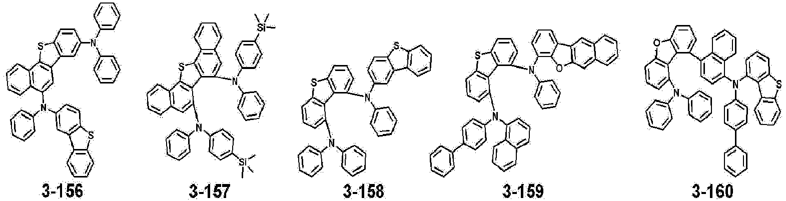

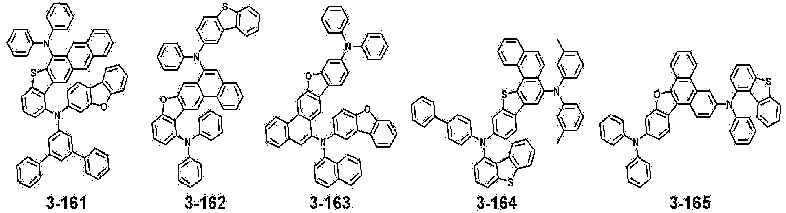

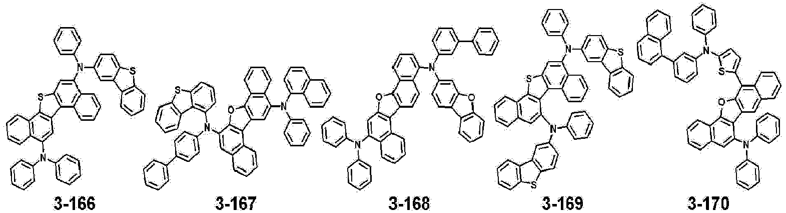

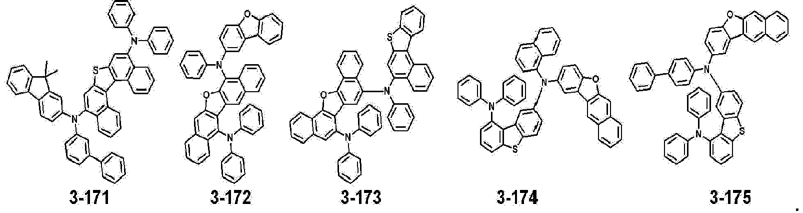

7. The organic electric element of claim 1, wherein Formula 2 is represented by the following Formula 4: 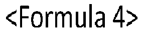 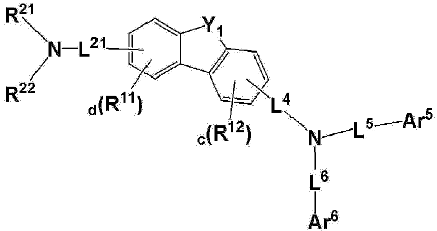 wherein,
Y₁ is O, S or C(R')(R"),
Ar⁵ and Ar⁶ are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, a C₁-C₃₀ alkyl group, a C₂-C₃₀ alkenyl group, a C₂-C₃₀ alkynyl group, a C₁-C₃₀ alkoxyl group and a C₆-C₃₀ aryloxy group,
L⁴ to L⁶, and L²¹ are each independently selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring and a combination thereof,
R¹¹, R¹², R²¹, R²², R' and R" are each independently selected from the group consisting of hydrogen, deuterium, halogen, a silane group substituted or unsubstituted with C₁-C₂₀ alkyl group or C₆-C₂₀ aryl group, a cyano group, a nitro group, a C₁-C₂₀ alkoxyl group, a C₆-C₃₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring and -L'-N(Rₐ)(R_{b}), and adjacent groups may optionally be linked to each other to form a ring,
c and d are each an integer of 0^{~}3, and where c or d is an integer of 2 or more, each of a plurality of R¹² is the same as or different from each other.
L' is selected from the group consisting of a single bond, a C₆-C₆₀ arylene group, a fluorenylene group, a C₂-C₆₀ heterocyclic group comprising at least one heteroatom selected from the group consisting of O, N, S, Si and P, a C₃-C₆₀ aliphatic ring, and a combination thereof,
Rₐ and R_{b} are each independently selected from the group consisting of a C₆-C₆₀ aryl group, a fluorenyl group, a C₂-C₆₀ heterocyclic group comprising at least one heteroatom selected from the group consisting of O, N, S, Si and P, a C₃-C₆₀ aliphatic ring, and a combination thereof,
Ar⁵, Ar⁶, L⁴ to L⁶, L²¹, R¹¹, R¹², R²¹, R²², R', R", L', Rₐ, R_{b} and the ring formed by bonding adjacent groups to each other may be each optionally further substituted with one or more substituents selected from the group consisting of deuterium, halogen, a silane group substituted or unsubstituted with C₁-C₂₀ alkyl group or C₆-C₂₀ aryl group, a siloxane group, a boron group, a germanium group, a cyano group, a nitro group, a C₁-C₂₀ alkylthio group, a C₁-C₂₀ alkoxyl group, a C₆-C₂₀ aryloxy group, a C₁-C₂₀ alkyl group, a C₂-C₂₀ alkenyl group, a C₂-C₂₀ alkynyl group, a C₆-C₂₀ aryl group, a fluorenyl group, a C₂-C₂₀ heterocyclic group comprising at least one heteroatom selected from the group consisting of O, N, S, Si and P, a C₃-C₂₀ aliphatic ring, a C₇-C₂₀ arylalkyl group and a C₈-C₂₀ arylalkenyl group.

8. The organic electric element of claim 7, wherein Formula 4 is represented by one of the following Formulas 4-1 to 4-9: 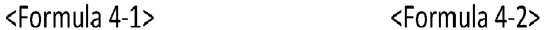 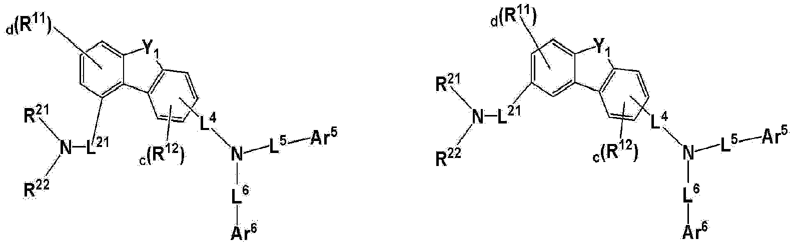 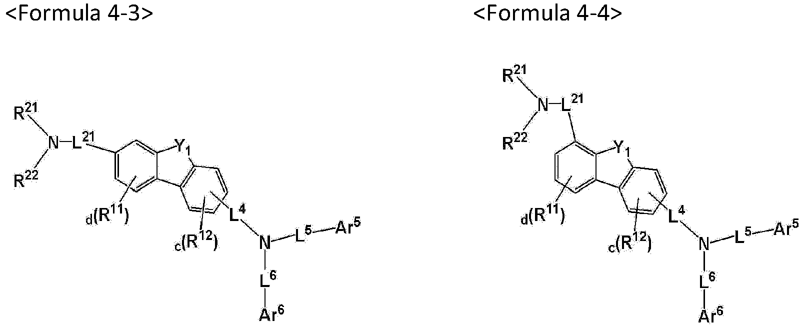 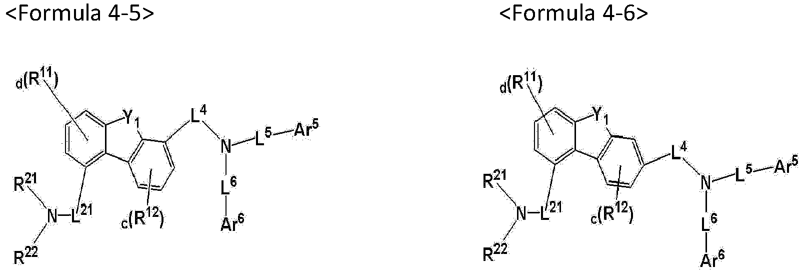 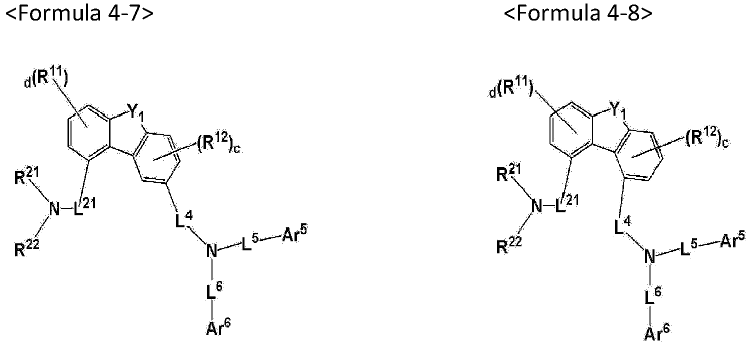 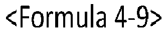 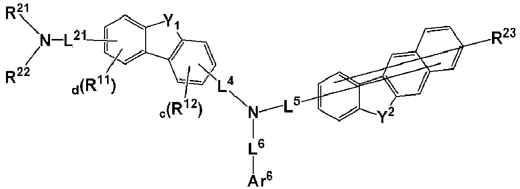 wherein, Y₁, Ar⁵, Ar⁶, L⁴ to L⁶, L²¹, R¹¹, R¹², R²¹, R²², c and d are the same as defined in claim 7, Y² is O or S, and R²³ is defined to be the same as R¹¹.

9. The organic electric element of claim 7, wherein the organic material layer comprises an emission-auxiliary layer, and the emission-auxiliary layer comprises the compound represented by Formula 4.

10. The compound of claim 7, wherein the compound represented by Formula 4 is one of the following compounds:

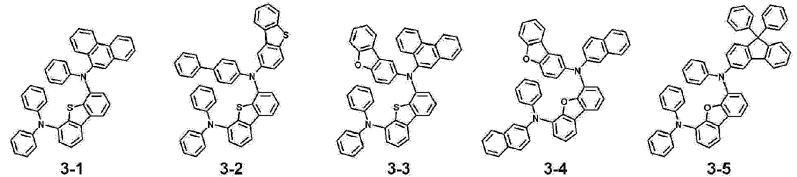

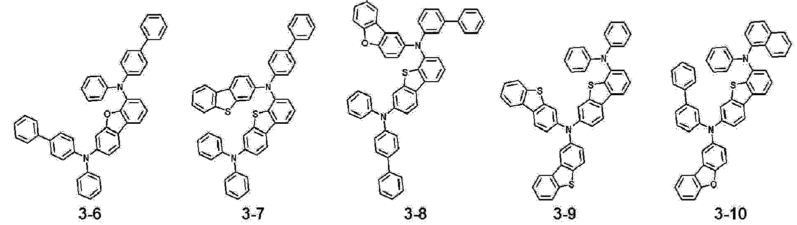

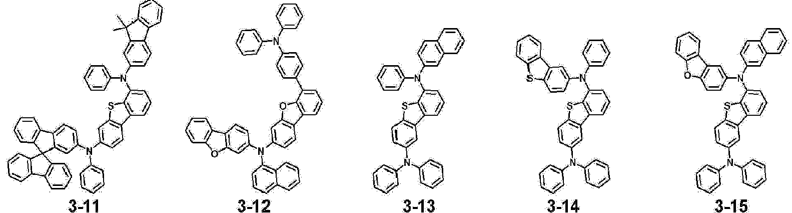

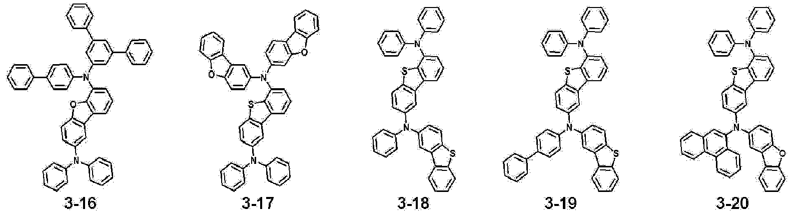

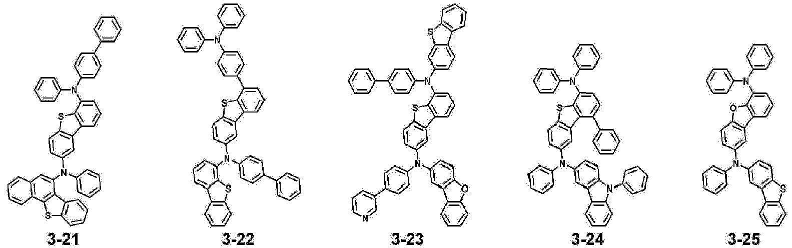

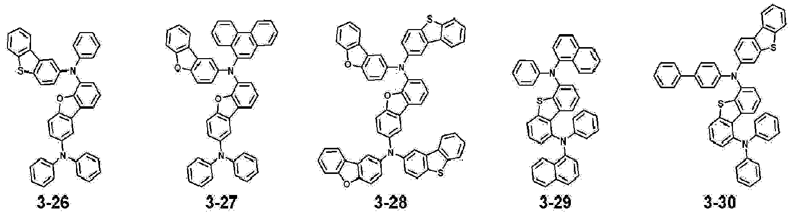

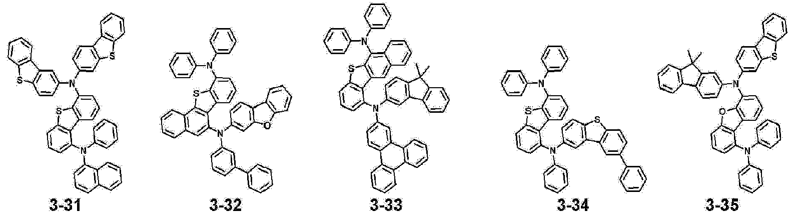

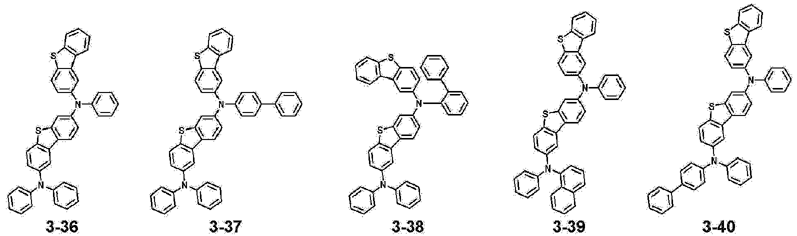

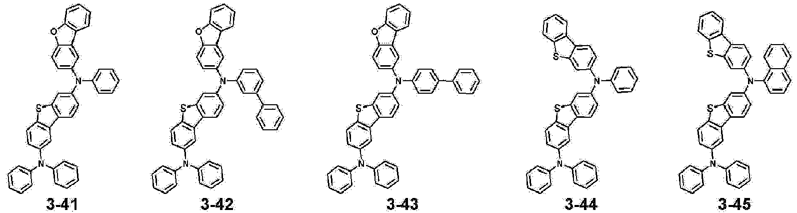

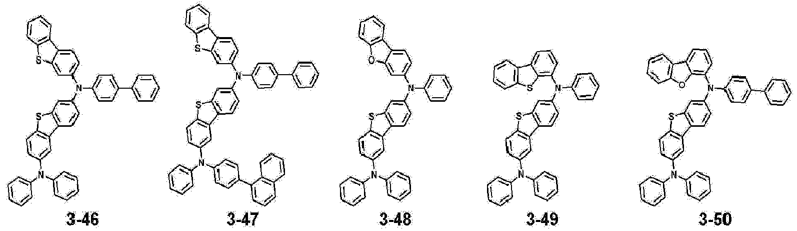

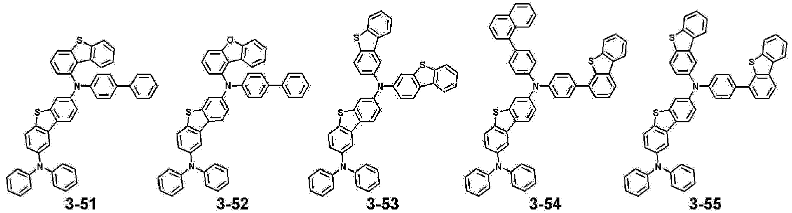

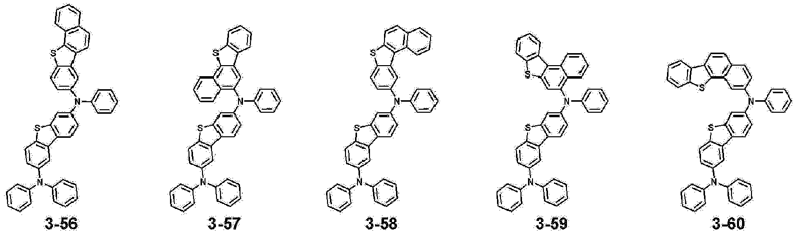

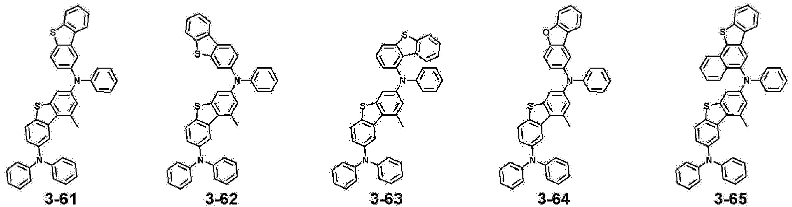

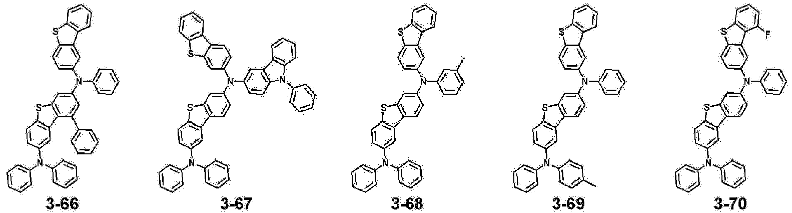

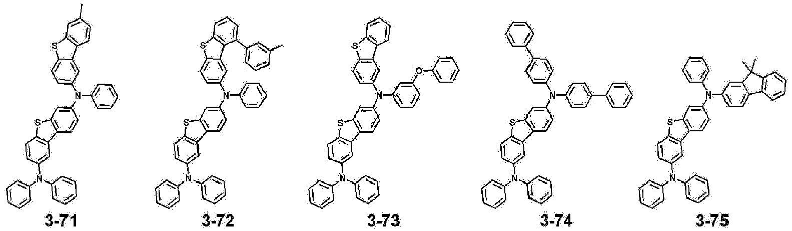

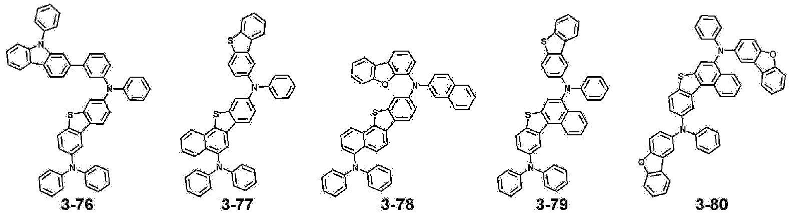

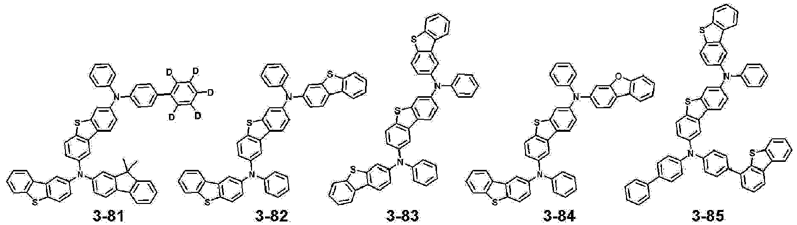

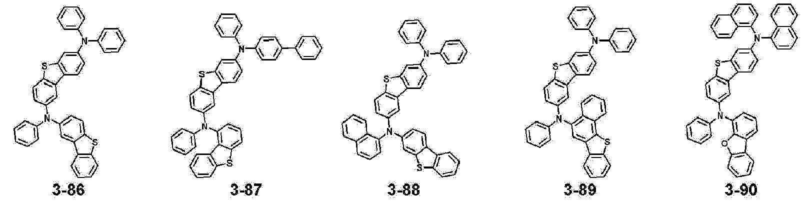

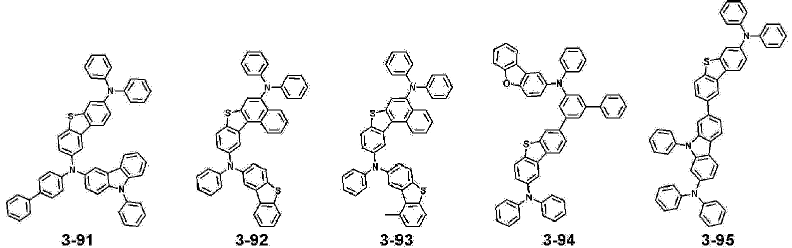

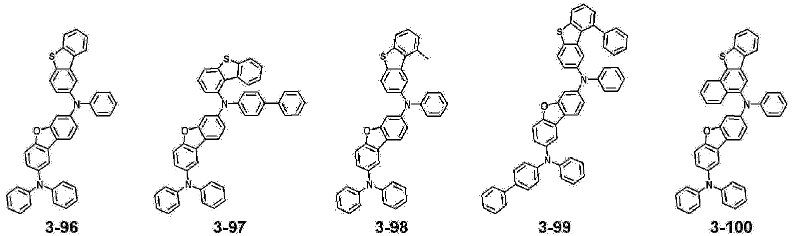

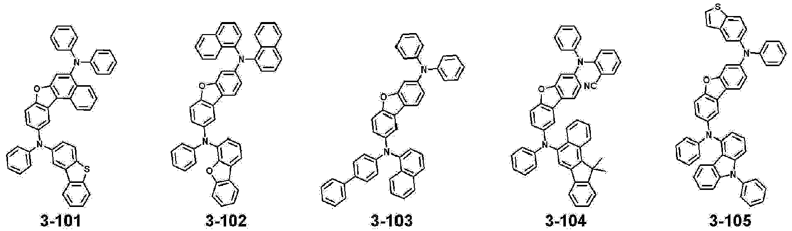

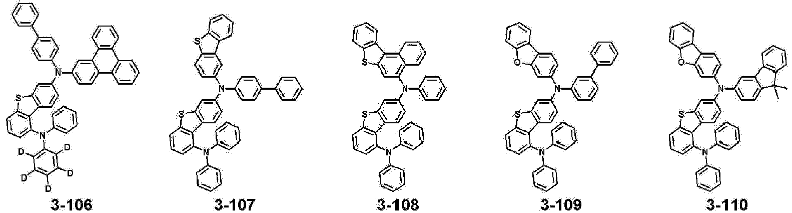

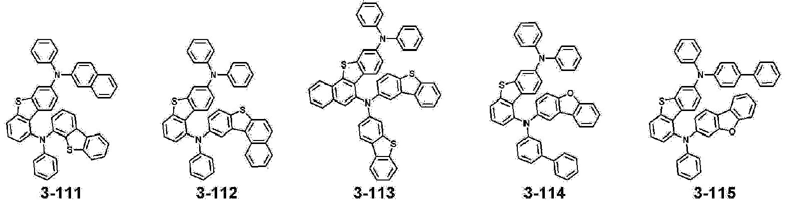

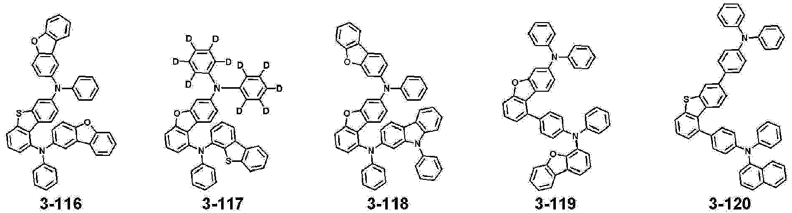

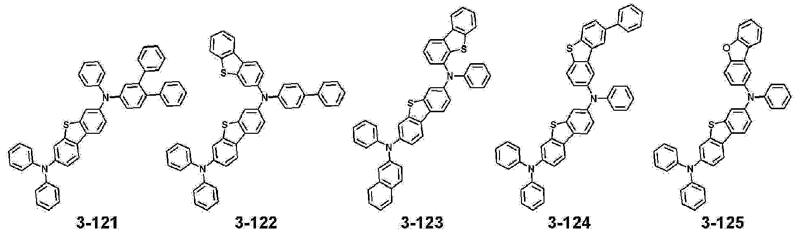

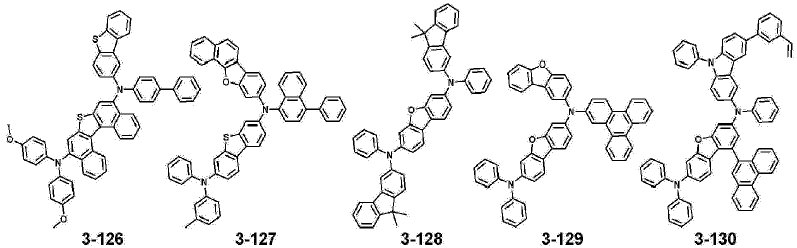

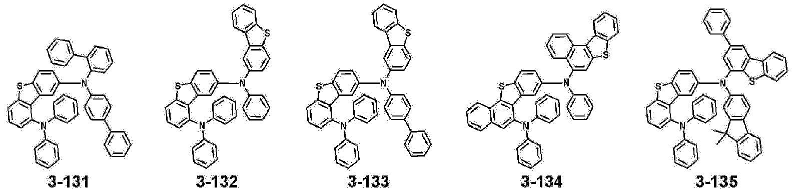

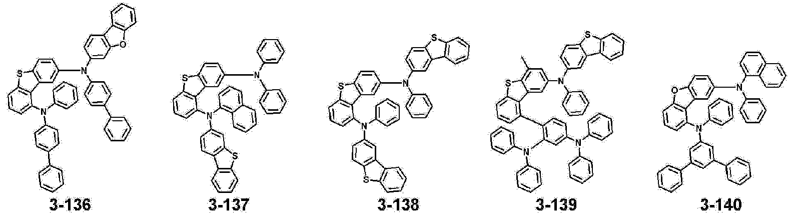

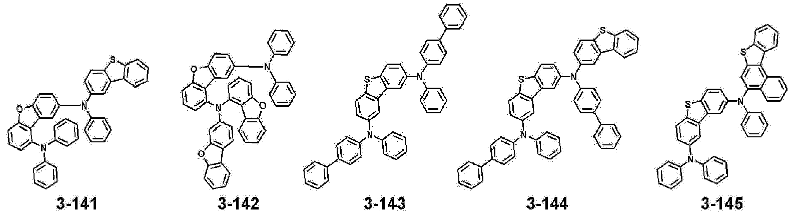

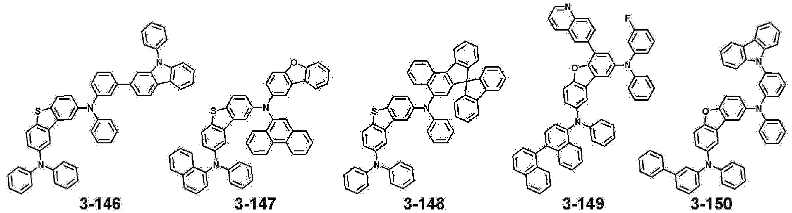

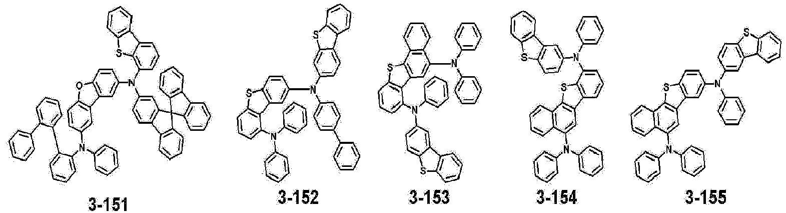

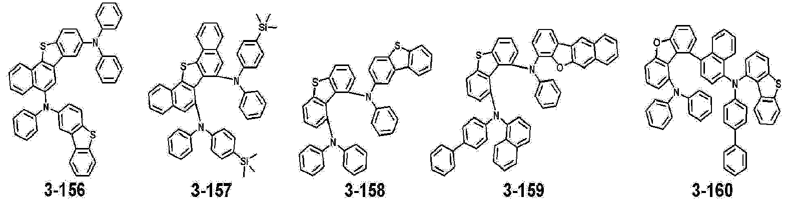

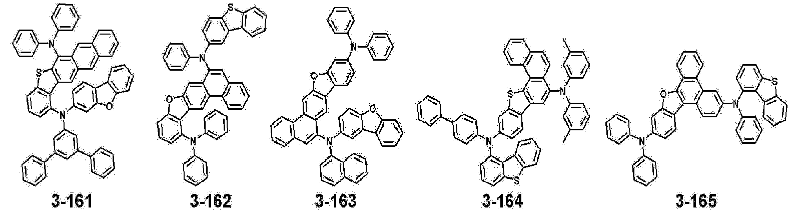

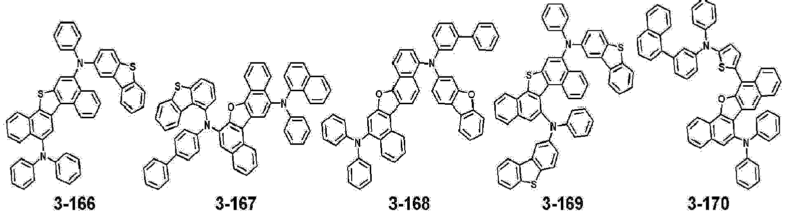

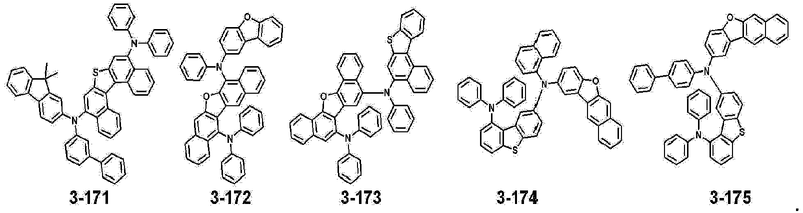

11. The organic electric element of claim 1, further comprising a layer for improving luminous efficiency formed on one side of the first electrode or one side of the second electrode, the side not facing the organic material layer.

12. The organic electric element of claim 1, wherein the organic material layer comprises two or more stacks, the stacks comprising a hole transport layer, a light emitting layer, and an electron transport layer in sequence formed on the anode.

13. The organic electric element of claim 12, wherein the organic material layer further comprises a charge generation layer formed between the two or more stacks.

14. An electronic device comprising a display device and a control unit for driving the display device, wherein the display device comprises the organic electric element of claim 1.

15. The electronic device of claim 14, wherein the organic electric element is selected from the group consisting of an organic light emitting diode, an organic solar cell, an organic photo conductor, an organic transistor, an element for monochromatic illumination and a quantum dot display.
